# EUROPEAN PATENT APPLICATION

(11) **EP 4 567 033 A1**
(43) Date of publication of application: **11.06.2025**
(21) Application number: 23850166.2
(22) Date of filing: 04.08.2023
(51) Int. Cl.: C07D 401/14, A61K 31/4709, A61K 31/517, A61K 31/5377, A61K 38/05, A61P 1/18, A61P 35/00, A61P 43/00, C07D 403/14, C07D 405/14, C07D 413/14, C07D 417/14

(54) **HETEROCYCLIC COMPOUND FOR INDUCING DEGRADATION OF MUTANT KRAS PROTEIN**

(30) Priority: 05.08.2022 JP 2022125351
(71) Applicant: Astellas Pharma Inc., Chuo-ku Tokyo 103-8411 (JP)
(72) Inventor: MORIKAWA, Takahiro, Tokyo 103-8411 (JP); IMADA, Sunao, Tokyo 103-8411 (JP); HAMAGUCHI, Hisao, Tokyo 103-8411 (JP); OKUMURA, Mitsuaki, Tokyo 103-8411 (JP); IMAIZUMI, Tomoyoshi, Tokyo 103-8411 (JP); KOGANEMARU, Yohei, Tokyo 103-8411 (JP); HONJO, Eriko, Tokyo 103-8411 (JP); FUJIWARA, Yuta, Tokyo 103-8411 (JP); KAWAMINAMI, Eiji, Tokyo 103-8411 (JP); YOSHINARI, Tomohiro, Tokyo 103-8411 (JP); ISHIOKA, Hiroki, Tokyo 103-8411 (JP); KURAMOTO, Kazuyuki, Tokyo 103-8411 (JP); NISHIZONO, Yoshihiro, Tokyo 103-8411 (JP)
(74) Representative: Potter Clarkson
(86) International application number: PCT/JP2023/028510
(87) International publication number: WO 2024/029613

(57) **Abstract**

A compound useful as an active ingredient of a pharmaceutical composition for treating pancreatic cancer is provided.

The present inventors have studied about a compound that is useful as an active ingredient of a pharmaceutical composition for treating pancreatic cancer and have found that the heterocyclic compound represented by the formula (I) has an excellent degradation-inducing action on a mutant KRAS protein, in particular, a degradation-inducing action on a G12V mutant, G12D mutant and G12C mutant KRAS protein, and has a mutant KRAS inhibition activity, in particular, an inhibition activity on a G12V mutant, G12D mutant and G12C mutant KRAS, thus completing the present invention. The heterocyclic compound of the present invention or a salt thereof can be used as a therapeutic agent for pancreatic cancer.

## Description

### [Technical Field]

The present invention relates to pharmaceutical compositions and to a heterocyclic compound that is excellent in a degradation-inducing action on a mutant KRAS protein, in particular, in a degradation-inducing action on a G12V mutant, G12D mutant and G12C mutant KRAS protein, and that is expected to be useful as a mutant KRAS inhibitor, in particular, as a G12V mutant, G12D mutant and G12C mutant KRAS inhibitor, and to be useful as an active ingredient of, for example, a pharmaceutical composition for treating pancreatic cancer.

### [Background Art]

Pancreatic cancer mainly including pancreatic ductal adenocarcinoma is a cancer with a very poor prognosis having a five years survival rate of 10% or less (CA Cancer J. Clin., 2016, 66, p.7-30), and about 460,000 new cases are reported per year in the world (CA Cancer J. Clin., 2018, 68, p.394-424). The most effective therapy for treating pancreatic cancer is a surgery. However, the cancer has often metastasized since early detection is difficult, and therapeutic effects of a surgery cannot be expected in many cases. When the cancer is not treated by a surgery, chemotherapy or radiotherapy is adopted but the survival rate is not so good. Currently, the FOLFIRINOX therapy (multidrug treatment of three chemotherapy agents of 5-FU, irinotecan and oxaliplatin, plus levofolinate) is used as a standard therapy of pancreatic cancer. However, due to the strong toxicity, the subject patient has to be cautiously selected, for example, the therapy is to be applied only to patients of an ECOG performance status of 1 or less (J. Clin. Oncol., 2018, 36, p.2545-2556). As a molecular target drug, an epidermal growth factor receptor (EGFR) inhibitor, Erlotinib, has been approved in a combination therapy with Gemcitabine. However, the extension of the overall survival is only about two weeks as compared with Gemcitabine alone, and no satisfying therapeutic effect has been achieved. A highly effective therapeutic agent remains needed (J. Clin. Oncol., 2007, 25, p.1960-1966).

RAS proteins are low molecular weight guanosine triphosphate (GTP)-binding proteins of about 21 kDa constituted of 188-189 amino acids and include four main types of proteins (KRAS (KRAS 4A and KRAS 4B), NRAS and HRAS) produced by three genes of a KRAS gene, an NRAS gene and an HRAS gene. RAS proteins are divided into an active GTP-binding type and an inactive GDP-binding type. A RAS protein is activated by replacement of guanosine diphosphate (GDP) with GTP due to, for example, ligand stimulation to a membrane receptor, such as EGFR. The active RAS binds to effector proteins as much as twenty, such as RAF, PI3K and RALGDS, to activate the downstream signal cascade. On the other hand, the active RAS is converted to the inactive type by replacement of GTP with GDP due to the intrinsic GTP hydrolysis (GTPase) activity. The GTPase activity is enhanced by a GTPase-activating protein (GAP). As can be seen from the above statement, RAS bears an important function of "molecular switch" in an intracellular signal transduction pathway for EGFR or the like and plays a critical role in the processes of cell growth, proliferation, angiogenesis and the like (Nature Rev. Cancer, 2011, 11, p.761-774, Nature Rev. Drug Discov., 2014, 13, p.828-851, Nature Rev. Drug Discov., 2016, 15, p.771-785).

Substitution of an amino acid by spontaneous mutation of the RAS gene results in a constant activated state due to hypofunction of RAS as GTPase or hyporeactivity to GAP, and then, signals are continuously transmitted to downstream. The excessive signaling causes carcinogenesis or cancer growth acceleration. It is said that pancreatic ductal adenocarcinoma occurs through a weakly heteromorphic stage and a subsequent highly heteromorphic stage in the pancreatic intraepithelial neoplasia (PanIN), and mutation of the KRAS gene has already been recognized in an initial stage of PanIN. Subsequently, abnormality occurs in INK4A, p53 and SMAD4, which are tumor suppression genes, leading to malignancy (Nature Rev. Cancer, 2010, 10, p.683-695). Furthermore, in 90% or more of the cases of pancreatic ductal adenocarcinoma, mutation is seen in the KRAS gene, and a majority of them are a spontaneous point mutation in the codon 12 located in the KRAS exon 2 (Cancer Cell 2017, 32, p.185-203). As can be seen from the above statement, KRAS plays a critical role in the processes of carcinogenesis and development of pancreatic cancer.

As a mutation of a KRAS gene, KRAS G12C mutation, KRAS G12D mutation and the like are known. G12C mutant KRAS frequently occurs in non-small-cell lung cancer but occurs few percent in pancreatic cancer (Cancer Cell 2014, 25, p.272-281), and a therapeutic agent against another KRAS mutation is desired. G12D mutant KRAS is seen in about 34% of the cases of pancreatic cancer, and this rate is reported to be the highest in KRAS mutations. Moreover, G12V mutant KRAS is seen in about 22% of the cases of pancreatic cancer, and this rate is reported to be the second highest in KRAS mutations after G12D mutation (Nat. Rev. Cancer, 2018, 18, p.767-777).

Patent Documents 1, 2 and 3 disclose RAS inhibitors, and Patent Documents 2 and 3 disclose compounds represented by the following formulae (A) and (B), respectively (see the publications for the meanings of the symbols in the formulae). Patent Documents 1, 2 and 3 state that the inhibitors are useful for a cancer with a mutation in the codon 12 of KRAS. The G12D mutation is one of such mutations, but any effect on the G12D mutant KRAS cancer is not described.

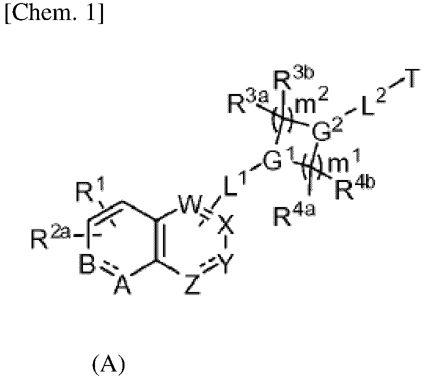

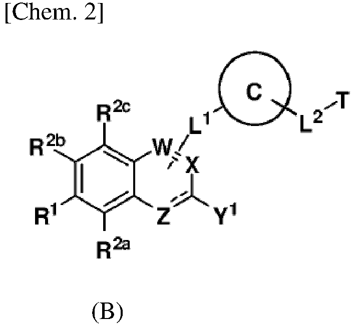

Patent Documents 9, 10 and 11 disclose KRAS G12D inhibitors.

Patent Document 12 discloses a pan-KRAS inhibitor.

In recent years, as a technique for inducing degradation of a target protein, bifunctional compounds collectively called as PROTAC (PROteolysis-TArgeting Chimera) or SNIPER (Specific and Nongenetic IAP-dependent Protein Eraser) are found and are expected as one novel technique of drug development modality (Drug. Discov. Today Technol., 2019, 31, p15-27). Such a bifunctional compound promotes formation of a composite of the target protein and an E3 ligase in a cell, and degradation of the target protein is induced by using the ubiquitin-proteasome system. The ubiquitin-proteasome system is one of intracellular protein degradation mechanisms. A protein called E3 ligase recognizes a protein to be degraded to convert the protein into ubiquitin, whereby degradation by proteasome is promoted.

Six hundred E3 ligases or more are present in an organism and are roughly divided into four types of HECT-domain E3s, U-box E3s, monomeric RING E3s and multi-subunit E3s. E3 ligases used as a bifunctional degradation inducer which are called PROTAC, SNIPER or the like are currently limited, and typical examples thereof include Von Hippel-Lindau (VHL), celebron (CRBN), inhibitor of apoptosis protein (IAP), mouse double minute 2 homolog (MDM2) and the like. In particular, VHL is reported in Patent Document 4, and CRBN is reported in Patent Document 5.

The bifunctional compounds are compounds in which a ligand of a target protein and a ligand of an E3 ligase are bound via a linker, and bifunctional compounds for degrading a KRAS protein have ever been reported (Non-patent Document 1, Non-patent Document 2, Patent Document 6, Patent Document 7, Patent Document 8, Patent Document 13, Patent Document 14, Patent Document 15, Patent Document 16 and Patent Document 17).

### [Related Art Document]

### [Patent Document]

[Patent Document 1] WO2016/049565
[Patent Document 2] WO2016/049568
[Patent Document 3] WO2017/172979
[Patent Document 4] WO2013/106643
[Patent Document 5] WO2015/160845
[Patent Document 6] US2018/0015087
[Patent Document 7] WO2019/195609
[Patent Document 8] WO2020/018788
[Patent Document 9] WO2021/041671
[Patent Document 10] WO2021/106231
[Patent Document 11] WO2021/107160
[Patent Document 12] WO2022/132200
[Patent Document 13] WO2021/051034
[Patent Document 14] WO2022/087335
[Patent Document 15] WO2022/061348
[Patent Document 16] WO2022/148421
[Patent Document 17] WO2022/173032

### [Non-patent Document]

[Non-patent Document 1] Cell. Chem. Biol., 2020, 27, p19-31
[Non-patent Document 2] ACS Cent. Sci., 2020, 6, p1367-1375

### [Summary of the Invention]

### [Problem that the invention is to solve]

A pharmaceutical composition, for example, a heterocyclic compound that is excellent in a degradation-inducing action on a mutant KRAS protein, in particular, in a degradation-inducing action on a G12V mutant, G12D mutant and G12C mutant KRAS protein, and that is expected to be useful as a mutant KRAS inhibitor, in particular, as a G12V mutant, G12D mutant and G12C mutant KRAS inhibitor and to be useful as an active ingredient of a pharmaceutical composition for treating pancreatic cancer, in particular, mutant KRAS-positive pancreatic cancer, in particular, G12V mutant, G12D mutant and G12C mutant KRAS-positive pancreatic cancer, is provided.

### [Means for solving the problem]

The present inventors have intensively and extensively studied about a compound that is useful as an active ingredient of a pharmaceutical composition for treating pancreatic cancer. As a result, the present inventors have found that a heterocyclic compound of the formula (I), in particular, a bifunctional compound of the formula (I) characterized in that a substituent on the position 8 of a heterocyclic compound selected from the group consisting of quinazoline and quinoline is bound to a ligand of an E3 ligase or that a substituent on the position 8 of a heterocyclic compound selected from the group consisting of quinazoline and quinoline is bound to a ligand of an E3 ligase via a linker, has an excellent degradation-inducing action on a mutant KRAS protein, in particular, a degradation-inducing action on a G12V mutant, G12D mutant and G12C mutant KRAS protein, and has a mutant KRAS inhibition activity, in particular, a G12V mutant, G12D mutant and G12C mutant KRAS inhibition activity, thus completing the present invention.

Specifically, the present invention relates to a compound of the formula (I) or a salt thereof and a pharmaceutical composition that contains a compound of the formula (I) or a salt thereof and one or more pharmaceutically acceptable excipients. 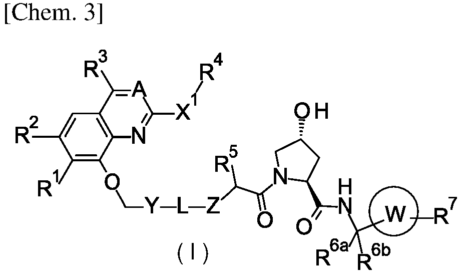 (In the formula,
A is CR^{A} or N,
wherein R^{A} is H or C₁₋₃ alkyl,
X¹ is -CH₂- or -O-,
R¹ is naphthyl optionally substituted with OH or the formula (II) below, 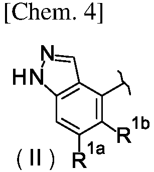
wherein R^{1a} is H, methyl, F or Cl, and
R^{1b} is F, Cl, methyl or ethyl,
R² is H, halogen, C₁₋₃ alkyl optionally substituted with a group selected from the group consisting of OH and OCH₃, cyclopropyl or vinyl,
R³ is a group selected from the group consisting of the formula (III), the formula (IV), the formula (V), the formula (VI), the formula (VII), the formula (VIII), the formula (IX), the formula (X), the formula (XI) and the formula (XXXV) below, 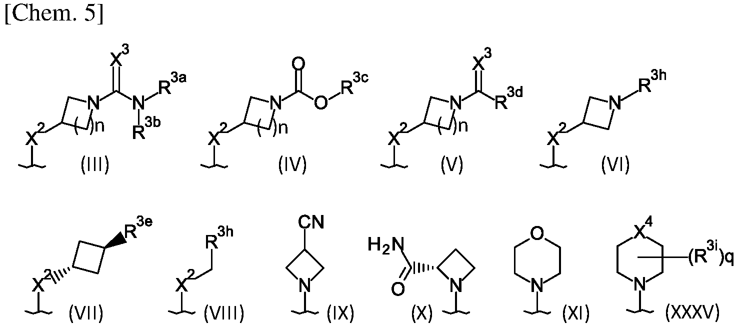
wherein R^{3a} is -(CH₂)ₚCHR^{3f}-NR^{N1}R^{N2}; -(CH₂)ₚCHR^{3f}-OR^{3g}; a 5-membered or 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3b} is H or C₁₋₃ alkyl,
R^{3c} and R^{3d} are -(CH₂)ₚCHR^{3f}-NR^{N1}R^{N2}; -(CH₂)ₚCHR^{3f}-OR^{3g}; a 4-membered to 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3e} is -O-C₂₋₃ alkylene-NR^{N1}R^{N2},
R^{3f} is H, F or C₁₋₃ alkyl,
R^{3g} is H or C₁₋₃ alkyl,
R^{3h} is optionally substituted 5-membered heteroaryl containing one to four hetero atoms selected from the group consisting of oxygen, sulfur and nitrogen or optionally substituted 6-membered heteroaryl containing one to three nitrogen atoms,
R³ⁱ, which are the same as or different from each other, are groups selected from the group consisting of H, OH, optionally substituted C₁₋₃ alkyl, -O-optionally substituted C₁₋₃ alkyl, -NH-optionally substituted C₁₋₃ alkyl, -N-(optionally substituted C₁₋₃ alkyl)₂, halogen, -CN and oxo, or
two R³ⁱ on a same carbon atom, together with the neighboring carbon atom, may form a ring selected from the group consisting of C₃₋₆ cycloalkane and a 4-membered to 6-membered saturated hetero ring containing one or two hetero atoms selected from the group consisting of oxygen, sulfur and nitrogen as ring-constituting atoms to form a spiro ring as the group in the formula (XXXV), wherein the spiro ring is optionally substituted with one or two groups selected from the group consisting of C₁₋₃ alkyl, -O-(C₁₋₃ alkyl), OH, halogen and oxo, or
R³ⁱ on two neighboring carbon atoms, together with the two carbon atoms, may form a ring selected from the group consisting of C₃₋₆ cycloalkane and a 4-membered to 6-membered saturated hetero ring containing one or two hetero atoms selected from the group consisting of oxygen, sulfur and nitrogen as ring-constituting atoms to form a condensed ring as the group in the formula (XXXV), wherein the condensed ring is optionally substituted with one or two groups selected from the group consisting of C₁₋₃ alkyl, -O-(C₁₋₃ alkyl), OH, halogen and oxo, or
R³ⁱ on two carbon atoms which are not neighboring, together with the two carbon atoms, may form a cross-linked structure composed of one or two carbon atoms, wherein the group in the formula (XXXV), which is a ring having the cross-linked structure, is optionally substituted with one or two groups selected from the group consisting of C₁₋₃ alkyl, -O-(C₁₋₃ alkyl), OH, halogen and oxo,
R^{N1} and R^{N2}, which are the same as or different from each other, are H or C₁₋₃ alkyl, or
R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms, or
R^{3f} and R^{N1}, together with the carbon atom and the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms,
X² is -O-, -NH- or -N(C₁₋₃ alkyl)-,
X³ is O or S,
X⁴ is -CH₂-, -CH₂-CH₂- or -O-CH₂-,
n is 1 or 2, p is 1 or 2, and
q is an integer of 1 to 8,
with the proviso that X² in the formula (IV) is -O-, -NH- or -N(C₂₋₃ alkyl)- when R^{3c} is -(CH₂)ₚCHR^{3f}-NR^{N1}R^{N2},
R⁴ is C₁₋₆ alkyl optionally substituted with a group selected from the group consisting of F, OH, OCH₃, R^{4a}, cyclopropyl, N(R^{4a})₂, pyrrolidinyl optionally substituted with R^{4a} and tetrahydrofuranyl optionally substituted with R^{4a}; piperidinyl optionally substituted with R^{4b}; or tetrahydropyranyl optionally substituted with R^{4a},
wherein R^{4a} is C₁₋₃ alkyl optionally substituted with F, and
R^{4b} is C₁₋₃ alkyl substituted with one to three F,
R⁵ is methyl, ethyl, isopropyl, isobutyl, sec-butyl, tert-butyl, C₃₋₆ cycloalkylmethyl or C₃₋₆ cycloalkyl,
R^{6a} and R^{6b}, which are the same as or different from each other, are H or C₁₋₆ alkyl optionally substituted with a group selected from the group consisting of F, OH, OCH₃ and N(CH₃)₂, or
R^{6a} and R^{6b}, together with the carbon to which they are attached, may form optionally substituted C₃₋₆ cycloalkane or an optionally substituted 4-membered to 6-membered saturated hetero ring containing one or two hetero atoms selected from the group consisting of oxygen, sulfur and nitrogen as ring-constituting atoms,
R⁷ is an optionally substituted 4-membered to 6-membered saturated heterocyclic group containing one or two hetero atoms selected from the group consisting of oxygen, sulfur and nitrogen as ring-constituting atoms, optionally substituted 5-membered heteroaryl containing one to four hetero atoms selected from the group consisting of oxygen, sulfur and nitrogen or 6-membered heteroaryl containing one to three nitrogen atoms,
W is optionally substituted phenylene or optionally substituted 6-membered heteroarenediyl containing one to three nitrogen atoms as ring-constituting atoms,
Y is phenylene optionally substituted with F or Cl or pyridinediyl,
L is -(L¹-L²-L³-L⁴)-,
wherein L¹, L², L³ and L⁴, which are the same as or different from each other, are groups selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O,
wherein R^{L1} is H or C₁₋₃ alkyl, and
Z is NH or 5-membered heteroarenediyl containing one to four hetero atoms selected from the group consisting of oxygen, sulfur and nitrogen as ring-constituting atoms,
or Y-L-Z is the formula (XII) below.)

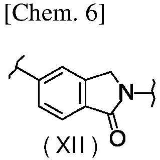

Note that, when a sign in a chemical formula in this specification is used in another chemical formula, the same sign represents the same meaning unless otherwise specified.

The present invention relates to a pharmaceutical composition containing the compound of the formula (I) or a salt thereof and one or more pharmaceutically acceptable excipients, a pharmaceutical composition for treating pancreatic cancer in an aspect, a pharmaceutical composition for treating mutant KRAS-positive pancreatic cancer in an aspect, in particular, a pharmaceutical composition for treating G12V mutant, G12D mutant and G12C mutant KRAS-positive pancreatic cancer, a pharmaceutical composition for treating metastatic pancreatic cancer in an aspect, a pharmaceutical composition for treating locally advanced pancreatic cancer in an aspect, a pharmaceutical composition for treating recurrent or refractory pancreatic cancer in an aspect, a pharmaceutical composition for treating pancreatic cancer of an untreated patient and/or a patient with history of treatment in an aspect, a pharmaceutical composition for treating mutant KRAS-positive metastatic pancreatic cancer in an aspect, in particular, a pharmaceutical composition for treating G12V mutant, G12D mutant and G12C mutant KRAS-positive metastatic pancreatic cancer, a pharmaceutical composition for treating G12D mutant KRAS-positive locally advanced pancreatic cancer in an aspect, in particular, a pharmaceutical composition for treating G12V mutant, G12D mutant and G12C mutant KRAS-positive locally advanced pancreatic cancer, a pharmaceutical composition for treating G12D mutant KRAS-positive recurrent or refractory pancreatic cancer in an aspect, in particular, a pharmaceutical composition for treating G12V mutant, G12D mutant and G12C mutant KRAS-positive recurrent or refractory pancreatic cancer, and a pharmaceutical composition for treating G12D mutant KRAS-positive pancreatic cancer of an untreated patient and/or a patient with history of treatment in an aspect, in particular, a pharmaceutical composition for treating G12V mutant, G12D mutant and G12C mutant KRAS-positive pancreatic cancer of an untreated patient and/or a patient with history of treatment. The pharmaceutical composition includes a therapeutic agent for pancreatic cancer, or mutant KRAS-positive pancreatic cancer in an aspect, containing the compound of the formula (I) or a salt thereof, in particular, a therapeutic agent for G12V mutant, G12D mutant and G12C mutant KRAS-positive pancreatic cancer.

The present invention relates to a pharmaceutical composition containing the compound of the formula (I) or a salt thereof and one or more pharmaceutically acceptable excipients, a pharmaceutical composition for treating pancreatic cancer in an aspect, a pharmaceutical composition for treating G12V mutant and G12D mutant KRAS-positive pancreatic cancer in an aspect, a pharmaceutical composition for treating metastatic pancreatic cancer in an aspect, a pharmaceutical composition for treating locally advanced pancreatic cancer in an aspect, a pharmaceutical composition for treating recurrent or refractory pancreatic cancer in an aspect, a pharmaceutical composition for treating pancreatic cancer of an untreated patient and/or a patient with history of treatment in an aspect, a pharmaceutical composition for treating G12V mutant and G12D mutant KRAS-positive metastatic pancreatic cancer in an aspect, a pharmaceutical composition for treating G12V mutant and G12D mutant KRAS-positive locally advanced pancreatic cancer in an aspect, a pharmaceutical composition for treating G12V mutant and G12D mutant KRAS-positive recurrent or refractory pancreatic cancer in an aspect and a pharmaceutical composition for treating G12V mutant and G12D mutant KRAS-positive pancreatic cancer of an untreated patient and/or a patient with history of treatment in an aspect. The pharmaceutical composition includes a therapeutic agent for pancreatic cancer, or G12V mutant and G12D mutant KRAS-positive pancreatic cancer in an aspect, containing the compound of the formula (I) or a salt thereof.

The present invention relates to a pharmaceutical composition containing the compound of the formula (I) or a salt thereof and one or more pharmaceutically acceptable excipients, a pharmaceutical composition for treating pancreatic cancer in an aspect, a pharmaceutical composition for treating G12V mutant KRAS-positive pancreatic cancer in an aspect, a pharmaceutical composition for treating metastatic pancreatic cancer in an aspect, a pharmaceutical composition for treating locally advanced pancreatic cancer in an aspect, a pharmaceutical composition for treating recurrent or refractory pancreatic cancer in an aspect, a pharmaceutical composition for treating pancreatic cancer of an untreated patient and/or a patient with history of treatment in an aspect, a pharmaceutical composition for treating G12V mutant KRAS-positive metastatic pancreatic cancer in an aspect, a pharmaceutical composition for treating G12V mutant KRAS-positive locally advanced pancreatic cancer in an aspect, a pharmaceutical composition for treating G12V mutant KRAS-positive recurrent or refractory pancreatic cancer in an aspect and a pharmaceutical composition for treating G12V mutant KRAS-positive pancreatic cancer of an untreated patient and/or a patient with history of treatment in an aspect. The pharmaceutical composition includes a therapeutic agent for pancreatic cancer, or G12V mutant KRAS-positive pancreatic cancer in an aspect, containing the compound of the formula (I) or a salt thereof.

The present invention relates to a pharmaceutical composition containing the compound of the formula (I) or a salt thereof and one or more pharmaceutically acceptable excipients, a pharmaceutical composition for treating pancreatic cancer in an aspect, a pharmaceutical composition for treating G12D mutant KRAS-positive pancreatic cancer in an aspect, a pharmaceutical composition for treating metastatic pancreatic cancer in an aspect, a pharmaceutical composition for treating locally advanced pancreatic cancer in an aspect, a pharmaceutical composition for treating recurrent or refractory pancreatic cancer in an aspect, a pharmaceutical composition for treating pancreatic cancer of an untreated patient and/or a patient with history of treatment in an aspect, a pharmaceutical composition for treating G12D mutant KRAS-positive metastatic pancreatic cancer in an aspect, a pharmaceutical composition for treating G12D mutant KRAS-positive locally advanced pancreatic cancer in an aspect, a pharmaceutical composition for treating G12D mutant KRAS-positive recurrent or refractory pancreatic cancer in an aspect and a pharmaceutical composition for treating G12D mutant KRAS-positive pancreatic cancer of an untreated patient and/or a patient with history of treatment in an aspect. The pharmaceutical composition includes a therapeutic agent for pancreatic cancer, or G12D mutant KRAS-positive pancreatic cancer in an aspect, containing the compound of the formula (I) or a salt thereof.

The present invention relates to a pharmaceutical composition containing the compound of the formula (I) or a salt thereof and one or more pharmaceutically acceptable excipients, a pharmaceutical composition for treating pancreatic cancer in an aspect, a pharmaceutical composition for treating G12C mutant KRAS-positive pancreatic cancer in an aspect, a pharmaceutical composition for treating metastatic pancreatic cancer in an aspect, a pharmaceutical composition for treating locally advanced pancreatic cancer in an aspect, a pharmaceutical composition for treating recurrent or refractory pancreatic cancer in an aspect, a pharmaceutical composition for treating pancreatic cancer of an untreated patient and/or a patient with history of treatment in an aspect, a pharmaceutical composition for treating G12C mutant KRAS-positive metastatic pancreatic cancer in an aspect, a pharmaceutical composition for treating G12C mutant KRAS-positive locally advanced pancreatic cancer in an aspect, a pharmaceutical composition for treating G12C mutant KRAS-positive recurrent or refractory pancreatic cancer in an aspect and a pharmaceutical composition for treating G12C mutant KRAS-positive pancreatic cancer of an untreated patient and/or a patient with history of treatment in an aspect. The pharmaceutical composition includes a therapeutic agent for pancreatic cancer, or G12C mutant KRAS-positive pancreatic cancer in an aspect, containing the compound of the formula (I) or a salt thereof.

The present invention relates to use of the compound of the formula (I) or a salt thereof for producing a pharmaceutical composition for treating pancreatic cancer, mutant KRAS-positive pancreatic cancer in an aspect, in particular, G12V mutant, G12D mutant and G12C mutant KRAS-positive pancreatic cancer, metastatic pancreatic cancer in an aspect, locally advanced pancreatic cancer in an aspect, recurrent or refractory pancreatic cancer in an aspect, pancreatic cancer of an untreated patient and/or a patient with history of treatment in an aspect, mutant KRAS-positive metastatic pancreatic cancer in an aspect, in particular, G12V mutant, G12D mutant and G12C mutant KRAS-positive metastatic pancreatic cancer, mutant KRAS-positive locally advanced pancreatic cancer in an aspect, in particular, G12V mutant, G12D mutant and G12C mutant KRAS-positive locally advanced pancreatic cancer, mutant KRAS-positive recurrent or refractory pancreatic cancer in an aspect, in particular, G12V mutant, G12D mutant and G12C mutant KRAS-positive recurrent or refractory pancreatic cancer, or mutant KRAS-positive pancreatic cancer of an untreated patient and/or a patient with history of treatment in an aspect, in particular, G12V mutant, G12D mutant and G12C mutant KRAS-positive metastatic pancreatic cancer of an untreated patient and/or a patient with history of treatment, to use of the compound of the formula (I) or a salt thereof for treating pancreatic cancer, or mutant KRAS-positive pancreatic cancer in an aspect, in particular, G12V mutant, G12D mutant and G12C mutant KRAS-positive pancreatic cancer, to the compound of the formula (I) or a salt thereof for use in treatment of pancreatic cancer, or mutant KRAS-positive pancreatic cancer in an aspect, in particular, G12V mutant, G12D mutant and G12C mutant KRAS-positive pancreatic cancer and to a method for treating pancreatic cancer, or mutant KRAS-positive pancreatic cancer in an aspect, in particular, G12V mutant, G12D mutant and G12C mutant KRAS-positive pancreatic cancer, including administering an effective amount of the compound of the formula (I) or a salt thereof to a subject.

The present invention relates to use of the compound of the formula (I) or a salt thereof for producing a pharmaceutical composition for treating pancreatic cancer, G12V mutant and G12D mutant KRAS-positive pancreatic cancer in an aspect, metastatic pancreatic cancer in an aspect, locally advanced pancreatic cancer in an aspect, recurrent or refractory pancreatic cancer in an aspect, pancreatic cancer of an untreated patient and/or a patient with history of treatment in an aspect, G12V mutant and G12D mutant KRAS-positive metastatic pancreatic cancer in an aspect, G12V mutant and G12D mutant KRAS-positive locally advanced pancreatic cancer in an aspect, G12V mutant and G12D mutant KRAS-positive recurrent or refractory pancreatic cancer in an aspect, or G12V mutant and G12D mutant KRAS-positive pancreatic cancer of an untreated patient and/or a patient with history of treatment in an aspect, to use of the compound of the formula (I) or a salt thereof for treating pancreatic cancer, or G12V mutant and G12D mutant KRAS-positive pancreatic cancer in an aspect, to the compound of the formula (I) or a salt thereof for use in treatment of pancreatic cancer, or G12V mutant and G12D mutant KRAS-positive pancreatic cancer in an aspect, and to a method for treating pancreatic cancer, or G12V mutant and G12D mutant KRAS-positive pancreatic cancer in an aspect, including administering an effective amount of the compound of the formula (I) or a salt thereof to a subject.

The present invention relates to use of the compound of the formula (I) or a salt thereof for producing a pharmaceutical composition for treating pancreatic cancer, G12V mutant KRAS-positive pancreatic cancer in an aspect, metastatic pancreatic cancer in an aspect, locally advanced pancreatic cancer in an aspect, recurrent or refractory pancreatic cancer in an aspect, pancreatic cancer of an untreated patient and/or a patient with history of treatment in an aspect, G12V mutant KRAS-positive metastatic pancreatic cancer in an aspect, G12V mutant KRAS-positive locally advanced pancreatic cancer in an aspect, G12V mutant KRAS-positive recurrent or refractory pancreatic cancer in an aspect, or G12V mutant KRAS-positive pancreatic cancer of an untreated patient and/or a patient with history of treatment in an aspect, to use of the compound of the formula (I) or a salt thereof for treating pancreatic cancer, or G12V mutant KRAS-positive pancreatic cancer in an aspect, to the compound of the formula (I) or a salt thereof for use in treatment of pancreatic cancer, or G12V mutant KRAS-positive pancreatic cancer in an aspect, and to a method for treating pancreatic cancer, or G12V mutant KRAS-positive pancreatic cancer in an aspect, including administering an effective amount of the compound of the formula (I) or a salt thereof to a subject.

The present invention relates to use of the compound of the formula (I) or a salt thereof for producing a pharmaceutical composition for treating pancreatic cancer, G12D mutant KRAS-positive pancreatic cancer in an aspect, metastatic pancreatic cancer in an aspect, locally advanced pancreatic cancer in an aspect, recurrent or refractory pancreatic cancer in an aspect, pancreatic cancer of an untreated patient and/or a patient with history of treatment in an aspect, G12D mutant KRAS-positive metastatic pancreatic cancer in an aspect, G12D mutant KRAS-positive locally advanced pancreatic cancer in an aspect, G12D mutant KRAS-positive recurrent or refractory pancreatic cancer in an aspect, or G12D mutant KRAS-positive pancreatic cancer of an untreated patient and/or a patient with history of treatment in an aspect, to use of the compound of the formula (I) or a salt thereof for treating pancreatic cancer, or G12D mutant KRAS-positive pancreatic cancer in an aspect, to the compound of the formula (I) or a salt thereof for use in treatment of pancreatic cancer, or G12D mutant KRAS-positive pancreatic cancer in an aspect, and to a method for treating pancreatic cancer, or G12D mutant KRAS-positive pancreatic cancer in an aspect, including administering an effective amount of the compound of the formula (I) or a salt thereof to a subject.

The present invention relates to use of the compound of the formula (I) or a salt thereof for producing a pharmaceutical composition for treating pancreatic cancer, G12C mutant KRAS-positive pancreatic cancer in an aspect, metastatic pancreatic cancer in an aspect, locally advanced pancreatic cancer in an aspect, recurrent or refractory pancreatic cancer in an aspect, pancreatic cancer of an untreated patient and/or a patient with history of treatment in an aspect, G12C mutant KRAS-positive metastatic pancreatic cancer in an aspect, G12C mutant KRAS-positive locally advanced pancreatic cancer in an aspect, G12C mutant KRAS-positive recurrent or refractory pancreatic cancer in an aspect, or G12C mutant KRAS-positive pancreatic cancer of an untreated patient and/or a patient with history of treatment in an aspect, to use of the compound of the formula (I) or a salt thereof for treating pancreatic cancer, or G12C mutant KRAS-positive pancreatic cancer in an aspect, to the compound of the formula (I) or a salt thereof for use in treatment of pancreatic cancer, or G12C mutant KRAS-positive pancreatic cancer in an aspect, and to a method for treating pancreatic cancer, or G12C mutant KRAS-positive pancreatic cancer in an aspect, including administering an effective amount of the compound of the formula (I) or a salt thereof to a subject.

The present invention also relates to the compound of the formula (I) or a salt thereof which is a mutant KRAS protein degradation inducer and/or a mutant KRAS inhibitor, in particular, a G12V mutant, G12D mutant and G12C mutant KRAS protein degradation inducer and/or a G12V mutant, G12D mutant and G12C mutant KRAS inhibitor, to the compound of the formula (I) or a salt thereof for use as a mutant KRAS protein degradation inducer and/or a mutant KRAS inhibitor, in particular, a G12V mutant, G12D mutant and G12C mutant KRAS protein degradation inducer and/or a G12V mutant, G12D mutant and G12C mutant KRAS inhibitor, and to a mutant KRAS protein degradation inducer and/or a mutant KRAS inhibitor, in particular, a G12V mutant, G12D mutant and G12C mutant KRAS protein degradation inducer and/or a G12V mutant, G12D mutant and G12C mutant KRAS inhibitor, containing the compound of the formula (I) or a salt thereof.

The present invention also relates to the compound of the formula (I) or a salt thereof which is a G12V mutant and G12D mutant KRAS protein degradation inducer and/or a G12V mutant and G12D mutant KRAS inhibitor, to the compound of the formula (I) or a salt thereof for use as a G12V mutant and G12D mutant KRAS protein degradation inducer and/or a G12V mutant and G12D mutant KRAS inhibitor and to a G12V mutant and G12D mutant KRAS protein degradation inducer and/or a G12V mutant and G12D mutant KRAS inhibitor containing the compound of the formula (I) or a salt thereof.

The present invention also relates to the compound of the formula (I) or a salt thereof which is a G12V mutant KRAS protein degradation inducer and/or a G12V mutant KRAS inhibitor, to the compound of the formula (I) or a salt thereof for use as a G12V mutant KRAS protein degradation inducer and/or a G12V mutant KRAS inhibitor and to a G12V mutant KRAS protein degradation inducer and/or a G12V mutant KRAS inhibitor containing the compound of the formula (I) or a salt thereof.

The present invention also relates to the compound of the formula (I) or a salt thereof which is a G12D mutant KRAS protein degradation inducer and/or a G12D mutant KRAS inhibitor, to the compound of the formula (I) or a salt thereof for use as a G12D mutant KRAS protein degradation inducer and/or a G12D mutant KRAS inhibitor and to a G12D mutant KRAS protein degradation inducer and/or a G12D mutant KRAS inhibitor containing the compound of the formula (I) or a salt thereof.

The present invention also relates to the compound of the formula (I) or a salt thereof which is a G12C mutant KRAS protein degradation inducer and/or a G12C mutant KRAS inhibitor, to the compound of the formula (I) or a salt thereof for use as a G12C mutant KRAS protein degradation inducer and/or a G12C mutant KRAS inhibitor and to a G12C mutant KRAS protein degradation inducer and/or a G12C mutant KRAS inhibitor containing the compound of the formula (I) or a salt thereof.

Note that the "subject" is a human or another animal that needs the treatment, and an aspect thereof is a human who needs the prevention or treatment.

### [Advantageous Effects of the Invention]

The compound of the formula (I) or a salt thereof has a degradation-inducing action on a mutant KRAS protein and a mutant KRAS inhibition activity, in particular, has a degradation-inducing action on a G12V mutant, G12D mutant and G12C mutant KRAS protein and a G12V mutant, G12D mutant and G12C mutant KRAS inhibition action and can be used as a therapeutic agent for pancreatic cancer, in particular, mutant KRAS-positive pancreatic cancer, in particular, G12V mutant, G12D mutant and G12C mutant KRAS-positive pancreatic cancer.

### [Mode for Carrying Out the Invention]

The present invention will be explained in detail below.

In this specification, "optionally substituted" means being unsubstituted or having one to five substituents. In an aspect, the term means being unsubstituted or having one to three substituents. Note that when there are multiple substituents, the substituents may be the same as or different from each other.

The "C₁₋₁₂ alkyl" is linear or branched alkyl having 1 to 12 carbon atoms, and examples thereof include methyl, ethyl, n-propyl, isopropyl, n-butyl, isobutyl, sec-butyl, tert-butyl, n-pentyl, n-hexyl, dodecyl and the like (the numbers of carbon atoms are described similarly below). An aspect thereof is ethyl or dodecyl.

Similarly, the "C₁₋₆ alkyl" is linear or branched alkyl having one to six carbon atoms, and examples thereof include methyl, ethyl, n-propyl, isopropyl, n-butyl, isobutyl, sec-butyl, tert-butyl, n-pentyl and n-hexyl. An aspect thereof is methyl, ethyl, n-propyl, isopropyl or sec-butyl; another aspect is methyl, ethyl, isopropyl or tert-butyl; and another aspect is methyl, ethyl, n-propyl, isopropyl or n-butyl.

Similarly, the "C₁₋₃ alkyl" is linear or branched alkyl having one to three carbon atoms, and examples thereof include methyl, ethyl, n-propyl and isopropyl. An aspect thereof is methyl or ethyl; another aspect is n-propyl or isopropyl; another aspect is methyl or isopropyl; another aspect is ethyl or isopropyl; another aspect is methyl; another aspect is ethyl; another aspect is isopropyl; and another aspect is n-propyl.

Similarly, the "C₂₋₃ alkyl" is linear or branched alkyl having two or three carbon atoms, and examples thereof include ethyl, n-propyl and isopropyl. An aspect thereof is ethyl; another aspect is isopropyl; and another aspect is n-propyl.

The "C₃₋₆ cycloalkyl" is cycloalkyl having three to six carbon atoms, and examples thereof include cyclopropyl, cyclobutyl, cyclopentyl and cyclohexyl. An aspect thereof is cyclobutyl, cyclopentyl or cyclohexyl; another aspect is cyclobutyl or cyclopentyl; another aspect is cyclopentyl or cyclohexyl; another aspect is cyclopropyl or cyclobutyl; another aspect is cyclopropyl; another aspect is cyclobutyl; another aspect is cyclopentyl; and another aspect is cyclohexyl.

The "C₁₋₃ alkylene" is a divalent group formed by removing a hydrogen atom from C₁₋₃ alkyl and is linear or branched C₁₋₃ alkylene, and examples thereof include methylene, ethylene, trimethylene, methylmethylene, methylethylene and 1,1-dimethylmethylene. An aspect thereof is linear or branched C₁₋₃ alkylene; another aspect is methylene, ethylene or trimethylene; another aspect is methylene or ethylene; another aspect is methylene; and another aspect is ethylene. Similarly, the "C₂₋₃ alkylene" is a divalent group formed by removing a hydrogen atom from C₂₋₃ alkyl and is linear or branched C₂₋₃ alkylene, and examples thereof include ethylene, trimethylene, methylmethylene, methylethylene and 1,1-dimethylmethylene. An aspect thereof is ethylene or trimethylene; another aspect is ethylene; and another aspect is trimethylene.

The "saturated heterocyclic group" is a saturated hydrocarbon ring group containing a hetero atom selected from the group consisting of oxygen, sulfur and nitrogen as a ring-constituting atom. The sulfur atom as the ring-constituting atom of the saturated heterocyclic group may be oxidized.

Thus, the "4-membered to 6-membered saturated heterocyclic group" is a 4-membered to 6-membered saturated heterocyclic group containing a hetero atom selected from the group consisting of oxygen, sulfur and nitrogen as a ring-constituting atom. An aspect of the "4-membered to 6-membered saturated heterocyclic group" is a 4-membered to 6-membered saturated heterocyclic group containing one or two hetero atoms selected from the group consisting of oxygen, sulfur and nitrogen as ring-constituting atoms. An aspect of the 4-membered to 6-membered saturated heterocyclic group containing one or two hetero atoms selected from the group consisting of oxygen, sulfur and nitrogen as ring-constituting atoms is a 4-membered to 6-membered saturated heterocyclic group containing one hetero atom selected from the group consisting of oxygen, sulfur and nitrogen as a ring-constituting atom; another aspect is a 5-membered or 6-membered saturated heterocyclic group containing one or two hetero atoms selected from the group consisting of oxygen, sulfur and nitrogen as ring-constituting atoms; another aspect is a 4-membered saturated heterocyclic group containing one hetero atom selected from the group consisting of oxygen, sulfur and nitrogen as a ring-constituting atom; another aspect is a 5-membered saturated heterocyclic group containing one or two hetero atoms selected from the group consisting of oxygen, sulfur and nitrogen as ring-constituting atoms; another aspect is a 6-membered saturated heterocyclic group containing one or two hetero atoms selected from the group consisting of oxygen, sulfur and nitrogen as ring-constituting atoms; another aspect is oxetanyl, tetrahydrofuranyl, tetrahydropyranyl, azetidinyl, pyrrolidinyl, piperidinyl, oxazolidinyl, imidazolidinyl, piperazinyl, morpholinyl, thiomorpholinyl or dioxothiomorpholinyl; another aspect is oxetanyl, tetrahydrofuranyl, tetrahydropyranyl, azetidinyl, pyrrolidinyl, piperidinyl, piperazinyl, morpholinyl, thiomorpholinyl or dioxothiomorpholinyl; another aspect is oxetanyl, tetrahydrofuranyl, tetrahydropyranyl, pyrrolidinyl, piperidinyl or morpholinyl; another aspect is oxetanyl, tetrahydrofuranyl, tetrahydropyranyl, pyrrolidinyl or piperidinyl; another aspect is oxetanyl, tetrahydrofuranyl or tetrahydropyranyl; another aspect is pyrrolidinyl or piperidinyl; another aspect is oxetanyl; another aspect is tetrahydrofuranyl; another aspect is tetrahydropyranyl; another aspect is pyrrolidinyl; another aspect is piperidinyl; another aspect is morpholinyl; and another aspect is oxazolidinyl.

The "heteroaryl" is a heterocyclic group containing a hetero atom selected from the group consisting of oxygen, sulfur and nitrogen as a ring-constituting atom.

Thus, the "5-membered heteroaryl" is a heterocyclic group of a 5-membered ring containing one to four hetero atoms selected from the group consisting of oxygen, sulfur and nitrogen as ring-constituting atoms.

An aspect of the "5-membered heteroaryl" is a heterocyclic group of a 5-membered ring containing one to three hetero atoms selected from the group consisting of oxygen, sulfur and nitrogen as ring-constituting atoms; another aspect is pyrazolyl, imidazolyl, triazolyl, tetrazolyl, oxazolyl, isoxazolyl, thiazolyl, isothiazolyl, oxadiazolyl or thiadiazolyl; another aspect is pyrazolyl, imidazolyl, triazolyl, oxazolyl or thiazolyl; another aspect is pyrazolyl, imidazolyl, oxazolyl or thiazolyl; another aspect is pyrazolyl, imidazolyl, triazolyl or isoxazolyl; another aspect is pyrazolyl, oxazolyl or thiazolyl; another aspect is pyrazolyl, triazolyl or isoxazolyl; another aspect is pyrazolyl or thiazolyl; another aspect is pyrazolyl or triazolyl; another aspect is pyrazolyl; another aspect is imidazolyl; another aspect is oxazolyl; another aspect is thiazolyl; and another aspect is triazolyl. The "5-membered heteroarenediyl" is a divalent group obtained by removing any one hydrogen from "5-membered heteroaryl".

The "6-membered heteroaryl" is a heterocyclic group of a 6-membered ring containing one to three nitrogen atoms as ring-constituting atoms. An aspect of the "6-membered heteroaryl" is pyridyl, pyrimidinyl, pyrazinyl, pyridazinyl or triazinyl; another aspect is pyridyl or pyridazinyl; another aspect is pyridyl or pyrimidinyl; another aspect is pyridyl; and another aspect is pyrimidinyl. The "6-membered heteroarenediyl" is a divalent group obtained by removing any one hydrogen from "6-membered heteroaryl".

The "C₃₋₆ cycloalkane" is cycloalkane having three to six carbon atoms, such as for example cyclopropane, cyclobutane, cyclopentane and cyclohexane.

The "saturated hetero ring" is a saturated hydrocarbon ring containing a hetero atom selected from the group consisting of oxygen, sulfur and nitrogen as a ring-constituting atom. The sulfur atom as the ring-constituting atom of the saturated hetero ring may be oxidized. Thus, the "4-membered to 6-membered saturated hetero ring" is a saturated hydrocarbon ring of a 4-membered ring to 6-membered ring containing a hetero atom selected from the group consisting of oxygen, sulfur and nitrogen as a ring-constituting atom. The sulfur atom as the ring-constituting atom of the saturated hetero ring may be oxidized. An aspect of the "4-membered to 6-membered saturated hetero ring" is a 4-membered to 6-membered saturated hetero ring containing one or two hetero atoms selected from the group consisting of oxygen, sulfur and nitrogen as ring-constituting atoms. An aspect of the "4-membered to 6-membered saturated hetero ring" is oxetane, tetrahydrofuran, tetrahydropyran, azetidine, pyrrolidine, piperidine, oxazolidine, imidazolidine, piperazine, morpholine, thiomorpholine or dioxothiomorpholine.

The "spiro ring" is a multicyclic structure in which two cyclic structures are bound with one common spiro atom, which is a quaternary carbon.

The "ring having a cross-linked structure" is a cyclic structure having a divalent chain structure linked to two non-neighboring atoms of the ring-constituting atoms of one ring. An aspect of the "ring having a cross-linked structure" is azabicyclo[3.2.1]octane, azabicyclo[3.1.1]heptane, azabicyclo[2.2.1]heptane or azaoxabicyclo[3.2.1]octane.

The "halogen" means F, Cl, Br and I. An aspect thereof is F, Cl or Br; another aspect is F or Cl; another aspect is F or Br; another aspect is F; another aspect is Cl; and another aspect is Br.

An aspect of the substituent acceptable in the "optionally substituted C₁₋₆ alkyl" and the "optionally substituted C₁₋₃ alkyl" is F, OH, OCH₃, N(CH₃)₂, optionally substituted C₃₋₆ cycloalkyl, azabicyclo[3.3.0]octanyl or an optionally substituted 4-membered to 6-membered saturated heterocyclic group containing one or two hetero atoms selected from the group consisting of oxygen, sulfur and nitrogen. An aspect thereof is F, OH, OCH₃, N(CH₃)₂, hydroxymethyl, methoxymethyl, difluoroethyl, optionally substituted cyclopropyl, tetrahydrofuranyl, optionally substituted tetrahydropyranyl, morpholinyl, optionally substituted pyrrolidinyl, optionally substituted piperidinyl or azabicyclo[3.3.0]octanyl; another aspect is F, OH, OCH₃, N(CH₃)₂, hydroxymethyl, methoxymethyl, optionally substituted cyclopropyl, tetrahydrofuranyl, optionally substituted tetrahydropyranyl or optionally substituted pyrrolidinyl; another aspect is F, OH, OCH₃, N(CH₃)₂, hydroxymethyl, methoxymethyl, cyclopropyl, (hydroxymethyl)cyclopropyl, (methoxymethyl)cyclopropyl, tetrahydrofuranyl, tetrahydropyranyl, (hydroxymethyl)tetrahydropyranyl, (methoxymethyl)tetrahydropyranyl, pyrrolidinyl or methylpyrrolidinyl; another aspect is F, OH, OCH₃, (methoxymethyl)cyclopropyl, tetrahydrofuranyl or methylpyrrolidinyl; another aspect is F, OH or cyclopropyl; another aspect is F, OH or OCH₃; another aspect is OH or OCH₃; another aspect is F or OCH₃; another aspect is OH; another aspect is F; and another aspect is OCH₃.

An aspect of the substituent acceptable in the "optionally substituted 5-membered heteroaryl", the "optionally substituted 6-membered heteroaryl", the "optionally substituted 6-membered heteroarenediyl", the "optionally substituted C₃₋₆ cycloalkyl", the "optionally substituted pyrazolyl", the "optionally substituted pyridyl", the "optionally substituted pyrimidinyl", the "optionally substituted phenylene" and the "optionally substituted cyclopropyl" is C₁₋₃ alkyl optionally substituted with a group selected from the group consisting of OH and OCH₃, -SO₂CH₃, halogen, OH, OCH₃ or C₃₋₆ cycloalkyl. An aspect thereof is C₁₋₃ alkyl optionally substituted with a group selected from the group consisting of OH and OCH₃; another aspect is C₁₋₃ alkyl optionally substituted with OH; another aspect is C₁₋₃ alkyl optionally substituted with OCH₃; another aspect is C₁₋₃ alkyl or halogen; another aspect is methyl, ethyl, methoxymethyl or F; and another aspect is methyl, ethyl or F.

An aspect of the substituent acceptable in the "optionally substituted 4-membered to 6-membered saturated heterocyclic group", the "optionally substituted pyrrolidinyl", the "optionally substituted piperidinyl", the "optionally substituted oxetanyl", the "optionally substituted tetrahydrofuranyl" and the "optionally substituted tetrahydropyranyl" is C₁₋₃ alkyl optionally substituted with a group selected from the group consisting of F, OH and OCH₃, F, OH, OCH₃, oxo or oxetanyl. An aspect thereof is F, OH or OCH₃; another aspect is OH or methyl; another aspect is C₁₋₃ alkyl optionally substituted with a group selected from the group consisting of F, OH and OCH₃, F, oxo or oxetanyl; another aspect is C₁₋₃ alkyl optionally substituted with a group selected from the group consisting of F, OH and OCH₃ or oxo; another aspect is C₁₋₃ alkyl optionally substituted with a group selected from the group consisting of F, OH and OCH₃; another aspect is C₁₋₃ alkyl optionally substituted with F; another aspect is C₁₋₃ alkyl optionally substituted with OH; another aspect is C₁₋₃ alkyl optionally substituted with OCH₃; and another aspect is C₁₋₃ alkyl.

An aspect of the substituent acceptable in the "optionally substituted pyrrolidinediyl", the "optionally substituted piperidinediyl", the "optionally substituted piperazinediyl" and the "optionally substituted C₁₋₃ alkylene" is F, OH, OCH₃ or optionally substituted C₁₋₃ alkyl. An aspect thereof is F, OH, OCH₃, methyl, ethyl, hydroxymethyl or methoxymethyl; and another aspect is F, OH, OCH₃ or methyl.

An aspect of the "C₁₋₃ alkyl optionally substituted with F" is methyl optionally substituted with F or ethyl optionally substituted with F. Examples thereof include methyl, ethyl, monofluoromethyl, difluoromethyl, trifluoromethyl, monofluoroethyl, difluoroethyl and trifluoroethyl. An aspect thereof is methyl, ethyl, monofluoromethyl, difluoromethyl or difluoroethyl; another aspect is monofluoromethyl or difluoromethyl; another aspect is monofluoromethyl or difluoroethyl; another aspect is difluoromethyl or difluoroethyl; another aspect is monofluoromethyl; another aspect is difluoromethyl; another aspect is difluoroethyl; and another aspect is 2,2-difluoroethyl.

An aspect of the "C₁₋₃ alkyl optionally substituted with OH" is methyl optionally substituted with one OH or ethyl optionally substituted with one or two OH. Examples thereof include methyl, ethyl, hydroxymethyl, 1-hydroxyethyl, 2-hydroxyethyl and 1,2-dihydroxyethyl. An aspect thereof is methyl, ethyl or hydroxymethyl; another aspect is methyl or hydroxymethyl; another aspect is hydroxymethyl or hydroxyethyl; another aspect is hydroxymethyl; and another aspect is hydroxyethyl.

An aspect of the "C₁₋₃ alkyl optionally substituted with OCH₃" is methyl optionally substituted with one OCH₃, ethyl optionally substituted with one or two OCH₃ or propyl optionally substituted with one to three OCH₃. Examples thereof include methyl, ethyl, methoxymethyl, 1-methoxyethyl, 2-methoxyethyl, 1,2-dimethoxyethyl and 2-methoxypropyl. An aspect thereof is methoxymethyl or methoxyethyl; another aspect is methoxymethyl; another aspect is methoxyethyl; and another aspect is 2-methoxypropyl.

An aspect of the "phenylene optionally substituted with F" is phenylene optionally substituted with one or two F. An aspect thereof is phenylene optionally substituted with one F; another aspect is phenylene or fluorophenylene; another aspect is phenylene; another aspect is 2-fluoro-1,4-phenylene; and another aspect is 3-fluoro-1,4-phenylene.

The "mutant KRAS" is KRAS having a mutation, and examples thereof include G12V mutant KRAS, G12D mutant KRAS and G12C mutant KRAS.

The "G12V mutation" represents a mutation in which the amino acid residue corresponding to the codon 12 in a wild type protein is converted from glycine to valine.

The "G12V mutant KRAS" represents KRAS having the "G12V mutation".

The "G12D mutation" represents a mutation in which the amino acid residue corresponding to the codon 12 in a wild type protein is converted from glycine to aspartic acid.

The "G12D mutant KRAS" represents KRAS having the "G12D mutation".

The "G12C mutation" represents a mutation in which the amino acid residue corresponding to the codon 12 in a wild type protein is converted from glycine to cysteine.

The "G12C mutant KRAS" represents KRAS having the "G12C mutation".

The "pancreatic cancer" is a malignant tumor occurring in the pancreas. Examples thereof include pancreatic ductal carcinoma and pancreatic ductal adenocarcinoma. An aspect thereof is pancreatic ductal carcinoma, and another aspect is pancreatic ductal adenocarcinoma. Another aspect thereof is metastatic pancreatic cancer; another aspect is locally advanced pancreatic cancer; another aspect is recurrent or refractory pancreatic cancer; and another aspect is pancreatic cancer of an untreated patient and/or a patient with history of treatment.

The "mutant KRAS-positive pancreatic cancer" is pancreatic cancer that is positive for mutant KRAS, in particular, pancreatic cancer that is positive for G12V mutant, G12D mutant and G12C mutant KRAS. Examples thereof include pancreatic cancer having KRAS G12V mutation, G12D mutation and G12C mutation and pancreatic cancer which has a high positive rate for G12V mutant, G12D mutant and G12C mutant KRAS. An aspect thereof is mutant KRAS-positive pancreatic ductal carcinoma, in particular, G12V mutant, G12D mutant and G12C mutant KRAS-positive pancreatic ductal carcinoma, and another aspect is G12D mutant KRAS-positive pancreatic ductal adenocarcinoma, in particular, G12V mutant, G12D mutant and G12C mutant KRAS-positive pancreatic ductal adenocarcinoma.

The "G12V mutant and G12D mutant KRAS-positive pancreatic cancer" is pancreatic cancer that is positive for G12V mutant and G12D mutant KRAS. Examples thereof include pancreatic cancer having KRAS G12V mutation and G12D mutation and pancreatic cancer which has a high positive rate for G12V mutant and G12D mutant KRAS. An aspect thereof is G12V mutant and G12D mutant KRAS-positive pancreatic ductal carcinoma, and another aspect is G12V mutant and G12D mutant KRAS-positive pancreatic ductal adenocarcinoma.

The "G12V mutant KRAS-positive pancreatic cancer" is pancreatic cancer that is positive for G12V mutant KRAS. Examples thereof include pancreatic cancer having KRAS G12V mutation and pancreatic cancer which has a high positive rate for G12V mutant KRAS. An aspect thereof is G12V mutant KRAS-positive pancreatic ductal carcinoma, and another aspect is G12V mutant KRAS-positive pancreatic ductal adenocarcinoma.

The "G12D mutant KRAS-positive pancreatic cancer" is pancreatic cancer that is positive for G12D mutant KRAS. Examples thereof include pancreatic cancer having KRAS G12D mutation and pancreatic cancer which has a high positive rate for G12D mutant KRAS. An aspect thereof is G12D mutant KRAS-positive pancreatic ductal carcinoma, and another aspect is G12D mutant KRAS-positive pancreatic ductal adenocarcinoma.

The "G12C mutant KRAS-positive pancreatic cancer" is pancreatic cancer that is positive for G12C mutant KRAS. Examples thereof include pancreatic cancer having KRAS G12C mutation and pancreatic cancer which has a high positive rate for G12C mutant KRAS. An aspect thereof is G12C mutant KRAS-positive pancreatic ductal carcinoma, and another aspect is G12C mutant KRAS-positive pancreatic ductal adenocarcinoma.

Aspects of the compound of the formula (I) or a salt thereof in the present invention are shown below.
(1-1) The compound or a salt thereof in which A is CR^{A} or N, where R^{A} is H or C₁₋₃ alkyl.
(1-2) The compound or a salt thereof in which A is CR^{A} or N, where R^{A} is H.
(1-3) The compound or a salt thereof in which A is N.
(1-4) The compound or a salt thereof in which A is CH.
(2-1) The compound or a salt thereof in which X¹ is -CH₂- or -O-.
(2-2) The compound or a salt thereof in which X¹ is -O-.
(3-1) The compound or a salt thereof in which R¹ is naphthyl optionally substituted with OH or the formula (II) below, 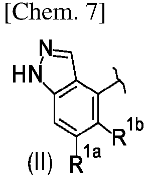
   where R^{1a} is H, methyl, F or Cl, and
   R^{1b} is F, Cl, methyl or ethyl.
(3-2) The compound or a salt thereof in which R¹ is the formula (II),
   where R^{1a} is H, methyl, F or Cl, and
   R^{1b} is F, Cl, methyl or ethyl.
(3-3) The compound or a salt thereof in which R¹ is the formula (II),
   where R^{1a} is H, methyl, F or Cl, and
   R^{1b} is methyl.
(3-4) The compound or a salt thereof in which R¹ is the formula (II-a) below. 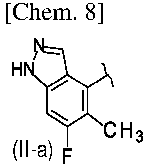
(4-1) The compound or a salt thereof in which R² is H, halogen, C₁₋₃ alkyl optionally substituted with a group selected from the group consisting of OH and OCH₃, cyclopropyl or vinyl.
(4-2) The compound or a salt thereof in which R² is halogen, C₁₋₃ alkyl optionally substituted with a group selected from the group consisting of OH and OCH₃, cyclopropyl or vinyl.
(4-3) The compound or a salt thereof in which R² is halogen, C₁₋₃ alkyl, cyclopropyl or vinyl.
(4-4) The compound or a salt thereof in which R² is cyclopropyl.
(5-1-1) The compound or a salt thereof in which R³ is a group selected from the group consisting of the formula (III), the formula (IV), the formula (V), the formula (VI), the formula (VII), the formula (VIII), the formula (IX), the formula (X), the formula (XI) and the formula (XXXV) below. 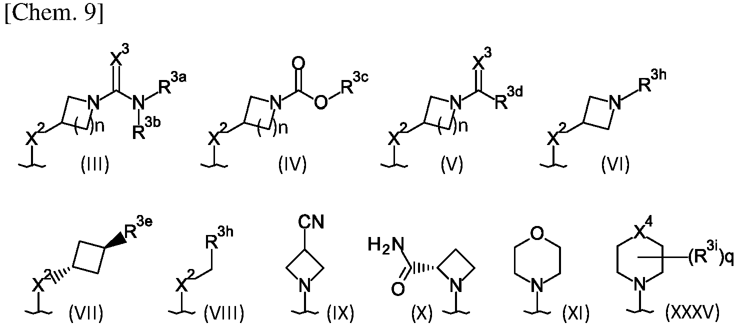
(5-1-2) The compound or a salt thereof in which R³ is a group selected from the group consisting of the formula (III), the formula (IV), the formula (V), the formula (VI), the formula (VII), the formula (VIII), the formula (IX), the formula (X) and the formula (XI) below. 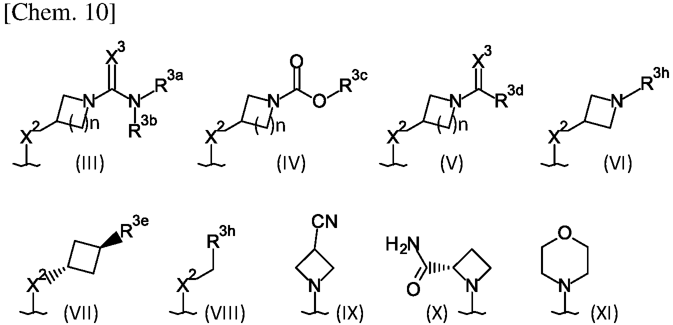
(5-1-3) The compound or a salt thereof in which R³ is a group selected from the group consisting of the formula (III), the formula (IV), the formula (V), the formula (VIII-a) and the formula (X) below. 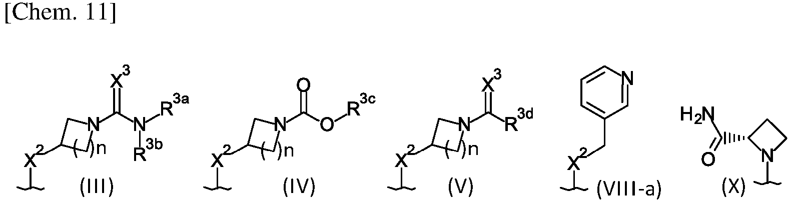
(5-1-4) The compound or a salt thereof in which R³ is a group selected from the group consisting of the formula (III-a), the formula (IV-a), the formula (V-a), the formula (VIII-a) and the formula (X) below. 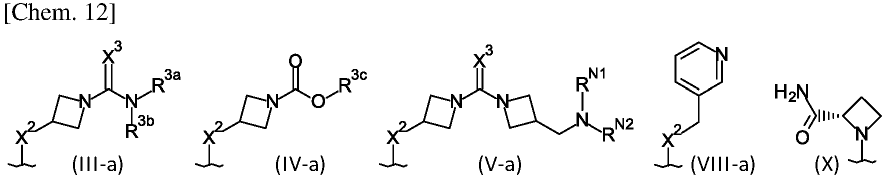
(5-1-5) The compound or a salt thereof in which R³ is a group selected from the group consisting of the formula (III-a), the formula (IV-a) and the formula (V-a) below. 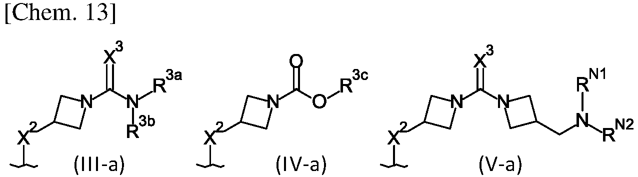
(5-2-1) The compound or a salt thereof in which R^{3a} is -(CH₂)ₚCHR^{3f}-NR^{N1}R^{N2}; -(CH₂)ₚCHR^{3f}-OR^{3g}; a 5-membered or 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2}.
(5-2-2) The compound or a salt thereof in which R^{3a} is -(CH₂)ₚCHR^{3f}-NR^{N1}R^{N2}; -(CH₂)ₚCHR^{3f}-OR^{3g}; pyrrolidinyl optionally substituted with C₁₋₃ alkyl; piperidinyl optionally substituted with C₁₋₃ alkyl; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2}.
(5-2-3) The compound or a salt thereof in which R^{3a} is -(CH₂)ₚCHR^{3f}-NR^{N1}R^{N2} or -(CH₂)ₚCHR^{3f}-OR^{3g}.
(5-2-4) The compound or a salt thereof in which R^{3a} is -(CH₂)ₚCHR^{3f}-NR^{N1}R^{N2},
(5-3-1) The compound or a salt thereof in which R^{3b} is H or C₁₋₃ alkyl.
(5-3-2) The compound or a salt thereof in which R^{3b} is H or methyl.
(5-3-3) The compound or a salt thereof in which R^{3b} is H.
(5-4-1) The compound or a salt thereof in which R^{3c} and R^{3d} are -(CH₂)ₚCHR^{3f}-NR^{N1}R^{N2}; -(CH₂)ₚCHR^{3f}-OR^{3g}; a 4-membered to 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2}. with the proviso that X² in the formula (IV) is -O-, -NH- or -N(C₂₋₃ alkyl)- when R^{3c} is -(CH₂)ₚCHR^{3f}-NR^{N1}R^{N2}.
(5-4-2) The compound or a salt thereof in which R^{3c} is -(CH₂)ₚCHR^{3f}-NR^{N1}R^{N2}; -(CH₂)ₚCHR^{3f}-OR^{3g}; pyrrolidinyl optionally substituted with C₁₋₃ alkyl; piperidinyl optionally substituted with C₁₋₃ alkyl; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2}. with the proviso that X² in the formula (IV-a) is -O- or -NH- when R^{3c} is -(CH₂)ₚCHR^{3f}-NR^{N1}R^{N2}.
(5-4-3) The compound or a salt thereof in which R^{3c} is -(CH₂)ₚCHR^{3f}-NR^{N1}R^{N2}; pyrrolidinyl optionally substituted with C₁₋₃ alkyl; or C₃₋₆cycloalkyl optionally substituted with -NR^{N1}R^{N2}. and X² in the formula (IV-a) is -O- or -NH-.
(5-4-4) The compound or a salt thereof in which R^{3c} is C₃₋₆ cycloalkyl optionally substituted with -NR^{N1}R^{N2}.
(5-4-5) The compound or a salt thereof in which R^{3d} is -(CH₂)ₚCHR^{3f}-NR^{N1}R^{N2} or a 4-membered to 6-membered saturated heterocyclic group which is optionally substituted with a group selected from the group consisting of C₁₋₃ alkyl, C₁₋₃ alkylene-NR^{N1}R^{N2} and -NR^{N1}R^{N2} and which contains one or two hetero atoms selected from the group consisting of oxygen, sulfur and nitrogen as ring-constituting atoms.
(5-5-1) The compound or a salt thereof in which R^{3e} is -O-C₂₋₃ alkylene-NR^{N1}R^{N2},
(5-6-1) The compound or a salt thereof in which R^{3f} is H, F or C₁₋₃ alkyl.
(5-6-2) The compound or a salt thereof in which R^{3f} is H or C₁₋₃ alkyl.
(5-6-3) The compound or a salt thereof in which R^{3f} is H.
(5-7-1) The compound or a salt thereof in which R^{3g} is H or C₁₋₃ alkyl.
(5-7-2) The compound or a salt thereof in which R^{3g} is H.
(5-8-1) The compound or a salt thereof in which R^{3h} is optionally substituted 5-membered heteroaryl containing one to four hetero atoms selected from the group consisting of oxygen, sulfur and nitrogen or optionally substituted 6-membered heteroaryl containing one to three nitrogen atoms.
(5-8-2) The compound or a salt thereof in which R^{3h} is optionally substituted 6-membered heteroaryl containing one to three nitrogen atoms.
(5-9-1) The compound or a salt thereof in which R³ⁱ, which are the same as or different from each other, are groups selected from the group consisting of H, OH, optionally substituted C₁₋₃ alkyl, -O-optionally substituted C₁₋₃ alkyl, -NH-optionally substituted C₁₋₃ alkyl, -N-(optionally substituted C₁₋₃ alkyl)₂, halogen, -CN and oxo, or
   two R³ⁱ on a same carbon atom, together with the neighboring carbon atom, may form a ring selected from the group consisting of C₃₋₆ cycloalkane and a 4-membered to 6-membered saturated hetero ring containing one or two hetero atoms selected from the group consisting of oxygen, sulfur and nitrogen as ring-constituting atoms to form a spiro ring as the group in the formula (XXXV), where the spiro ring is optionally substituted with one or two groups selected from the group consisting of C₁₋₃ alkyl, -O-(C₁₋₃ alkyl), OH, halogen and oxo, or
   R³ⁱ on two neighboring carbon atoms, together with the two carbon atoms, may form a ring selected from the group consisting of C₃₋₆ cycloalkane and a 4-membered to 6-membered saturated hetero ring containing one or two hetero atoms selected from the group consisting of oxygen, sulfur and nitrogen as ring-constituting atoms to form a condensed ring as the group in the formula (XXXV), where the condensed ring is optionally substituted with one or two groups selected from the group consisting of C₁₋₃ alkyl, -O-(C₁₋₃ alkyl), OH, halogen and oxo, or
   R³ⁱ on two carbon atoms which are not neighboring, together with the two carbon atoms, may form a cross-linked structure composed of one or two carbon atoms, where the group in the formula (XXXV), which is a ring having the cross-linked structure, is optionally substituted with one or two groups selected from the group consisting of C₁₋₃ alkyl, -O-(C₁₋₃ alkyl), OH, halogen and oxo.
(5-10-1) The compound or a salt thereof in which R^{N1} and R^{N2}, which are the same as or different from each other, are H or C₁₋₃ alkyl, or
   R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms, or
   R^{3f} and R^{N1}, together with the carbon atom and the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms.
(5-10-2) The compound or a salt thereof in which R^{N1} and R^{N2}, which are the same as or different from each other, are H or C₁₋₃ alkyl, or
   R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms.
(5-10-3) The compound or a salt thereof in which R^{N1} and R^{N2}, which are the same as or different from each other, are both C₁₋₃ alkyl.
(5-10-4) The compound or a salt thereof in which R^{N1} is C₁₋₃ alkyl, and R^{N2} is H.
(5-11-1) The compound or a salt thereof in which X² is -O-, -NH- or -N(C₁₋₃ alkyl)-.
(5-11-2) The compound or a salt thereof in which X² is -O- or -NH- or -N(CH₃)-.
(5-11-3) The compound or a salt thereof in which X² is -O- or -NH-.
(5-11-4) The compound or a salt thereof in which X² is -NH-.
(5-11-5) The compound or a salt thereof in which X² is -O-.
(5-12-1) The compound or a salt thereof in which X³ is O or S.
(5-12-2) The compound or a salt thereof in which X³ is O.
(5-13-1) The compound or a salt thereof in which X⁴ is -CH₂-, -CH₂-CH₂- or -O-CH₂-.
(5-14-1) The compound or a salt thereof in which n is 1 or 2, and p is 1 or 2.
(5-14-2) The compound or a salt thereof in which p is 1 or 2.
(5-14-3) The compound or a salt thereof in which p is 1.
(5-14-4) The compound or a salt thereof in which n is 1 or 2.
(5-14-5) The compound or a salt thereof in which n is 1.
(5-15-1) The compound or a salt thereof in which q is an integer of 1 to 8.
(6-1) The compound or a salt thereof in which R⁴ is C₁₋₆ alkyl optionally substituted with a group selected from the group consisting of F, OH, OCH₃, R^{4a}, cyclopropyl, N(R^{4a})₂, pyrrolidinyl optionally substituted with R^{4a} and tetrahydrofuranyl optionally substituted with R^{4a}; piperidinyl optionally substituted with R^{4b}; or tetrahydropyranyl optionally substituted with R^{4a},
   where R^{4a} is C₁₋₃ alkyl optionally substituted with F, and
   R^{4b} is C₁₋₃ alkyl substituted with one to three F.
(6-2) The compound or a salt thereof in which R⁴ is C₁₋₆ alkyl optionally substituted with a group selected from the group consisting of F, OH, OCH₃, R^{4a}, cyclopropyl, N(R^{4a})₂, pyrrolidinyl optionally substituted with R^{4a} and tetrahydrofuranyl; piperidinyl optionally substituted with R^{4b}; or tetrahydropyranyl,
   where R^{4a} is C₁₋₃ alkyl, and
   R^{4b} is C₁₋₃ alkyl substituted with one to three F.
(6-3) The compound or a salt thereof in which R⁴ is C₁₋₆ alkyl optionally substituted with a group selected from the group consisting of OCH₃, R^{4a}, N(R^{4a})₂, pyrrolidinyl optionally substituted with R^{4a} and tetrahydrofuranyl; piperidinyl optionally substituted with R^{4b}; or tetrahydropyranyl,
   where R^{4a} is C₁₋₃ alkyl, and
   R^{4b} is C₁₋₃ alkyl substituted with one to three F.
(6-4) The compound or a salt thereof in which R⁴ is C₁₋₆ alkyl optionally substituted with a group selected from the group consisting of OCH₃ and tetrahydrofuranyl; or tetrahydropyranyl.
(6-5) The compound or a salt thereof in which R⁴ is C₁₋₆ alkyl optionally substituted with OCH₃ or tetrahydropyranyl.
(6-6) The compound or a salt thereof in which R⁴ is C₁₋₆ alkyl optionally substituted with OCH₃.
(7-1) The compound or a salt thereof in which R⁵ is methyl, ethyl, isopropyl, isobutyl, sec-butyl, tert-butyl, C₃₋₆ cycloalkylmethyl or C₃₋₆ cycloalkyl.
(7-2) The compound or a salt thereof in which R⁵ is ethyl, isopropyl, isobutyl, sec-butyl, tert-butyl or C₃₋₆ cycloalkyl.
(7-3) The compound or a salt thereof in which R⁵ is isopropyl or sec-butyl.
(7-4) The compound or a salt thereof in which R⁵ is isopropyl.
(8-1) The compound or a salt thereof in which
   R^{6a} and R^{6b}, which are the same as or different from each other, are H or C₁₋₆ alkyl optionally substituted with a group selected from the group consisting of F, OH, OCH₃ and N(CH₃)₂, or
   R^{6a} and R^{6b}, together with the carbon to which they are attached, may form optionally substituted C₃₋₆ cycloalkane or an optionally substituted 4-membered to 6-membered saturated hetero ring containing one or two hetero atoms selected from the group consisting of oxygen, sulfur and nitrogen as ring-constituting atoms.
(8-2) The compound or a salt thereof in which
   R^{6a} and R^{6b}, which are the same as or different from each other, are H or C₁₋₆ alkyl optionally substituted with a group selected from the group consisting of F, OH, OCH₃ and N(CH₃)₂, or
   R^{6a} and R^{6b}, together with the carbon to which they are attached, may form optionally substituted C₃₋₆ cycloalkane.
(8-3) The compound or a salt thereof in which R^{6a} and R^{6b}, which are the same as or different from each other, are H or C₁₋₆ alkyl optionally substituted with a group selected from the group consisting of OH and N(CH₃)₂.
(8-4) The compound or a salt thereof in which
   R^{6a} is H, and R^{6b} is C₁₋₆ alkyl optionally substituted with OH.
(8-5) The compound or a salt thereof in which R^{6a} is H, and R^{6b} is hydroxymethyl.
(9-1) The compound or a salt thereof in which R⁷ is an optionally substituted 4-membered to 6-membered saturated heterocyclic group containing one or two hetero atoms selected from the group consisting of oxygen, sulfur and nitrogen as ring-constituting atoms, optionally substituted 5-membered heteroaryl containing one to four hetero atoms selected from the group consisting of oxygen, sulfur and nitrogen or 6-membered heteroaryl containing one to three nitrogen atoms.
(9-2) The compound or a salt thereof in which R⁷ is a group selected from the group consisting of the formula (XXIII), the formula (XXIV), the formula (XXV), the formula (XXVI), the formula (XXVII), the formula (XXVIII), the formula (XXIX), the formula (XXX), the formula (XXXI), the formula (XXXII) and the formula (XXXIII) below, 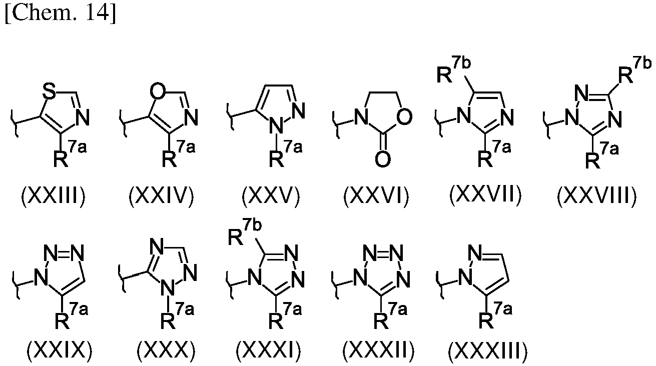 where R^{7a} and R^{7b}, which are the same as or different from each other, are H or C₁₋₃ alkyl optionally substituted with OH.
(9-3) The compound or a salt thereof in which R⁷ is a group selected from the group consisting of the formula (XXIII), the formula (XXIV), the formula (XXVI), the formula (XXVIII) and the formula (XXXIII) below, 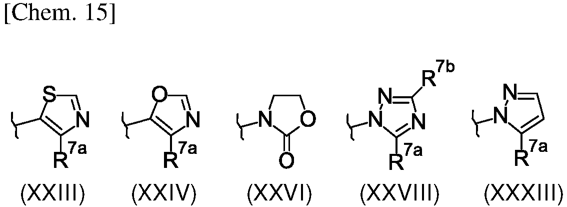 where R^{7a} and R^{7b}, which are the same as or different from each other, are H or C₁₋₃ alkyl optionally substituted with OH.
(9-4) The compound or a salt thereof in which R⁷ is a group selected from the group consisting of the formula (XXIII), the formula (XXIV), the formula (XXVI), the formula (XXVIII) and the formula (XXXIII),
   where R^{7a} and R^{7b}, which are the same as or different from each other, are H or C₁₋₃ alkyl.
(9-5) The compound or a salt thereof in which R⁷ is a group selected from the group consisting of the formula (XXIII), the formula (XXIV) and the formula (XXVIII) below, 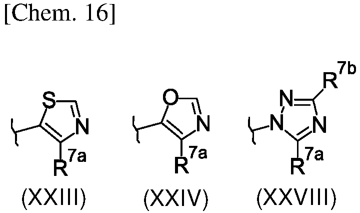 where R^{7a} and R^{7b}, which are the same as or different from each other, are H or C₁₋₃ alkyl.
(9-6) The compound or a salt thereof in which R⁷ is a group selected from the group consisting of the formula (XXIII-a), the formula (XXIV-a) and the formula (XXVIII-a) below. 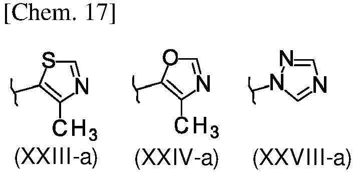
(9-7) The compound or a salt thereof in which R⁷ is the formula (XXIII-a) below. 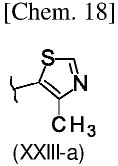
(9-8) The compound or a salt thereof in which R⁷ is the formula (XXVIII-a) below. 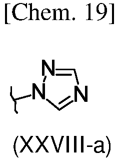
(9-9) The compound or a salt thereof in which R⁷ is the formula (XXIV-a) below. 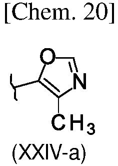
(10-1) The compound or a salt thereof in which W is optionally substituted phenylene or optionally substituted 6-membered heteroarenediyl containing one to three nitrogen atoms as ring-constituting atoms.
(10-2) The compound or a salt thereof in which W is the formula (XXXIV) below, 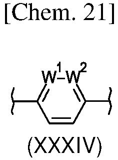
   where W¹ is CH, CF, CCl or CCH₃, and
   W² is CH, CF, CCl, CCH₃ or N.
(10-3) The compound or a salt thereof in which W is the formula (XXXIV),
   where W¹ is CH, and
   W² is CH or N.
(10-4) The compound or a salt thereof in which W is the formula (XXXIV),
   where W¹ and W² are both CH.
(11-1) The compound or a salt thereof in which Y is phenylene optionally substituted with F or Cl or pyridinediyl.
(11-2) The compound or a salt thereof in which Y is phenylene optionally substituted with F or Cl.
(11-3) The compound or a salt thereof in which Y is phenylene.
(12-1) The compound or a salt thereof in which L is -(L¹-L²-L³-L⁴)-,
   where L¹, L², L³ and L⁴, which are the same as or different from each other, are groups selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O,
   where R^{L1} is H or C₁₋₃ alkyl.
(12-2) The compound or a salt thereof in which L is a bond, C₁₋₃ alkylene, C=O or a group selected from the group consisting of the formula (XIII), the formula (XIV), the formula (XV), the formula (XVI), the formula (XVII) and the formula (XVIII) below, 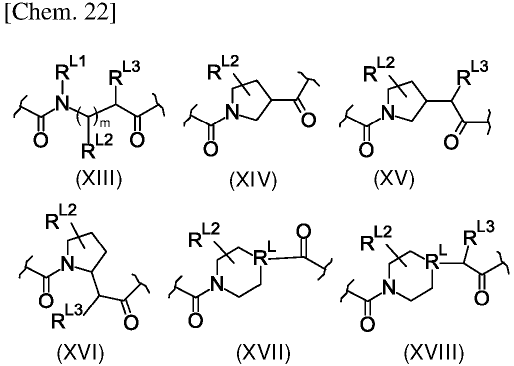
   where R^{L1} is H or C₁₋₃ alkyl,
   R^{L2} and R^{L3}, which are the same as or different from each other, are H, F, OH, OCH₃ or optionally substituted C₁₋₃ alkyl,
   R^{L} is CH or N, and
   m is 1 or 2.
(12-3) The compound or a salt thereof in which L is a bond, C=O or a group selected from the group consisting of the formula (XIII) and the formula (XIV) below, 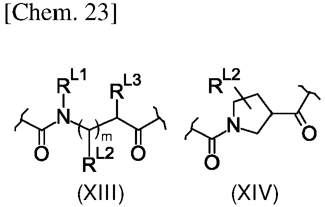
   where R^{L1} is H or C₁₋₃ alkyl,
   R^{L2} and R^{L3}, which are the same as or different from each other, are H, F, OH, OCH₃ or optionally substituted C₁₋₃ alkyl, and
   m is 1 or 2.
(12-4) The compound or a salt thereof in which L is a bond, C=O or a group selected from the group consisting of the formula (XIII) and the formula (XIV),
   R^{L1} is C₁₋₃ alkyl,
   R^{L2} and R^{L3} are both H, and
   m is 1.
(12-5) The compound or a salt thereof in which L is a bond or C=O.
(12-6) The compound or a salt thereof in which L is a bond.
(12-7) The compound or a salt thereof in which L is C=O.
(13-1) The compound or a salt thereof in which Z is NH or 5-membered heteroarenediyl containing one to four hetero atoms selected from the group consisting of oxygen, sulfur and nitrogen as ring-constituting atoms.
(13-2) The compound or a salt thereof in which Z is NH or a group selected from the group consisting of the formula (XIX), the formula (XX), the formula (XXI) and the formula (XXII) below. 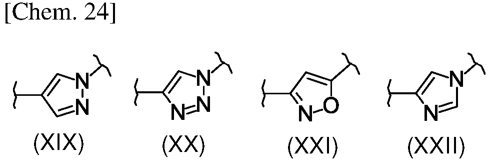
(13-3) The compound or a salt thereof in which Z is NH or a group selected from the group consisting of the formula (XIX-a), the formula (XX-a), the formula (XXI-a) and the formula (XXII-a) below. 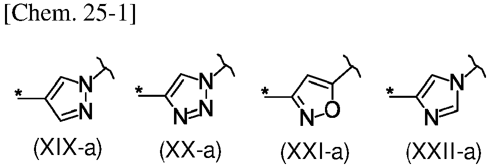 (In the formulae, * represents bond to L.)
(13-4) The compound or a salt thereof in which Z is a group selected from the group consisting of the formula (XIX), the formula (XX), the formula (XXI) and the formula (XXII).
(13-5) The compound or a salt thereof in which Z is a group selected from the group consisting of the formula (XIX-a), the formula (XX-a), the formula (XXI-a) and the formula (XXII-a). 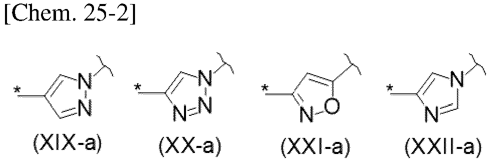 (In the formulae, * represents bond to L.)
(13-6) The compound or a salt thereof in which Z is the formula (XX) below. 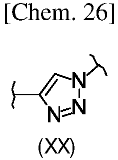
(13-7) The compound or a salt thereof in which Z is the formula (XX-a) below. 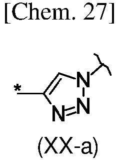 (In the formula, * represents bond to L.)
(13-8) The compound or a salt thereof in which Z is NH.
(14-1) The compound or a salt thereof in which Y-L-Z is the formula (XII) below. 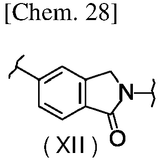
(14-2) The compound or a salt thereof in which Y-L-Z is the formula (XII-a) below. 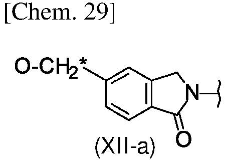 (In the formula, O-CH₂* represents bond to the carbon atom of O-CH₂ which is attached to Y-L-Z.)
(15) The compound or a salt thereof which is a combination of any compatible two or more of the aspects described in (1-1) to (14-2) above.
Specific examples of the combination described in (15) above include the aspects below.
(16-1) The compound of the formula (I) or a salt thereof. 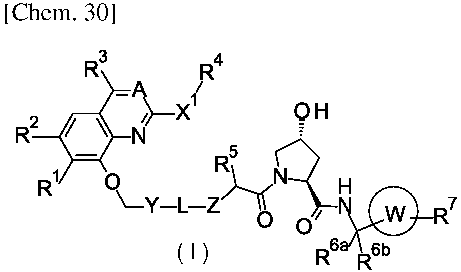 (In the formula,
   A is CR^{A} or N,
   where R^{A} is H or C₁₋₃ alkyl,
   X¹ is -CH₂- or -O-,
   R¹ is naphthyl optionally substituted with OH or the formula (II) below, 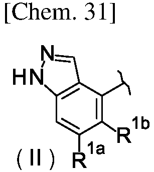
      where R^{1a} is H, methyl, F or Cl, and
      R^{1b} is F, Cl, methyl or ethyl,
      R² is H, halogen, C₁₋₃ alkyl optionally substituted with a group selected from the group consisting of OH and OCH₃, cyclopropyl or vinyl,
      R³ is a group selected from the group consisting of the formula (III), the formula (IV), the formula (V), the formula (VI), the formula (VII), the formula (VIII), the formula (IX), the formula (X), the formula (XI) and the formula (XXXV) below, 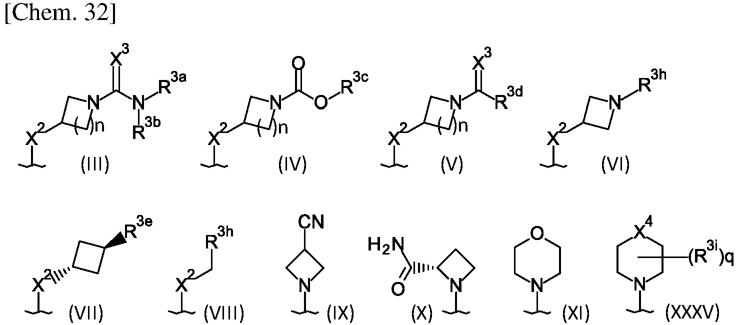
      where R^{3a} is -(CH₂)ₚCHR^{3f}-NR^{N1}R^{N2}; -(CH₂)ₚCHR^{3f}-OR^{3g}; a 5-membered or 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
      R^{3b} is H or C₁₋₃ alkyl,
      R^{3c} and R^{3d} are -(CH₂)ₚCHR^{3f}-NR^{N1}R^{N2}; -(CH₂)ₚCHR^{3f}-OR^{3g}; a 4-membered to 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
      R^{3e} is -O-C₂₋₃ alkylene-NR^{N1}R^{N2},
      R^{3f} is H, F or C₁₋₃ alkyl,
      R^{3g} is H or C₁₋₃ alkyl,
      R^{3h} is optionally substituted 5-membered heteroaryl containing one to four hetero atoms selected from the group consisting of oxygen, sulfur and nitrogen or optionally substituted 6-membered heteroaryl containing one to three nitrogen atoms,
      R³ⁱ, which are the same as or different from each other, are groups selected from the group consisting of H, OH, optionally substituted C₁₋₃ alkyl, -O-optionally substituted C₁₋₃ alkyl, -NH-optionally substituted C₁₋₃ alkyl, -N-(optionally substituted C₁₋₃ alkyl)₂, halogen, -CN and oxo, or
      two R³ⁱ on a same carbon atom, together with the neighboring carbon atom, may form a ring selected from the group consisting of C₃₋₆ cycloalkane and a 4-membered to 6-membered saturated hetero ring containing one or two hetero atoms selected from the group consisting of oxygen, sulfur and nitrogen as ring-constituting atoms to form a spiro ring as the group in the formula (XXXV), where the spiro ring is optionally substituted with one or two groups selected from the group consisting of C₁₋₃ alkyl, -O-(C₁₋₃ alkyl), OH, halogen and oxo, or
      R³¹ on two neighboring carbon atoms, together with the two carbon atoms, may form a ring selected from the group consisting of C₃₋₆ cycloalkane and a 4-membered to 6-membered saturated hetero ring containing one or two hetero atoms selected from the group consisting of oxygen, sulfur and nitrogen as ring-constituting atoms to form a condensed ring as the group in the formula (XXXV), where the condensed ring is optionally substituted with one or two groups selected from the group consisting of C₁₋₃ alkyl, -O-(C₁₋₃ alkyl), OH, halogen and oxo, or
      R³ⁱ on two carbon atoms which are not neighboring, together with the two carbon atoms, may form a cross-linked structure composed of one or two carbon atoms, where the group in the formula (XXXV), which is a ring having the cross-linked structure, is optionally substituted with one or two groups selected from the group consisting of C₁₋₃ alkyl, -O-(C₁₋₃ alkyl), OH, halogen and oxo,
      R^{N1} and R^{N2}, which are the same as or different from each other, are H or C₁₋₃ alkyl, or
      R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms, or
      R^{3f} and R^{N1}, together with the carbon atom and the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms,
      X² is -O-, -NH- or -N(C₁₋₃ alkyl)-,
      X³ is O or S,
      X⁴ is -CH₂-, -CH₂-CH₂- or -O-CH₂-,
      n is 1 or 2, p is 1 or 2, and
      q is an integer of 1 to 8,
      with the proviso that X² in the formula (IV) is -O-, -NH- or -N(C₂₋₃ alkyl)- when R^{3c} is -(CH₂)ₚCHR^{3f}-NR^{N1}R^{N2},
      R⁴ is C₁₋₆ alkyl optionally substituted with a group selected from the group consisting of F, OH, OCH₃, R^{4a}, cyclopropyl, N(R^{4a})₂, pyrrolidinyl optionally substituted with R^{4a} and tetrahydrofuranyl optionally substituted with R^{4a}; piperidinyl optionally substituted with R^{4b}; or tetrahydropyranyl optionally substituted with R^{4a},
      where R^{4a} is C₁₋₃ alkyl optionally substituted with F, and
      R^{4b} is C₁₋₃ alkyl substituted with one to three F,
      R⁵ is methyl, ethyl, isopropyl, isobutyl, sec-butyl, tert-butyl, C₃₋₆ cycloalkylmethyl or C₃₋₆ cycloalkyl,
      R^{6a} and R^{6b}, which are the same as or different from each other, are H or C₁₋₆ alkyl optionally substituted with a group selected from the group consisting of F, OH, OCH₃ and N(CH₃)₂, or
      R^{6a} and R^{6b}, together with the carbon to which they are attached, may form optionally substituted C₃₋₆ cycloalkane or an optionally substituted 4-membered to 6-membered saturated hetero ring containing one or two hetero atoms selected from the group consisting of oxygen, sulfur and nitrogen as ring-constituting atoms,
      R⁷ is an optionally substituted 4-membered to 6-membered saturated heterocyclic group containing one or two hetero atoms selected from the group consisting of oxygen, sulfur and nitrogen as ring-constituting atoms, optionally substituted 5-membered heteroaryl containing one to four hetero atoms selected from the group consisting of oxygen, sulfur and nitrogen or 6-membered heteroaryl containing one to three nitrogen atoms,
      W is optionally substituted phenylene or optionally substituted 6-membered heteroarenediyl containing one to three nitrogen atoms as ring-constituting atoms,
      Y is phenylene optionally substituted with F or Cl or pyridinediyl,
      L is -(L¹-L²-L³-L⁴)-,
      where L¹, L², L³ and L⁴, which are the same as or different from each other, are groups selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O,
      where R^{L1} is H or C₁₋₃ alkyl, and
      Z is NH or 5-membered heteroarenediyl containing one to four hetero atoms selected from the group consisting of oxygen, sulfur and nitrogen as ring-constituting atoms,
      or Y-L-Z is the formula (XII) below.) 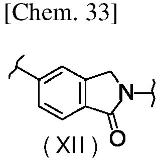
(16-2) The compound described in (16-1) above or a salt thereof in which R³ is a group selected from the group consisting of the formula (III), the formula (IV), the formula (V), the formula (VI), the formula (VII), the formula (VIII), the formula (IX), the formula (X) and the formula (XI) below, 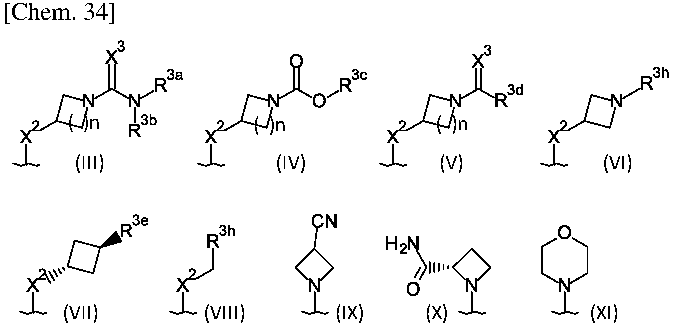
   where R^{3a} is -(CH₂)ₚCHR^{3f}-NR^{N1}R^{N2}; -(CH₂)ₚCHR^{3f}-OR^{3g}; a 5-membered or 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
   R^{3b} is H or C₁₋₃ alkyl,
   R^{3c} and R^{3d} are -(CH₂)ₚCHR^{3f}-NR^{N1}R^{N2}; -(CH₂)ₚCHR^{3f}-OR^{3g}; a 4-membered to 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
   R^{3e} is -O-C₂₋₃ alkylene-NR^{N1}R^{N2},
   R^{3f} is H, F or C₁₋₃ alkyl,
   R^{3g} is H or C₁₋₃ alkyl,
   R^{3h} is optionally substituted 5-membered heteroaryl containing one to four hetero atoms selected from the group consisting of oxygen, sulfur and nitrogen or optionally substituted 6-membered heteroaryl containing one to three nitrogen atoms,
   R^{N1} and R^{N2}, which are the same as or different from each other, are H or C₁₋₃ alkyl, or
   R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms, or
   R^{3f} and R^{N1}, together with the carbon atom and the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms,
   X² is -O-, -NH- or -N(C₁₋₃ alkyl)-,
   X³ is O or S,
   n is 1 or 2, and p is 1 or 2,
   with the proviso that X² in the formula (IV) is -O-, -NH- or -N(C₂₋₃ alkyl)- when R^{3c} is -(CH₂)ₚCHR^{3f}-NR^{N1}R^{N2},
(16-3) The compound described in (16-2) above or a salt thereof in which Y is phenylene optionally substituted with F or Cl or pyridinediyl,
   L is a bond, C₁₋₃ alkylene, C=O or a group selected from the group consisting of the formula (XIII), the formula (XIV), the formula (XV), the formula (XVI), the formula (XVII) and the formula (XVIII) below, 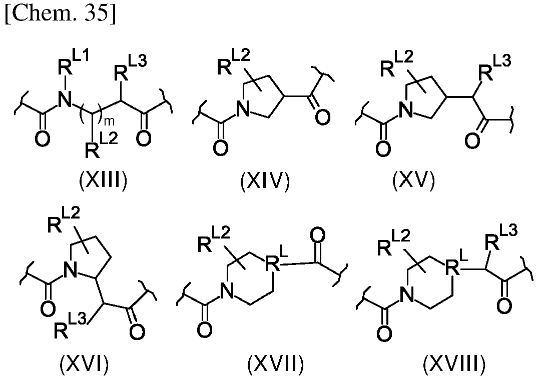
   where R^{L1} is H or C₁₋₃ alkyl,
   R^{L2} and R^{L3}, which are the same as or different from each other, are H, F, OH, OCH₃ or optionally substituted C₁₋₃ alkyl,
   R^{L} is CH or N, and
   m is 1 or 2, and
   Z is NH or a group selected from the group consisting of the formula (XIX), the formula (XX), the formula (XXI) and the formula (XXII) below, 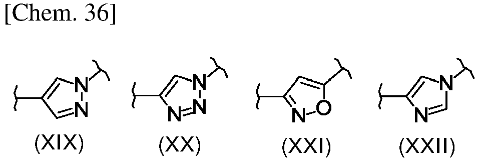
   or Y-L-Z is the formula (XII) below. 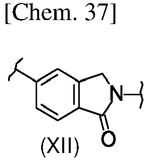
(16-4) The compound described in (16-3) above or a salt thereof in which A is CR^{A} or N,
   where R^{A} is H,
   X¹ is -O-,
   R^{6a} and R^{6b}, which are the same as or different from each other, are H or C₁₋₆ alkyl optionally substituted with a group selected from the group consisting of F, OH, OCH₃ and N(CH₃)₂, or
   R^{6a} and R^{6b}, together with the carbon to which they are attached, may form optionally substituted C₃₋₆ cycloalkane,
   R⁷ is a group selected from the group consisting of the formula (XXIII), the formula (XXIV), the formula (XXV), the formula (XXVI), the formula (XXVII), the formula (XXVIII), the formula (XXIX), the formula (XXX), the formula (XXXI), the formula (XXXII) and the formula (XXXIII) below, 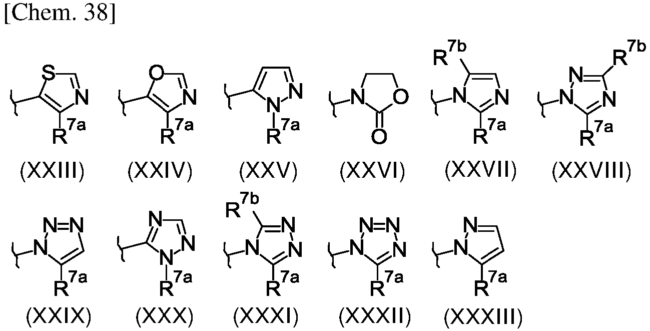
   where R^{7a} and R^{7b}, which are the same as or different from each other, are H or C₁₋₃ alkyl optionally substituted with OH, and
   W is the formula (XXXIV) below, 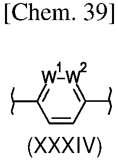
   where W¹ is CH, CF, CCl or CCH₃, and
   W² is CH, CF, CCl, CCH₃ or N.
(16-5) The compound described in (16-4) above or a salt thereof in which R¹ is the formula (II) below, 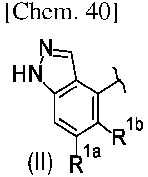
   where R^{1a} is H, methyl, F or Cl, and
   R^{1b} is F, Cl, methyl or ethyl,
   R² is halogen, C₁₋₃ alkyl optionally substituted with a group selected from the group consisting of OH and OCH₃, cyclopropyl or vinyl,
   R³ is a group selected from the group consisting of the formula (III), the formula (IV), the formula (V), the formula (VIII-a) and the formula (X) below, 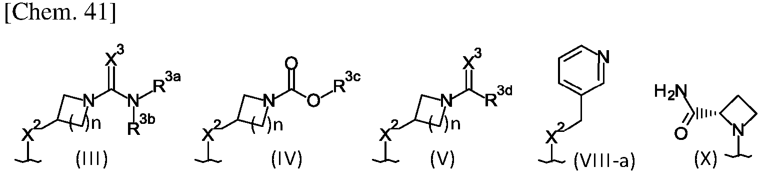
   where R^{3a} is -(CH₂)ₚCHR^{3f}-NR^{N1}R^{N2}; -(CH₂)ₚCHR^{3f}-OR^{3g}; a 5-membered or 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
   R^{3b} is H or methyl,
   R^{3c} and R^{3d} are -(CH₂)ₚCHR^{3f}-NR^{N1}R^{N2}; -(CH₂)ₚCHR^{3f}-OR^{3g}; a 4-membered to 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
   R^{3f} is H, F or C₁₋₃ alkyl,
   R^{3g} is H or C₁₋₃ alkyl,
   R^{N1} and R^{N2}, which are the same as or different from each other, are H or C₁₋₃ alkyl, or
   R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms, or
   R^{3f} and R^{N1}, together with the carbon atom and the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms,
   X² is -O-, -NH- or -N(C₁₋₃ alkyl)-,
   X³ is O or S,
   n is 1 or 2, and p is 1 or 2,
   with the proviso that X² in the formula (IV) is -O-, -NH- or -N(C₂₋₃ alkyl)- when R^{3c} is -(CH₂)ₚCHR^{3f}-NR^{N1}R^{N2},
   Y is phenylene optionally substituted with F or Cl or pyridinediyl,
   L is a bond, C=O or a group selected from the group consisting of the formula (XIII) and the formula (XIV) below, 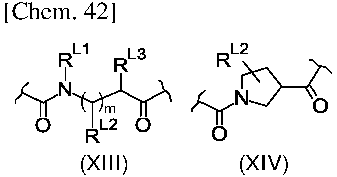
   where R^{L1} is H or C₁₋₃ alkyl,
   R^{L2} and R^{L3}, which are the same as or different from each other, are H, F, OH, OCH₃ or optionally substituted C₁₋₃ alkyl, and
   m is 1 or 2, and
   Z is NH or a group selected from the group consisting of the formula (XIX), the formula (XX), the formula (XXI) and the formula (XXII) below, 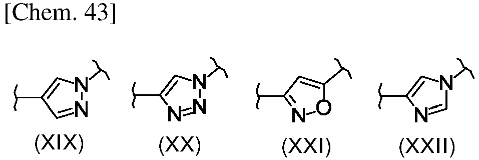
   or Y-L-Z is the formula (XII) below. 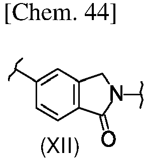
(16-6) The compound described in (16-5) above or a salt thereof in which R³ is a group selected from the group consisting of the formula (III-a), the formula (IV-a), the formula (V-a), the formula (VIII-a) and the formula (X) below, 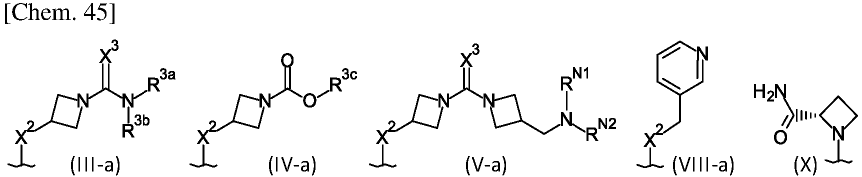
   where R^{3a} is -(CH₂)ₚCHR^{3f}-NR^{N1}R^{N2}; -(CH₂)ₚCHR^{3f}-OR^{3g}; pyrrolidinyl optionally substituted with C₁₋₃ alkyl; piperidinyl optionally substituted with C₁₋₃ alkyl; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
   R^{3b} is H or methyl,
   R^{3c} is -(CH₂)ₚCHR^{3f}-NR^{N1}R^{N2}; -(CH₂)ₚCHR^{3f}-OR^{3g}; pyrrolidinyl optionally substituted with C₁₋₃ alkyl; piperidinyl optionally substituted with C₁₋₃ alkyl; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
   R^{3f} is H, F or C₁₋₃ alkyl,
   R^{3g} is H or C₁₋₃ alkyl,
   R^{N1} and R^{N2}, which are the same as or different from each other, are H or C₁₋₃ alkyl, or
   R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms, or
   R^{3f} and R^{N1}, together with the carbon atom and the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms,
   X² is -O- or -NH- or -N(CH₃)-,
   X³ is O or S, and
   p is 1 or 2,
   with the proviso that X² in the formula (IV-a) is -O- or -NH- when R^{3c} is -(CH₂)ₚCHR^{3f}-NR^{N1}R^{N2},
   R⁴ is C₁₋₆ alkyl optionally substituted with a group selected from the group consisting of F, OH, OCH₃, R^{4a}, cyclopropyl, N(R^{4a})₂, pyrrolidinyl optionally substituted with R^{4a} and tetrahydrofuranyl; piperidinyl optionally substituted with R^{4b}; or tetrahydropyranyl,
   where R^{4a} is C₁₋₃ alkyl, and
   R^{4b} is C₁₋₃ alkyl substituted with one to three F,
   R⁷ is a group selected from the group consisting of the formula (XXIII), the formula (XXIV), the formula (XXVI), the formula (XXVIII) and the formula (XXXIII) below, 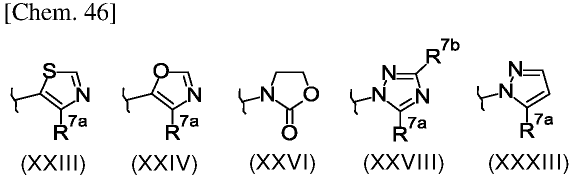
   where R^{7a} and R^{7b}, which are the same as or different from each other, are H or C₁₋₃ alkyl optionally substituted with OH,
   Y is phenylene optionally substituted with F or Cl, and
   Z is NH or a group selected from the group consisting of the formula (XIX), the formula (XX), the formula (XXI) and the formula (XXII) below. 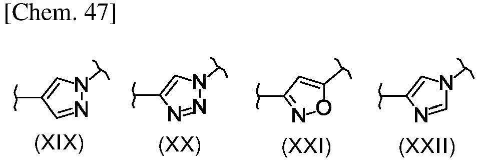
(16-7) The compound described in (16-6) above or a salt thereof in which R¹ is the formula (II-a) below, 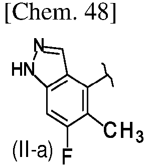
   R² is cyclopropyl,
   R³ is a group selected from the group consisting of the formula (III-a), the formula (IV-a), the formula (V-a), the formula (VIII-a) and the formula (X) below, 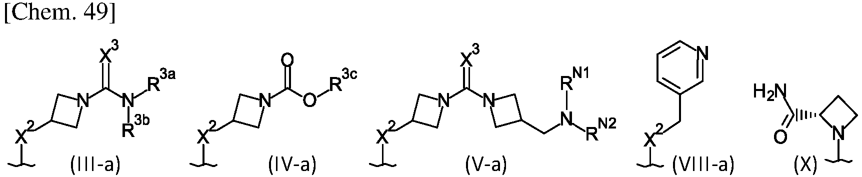
   where R^{3a} is -(CH₂)ₚCHR^{3f}-NR^{N1}R^{N2} or -(CH₂)ₚCHR^{3f}-OR^{3g},
   R³⁶ is H,
   R^{3c} is -(CH₂)ₚCHR^{3f}-NR^{N1}R^{N2}; pyrrolidinyl optionally substituted with C₁₋₃ alkyl; or C₃₋₆ cycloalkyl optionally substituted with -NR^{N1}R^{N2},
   R^{3f} is H,
   R^{3g} is H,
   R^{N1} and R^{N2}, which are the same as or different from each other, are H or C₁₋₃ alkyl, or
   R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms,
   X² is -O- or -NH-,
   X³ is O or S, and
   p is 1 or 2,
   R⁴ is C₁₋₆ alkyl optionally substituted with a group selected from the group consisting of OCH₃, R^{4a}, N(R^{4a})₂, pyrrolidinyl optionally substituted with R^{4a} and tetrahydrofuranyl; piperidinyl optionally substituted with R^{4b}; or tetrahydropyranyl,
   where R^{4a} is C₁₋₃ alkyl, and
   R^{4b} is C₁₋₃ alkyl substituted with one to three F,
   R⁵ is isopropyl or sec-butyl,
   R^{6a} and R^{6b}, which are the same as or different from each other, are H or C₁₋₆ alkyl optionally substituted with a group selected from the group consisting of OH and N(CH₃)₂,
   R⁷ is a group selected from the group consisting of the formula (XXIII), the formula (XXIV), the formula (XXVI), the formula (XXVIII) and the formula (XXXIII) below, 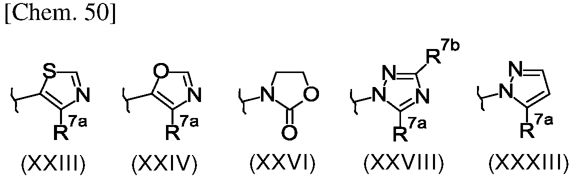
   where R^{7a} and R^{7b}, which are the same as or different from each other, are H or C₁₋₃ alkyl,
   W is the formula (XXXIV) below, 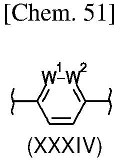
   where W¹ is CH, and
   W² is CH or N,
   Y is phenylene, and
   L is a bond or C=O.
(16-8) The compound described in (16-7) above or a salt thereof in which R³ is a group selected from the group consisting of the formula (III-a), the formula (IV-a) and the formula (V-a) below, 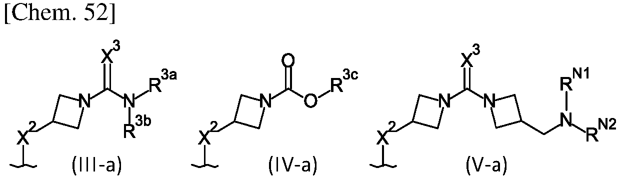
   where R^{3a} is -(CH₂)ₚCHR^{3f}-NR^{N1}R^{N2},
   R^{3b} is H,
   R^{3c} is C₃₋₆cycloalkyl optionally substituted with -NR^{N1}R^{N2},
   R^{3f} is H,
   R^{N1} and R^{N2}, which are the same as or different from each other, are both C₁₋₃ alkyl,
   X² is -O- or -NH-,
   X³ is O, and
   p is 1,
   R⁴ is C₁₋₆ alkyl optionally substituted with OCH₃,
   R⁵ is isopropyl,
   R^{6a} is H,
   R^{6b} is C₁₋₆ alkyl optionally substituted with OH,
   R⁷ is a group selected from the group consisting of the formula (XXIII), the formula (XXIV) and the formula (XXVIII) below, 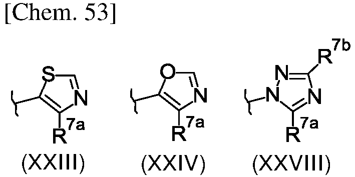
   where R^{7a} and R^{7b}, which are the same as or different from each other, are H or C₁₋₃ alkyl,
   W is the formula (XXXIV) below, 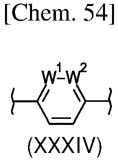
   where W¹ and W² are both CH,
   L is a bond, and
   Z is a group selected from the group consisting of the formula (XIX), the formula (XX), the formula (XXI) and the formula (XXII) below.

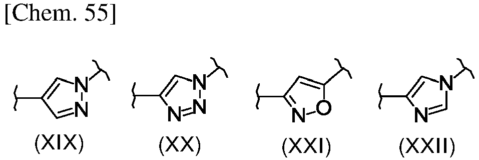

Examples of specific compounds included in the present invention as an aspect include the following compounds.

A compound selected from the group consisting of
(1s,3R)-3-(dimethylamino)cyclobutyl 3-({6-cyclopropyl-7-(6-fluoro-5-methyl-1H-indazol-4-yl)-8-{[4-(1-{(2S)-1-[(2S,4R)-4-hydro xy-2-({(1R)-2-hydroxy-1-[4-(4-methyl-l,3-oxazol-5-yl)phenyl]ethyl}carbamoyl)pyrrolidin-1 -yl]-3-methyl-1-oxobutan-2-yl}-1H-1,2,3-triazol-4-yl)phenyl]methoxy}-2-[(2S)-2-methoxypr opoxy]quinazolin-4-yl }amino)azetidine-1-carboxylate,
(1s,3R)-3-(dimethylamino)cyclobutyl 3-({6-cyclopropyl-7-(6-fluoro-5-methyl-1H-indazol-4-yl)-8-{[4-(1-{(2S)-1-[(2S,4R)-4-hydro xy-2-({(1R)-2-hydroxy-1-[4-(1H-1,2,4-triazol-1-yl)phenyl]ethyl}carbamoyl)pyrrolidin-1-yl]-3-methyl-1-oxobutan-2-yl}-1H-1,2,3-triazol-4-yl)phenyl]methoxy}-2-[(2S)-2-methoxypropo xy]quinazolin-4-yl}amino)azetidine-1-carboxylate,
(4R)-1-[(2S)-2-(4-{4-[({6-cyclopropyl-4-[(1-{3-[(dimethylamino)methyl]azetidine-1-carbonyl}azetidin-3-yl)oxy]-7-(6-fluoro-5-methyl-1H-indazol-4-yl)-2-[(2S)-2-methoxypro poxy]quinolin-8-yl}oxy)methyl]phenyl}-1H-1,2,3-triazol-1-yl)-3-methylbutanoyl]-4-hydrox y-N-{(1R)-2-hydroxy-1-[4-(1H-1,2,4-triazol-1-yl)phenyl]ethyl}-L-prolinamide,
(4R)-1-[(2S)-2-(4-{4-[({6-cyclopropyl-4-[(1-{3-[(dimethylamino)methyl]azetidine-1-carbonyl}azetidin-3-yl)oxy]-7-(6-fluoro-5-methyl-1H-indazol-4-yl)-2-[(2S)-2-methoxypro poxy]quinolin-8-yl}oxy)methyl]phenyl}-1H-1,2,3-triazol-1-yl)-3-methylbutanoyl]-4-hydrox y-N-{(1R)-2-hydroxy-1-[4-(4-methyl-1,3-oxazol-5-yl)phenyl]ethyl}-L-prolinamide,
(1s,3R)-3-(dimethylamino)cyclobutyl 3-({6-cyclopropyl-7-(6-fluoro-5-methyl-1H-indazol-4-yl)-8-{[4-(1-{(2S)-1-[(2S,4R)-4-hydro xy-2-({(1R)-2-hydroxy-1-[4-(4-methyl-1,3-thiazol-5-yl)phenyl]ethyl}carbamoyl)pyrrolidin-1 -yl]-3-methyl-1-oxobutan-2-yl}-1H-1,2,3-triazol-4-yl)phenyl]methoxy}-2-[(2S)-2-methoxypr opoxy]quinazolin-4-yl}amino)azetidine-1-carboxylate,
(4R)-1-[(2S)-2-(4-{4-[({6-cyclopropyl-4-[(1-{[2-(dimethylamino)ethyl]carbamoyl}a zetidin-3-yl)oxy]-7-(6-fluoro-5-methyl-1H-indazol-4-yl)-2-[(2S)-2-methoxypropoxy]quinolin -8-yl}oxy)methyl]phenyl}-1H-1,2,3-triazol-1-yl)-3-methylbutanoyl]-4-hydroxy-N-{(1R)-2-h ydroxy-1-[4-(1H-1,2,4-triazol-1-yl)phenyl]ethyl}-L-prolinamide,
(1s,3R)-3-(dimethylamino)cyclobutyl 3-({6-cyclopropyl-7-(6-fluoro-5-methyl-1H-indazol-4-yl)-8-{[4-(1-{(2S)-1-[(2S,4R)-4-hydro xy-2-({(1R)-2-hydroxy-1-[4-(1H-1,2,4-triazol-1-yl)phenyl]ethyl}carbamoyl)pyrrolidin-1-yl]-3-methyl-1-oxobutan-2-yl}-1H-,2,3-triazol-4-yl)phenyl]methoxy}-2-[(2S)-2-methoxypropo xy]quinolin-4-yl}oxy)azetidine-1-carboxylate and
(4R)-1-[(2S)-2-(4-{4-[({6-cyclopropyl-4-[(1-{3-[(dimethylamino)methyl]azetidine-1-carbonyl}azetidin-3-yl)oxy]-7-(6-fluoro-5-methyl-1H-indazol-4-yl)-2-[(2S)-2-methoxypro poxy]quinolin-8-yl}oxy)methyl]phenyl}-1H-1,2,3-triazol-1-yl)-3-methylbutanoyl]-4-hydrox y-N-{(1R)-2-hydroxy-1-[4-(4-methyl-1,3-thiazol-5-yl)phenyl]ethyl}-L-prolinamide,
or a salt thereof.

Examples of specific compounds included in the present invention as an aspect include the following compounds.

A compound selected from the group consisting of
(1s,3R)-3-(dimethylamino)cyclobutyl 3-({(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-oxazol-5-yl)phenyl]ethyl}carbamoyl)pyrrol idin-1-yl]-3-methyl-1-oxobutan-2-yl}-1H-1,2,3-triazol-4-yl)phenyl]methoxy}-2-[(2S)-2-met hoxypropoxy]quinazolin-4-yl}amino)azetidine-1-carboxylate,
(1s,3R)-3-(dimethylamino)cyclobutyl 3-({(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(1H-1,2,4-triazol-1-yl)phenyl]ethyl}carbamoyl)pyrrolidin-1-yl]-3-methyl-l-oxobutan-2-yl}-1H-1,2,3-triazol-4-yl)phenyl]methoxy}-2-[(2S)-2-methoxy propoxy]quinazolin-4-yl}amino)azetidine-1-carboxylate,
(4R)-1-[(2S)-2-(4-{4-[({(7M)-6-cyclopropyl-4-[(1-{3-[(dimethylamino)methyl]azeti dine-1-carbonyl}azetidin-3-yl)oxy]-7-(6-fluoro-5-methyl-1H-indazol-4-yl)-2-[(2S)-2-methox ypropoxy]quinolin-8-yl}oxy)methyl]phenyl}-1H-1,2,3-triazol-1-yl)-3-methylbutanoyl]-4-hy droxy-N-{(1R)-2-hydroxy-1-[4-(1H-1,2,4-triazol-1-yl)phenyl]ethyl}-L-prolinamide,
(4R)-1-[(2S)-2-(4-{4-[({(7M)-6-cyclopropyl-4-[(1-{3-[(dimethylamino)methyl]azeti dine-1-carbonyl}azetidin-3-yl)oxy]-7-(6-fluoro-5-methyl-1H-indazol-4-yl)-2-[(2S)-2-methox ypropoxy]quinolin-8-yl}oxy)methyl]phenyl}-1H-1,2,3-triazol-1-yl)-3-methylbutanoyl]-4-hy droxy-N-{(1R)-2-hydroxy-1-[4-(4-methyl-1,3-oxazol-5-yl)phenyl]ethyl}-L-prolinamide,
(1s,3R)-3-(dimethylamino)cyclobutyl 3-({(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrol idin-1-yl]-3-methyl-1-oxobutan-2-yl}-1H-1,2,3-triazol-4-yl)phenyl]methoxy}-2-[(2S)-2-met hoxypropoxy]quinazolin-4-yl}amino)azetidine-1-carboxylate,
(4R)-1-[(2S)-2-(4-{4-[({(7M)-6-cyclopropyl-4-[(1-{[2-(dimethylamino)ethyl]carba moyl}azetidin-3-yl)oxy]-7-(6-fluoro-5-methyl-1H-indazol-4-yl)-2-[(2S)-2-methoxypropoxy] quinolin-8-yl}oxy)methyl]phenyl}-1H-1,2,3-triazol-1-yl)-3-methylbutanoyl]-4-hydroxy-N-{( 1R)-2-hydroxy-1-[4-(1H-1,2,4-triazol-1-yl)phenyl]ethyl}-L-prolinamide,
(1s,3R)-3-(dimethylamino)cyclobutyl 3-({(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(1H-1,2,4-triazol-1-yl)phenyl]ethyl}carbamoyl)pyrrolidin-1-yl]-3-methyl-l-oxobutan-2-yl}-1H-1,2,3-triazol-4-yl)phenyl]methoxy}-2-[(2S)-2-methoxy propoxy]quinolin-4-yl}oxy)azetidine-1-carboxylate and
(4R)-1-[(2S)-2-(4-{4-[({(7M)-6-cyclopropyl-4-[(1-{3-[(dimethylamino)methyl]azeti dine-1-carbonyl}azetidin-3-yl)oxy]-7-(6-fluoro-5-methyl-1H-indazol-4-yl)-2-[(2S)-2-methox ypropoxy]quinolin-8-yl}oxy)methyl]phenyl}-1H-1,2,3-triazol-1-yl)-3-methylbutanoyl]-4-hy droxy-N-{(1R)-2-hydroxy-1-[4-(4-methyl-1,3-thiazol-5-yl)phenyl]ethyl}-L-prolinamide,
or a salt thereof.

Examples of specific compounds included in the present invention as an aspect include the following compounds.

A compound selected from the group consisting of
(1s,3R)-3-(dimethylamino)cyclobutyl 3-({(7P)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-oxazol-5-yl)phenyl]ethyl}carbamoyl)pyrrol idin-1-yl]-3-methyl-1-oxobutan-2-yl}-1H-1,2,3-triazol-4-yl)phenyl]methoxy}-2-[(2S)-2-met hoxypropoxy]quinazolin-4-yl}amino)azetidine-1-carboxylate,
(1s,3R)-3-(dimethylamino)cyclobutyl 3-({(7P)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(1H-1,2,4-triazol-1-yl)phenyl]ethyl}carbamoyl)pyrrolidin-1-yl]-3-methyl-l-oxobutan-2-yl}-1H-1,2,3-triazol-4-yl)phenyl]methoxy}-2-[(2S)-2-methoxy propoxy]quinazolin-4-yl}amino)azetidine-1-carboxylate,
(4R)-1-[(2S)-2-(4-{4-[({(7P)-6-cyclopropyl-4-[(1-{3-[(dimethylamino)methyl]azeti dine-1-carbonyl}azetidin-3-yl)oxy]-7-(6-fluoro-5-methyl-1H-indazol-4-yl)-2-[(2S)-2-methox ypropoxy]quinolin-8-yl}oxy)methyl]phenyl}-1H-1,2,3-triazol-1-yl)-3-methylbutanoyl]-4-hy droxy-N-{(1R)-2-hydroxy-1-[4-(1H-1,2,4-triazol-1-yl)phenyl]ethyl}-L-prolinamide,
(4R)-1-[(2S)-2-(4-{4-[({(7P)-6-cyclopropyl-4-[(1-{3-[(dimethylamino)methyl]azeti dine-1-carbonyl}azetidin-3-yl)oxy]-7-(6-fluoro-5-methyl-1H-indazol-4-yl)-2-[(2S)-2-methox ypropoxy]quinolin-8-yl}oxy)methyl]phenyl}-1H-1,2,3-triazol-1-yl)-3-methylbutanoyl]-4-hy droxy-N-{(1R)-2-hydroxy-1-[4-(4-methyl-1,3-oxazol-5-yl)phenyl]ethyl}-L-prolinamide,
(1s,3R)-3-(dimethylamino)cyclobutyl 3-({(7P)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrol idin-1-yl]-3-methyl-1-oxobutan-2-yl}-1H-1,2,3-triazol-4-yl)phenyl]methoxy}-2-[(2S)-2-met hoxypropoxy]quinazolin-4-yl}amino)azetidine-1-carboxylate,
(4R)-1-[(2S)-2-(4-{4-[({(7P)-6-cyclopropyl-4-[(1-{[2-(dimethylamino)ethyl]carbam oyl}azetidin-3-yl)oxy]-7-(6-fluoro-5-methyl-1H-indazol-4-yl)-2-[(2S)-2-methoxypropoxy]qu inolin-8-yl}oxy)methyl]phenyl}-1H-1,2,3-triazol-1-yl)-3-methylbutanoyl]-4-hydroxy-N-{(1 R)-2-hydroxy-1-[4-(1H-1,2,4-triazol-1-yl)phenyl]ethyl}-L-prolinamide,
(1s,3R)-3-(dimethylamino)cyclobutyl 3-({(7P)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(1H-1,2,4-triazol-1-yl)phenyl]ethyl}carbamoyl)pyrrolidin-1-yl]-3-methyl-l-oxobutan-2-yl}-1H-1,2,3-triazol-4-yl)phenyl]methoxy}-2-[(2S)-2-methoxy propoxy]quinolin-4-yl}oxy)azetidine-1-carboxylate and
(4R)-1-[(2S)-2-(4-{4-[({(7P)-6-cyclopropyl-4-[(1-{3-[(dimethylamino)methyl]azeti dine-1-carbonyl}azetidin-3-yl)oxy]-7-(6-fluoro-5-methyl-1H-indazol-4-yl)-2-[(2S)-2-methox ypropoxy]quinolin-8-yl}oxy)methyl]phenyl}-1H-1,2,3-triazol-1-yl)-3-methylbutanoyl]-4-hy droxy-N-{(1R)-2-hydroxy-1-[4-(4-methyl-1,3-thiazol-5-yl)phenyl]ethyl}-L-prolinamide,
or a salt thereof.

The compound of the formula (I) may have tautomers or geometrical isomers depending on the type of the substituent. In this specification, the compound of the formula (I) is sometimes described only as one of isomers, but the present invention includes isomers other than the above one and includes separated isomers or mixtures thereof.

In addition, the compound of the formula (I) may have an asymmetric carbon atom or an axial chirality and may have diastereomers based on them. The present invention includes separated diastereomers of the compound of the formula (I) or mixtures thereof.

Furthermore, the present invention also includes pharmaceutically acceptable prodrugs of the compound represented by the formula (I). A pharmaceutically acceptable prodrug is a compound having a group that can be converted into an amino group, a hydroxy group, a carboxyl group or the like by solvolysis or under physiological conditions. Examples of groups to form a prodrug include groups described in Prog. Med., 1985, 5, p.2157-2161 or in "Iyakuhin no Kaihatsu (development of pharmaceuticals)", Vol.7, Bunshi-sekkei (molecular design), Hirokawa Shoten, 1990, p.163-198.

In addition, the salt of the compound of the formula (I) is a pharmaceutically acceptable salt of the compound of the formula (I) and may be an acid addition salt or a salt formed with a base depending on the type of the substituent. Examples thereof include salts shown in P. Heinrich Stahl, Handbook of Pharmaceutical Salts Properties, Selection, and Use, Wiley-VCH, 2008. Specific examples include an acid addition salt with an inorganic acid, such as hydrochloric acid, hydrobromic acid, hydroiodic acid, sulfuric acid, nitric acid and phosphoric acid, or with an organic acid, such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid and glutamic acid, a salt with an inorganic metal, such as sodium, potassium, magnesium, calcium and aluminum, a salt with an organic base, such as methylamine, ethylamine and ethanolamine, a salt with various amino acids and amino acid derivatives, such as acetylleucine, lysine and ornithine, an ammonium salt and the like.

Furthermore, the present invention also includes various hydrates, solvates and crystal polymorphism substances of the compound of the formula (I) and a salt thereof.

The present invention also includes all the compounds of the formula (I) or salts thereof which are pharmaceutically acceptable and labeled with one or more radioactive or nonradioactive isotopes. Examples of preferable isotopes used for isotope labeling of the compound of the present invention include isotopes of hydrogen (²H, ³H and the like), carbon (¹¹C, ¹³C, ¹⁴C and the like), nitrogen (¹³N, ¹⁵N and the like), oxygen (¹⁵O, ¹⁷O, ¹⁸O and the like), fluorine (¹⁸F and the like), chlorine (³⁶Cl and the like) and iodine (¹²³I, ¹²⁵I and the like) and sulfur (³⁵S and the like).

The isotopically labeled compound of the invention of the present application can be used for studies such as histological distribution study of drugs and/or substrates and the like. For example, a radioisotope such as tritium (³H) and carbon-14 (¹⁴C) can be used for the purpose due to easiness of labeling and convenience of detection.

Replacement with a heavier isotope, for example replacement of hydrogen with deuterium (²H), is sometimes therapeutically advantageous because metabolic stability improves (for example, increased *in vivo* half-life, decreased required dose and declined drug interaction).

Replacement with a positron-emitting isotope (¹¹C, ¹⁸F, ¹⁵O, ¹³N or the like) can be used for positron emission tomography (PET) for testing the substrate acceptor occupancy.

The isotopically labeled compound of the present invention can be generally produced by a conventional method known to a person skilled in the art or by a production method similar to those in the Examples or the Production Examples or the like using an appropriate isotopically labeled reagent instead of an unlabeled reagent.

### (Production Method)

The compound of the formula (I) and a salt thereof can be produced by applying various known synthetic methods using characteristics based on the basic structure or the type of substituent thereof. Here, depending on the type of functional group, it is sometimes effective as a production technique to substitute the functional group with an appropriate protective group (a group that can be easily converted to the functional group) in the process from a raw material to an intermediate. Examples of the protective group include protective groups described in P. G. M. Wuts and T. W. Greene, "Greene's Protective Groups in Organic Synthesis", 5th edition, John Wiley & Sons Inc., 2014 and the like, and a group appropriately selected from the protective groups is used depending on the reaction conditions. In such a method, a reaction is carried out with the protective group introduced, and then the protective group is removed, as required, whereby a desired compound can be obtained.

In addition, a prodrug of the compound of the formula (I) can be produced by introducing a special group in a process from a raw material to an intermediate as for the above protective group or by further carrying out a reaction using the compound of the formula (I) obtained. This reaction can be carried out by applying a method known to a person skilled in the art, such as common esterification, amidation and dehydration.

Typical methods for producing the compound of the formula (I) will be explained below. The production methods can also be carried out referring to a reference attached to the explanation. Note that the production method of the present invention is not limited to the examples described below.

In this specification, the following abbreviations are sometimes used.

DMF: N,N-dimethylformamide, DMAc: N,N-dimethylacetamide, THF: tetrahydrofuran, MeCN: acetonitrile, MeOH: methanol, EtOH: ethanol, iPrOH: isopropylalcohol, tBuOH: tert-butanol, DOX: 1,4-dioxane, DMSO: dimethyl sulfoxide, TFA: trifluoroacetic acid, TEA: triethylamine, DIPEA: N,N-diisopropylethylamine, tBuOK : potassium tert-butoxide, PdCl₂(dppf)·CH₂Cl₂:
[1,1'-bis(diphenylphosphino)ferrocene]palladium(II) dichloride-dichloromethane adduct, Pd/C: palladium carbon, PyBOP: (benzotriazol-1-yloxy)tripyrrolidinophosphonium hexafluorophosphate, TBAF: tetra-n-butylammonium fluoride, DABCO: 1,4-diazabicyclo[2.2.2]octane, CDI: 1,1'-carbonyldiimidazole, HATU: O-(7-azabenzotriazol-1-yl)-N,N,N',N'-tetramethyluronium hexafluorophosphate, Tr: trityl.

### (Production Method 1)

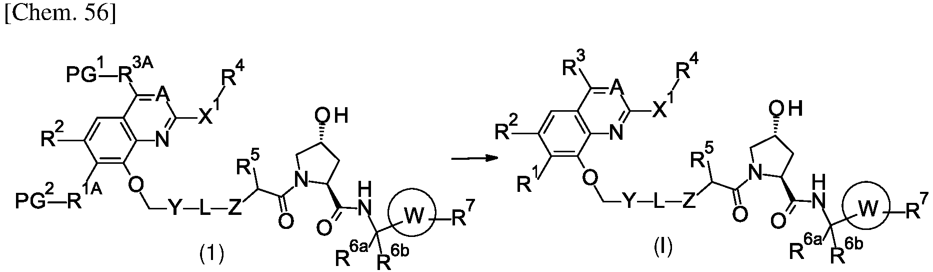

(In the formula, R^{1A} and R^{3A} represent divalent groups in which H has been removed from a functional group of R¹ or R³ to which a protective group can be introduced. PG¹and PG² represent protective groups. In some cases, one of the protective groups is absent. The same applies below.)

The compound of the formula (I) can be obtained by subjecting a compound (1) to a deprotection reaction. Furthermore, the compound can also be sometimes obtained by subjecting NH₂ contained in R³ to an alkylation reaction after subjecting to a deprotection reaction. Examples of the protective groups shown here include a tert-butoxycarbonyl group, a triphenylmethyl group, a tetrahydro-2H-pyran-2-yl group, a methoxymethyl group, a dimethylmethanediyl group, a tert-butylsulfinyl group and the like.

In the deprotection reaction, the compound is stirred from under cooling to under reflux with heat generally for 0.1 hours to five days. Examples of the solvent used here include, but are not particularly limited to, an alcohol, such as MeOH, EtOH and iPrOH, a halogenated hydrocarbon, such as dichloromethane, 1,2-dichloroethane and chloroform, an ether, such as diethyl ether, THF, DOX and dimethoxyethane, DMF, DMSO, MeCN or water and a mixture thereof. Examples of the deprotection reagent include, but are not particularly limited to, an acid, such as hydrogen chloride (DOX solution), trifluoroacetic acid, methanesulfonic acid, p-toluenesulfonic acid and phosphoric acid.

By selecting a protective group, the deprotection can be performed by a catalytic hydrogenation reaction. Examples of the protective group include a benzyl group, a p-methoxybenzyl group, a benzyloxycarbonyl group and the like. The deprotection can also be performed using a fluoride ion source such as tetra-n-butylammonium fluoride. Examples of the protective group include a tert-butyl(dimethyl)silyl group, a (trimethylsilyl)ethoxymethyl group and the like. Furthermore, examples of the protective group which can be deprotected under basic conditions include an acetyl group, a trifluoroacetyl group, a benzoyl group and the like. Moreover, protective groups which can be deprotected under different deprotection conditions can be selected for PG¹ and PG², and the deprotection can be performed stepwise.

The alkylation reaction is performed using formaldehyde or an alkyl having a formyl group, by stirring in the presence of a reductant, in a solvent inactive for the reaction, at -45°C to under reflux with heat, preferably at 0°C to at room temperature, generally for 0.1 hours to five days. Examples of the solvent used here include, but are not particularly limited to, an alcohol, such as methanol and ethanol, an ether, such as diethyl ether, tetrahydrofuran (THF), dioxane and dimethoxyethane, a halogenated hydrocarbon, such as dichloromethane, 1,2-dichloroethane and chloroform and a mixture thereof. The reductant is sodium cyanoborohydride, sodium triacetoxyborohydride, sodium borohydride or the like. Performing the reaction in the presence of a dehydrator such as molecular sieves or an acid such as acetic acid, hydrochloric acid and titanium(IV) isopropoxide complex is sometimes preferable.

For example, the following can be referred as a reference.

P. G. M. Wuts and T. W. Greene, "Greene's Protective Groups in Organic Synthesis", 5th edition, John Wiley & Sons Inc., 2014

A. R. Katritzky and R. J. K. Taylor, "Comprehensive Organic Functional Group Transformations II", vol. 2, Elsevier Pergamon, 2005

Note that when the compound (1) as a raw material has an axial chirality, a stereoisomer which is obtained by once separating the compound (1) may be used for this reaction.

By subjecting the compound of the formula (I), for example, to the following operation as a salt formation reaction, the hydrochloride of the compound of the formula (I) can be obtained.

The compound of the formula (I) which is believed to form a salt with hydrochloric acid from the characteristics of the chemical structure is dissolved in CH₂Cl₂ and MeOH and stirred under ice cooling for 30 minutes after adding hydrogen chloride (4M DOX solution, 10 equivalents) under ice cooling. The reaction mixture is concentrated under reduced pressure, and diethyl ether is added to the resulting residue. The produced solid is taken by filtration and is dried under reduced pressure, thus obtaining the hydrochloride of the compound of the formula (I).

By subjecting the hydrochloride of the compound of the formula (I), for example, to the following operation as a desalting reaction, the compound of the formula (I) can be obtained, but the method is not limited to this method.

The hydrochloride of the compound of the formula (I) is purified by ODS column chromatography (MeCN/0.1% aqueous formic acid solution), and a fraction containing the target substance is collected and is made basic with saturated aqueous sodium hydrogen carbonate solution. Then the solution is subjected to extraction with CHCl₃/MeOH (5/1). The combined organic layer is dried over anhydrous sodium sulfate, and the solution is concentrated under reduced pressure. The resulting solid is washed with diethyl ether and dried under reduced pressure, thus obtaining the compound of the formula (I).

### (Raw Material Synthesis 1)

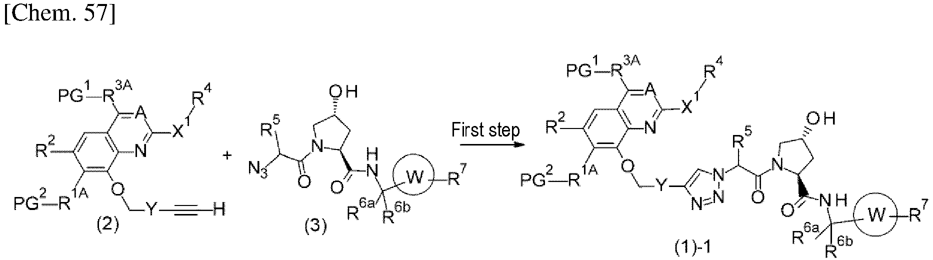

This production method is a first method for producing a compound (1)-1 included in the raw material compound (1).

### (First Step)

This step is a method for producing the compound (1)-1 by a cycloaddition reaction of a compound (2) and a compound (3).

In this reaction, the compound (2) and the compound (3) are used in an equal amount or with one compound thereof in an excess amount, and the mixture of the compounds is stirred preferably in the presence of a copper salt, further preferably in the presence of a copper salt and a reductant, in a solvent inactive for the reaction or with no solvent, from under cooling to under reflux with heat, preferably at 0°C to 100°C, generally for 0.1 hours to five days. Examples of the solvent used here include, but are not particularly limited to, a halogenated hydrocarbon, such as dichloromethane, 1,2-dichloroethane and chloroform, an aromatic hydrocarbon, such as benzene, toluene and xylene, an ether, such as diethyl ether, THF, DOX and 1,2-dimethoxyethane, DMF, DMSO, ethyl acetate, MeCN, tBuOH, water and a mixture thereof. The copper salt is CuI, CuSO₄, copper(I) trifluoromethanesulfonate (CuOTf) or the like. The reductant is sodium ascorbate or the like. Performing the reaction in the presence of TEA, DIPEA, N-methylmorpholine (NMM), 2,6-lutidine, tris[(1-benzyl-1H-1,2,3-triazol-4-yl)methyl]amine (TBTA) or the like is sometimes advantageous for smoothly promoting the reaction.

### [Reference]

Angew. Chem. Int. Ed. 2002, 41, p.2596-2599.

Note that a compound obtained by subjecting PG² of the compound (2) first to a deprotection reaction may be used for this reaction.

### (Raw Material Synthesis 2)

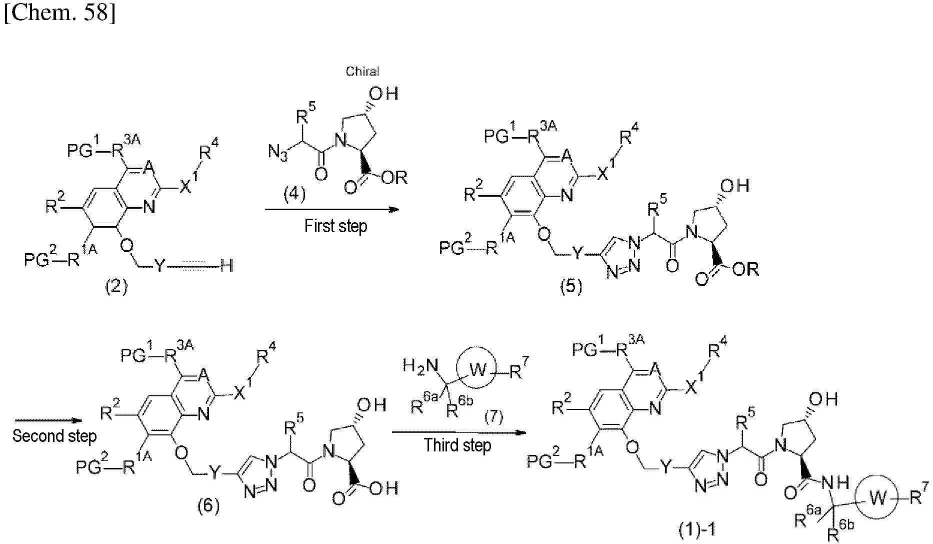

(In the formulae, R represents a C₁₋₃ alkyl group. The same applies below.)

This production method is a second method for producing the compound (1)-1 included in the raw material compound (1).

### (First Step)

This step is a method for producing a compound (5) by a cycloaddition reaction of the compound (2) and a compound (4).

The reaction conditions are the same as in the first step of the Raw Material Synthesis 1.

### (Second Step)

This step is a method for producing a compound (6) by hydrolysis of the compound (5).

This reaction is performed by stirring the compound (5) from under cooling to under reflux with heat generally for 0.1 hours to five days. Examples of the solvent used here include, but are not particularly limited to, an alcohol, acetone, DMF, THF and the like. In addition, a mixed solvent of the above solvent and water is sometimes suitable for the reaction. Examples of the hydrolysis reagent include, but are not particularly limited to, an aqueous sodium hydroxide solution, an aqueous potassium hydroxide solution, trimethyltin hydroxide and the like.

For example, the following can be referred as a reference about this reaction.

The Chemical Society of Japan, "Jikken Kagaku Koza (lectures on experimental chemistry) (5th edition)", Vol. 16 (2005) (Maruzen)

Angew. Chem. Int. Ed. 2005, 44, p.1378-1382.

### (Third Step)

This step is a method for producing the compound (1)-1 by an amidation reaction of the compound (6) and a compound (7).

In this reaction, the compound (6) and the compound (7) are used in an equal amount or with one compound thereof in an excess amount, and the mixture of the compounds is stirred in the presence of a condensing agent, in a solvent inactive for the reaction, from under cooling to under heating, preferably at -20°C to 60°C, generally for 0.1 hours to five days. Examples of the solvent include, but are not particularly limited to, an aromatic hydrocarbon, such as toluene, an ether, such as THF and DOX, a halogenated hydrocarbon, such as dichloromethane, an alcohol, DMF, DMSO, ethyl acetate, MeCN and a mixture thereof. Examples of the condensing agent include (benzotriazol-1-yloxy)tripyrrolidinophosphonium hexafluorophosphate (PyBOP), O-(7-azabenzotriazol-1-yl)-N,N,N',N'-tetramethyluronium hexafluorophosphate (HATU), 1-(3-dimethylaminopropyl)-3-ethylcarbodiimide or the hydrochloride thereof, N,N'-dicyclohexylcarbodiimide (DCC), 1,1'-carbonyldiimidazole (CDI), diphenylphosphoryl azide (DPPA) and the like. Use of an additive (for example, 1-hydroxybenzotriazole) is sometimes preferred for the reaction. Performing the reaction in the presence of an organic base, such as TEA, DIPEA and NMM, or an inorganic base, such as potassium carbonate, sodium carbonate and potassium hydroxide, is sometimes advantageous for smoothly promoting the reaction.

Alternatively, a method in which the compound (6) is converted into a reactive derivative, which is then subjected to an acylation reaction, can be used. Examples of the reactive derivative of a carboxylic acid include an acid halogenation product obtained by a reaction with a halogenating agent, such as phosphorus oxychloride and thionyl chloride, a mixed acid anhydride obtained by a reaction with isobutyl chloroformate or the like, an active ester obtained by condensation with 1-hydroxybenzotriazole or the like and the like. The reaction of such a reactive derivative and the compound (7) can be performed in a solvent inactive for the reaction, such as a halogenated hydrocarbon, an aromatic hydrocarbon and an ether, from under cooling to under heating, preferably at -20°C to 120°C.

### [Reference]

S. R. Sandler and W. Karo, "Organic Functional Group Preparations", 2nd edition, Vol. 1, Academic Press Inc., 1991

The Chemical Society of Japan, "Jikken Kagaku Koza (lectures on experimental chemistry (5th edition)", Vol. 16 (2005) (Maruzen)

### (Raw Material Synthesis 3)

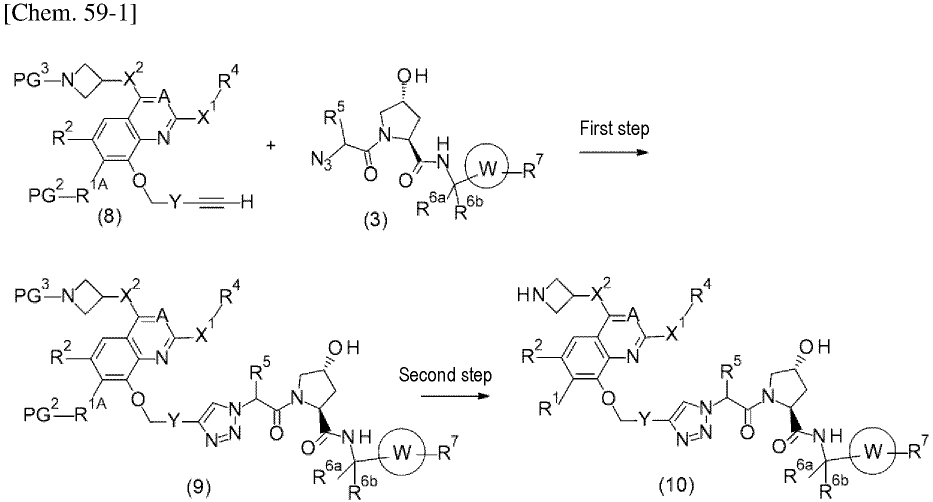

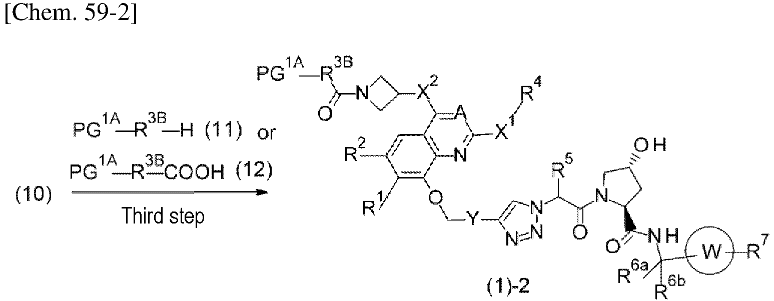

(In the formulae, R^{3B} represents NR^{3aa}R^{3bb}, OR^{3cc} or R^{3dd}, and PG^{1A} and PG³ represent protective groups. R^{3B} represents a divalent group in which H has been removed from a functional group to which a protective group can be introduced and from a functional group to which a carbonyl group or a carboxyl group can be introduced in any of NR^{3a}R^{3b}, OR^{3c} and R^{3d}. R¹ may have PG². The same applies below.)

This production method is a second method for producing a compound (1)-2 included in the raw material compound (1).

### (First Step)

This step is a method for producing a compound (9) by a cycloaddition reaction of a compound (8) and the compound (3).

The reaction conditions are the same as in the first step of the Raw Material Synthesis 1.

### (Second Step)

This step is a method for producing a compound (10) by subjecting the compound (9) to a deprotection reaction.

The reaction conditions are the same as in the deprotection reaction of the Production Method 1.

### (Third Step)

This step is a method for producing the compound (1)-2 by adding a compound (11) after a reaction of the compound (10) and a carbonylation reagent or a method for producing the compound (1)-2 by an amidation reaction of the compound (10) and a compound (12).

This step is performed by reacting the compound (10) with a carbonylation reagent in an equal amount or an excess amount in a solvent inactive for the reaction, from under cooling to under heating, preferably at -20°C to 80°C, generally for 0.1 hours to around a day. Subsequently, the compound (11) in an equal amount or an excess amount is added to the obtained reaction mixture, and this mixture is reacted from under cooling to under heating, preferably at -20°C to 80°C, for 0.1 hours to around a day. Examples of the carbonylation reagent include 1,1'-carbonyldiimidazole, 4-nitrophenyl chloroformate, diphosgene, triphosgene, phenyl chloroformate and the like. Examples of the solvent used here include, but are not particularly limited to, a halogenated hydrocarbon, such as dichloromethane, 1,2-dichloroethane and chloroform, an aromatic hydrocarbon, such as benzene, toluene and xylene, an ether, such as diethyl ether, tetrahydrofuran, dioxane and dimethoxyethane, N,N-dimethylformamide, dimethylsulfoxide, ethyl acetate, acetonitrile or a mixture thereof. Performing the reaction in the presence of a base, such as triethylamine is sometimes advantageous for smoothly promoting the reaction.

The reaction conditions of the amidation reaction of the compound (10) and the compound (12) are the same as in the third step of the Raw Material Synthesis 2.

### [Reference]

S. R. Sandler and W. Karo, "Organic Functional Group Preparations", 2nd edition, Vol. 2, Academic Press Inc., 1991

### (Raw Material Synthesis 4)

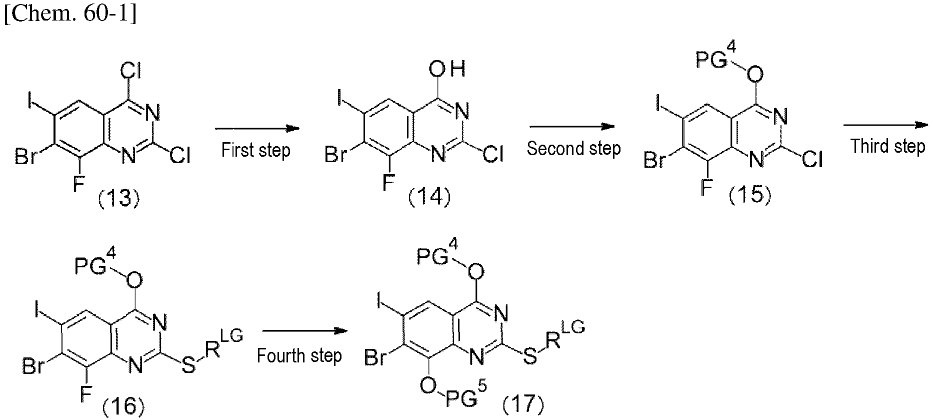

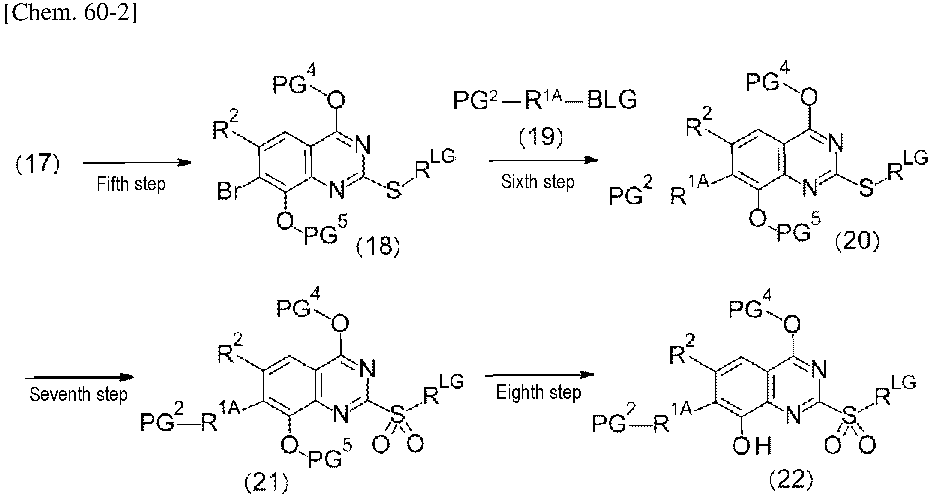

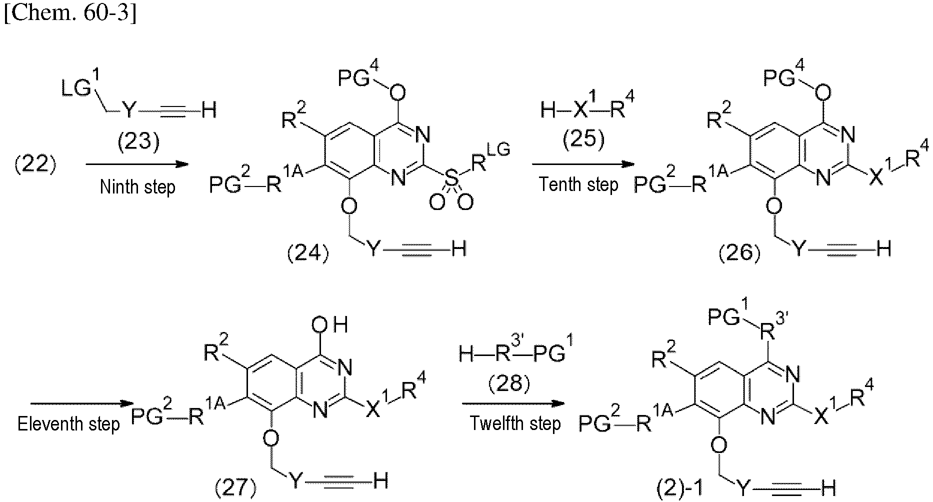

(In the formulae, PG⁴ and PG⁵ represent protective groups. R^{LG} represents a C₁₋₁₂ alkyl group, and LG¹ represents a leaving group. BLG represents a boronic acid group, a boronic acid group protected with a protective group of boronic acid, such as a boronic acid pinacol ester group, or a trifluoroboric acid salt group (sometimes referred to as a boronic acid group or the like below). Examples of the leaving group shown here include Cl, Br, a methanesulfonyloxy group, a p-toluenesulfonyloxy group and the like.)

This production method is a method for producing a compound (2)-1 included in the raw material compound (2).

### (First Step)

This step is a method for producing a compound (14) by hydrolysis of a compound (13).

This reaction is performed by stirring the compound (13) from under cooling to under reflux with heat, generally for 0.1 hours to five days. Examples of the solvent used here include, but are not particularly limited to, an alcohol, acetone, DMF, THF and the like. In addition, a mixed solvent of the above solvent and water is sometimes suitable for the reaction. Examples of the hydrolysis reagent include, but are not particularly limited to, an aqueous sodium hydroxide solution, an aqueous potassium hydroxide solution and the like.

For example, the following can be referred as a reference about this reaction.

The Chemical Society of Japan, "Jikken Kagaku Koza (lectures on experimental chemistry (5th edition)", Vol. 16 (2005) (Maruzen)
Angew. Chem. Int. Ed. 2005, 44, p.1378-1382.

### (Second Step)

This step is a method for producing a compound (15) by protecting the hydroxy group of the compound (14) with a protective group.

When the group is protected with a tert-butyl group as an example, this reaction is performed by stirring the compound (14) from under cooling to under reflux with heat, generally for 0.1 hours to five days. Examples of the solvent used here include, but are not particularly limited to, an ether, such as THF and DOX, a halogenated hydrocarbon, such as dichloromethane, tBuOH, DMF and the like. Examples of the tert-butyl protective reagent include, but are not particularly limited to, isobutene, 2-tert-butyl-1,3-diisopropylisourea and the like.

Moreover, the compound (15) can be produced by a dehydration condensation reaction of the compound (14) and tBuOH.

For example, the following can be referred as a reference about this reaction.
P. G. M. Wuts and T. W. Greene, "Greene's Protective Groups in Organic Synthesis", 5th edition, John Wiley & Sons Inc., 2014
Org. Lett., 2012, 14, 17, p.4678-4681

### (Third Step)

This step is a method for producing a compound (16) by an ipso substitution reaction of the compound (15) and R^{LG}-SH.

Examples of the R^{LG}-SH used here include C₁₋₁₂ alkylthiols, for example, ethanethiol and dodecanethiol. In this reaction, the compound (15) and the R^{LG}-SH are used in an equal amount or with one compound thereof in an excess amount, and the mixture of the compounds is stirred in a solvent inactive for the reaction or with no solvent, from under cooling to under reflux with heat, preferably at 0°C to 80°C, generally for 0.1 hours to five days. Examples of the solvent used here include, but are not particularly limited to, a halogenated hydrocarbon, such as dichloromethane, 1,2-dichloroethane and chloroform, an aromatic hydrocarbon, such as benzene, toluene and xylene, an ether, such as diethyl ether, THF, DOX and 1,2-dimethoxyethane, DMF, DMAc, DMSO, ethyl acetate, MeCN and a mixture thereof. Performing the reaction in the presence of an organic base, such as TEA, DIPEA, N-methylmorpholine (NMM), 1,4-diazabicyclo[2.2.2]octane (DABCO) and tBuOK, or an inorganic base, such as sodium hydride, potassium carbonate, sodium carbonate and cesium carbonate, is sometimes advantageous for smoothly promoting the reaction.

### (Fourth Step)

This step is a method for producing a compound (17) by an ipso substitution reaction of the compound (16) and PG⁵-OH. Examples of the PG⁵-OH used here include benzyl alcohol, p-methoxybenzyl alcohol and 1-phenylethanol.

The reaction conditions are the same as in the third step of the Raw Material Synthesis 4.

### (Fifth Step)

This step is a method for producing a compound (18) by a Suzuki-Miyaura coupling reaction of the compound (17) and a boronic acid derivative composed of an R²-boronic acid group or the like.

Examples of the boronic acid group or the like used here include, but are not particularly limited to, a boronic acid group, a boronic acid ester group, a boronic acid pinacol ester group, a triol borate salt group and a trifluoroboric acid salt group.

In this reaction, the compound (17) and the boronic acid derivative composed of an R²-boronic acid group or the like are used in an equal amount or with one compound thereof in an excess amount, and the mixture of the compounds is stirred in a solvent inactive for the reaction, in the presence of a base and a palladium catalyst, from at room temperature to under reflux with heat, preferably at 20°C to 140°C, generally for 0.1 hours to five days. Examples of the solvent used here include, but are not particularly limited to, a halogenated hydrocarbon, such as dichloromethane, 1,2-dichloroethane and chloroform, an aromatic hydrocarbon, such as benzene, toluene and xylene, an ether, such as diethyl ether, THF, DOX and 1,2-dimethoxyethane, an alcohol, such as MeOH, EtOH, isopropyl alcohol, butanol and amyl alcohol, DMF, DMSO, MeCN, 1,3-dimethylimidazolidin-2-one, water and a mixture thereof. The base is an inorganic base, such as tripotassium phosphate, sodium carbonate, potassium carbonate, sodium hydroxide and barium hydroxide. The palladium catalyst is tetrakis(triphenylphosphine)palladium, bis(triphenylphosphine)palladium(II) dichloride, [1,1'-bis(diphenylphosphino)ferrocene]palladium(II) dichloride · dichloromethane adduct, (1E,4E)-1,5-diphenylpenta-1,4-dien-3-one/palladium (3:2) and (2-dicyclohexylphosphino-2',6'-diisopropoxy-1,1'-biphenyl)[2-(2'-amino-1,1'-biphenyl)]palla dium(II) methanesulfonate, palladium(II) acetate or the like. Performing the reaction in the presence of a ligand, such as dicyclohexyl(2',6'-dimethoxybiphenyl-2-yl)phosphine, dicyclohexyl(2',6'-diisopropoxy-[1,1'-biphenyl]-2-yl)phosphine and 1,1'-bis(diphenylphosphino)ferrocene, is sometimes advantageous for smoothly promoting the reaction. In addition, heating the mixture by microwave irradiation is sometimes advantageous for smoothly promoting the reaction.

### [Reference]

J. Am. Chem. Soc., 2005, 127, p.4685-4696
Org. Lett. 2011, 13, p.3948-3951
Org. Lett. 2012, 14, p.1278-1281

The compound (18) (R²is hydrogen here) can be produced by a dehalogenation reaction of the compound (17) using a Pd catalyst and a reductant.

### [Reference]

J. Org. Chem., 1977, 42, p.3491-3494
Tetrahedron Letters 2013, 54, 5207-5210

### (Sixth Step)

This step is a method for producing a compound (20) by a Suzuki-Miyaura coupling reaction of the compound (18) and a compound (19).

The reaction conditions are the same as in the fifth step of the Raw Material Synthesis 4.

When the compound (18) has an axial chirality, the compound (18) is obtained as a mixture of stereoisomers, and each stereoisomer can be isolated by separation using a common separation operation, for example, ODS column chromatography or silica gel column chromatography.

### (Seventh Step)

This step is a method for producing a compound (21) by an oxidation reaction of the compound (20).

In this reaction, a compound (22) is treated with an oxidant in an equal amount or an excess amount in a solvent inactive for the reaction, from under cooling to under heating, preferably at -20°C to 80°C, generally for 0.1 hours to three days. In this reaction, oxidation with m-chloroperbenzoic acid, perbenzoic acid, peracetic acid, sodium hypochlorite or hydrogen peroxide is suitably used. Examples of the solvent include an aromatic hydrocarbon, an ether, a halogenated hydrocarbon such as dichloromethane, DMF, DMSO, ethyl acetate, MeCN and a mixture thereof. Other examples of the oxidant include cumene hydroperoxide, Oxone, active manganese dioxide, chromic acid, potassium permanganate, sodium periodate and the like.

### [Reference]

The Chemical Society of Japan, "Jikken Kagaku Koza (lectures on experimental chemistry)", 5th edition, Vol. 17, Maruzen, 2004

When the compound (21) has an axial chirality, the compound (21) is sometimes obtained as a mixture of stereoisomers, and each stereoisomer can be isolated by separation using a common separation operation, for example, ODS column chromatography or silica gel column chromatography.

After subjecting the compound (21) to a deprotection reaction, PG² is sometimes converted to another protective group so that deprotection can be performed under different conditions from those of the protective group PG¹ to be introduced later.

The reaction conditions of the deprotection reaction used here are the same as in the step described in the Production Method 1.

Examples of the protective group of PG² to be converted subsequently include a tetrahydro-2H-pyran-2-yl group and the like.

For example, the following can be referred as a reference about this reaction.
P. G. M. Wuts and T. W. Greene, "Greene's Protective Groups in Organic Synthesis", 5th edition, John Wiley & Sons Inc., 2014

### (Eighth Step)

This step is a method for producing the compound (22) by deprotection by a catalytic hydrogenation reaction of the compound (21).

This reaction can be performed by stirring the compound (21) under hydrogen atmosphere, from under normal pressure to under increased pressure, in a solvent inactive for the reaction, such as MeOH, EtOH, ethyl acetate and THF, in the presence of a metal catalyst, from under cooling to under heating, preferably at room temperature, for an hour to five days. As the metal catalyst, a palladium catalyst, such as Pd/C and palladium black, a platinum catalyst, such as a platinum plate and platinum oxide, a nickel catalyst, such as reduced nickel and Raney nickel, or the like is used.

### (Ninth Step)

This step is a method for producing a compound (24) by a reaction of the compound (22) and a compound (23).

This reaction is performed using the compound (22) and the compound (23) in an equal amount or with one compound thereof in an excess amount by reacting a mixture of the compounds in the presence of a base, in a solvent inactive for the reaction, from under cooling to under reflux with heat, preferably at 0°C to 80°C, generally for 0.1 hours to five days. The solvent used here is not particularly limited, and examples thereof include an aromatic hydrocarbon, such as benzene, toluene and xylene, an alcohol, such as MeOH and EtOH, an ether, such as diethyl ether, THF, DOX and 1,2-dimethoxyethane, a halogenated hydrocarbon, such as dichloromethane, 1,2-dichloroethane and chloroform, DMF, DMSO, ethyl acetate, MeCN and a mixture thereof. Examples of the base include, but are not particularly limited to, for example, an organic base, such as TEA, DIPEA, 1,8-diazabicyclo[5.4.0]-7-undecene, n-butyllithium and tBuOK, and an inorganic base, such as sodium hydroxide, sodium carbonate, potassium carbonate, cesium carbonate and sodium hydride. Performing the reaction in the presence of a phase transfer catalyst, such as tetra-n-butylammonium chloride, is sometimes advantageous.

For example, the following can be referred as a reference about this reaction.

The Chemical Society of Japan, "Jikken Kagaku Koza (lectures on experimental chemistry)", 5th edition, Vol. 14, Maruzen, 2005

The compound (24) in which LG¹ is halogen can be produced by halogenation of a compound in which the moiety corresponding to LG¹ is a hydroxy group. Examples of the halogenating agent used here include, but are not particularly limited to, for example, thionyl chloride, phosphorus oxychloride, hydrobromic acid, phosphorus tribromide and the like.

For example, the following can be referred as a reference about this reaction.

The Chemical Society of Japan, "Jikken Kagaku Koza (lectures on experimental chemistry)", 5th edition, Vol. 13, Maruzen, 2004

Furthermore, the compound (24) in which LG¹ is a sulfonyloxy group can be produced by sulfonylation of a compound in which the moiety corresponding to LG¹ is a hydroxy group in the presence of a base. Examples of the sulfonylation reagent used here include, but are not particularly limited to, for example, methanesulfonyl chloride, p-toluenesulfonylchloride, methanesulfonic anhydride and the like. Examples of the base include, but are not particularly limited to, for example, TEA, DIPEA, pyridine, tetramethylethylenediamine and the like.

For example, the following can be referred as a reference about this reaction.
Synthesis 1999, 9, p.1633-1636

### (Tenth Step)

This step is a method for producing a compound (26) by an ipso substitution reaction of the compound (24) and a compound (25).

The reaction conditions are the same as in the third step of the Raw Material Synthesis 4.

### (Eleventh Step)

This step is a method for producing a compound (27) by subjecting the compound (26) to a deprotection reaction.

The reaction conditions are the same as in the step described in the Production Method 1.

### (Twelfth Step)

This step is a method for producing the compound (2)-1 by a reaction of the compound (27) and a compound (28).

In this reaction, the compound (27) and the compound (28) are used in an equal amount or with one compound thereof in an excess amount, and the mixture of the compounds is stirred in the presence of a condensing agent, in a solvent inactive for the reaction, from under cooling to under heating, preferably at -20°C to 60°C, generally for 0.1 hours to five days. Examples of the solvent include, but are not particularly limited to, an aromatic hydrocarbon, such as toluene, an ether, such as THF and DOX, a halogenated hydrocarbon, such as dichloromethane, an alcohol, DMF, DMSO, ethyl acetate, MeCN and a mixture thereof. Examples of the condensing agent include PyBOP, HATU, CDI and the like. Performing the reaction in the presence of an organic base, such as TEA, DIPEA and NMM, or an inorganic base, such as potassium carbonate, sodium carbonate and cesium carbonate, is sometimes advantageous for smoothly promoting the reaction.

### (Raw Material Synthesis 5)

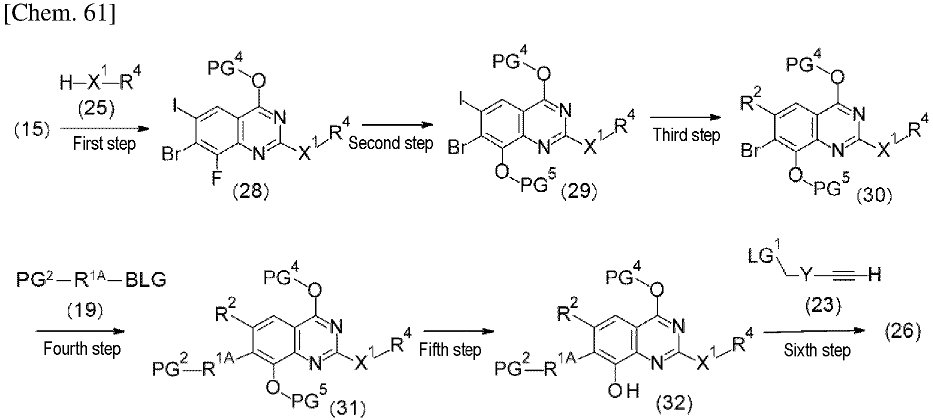

This production method is a method for producing the raw material compound (26).

### (First Step)

This step is a method for producing the compound (28) by an ipso substitution reaction of the compound (15) and the compound (25).

The reaction conditions are the same as in the third step of the Raw Material Synthesis 4.

Moreover, the compound (28) can be produced by Negishi coupling of a compound in which a hydrogen atom of the compound (25) has been converted to halogen and the compound (15).

### (Second Step)

This step is a method for producing a compound (29) by an ipso substitution reaction of the compound (28) and PG⁵-OH.

The reaction conditions are the same as in the third step of the Raw Material Synthesis 4.

### (Third Step)

This step is a method for producing a compound (30) by a Suzuki-Miyaura coupling reaction of the compound (29) and a boronic acid derivative composed of an R²-boronic acid group or the like.

The reaction conditions are the same as in the fifth step of the Raw Material Synthesis 4.

The compound (30) (R² is hydrogen here) can be produced by a dehalogenation reaction of the compound (29) using a Pd catalyst and a reductant.

### [Reference]

J. Org. Chem., 1977, 42, p.3491-3494
Tetrahedron Letters 2013, 54, 5207-5210

### (Fourth Step)

This step is a method for producing a compound (31) by a Suzuki-Miyaura coupling reaction of the compound (30) and the compound (19).

The reaction conditions are the same as in the fifth step of the Raw Material Synthesis 4.

### (Fifth Step)

This step is a method for producing a compound (32) by deprotection by a catalytic hydrogenation reaction of the compound (31).

The reaction conditions are the same as in the eighth step of the Raw Material Synthesis 4.

### (Sixth Step)

This step is a method for producing the compound (26) by a reaction of the compound (32) and the compound (23).

The reaction conditions are the same as in the ninth step of the Raw Material Synthesis 4.

### (Raw Material Synthesis 6)

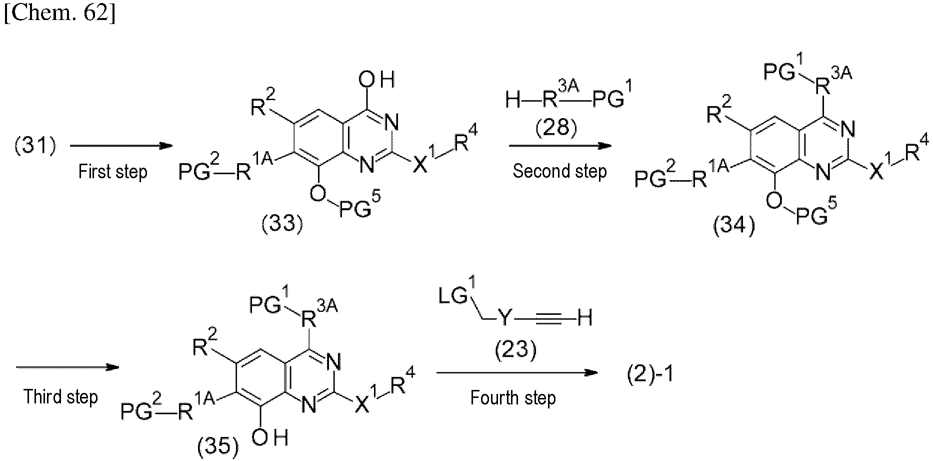

This production method is a method for producing the raw material compound (2)-1.

### (First Step)

This step is a method for producing a compound (33) by subjecting the compound (31) to a deprotection reaction.

The reaction conditions are the same as in the step described in the Production Method 1.

When the compound (31) has an axial chirality, the compound (31) is sometimes obtained as a mixture of stereoisomers, and each stereoisomer can be isolated by separation using a common separation operation, for example, ODS column chromatography or silica gel column chromatography.

After subjecting the compound (31) to a deprotection reaction, PG² is sometimes converted to another protective group so that deprotection can be performed under different conditions from those of the protective group PG¹ to be introduced later.

### (Second Step)

This step is a method for producing a compound (34) by a reaction of the compound (33) and the compound (28).

The reaction conditions are the same as in the twelfth step of the Raw Material Synthesis 4.

### (Third Step)

This step is a method for producing a compound (35) by deprotection by a catalytic hydrogenation reaction of the compound (34).

The reaction conditions are the same as in the eighth step of the Raw Material Synthesis 4.

### (Fourth Step)

This step is a method for producing the compound (2)-1 by a reaction of the compound (35) and the compound (23).

The reaction conditions are the same as in the ninth step of the Raw Material Synthesis 4.

### (Raw Material Synthesis 7)

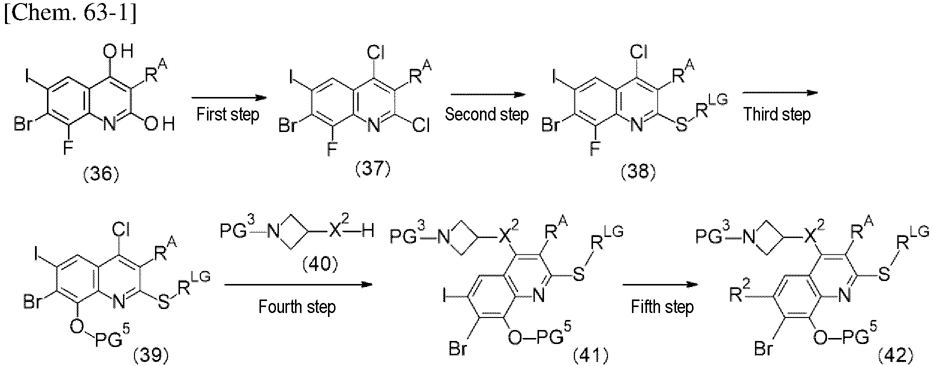

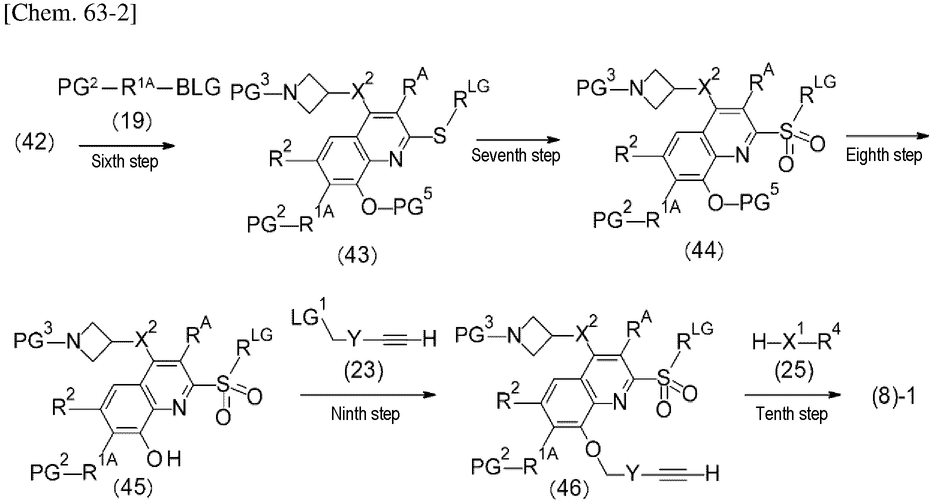

This production method is a method for producing a compound (8)-1 included in the raw material compound (8).

### (First Step)

This step is a method for producing a compound (37) by a chlorination reaction of a compound (36).

This reaction is performed using the compound (36) and a chlorinating agent in an equal amount or with one compound thereof in an excess amount by stirring the mixture of the compounds in a solvent inactive for the reaction or with no solvent, from under cooling to under reflux with heat, preferably at 60°C to under reflux with heat, generally for 0.1 hours to five days. Examples of the solvent used here include, but are not particularly limited to, an aromatic hydrocarbon such as toluene, an ether, such as THF and DOX, a halogenated hydrocarbon, such as dichloromethane, and the like. Examples of the chlorinating agent include phosphorus oxychloride, thionyl chloride and the like. Performing the reaction in the presence of an organic base, such as TEA, DIPEA and NMM, is sometimes advantageous for smoothly promoting the reaction.

### (Second Step)

This step is a method for producing a compound (38) by an ipso substitution reaction of the compound (37) and R^{LG}-SH.

The reaction conditions are the same as in the third step of the Raw Material Synthesis 4.

### (Third Step)

This step is a method for producing a compound (39) by an ipso substitution reaction of the compound (38) and PG⁵-OH.

The reaction conditions are the same as in the third step of the Raw Material Synthesis 4.

### (Fourth Step)

This step is a method for producing a compound (41) by an ipso substitution reaction of the compound (39) and a compound (40).

In this reaction, the compound (39) and the compound (40) are used in an equal amount or with one compound thereof in an excess amount, and the mixture of the compounds is stirred in a solvent inactive for the reaction or with no solvent, from under cooling to under reflux with heat, preferably at 0°C to 80°C, generally for 0.1 hours to five days. Examples of the solvent used here include, but are not particularly limited to, a halogenated hydrocarbon, such as dichloromethane, 1,2-dichloroethane and chloroform, an aromatic hydrocarbon, such as benzene, toluene and xylene, an ether, such as diethyl ether, THF, DOX and 1,2-dimethoxyethane, DMF, DMAc, DMSO, ethyl acetate, MeCN and a mixture thereof. Performing the reaction in the presence of an organic base such as TEA, DIPEA, N-methylmorpholine (NMM), 1,4-diazabicyclo[2.2.2]octane (DABCO) and tBuOK, or an inorganic base, such as sodium hydride, potassium carbonate, sodium carbonate and cesium carbonate, is sometimes advantageous for smoothly promoting the reaction.

### (Fifth Step)

This step is a method for producing a compound (42) by a Suzuki-Miyaura coupling reaction of the compound (41) and a boronic acid derivative composed of an R²-boronic acid group or the like.

The reaction conditions are the same as in the fifth step of the Raw Material Synthesis 4.

### (Sixth Step)

This step is a method for producing a compound (43) by a Suzuki-Miyaura coupling reaction of the compound (42) and the compound (19).

The reaction conditions are the same as in the fifth step of the Raw Material Synthesis 4.

### (Seventh Step)

This step is a method for producing a compound (44) by an oxidation reaction of the compound (43).

The reaction conditions are the same as in the seventh step of the Raw Material Synthesis 4.

### (Eighth Step)

This step is a method for producing a compound (45) by deprotection by a catalytic hydrogenation reaction of the compound (44).

The reaction conditions are the same as in the eighth step of the Raw Material Synthesis 4.

### (Ninth Step)

This step is a method for producing a compound (46) by a reaction of the compound (45) and the compound (23).

The reaction conditions are the same as in the ninth step of the Raw Material Synthesis 4.

### (Tenth Step)

This step is a method for producing the compound (8)-1 by an ipso substitution reaction of the compound (46) and the compound (25).

The reaction conditions are the same as in the third step of the Raw Material Synthesis 4.

### (Raw Material Synthesis 8)

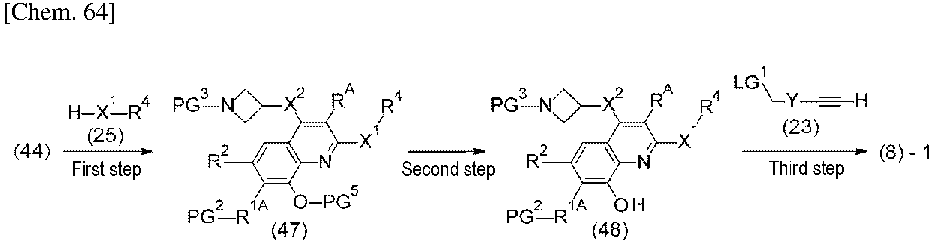

This production method is a method for producing the compound (8)-1 included in the raw material compound (8).

### (First Step)

This step is a method for producing a compound (47) by an ipso substitution reaction of the compound (44) and the compound (25).

The reaction conditions are the same as in the third step of the Raw Material Synthesis 4.

### (Second Step)

This step is a method for producing a compound (48) by deprotection by a catalytic hydrogenation reaction of the compound (47).

The reaction conditions are the same as in the eighth step of the Raw Material Synthesis 4.

### (Third Step)

This step is a method for producing the compound (8)-1 by a reaction of the compound (48) and the compound (23).

### (Raw Material Synthesis 9)

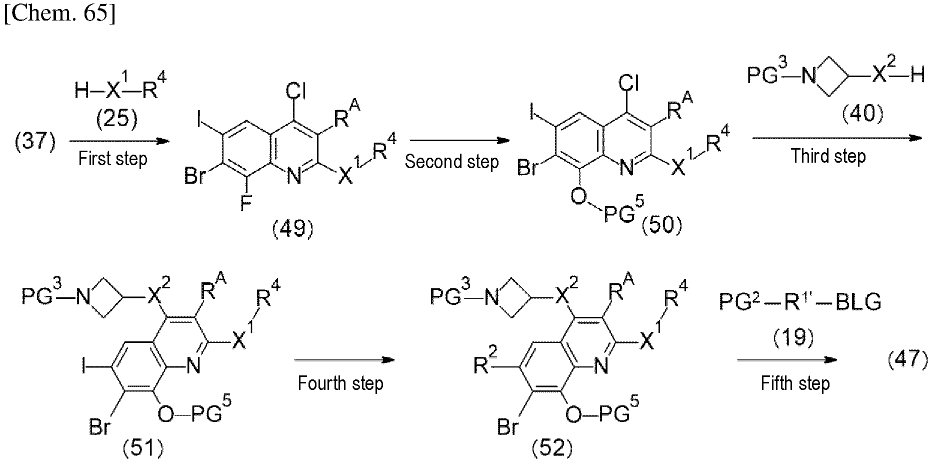

This production method is a method for producing the raw material compound (47).

### (First Step)

This step is a method for producing a compound (49) by an ipso substitution reaction of the compound (37) and the compound (25).

The reaction conditions are the same as in the third step of the Raw Material Synthesis 4.

Moreover, the compound (49) can be produced by Negishi coupling of a compound in which a hydrogen atom of the compound (25) has been converted to halogen and the compound (37).

### (Second Step)

This step is a method for producing a compound (50) by an ipso substitution reaction of the compound (49) and PG⁵-OH.

The reaction conditions are the same as in the third step of the Raw Material Synthesis 4.

### (Third Step)

This step is a method for producing a compound (51) by an ipso substitution reaction of the compound (50) and the compound (40).

The reaction conditions are the same as in the fourth step of the Raw Material Synthesis 7.

### (Fourth Step)

This step is a method for producing a compound (52) by a Suzuki-Miyaura coupling reaction of the compound (51) and a boronic acid derivative composed of an R²-boronic acid group or the like.

The reaction conditions are the same as in the fifth step of the Raw Material Synthesis 4.

### (Fifth Step)

This step is a method for producing the compound (47) by a Suzuki-Miyaura coupling reaction of the compound (52) and the compound (19).

The reaction conditions are the same as in the fifth step of the Raw Material Synthesis 4.

### (Raw Material Synthesis 10)

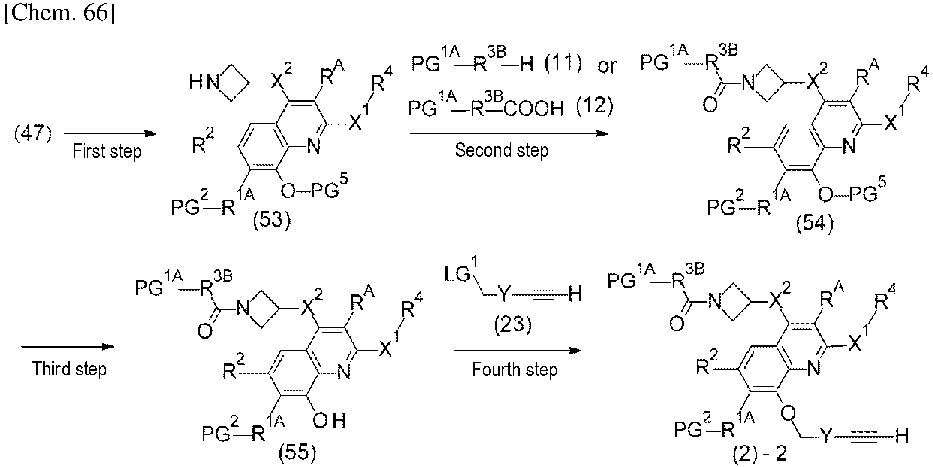

This production method is a method for producing a compound (2)-2 included in the raw material compound (2).

### (First Step)

This step is a method for producing a compound (53) by subjecting the compound (47) to a deprotection reaction.

The reaction conditions are the same as in the deprotection reaction of the Production Method 1.

### (Second Step)

This step is a method for producing a compound (54) by adding the compound (11) after a reaction of the compound (53) and a carbonylation reagent or a method for producing the compound (54) by an amidation reaction of the compound (53) and the compound (12).

The reaction conditions are the same as in the third step of the Raw Material Synthesis 3.

### (Third Step)

This step is a method for producing a compound (55) by deprotection by a catalytic hydrogenation reaction of the compound (54).

The reaction conditions are the same as in the eighth step of the Raw Material Synthesis 4.

### (Fourth Step)

This step is a method for producing the compound (2)-2 by a reaction of the compound (55) and the compound (23).

The reaction conditions are the same as in the ninth step of the Raw Material Synthesis 4.

### (Raw Material Synthesis 11)

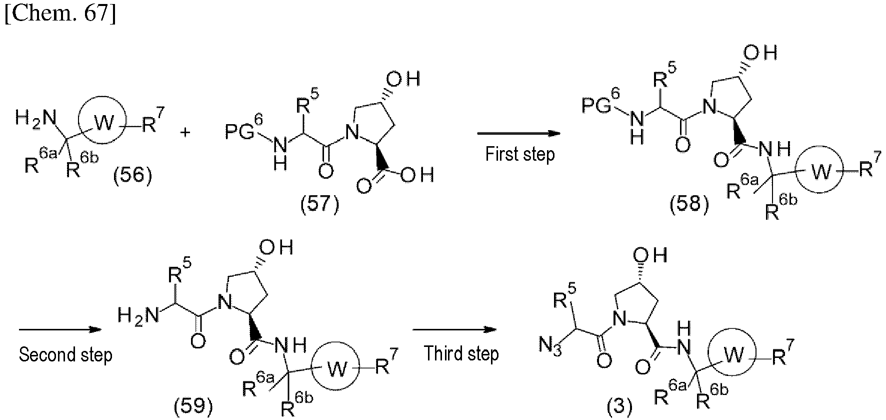

(In the formulae, PG⁶ represents a protective group.)

This production method is a method for producing the raw material compound (3).

### (First Step)

This step is a method for producing a compound (58) by an amidation reaction of a compound (56) and a compound (57).

The reaction conditions are the same as in the third step of the Raw Material Synthesis 2.

### (Second Step)

This step is a method for producing a compound (59) by subjecting the compound (58) to a deprotection reaction.

The reaction conditions are the same as in the step described in the Production Method 1.

### (Third Step)

This step is a method for producing the compound (3) by a reaction of the compound (59) and a diazo-transfer reagent.

In this reaction, the compound (59) is treated with the diazo-transfer reagent in an equal amount or an excess amount in a solvent inactive for the reaction, from under cooling to under heating, preferably at 0°C to 50°C, generally for 0.1 hours to three days. Examples of the diazo-transfer reagent include, but are not particularly limited to, for example, trifluoromethanesulfonyl azide, imidazole-1-sulfonyl azide or a salt thereof, 2-azido-1,3-dimethylimidazolinium hexafluorophosphate (ADMP) and the like. Performing the reaction in the presence of an organic base, such as TEA, 4-dimethylaminopyridine (DMAP) and 2,6-lutidine, and a catalytic amount of a copper salt, such as CuSO₄, is sometimes advantageous. Examples of the solvent include THF, a halogenated hydrocarbon such as dichloromethane, MeCN, an alcohol, water and a mixture thereof.

### [Reference]

J. Org. Chem. 2012, 77, p.1760-1764
Nature 2019, 574, p.86-89
Org. Biomol. Chem. 2014, 12, p.4397-4406

### (Raw Material Synthesis 12)

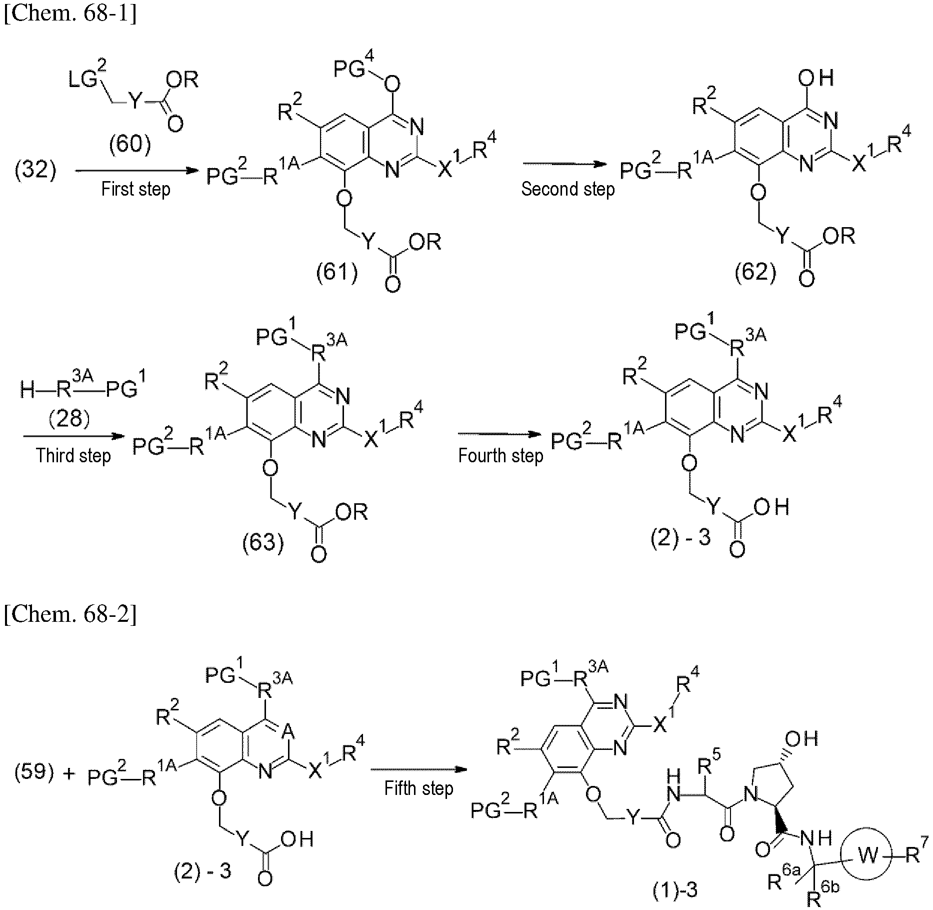

(In the formula, LG² represents a leaving group.)

This production method is a method for producing a raw material compound (1)-3 included in the raw material compound (1).

### (First Step)

This step is a method for producing a compound (61) by a reaction of the compound (32) and a compound (60).

The reaction conditions are the same as in the ninth step of the Raw Material Synthesis 4.

### (Second Step)

This step is a method for producing a compound (62) by subjecting the compound (61) to a deprotection reaction.

The reaction conditions are the same as in the step described in the Production Method 1.

### (Third Step)

This step is a method for producing a compound (63) by a reaction of the compound (62) and the compound (28).

The reaction conditions are the same as in the twelfth step of the Raw Material Synthesis 4.

### (Fourth Step)

This step is a method for producing a compound (2)-3 by hydrolysis of the compound (63).

The reaction conditions are the same as in the second step of the Raw Material Synthesis 2.

### (Fifth Step)

This step is a method for producing the compound (1)-3 by an amidation reaction of the compound (2)-3 and the compound (59).

The reaction conditions are the same as in the third step of the Raw Material Synthesis 2.

### (Raw Material Synthesis 13)

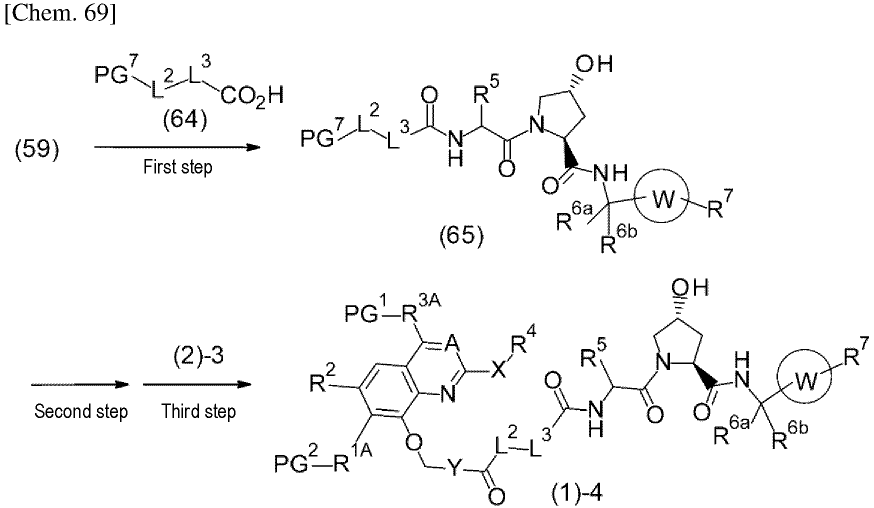

(In the formulae, PG⁷ represents a protective group of NH.)

This production method is a method for producing a raw material compound (1)-4 included in the raw material compound (1). Here, a production method of the raw material compound (1)-4 in which L² is NR^{L1}, pyrrolidinediyl, piperidinediyl or piperazinediyl is shown.

### (First Step)

This step is a method for producing a compound (65) by an amidation reaction of the compound (59) and a compound (64).

The reaction conditions are the same as in the third step of the Raw Material Synthesis 2.

### (Second Step and Third Step)

These steps are a method for producing the compound (1)-4 by an amidation reaction of a compound obtained by a deprotection reaction of the compound (65) and the compound (2)-3.

The reaction conditions of the deprotection reaction are the same as in the step described in the Production Method 1.

The reaction conditions of the amidation reaction are the same as in the third step of the Raw Material Synthesis 2.

### (Raw Material Synthesis 14)

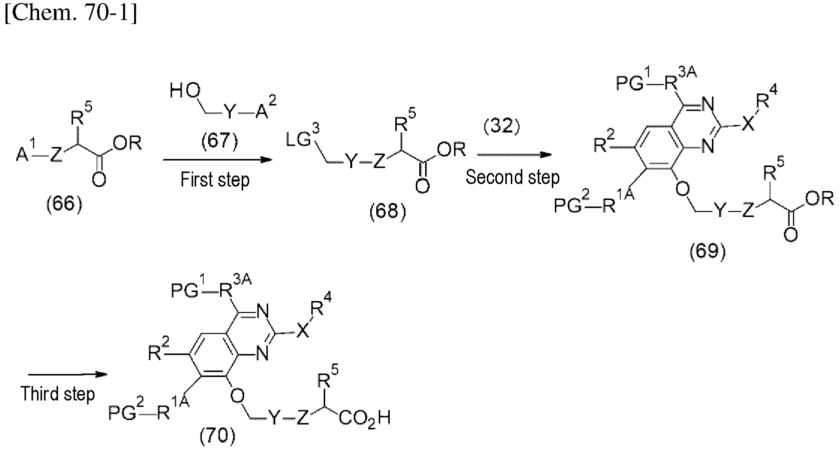

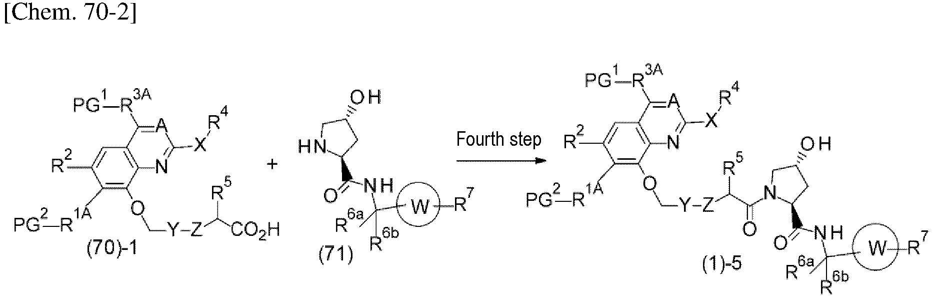

(In the formulae, LG³ represents a leaving group. When Z is NH, A¹ represents a hydrogen atom, and A² represents halogen. When Z is 5-membered heteroarenediyl containing one to four hetero atoms selected from the group consisting of oxygen, sulfur and nitrogen as ring-constituting atoms, A² represents a boronic acid group or the like in the case where A¹ is a group selected from the group consisting of Cl, Br and I, and A² represents a group selected from the group consisting of Cl, Br and I in the case where A¹ is a boronic acid group or the like.)

This production method is a method for producing a raw material compound (1)-5 included in the raw material compound (1).

### (First Step)

This step is a method for producing a compound (68) by an ipso reaction or a Buchwald-Hartwig amination reaction of a compound (66) and a compound (67) when Z is NH.

The reaction conditions of the ipso reaction are the same as in the fourth step of the Raw Material Synthesis 7.

For example, the following can be referred as a reference about the Buchwald-Hartwig amination reaction.
J. Am. Chem. Soc., 2020, 142, p.15027-15037

Moreover, this step is a method for producing the compound (68) by a Suzuki-Miyaura coupling reaction of the compound (66) and the compound (67) when Z is 5-membered heteroaryl containing one to four hetero atoms selected from the group consisting of oxygen, sulfur and nitrogen as ring-constituting atoms.

The reaction conditions are the same as in the fifth step of the Raw Material Synthesis 4.

For example, the following can be referred as a reference about the reaction in which Z is the formula (XXIX) here.
J. Org. Chem., 2000, 65, p.1516-1524
Chemical Communications 2014, 50, p.1867-1870
Bioorg. Med. Chem. Lett., 2001, 11, p.2061-2065

### (Second Step)

This step is a method for producing a compound (69) by a reaction of the compound (68) and the compound (32).

The reaction conditions are the same as in the ninth step of the Raw Material Synthesis 4.

Moreover, in this step, the compound (69) can also be produced by a Mitsunobu reaction of a compound in which the moiety corresponding to LG³ of the compound (68) is a hydroxy group and the compound (32).

For example, the following can be referred as a reference about the Mitsunobu reaction here.
Chem. Asian J. 2007, 2, p.1340 - 1355

### (Third Step)

This step is a method for producing a compound (70) by hydrolysis of the compound (69).

The reaction conditions are the same as in the second step of the Raw Material Synthesis 2.

### (Fourth Step)

This step is a method for producing the compound (1)-4 by an amidation reaction of a compound (70)-1 and a compound (71).

The reaction conditions are the same as in the third step of the Raw Material Synthesis 2.

### (Raw Material Synthesis 15)

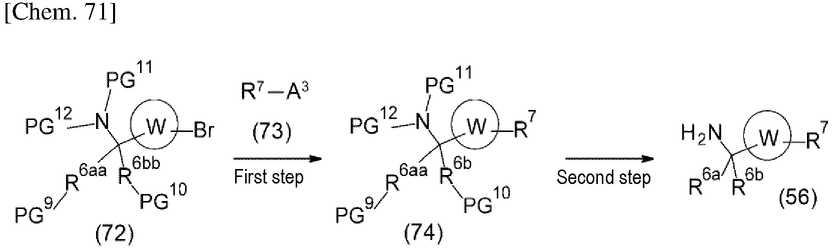

(In the formulae, R^{6aa} and R^{6bb} represent divalent groups in which H has been removed from a functional group of R^{6a} and R^{6b} into which a protective group can be introduced. PG⁹, PG¹⁰ and PG¹¹, which are the same as or different from each other, represent a hydrogen atom or a protective group, and PG¹² represents a protective group. A³ represents a hydrogen atom, a carboxyl group, a boronic acid group or the like.)

This production method is a method for producing the raw material compound (56).

### (First Step)

This step is a method for producing a compound (74) by a Mizoroki-Heck reaction of a compound (73) in which R⁷ is the formula (XXIII) for example and a compound (72) when A³ is a hydrogen atom.

In this reaction, the compound (72) and the compound (73) are used in an equal amount or with one compound thereof in an excess amount, and the mixture of the compounds is stirred in a solvent inactive for the reaction, in the presence of a base and a palladium catalyst, from at room temperature to under reflux with heat, preferably at 20°C to 140°C, generally for 0.1 hours to five days. Examples of the solvent used here include, but are not particularly limited to, an ether, such as diethyl ether, THF, DOX and 1,2-dimethoxyethane, DMF, DMAc, DMSO, MeCN, 1,3-dimethylimidazolidin-2-one, ethyl acetate, water and a mixture thereof. The base is a base, such as tripotassium phosphate, sodium carbonate, potassium carbonate and potassium acetate. The palladium catalyst is tetrakis(triphenylphosphine)palladium, bis(triphenylphosphine)palladium(II) dichloride, [1,1'-bis(diphenylphosphino)ferrocene]palladium(II) dichloride · dichloromethane adduct, (1E,4E)-1,5-diphenylpenta-1,4-dien-3-one/palladium (3:2), (2-dicyclohexylphosphino-2',6'-diisopropoxy-1,1'-biphenyl)[2-(2'-amino-1,1'-biphenyl)]palla dium(II) methanesulfonate, palladium(II) acetate or the like. In addition, heating the mixture by microwave irradiation is sometimes advantageous for smoothly promoting the reaction.

For example, the following can be referred as a reference about the reaction.
Synthesis 2020, 52, p.2521-2527
PNAS 2016, 113, p.7124-7129

Alternatively, this is a method for producing the compound (74) by an Ullmann reaction of the compound (73) in which R⁷ is a group selected from the group consisting of the formula (XXVI), the formula (XXVIII) and the formula (XXXIII) for example and the compound (72).

For example, the following can be referred as a reference about the reaction.
Angew. Chem. Int. Ed., 2003, 42, p.5400-5449

Moreover, this step is a method for producing the compound (74) by a decarbonation coupling reaction of the compound (73) in which R⁷ is the formula (XXIV) for example and the compound (72) when A³ is a carboxyl group.

For example, the following can be referred as a reference about the reaction.
Science, 2006, 313, p.662-664

Furthermore, this step is a method for producing the compound (74) by a Suzuki-Miyaura coupling reaction of the compound (73) in which R⁷ is the formula (XXIII) for example and the compound (72) when A³ is a boronic acid group or the like.

The reaction conditions are the same as in the fifth step of the Raw Material Synthesis 4.

### (Second Step)

This step is a method for producing the compound (56) by subjecting the compound (74) to a deprotection reaction.

The reaction conditions are the same as in the step described in the Production Method 1.

### (Raw Material Synthesis 16)

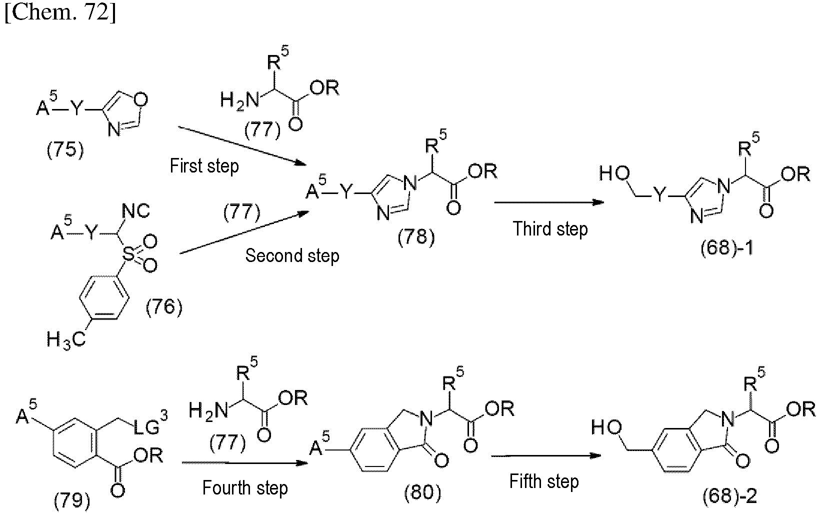

(In the formulae, A⁵ represents a group selected from the group consisting of Cl, Br, mesylate and triflate, and LG³ represents a leaving group.)

This production method is a method for producing a raw material compound (68)-1 and a raw material compound (68)-2 included in the raw material compound (68).

### (First Step)

This step is a method for producing a compound (78) by a reaction of a compound (75) and a compound (77).

For example, the following can be referred as a reference about the reaction.
Chem. Commun., 2014, 50, 15, p.1867-1870

### (Second Step)

This step is a method for producing the compound (78) by a reaction of the compound (77) and glyoxylic acid using a toluenesulfonylmethyl isocyanide (TosMIC) reagent substituted with aryl.

For example, the following can be referred as a reference about the reaction.
J. Org. Chem., 2000, 65, 5, p.1516-1524
J. Am. Chem. Soc., 2007, 129, 3, p.490-491

### (Third Step)

This step is a method for producing the compound (68)-1 by a Suzuki-Miyaura coupling reaction of the compound (78) and an alkoxymethyl boronic acid derivative and by subsequently subjecting to a deprotection reaction under acidic conditions.

The reaction conditions of the Suzuki-Miyaura coupling reaction are the same as in the fifth step of the Raw Material Synthesis 4.

An example of the alkoxymethyl boronic acid derivative used here is potassium (2-trimethylsilyl)-ethoxymethyl trifluoroborate.

For example, the following can be referred as a reference about the reaction.
Org. Lett., 2008, 10, 11, p.2135-2128
Org. Lett., 2011, 13, 15, p.3948-3951

The reaction conditions of the deprotection reaction under the acidic conditions performed subsequently are the same as in the step described in the Production Method 1. Examples of the acid used here include trifluoroacetic acid and the like.

For example, the following can be referred as a reference about the deprotection reaction.
P. G. M. Wuts and T. W. Greene, "Greene's Protective Groups in Organic Synthesis", 5th edition, John Wiley & Sons Inc., 2014

Moreover, in this step, the compound (68)-1 can be produced by a Suzuki-Miyaura coupling reaction of the compound (78) and an acetoxymethyl boronic acid derivative.

For example, the following can be referred as a reference about the reaction.
Org. Lett., 2012, 14, 5, p.1278-1281

### (Fourth Step)

This step is a method for producing a compound (80) by a reaction of a compound (79) and the compound (77).

This reaction is performed using the compound (79) and the compound (77) in an equal amount or with one compound thereof in an excess amount by stirring the mixture of the compounds in a solvent inactive for the reaction or with no solvent, from under cooling to under reflux with heat, preferably from at room temperature to under reflux with heat, generally for 0.1 hours to five days. Examples of the solvent used here include, but are not particularly limited to, an aromatic hydrocarbon, such as toluene, an ether, such as THF and DOX, a halogenated hydrocarbon, such as dichloromethane, DMF, DMSO, ethyl acetate, MeCN and the like. Performing the reaction in the presence of an organic base, such as TEA, DIPEA and NMM, or an inorganic base, such as potassium carbonate, sodium carbonate and cesium carbonate, is sometimes advantageous for smoothly promoting the reaction.

### (Fifth Step)

This step is a method for producing the compound (68)-2 by a Suzuki-Miyaura coupling reaction of the compound (80) and an alkoxymethyl boronic acid derivative and by subsequently subjecting to a deprotection reaction under acidic conditions.

The reaction conditions are the same as in the third step of the Raw Material Synthesis 16.

Moreover, in this step, the compound (68)-2 can be produced by a Suzuki-Miyaura coupling reaction of the compound (80) and an acetoxymethyl boronic acid derivative.

The compound of the formula (I) is isolated and purified as a free compound, a salt, hydrate, solvate or crystal polymorphous substance thereof or a substance in amorphous solid form. A salt of the compound of the formula (I) can also be produced by subjecting the compound to a salt formation reaction which is an ordinary method.

The isolation and purification are performed by applying a common chemical operation, such as extraction, fractional crystallization and various types of fraction chromatography.

Various types of isomers can be produced by selecting an appropriate raw material compound or can be separated by using a difference in physiochemical properties between the isomers. For example, an optical isomer can be obtained by a general optical resolution method of a racemate (for example, fractional crystallization for inducing to a diastereomer salt with an optically active base or acid, chromatography using a chiral column or the like and the like) and can also be produced from an appropriate optically active raw material compound.

In addition, the compound of the formula (I) or an intermediate thereof sometimes has an axial chirality and is obtained as a mixture of stereoisomers, and each stereoisomer can be isolated by separation using a common separation operation, for example, octadecylsilyl (ODS) column chromatography or silica gel column chromatography.

The pharmacological activities of the compounds of the formula (I) were confirmed by the following tests.

Test Example 1-1: Evaluation of RAS G12V degradation activity on human KRAS G12V mutant-positive pancreatic cancer line PA-TU-8902

The RAS G12V degradation activities of subject compounds were evaluated by measuring the expression levels of RAS G12V by a Cell ELISA method.

PA-TU-8902 cells (DSMZ, ACC 179) were seeded at 36 µL per well on a 384-well plate (from Greiner bio-one) to give 1.5 x 10⁴ cells per well. As for the cell culture conditions, DMEM medium (from Sigma-Aldrich) containing 10% fetal bovine serum (from Cytiva) was used in the presence of 5% CO₂ at 37°C.

The next day, the subject compounds (10 points having final concentrations in the range of 3 µM to 0.1 nM), Example compound 17 of a final concentration of 1 µM as a positive control and dimethyl sulfoxide (DMSO), which was the solvent for the subject compounds, as a negative control were diluted 100-fold with a fresh medium and were each added at 4 µL per well, followed by culturing for 24 hours.

The next day, the culture supernatant was removed, and a 4% paraformaldehyde phosphate buffer solution (from FUJIFILM Wako) was added at 20 µL per well. The plate was allowed to stand for 30 minutes at room temperature to thus immobilize the cells. Then, the supernatant was removed, and 0.1% Triton X-100 (from Amersham Biosciences)-containing Phosphate buffered saline (PBS) was added at 20 µL per well.
After allowing the plate to stand at room temperature for 10 minutes, the supernatant was removed, and the wells were washed twice in total by adding PBS at 25 µL per well and removing the supernatant. The washing operations below were performed in the same manner. Next, the supernatant was removed, and 0.5% sodium dodecyl sulfate (SDS; from ThermoFisher Scientific)-containing PBS was added at 20 µL per well. After allowing the plate to stand at room temperature for 10 minutes, the plate was centrifuged to remove the supernatant. After washing with PBS, the supernatant was removed by centrifugation, and a blocking solution (PVDF Blocking Reagent for Can Get Signal; from TOYOBO) was added at 20 µL per well. After allowing the plate to stand for 30 minutes at room temperature, the supernatant was removed by centrifugation, and a solution obtained by diluting an anti-Ras (G12V Mutant Specific) antibody (Ras (G12V Mutant Specific) (D2H12) Rabbit mAb; from Cell Signaling Technology; 500-fold dilution) and an anti-β-actin antibody (Anti-beta Actin antibody; from Abcam; 5,000-fold dilution) with Can Get Signal Solution 2 (from TOYOBO) was added at 15 µL per well as primary antibodies. The plate was allowed to stand overnight at 4°C.

The next day, the supernatant was removed by centrifugation, and the plate was washed with PBS. The supernatant was removed by centrifugation, and a solution obtained by diluting an anti-rabbit IgG antibody (IRDye 800CW Goat anti-Rabbit IgG; from LI-COR Biosciences) and an anti-mouse IgG antibody (IRDye 680RD Donkey anti-Mouse IgG; from LI-COR Biosciences) 1,000-fold with Can Get Signal Solution 2 was added at 15 µL per well as secondary antibodies. After allowing the plate to stand at room temperature for an hour, the supernatant was removed by centrifugation, and the plate was washed with PBS. After removing the supernatant, the plate was dried as it was with air at room temperature for two hours or more, and the 700-nm and 800-nm fluorescent signals were measured with Aerius (from LI-COR Biosciences).

With the RAS G12V signaling value at the time of addition of DMSO corrected with the signaling value of β-actin taken as 0% and with the RAS G12V signaling value at the time of addition of Example compound 17 of a final concentration of 1 µM taken as 100%, the RAS G12V degradation rates were calculated. The 50% degradation values (DC₅₀) were calculated by Sigmoid-Emax model nonlinear regression analysis. Dmax shows the degradation rate at the compound concentration which showed the highest degradation activity. The results of some subject compounds of the formula (I) are shown in the table below.

**[Table 1-1]**

| Ex | DC₅₀ (nM) | Ex | DC₅₀ (nM) |
|---|---|---|---|
| 1 | 31 | 18 | 69 |
| 2 | 18 | 19 | 26 |
| 4 | 45 | 20 | 48 |
| 5 | 26 | 21 | 35 |
| 6 | 22 | 23 | 112 (Dmax=62%) |
| 9 | 21 | 25 | 14 |
| 11 | 13 | 26 | 19 |
| 12 | 37 | 29 | 8 |
| 13 | 11 | 35 | 23 |
| 14 | 10 | 40 | 76 |
| 15 | 89 | 41 | 30 |
| 17 | 41 | 42 | 30 |

Test Example 1-2: Evaluation of RAS G12D degradation activity on human KRAS G12D mutant-positive pancreatic cancer line AsPC-1

The RAS G12D degradation activities of subject compounds were evaluated in the same manner as in Test Example 1-1 by measuring the expression levels of RAS G12D by a Cell ELISA method.

AsPC-1 cells (ATCC, CRL-1682) were seeded at 36 µL per well on a 384-well plate to give 2.0 x 10⁴ cells per well. As for the cell culture conditions, RPMI-1640 medium (from Sigma-Aldrich) containing 10% fetal bovine serum was used in the presence of 5% CO₂ at 37°C.

The next day, the subject compounds (10 points having final concentrations in the range of 3 µM to 0.1 nM) and DMSO, which was the solvent for the subject compounds, as a negative control were diluted 100-fold with a fresh medium and were each added at 4 µL per well, followed by culturing for 24 hours.

The next day, the procedures from the immobilization of the cells to before the addition of the blocking solution were performed by the same methods as in Test Example 1-1. A blocking solution (Intercept Blocking Buffer; from LI-COR Biosciences) was added at 20 µL per well. After allowing the plate to stand for 30 minutes at room temperature, the supernatant was removed by centrifugation. A solution obtained by diluting an anti-β-actin antibody (1,000-fold dilution) with the blocking solution was added to the wells of the positive control as a primary antibody, and a solution obtained by diluting an anti-Ras (G12D Mutant Specific) antibody (Ras (G12D Mutant Specific) (D8H7) Rabbit mAb; from Cell Signaling Technology; 1,000-fold dilution) and an anti-β-actin antibody (1,000-fold dilution) with the blocking solution was added to the other wells as primary antibodies, each at 15 µL per well. The plate was allowed to stand overnight at 4°C.

The next day, the supernatant was removed by centrifugation, and the plate was washed with PBS. The supernatant was removed by centrifugation, and a solution obtained by diluting an anti-rabbit IgG antibody and an anti-mouse IgG antibody 1,000-fold with the blocking solution was added at 15 µL per well as secondary antibodies. After allowing the plate to stand at room temperature for an hour, the supernatant was removed by centrifugation, and the plate was washed with PBS. After removing the supernatant, the plate was dried as it was with air at room temperature for two hours or more, and the 700-nm and 800-nm fluorescent signals were measured with Aerius.

With the RAS G12D signaling value at the time of addition of DMSO corrected with the signaling value of β-actin taken as 0% and with the RAS G12D signaling value under the conditions of staining with the anti-β-actin antibody alone taken as 100%, the RAS G12D degradation rates were calculated. The 50% degradation values (DC₅₀) were calculated by Sigmoid-Emax model nonlinear regression analysis. The results of some subject compounds of the formula (I) are shown in the table below.

**[Table 1-2]**

| Ex | DC₅₀ (nM) | Ex | DC₅₀ (nM) |
|---|---|---|---|
| 1 | 5 | 18 | 26 |
| 2 | 5 | 19 | 4 |
| 4 | 20 | 20 | 16 |
| 5 | 6 | 21 | 8 |
| 6 | 5 | 23 | 1 |
| 9 | 5 | 25 | 3 |
| 11 | 4 | 26 | 6 |
| 12 | 3 | 29 | 21 |
| 13 | 6 | 35 | 16 |
| 14 | 2 | 40 | 47 |
| 15 | 146 | 41 | 3 |
| 17 | 11 | 42 | 6 |

Test Example 2: Evaluation of ERK phosphorylation inhibitory activity on human KRAS G12V mutant-positive pancreatic cancer line PA-TU-8902

The ERK phosphorylation inhibitory activities of subject compounds were evaluated by measuring phosphorylation of the 202th threonine (Thr202) and the 204th tyrosine (Tyr204) of ERK located downstream of the KRAS signal by cell ELISA.

PA-TU-8902 cells were seeded at 36 µL/well on a 384-well plate (from Greiner bio-one) to give 5.0 x 10³ cells per well. The cell culture conditions were the same conditions as in Test Example 1-1.

The next day, the subject compounds (9 points having final concentrations in the range of 3 µM to 0.3 nM), trametinib (MEK inhibitor) of a final concentration of 300 nM as a positive control and DMSO, which was the solvent for the subject compounds, as a negative control were diluted 100-fold with a fresh medium and were each added at 4 µL per well, followed by culturing for 24 hours. After culturing, a 30% glyoxal solution (40% glyoxal [from Nacalai Tesque] was diluted with PBS) was quickly added at 30 µL per well, and the plate was allowed to stand for an hour and 30 minutes at room temperature to thus immobilize the cells. Then, the plate was centrifuged (110 x g, seven seconds, hereinafter centrifugation was performed under the same conditions unless otherwise specified) to remove the supernatant, and 0.1% Triton X-100-containing PBS was added at 20 µL per well. After allowing the plate to stand for 10 minutes at room temperature, the supernatant was removed by centrifugation, and the same operation was further repeated. Next, 0.5% SDS-containing PBS was added at 20 µL per well. The plate was allowed to stand at room temperature for 30 minutes and was then centrifuged to remove the supernatant. Subsequently, a blocking solution (Intercept Blocking Buffer) was added at 20 µL per well, and the plate was allowed to stand for an hour at room temperature. The supernatant was removed by centrifugation, and an ERK (Thr202/Tyr204) phosphorylation antibody (Phospho-p44/42 MAPK (Erk 1/2) (Thr202/Tyr204) (D13.14.4E) XP Rabbit mAb; from Cell Signaling Technology) diluted 2,500-fold with the blocking solution was added at 15 µL per well as a primary antibody. The plate was allowed to stand at 4°C overnight.

The next day, the plate was centrifuged to remove the supernatant, and 0.05% Tween-20-containing PBS (from Thermo Scientific; 20x PBS Tween-20 was diluted 20-fold with ion exchange water and used) was added at 50 µL per well. The supernatant was removed by centrifugation to thus wash each well. The washing was performed three times in total. After washing, an anti-rabbit IgG antibody diluted 1,000-fold with the blocking solution was added at 15 µL per well as a secondary antibody, and the plate was allowed to stand for an hour at room temperature. The plate was centrifuged to remove the supernatant, and each well was washed three times with 0.05% Tween-20-containing PBS in the same manner as after the primary antibody reaction. Centrifugation after the third washing was performed at 171 × g for 17 seconds. After removing the supernatant, the plate was dried as it was with air at room temperature for three hours or more, and the 800-nm fluorescent signals were measured with Aerius.

With the signaling value at the time of addition of DMSO taken as 0% inhibition and with the signaling value at the time of addition of 300 nM trametinib taken as 100% inhibition, the 50% inhibition values (IC₅₀) were calculated by Sigmoid-Emax model nonlinear regression analysis. The results of some subject compounds of the formula (I) are shown in the table below.

**[Table 2]**

| Ex | IC₅₀ (nM) | Ex | IC₅₀ (nM) |
|---|---|---|---|
| 1 | 19 | 18 | 33 |
| 2 | 16 | 19 | 31 |
| 4 | 48 | 20 | 34 |
| 5 | 8 | 21 | 19 |
| 6 | 7 | 23 | 107 |
| 9 | 12 | 25 | 13 |
| 11 | 14 | 26 | 15 |
| 12 | 18 | 29 | 15 |
| 13 | 9 | 35 | 11 |
| 14 | 10 | 40 | 53 |
| 15 | 42 | 41 | 24 |
| 17 | 23 | 42 | 14 |

Test Example 3: Evaluation of non-anchorage-dependent cell growth inhibitory activity on human KRAS G12V mutant-positive pancreatic cancer line PA-TU-8902, human KRAS G12D mutant-positive pancreatic cancer line AsPC-1 or human KRAS G12C mutant-positive pancreatic cancer line MIA PaCa-2

The non-anchorage-dependent cell growth inhibitory activities of subject compounds were evaluated by spheroid 3D cell culture.

PA-TU-8902 cells, AsPC-1 cells or MIA PaCa-2 cells (RIKEN BRC, RCB2094) were seeded at 36 µL/well on a low-cell-adhesive round bottom 384-well plate (Prime Surface: from Sumitomo Bakelite) to give 5 x 10² cells per well. The PA-TU-8902 cells were under the same conditions as in Test Example 1-1, and the AsPC-1 cells were under the same conditions as in Test Example 1-2. For the MIA PaCa-2 cells, RPMI-1640 medium containing 10% fetal bovine serum was used in the presence of 5% CO₂ at 37°C.

The next day, the subject compounds (6-9 points having final concentrations in the range of 3 µM to 0.3 nM) and DMSO, which was the solvent for the subject compounds, as a negative control were diluted 100-fold with a fresh medium and were each added at 4 µL per well. After culturing in the presence of 5% CO₂ at 37°C for six days, CellTiter-Glo 2.0 (from Promega) was added at 20 µL per well. After stirring with a plate mixer (from FINEPCR) at normal temperature for an hour, the luminescent signals were measured with ARVO X3 (from PerkinElmer).

With the signaling value in treatment with DMSO taken as 0% inhibition and with the signaling value in the medium alone without cells taken as 100% inhibition, the 50% inhibition values (IC₅₀) were calculated by Sigmoid-Emax model nonlinear regression analysis. The results of some subject compounds of the formula (I) are shown in the table below.

**[Table 3]**

| Ex | PA-TU-8902 IC₅₀ (nM) | AsPC-1 IC₅₀ (nM) | MIA PaCa-2 IC₅₀ (nM) |
|---|---|---|---|
| 1 | 57 | 28 | 11 |
| 2 | 33 | 11 | 12 |
| 3 | 160 | N.T. | N.T. |
| 4 | 72 | 38 | 22 |
| 5 | 29 | 8 | 5 |
| 6 | 32 | 14 | 7 |
| 7 | 81 | N.T. | N.T. |
| 8 | 102 | N.T. | N.T. |
| 9 | 53 | 19 | 9 |
| 10 | 69 | N.T. | N.T. |
| 11 | 45 | 11 | 13 |
| 12 | 36 | 17 | 20 |
| 13 | 36 | 18 | 13 |
| 14 | 47 | 19 | 10 |
| 15 | 161 | 126 | 6 |
| 16 | 261 | N.T. | N.T. |
| 17 | 46 | 16 | 10 |
| 18 | 69 | 32 | 26 |
| 19 | 58 | 16 | 27 |
| 20 | 63 | 68 | 14 |
| 21 | 60 | 43 | 11 |
| 22 | 65 | N.T. | N.T. |
| 23 | 182 | 9 | 17 |
| 24 | 125 | N.T. | N.T. |
| 25 | 41 | 14 | 16 |
| 26 | 41 | 15 | 7 |
| 27 | 122 | N.T. | N.T. |
| 28 | 159 | N.T. | N.T. |
| 29 | 63 | 25 | 5 |
| 30 | 235 | N.T. | N.T. |
| 31 | 67 | N.T. | N.T. |
| 32 | 105 | N.T. | N.T. |
| 33 | 103 | N.T. | N.T. |
| 34 | 109 | N.T. | N.T. |
| 35 | 32 | 10 | 5 |
| 36 | 143 | N.T. | N.T. |
| 37 | 78 | N.T. | N.T. |
| 38 | 177 | N.T. | N.T. |
| 39 | 52 | N.T. | N.T. |
| 40 | 100 | 62 | 20 |
| 41 | 52 | 29 | 23 |
| 42 | 32 | 10 | 9 |
| 43 | 61 | N.T. | N.T. |

| | | | |
|---|---|---|---|
| N.T.=Not tested. | | | |

Test Example 4: Evaluation of anti-tumor activity in human KRAS G12V mutant-positive pancreatic cancer line PA-TU-8902 or human KRAS G12D mutant-positive pancreatic cancer line PK-59 xenograft mouse

PA-TU-8902 cells or PK-59 cells (RIKEN BRC, RCB1901) were cultured using DMEM medium or RPMI-1640 medium containing 10% fetal bovine serum, respectively, in the presence of 5% CO₂ at 37°C. The PA-TU-8902 cells or the PK-59 cells were collected and suspended in PBS, and two equivalents of VitroGel Hydrogel Matrix (from TheWell Bioscience) was added. A cell suspension prepared at 1.0-2.0 x 10⁷ cells/mL was subcutaneously inoculated in a volume of 100 µL in 4- to 6-week-old male nude mice (BALB/c-nu (nu/nu), from The Jackson Laboratory Japan, Inc.). After about two weeks of the inoculation, the mice were divided into groups so that all the groups had approximately the same tumor volume and body weight, and administration of a subject compound was started on the next day. The test was conducted for five mice for each of a solvent group and a subject compound administration group. The solvent used was a solvent containing ethanol (from FUJIFILM Wako), an aqueous (2-hydroxypropyl)-β-cyclodextrin (HP-βCD) solution (from ROQUETTE), HCO-40 (from Nikko Chemicals Co., Ltd.) dissolved in a 5% glucose solution (from Otsuka Pharmaceutical) at a ratio of 4%, 0.5%, and 9%, respectively. The compound was dissolved using the solvent. The subject compound dissolved in the solvent or the solvent was administered into tail vein. The administration was performed once or twice a week for two weeks. The tumor size and the body weight were measured twice a week. The tumor volume was calculated by using the following formula. [Tumor volume (mm3)] = [Major axis of tumor (mm)] x [Minor axis of tumor (mm)]2 x 0.5

The tumor growth inhibition (%) by the subject compound was calculated with the tumor volume of the subject compound administration group on the previous day of the start of the administration taken as 100% inhibition and the tumor volume of the solvent group of the day of the final measurement taken as 0% inhibition. The results of some subject compounds of the formula (I) are shown in the table below.

**[Table 4-1]**

| Ex | Dose (mg/kg) | Frequency of administration | Anti-tumor activity at final measurement (PA-TU-8902 xenograft mouse) |
|---|---|---|---|
| 1 | 30 | Twice a week | 69% inhibition |
| 5 | 30 | Twice a week | 56% inhibition |
| 11 | 30 | Twice a week | 73% inhibition |
| 12 | 30 | Twice a week | 61% inhibition |
| 13 | 30 | Twice a week | 57% inhibition |

**Table 4-2]**

| Ex | Dose (mg/kg) | Frequency of administration | Anti-tumor activity at final measurement (PK-59 xenograft mouse) |
|---|---|---|---|
| 1 | 10 | Once a week | 68% inhibition |
| 5 | 10 | Once a week | 92% inhibition |
| 11 | 10 | Once a week | 95% inhibition |
| 12 | 10 | Once a week | 63% inhibition |
| 13 | 10 | Once a week | 78% inhibition |

As a result of the above tests, in some compounds of the formula (I), a G12V mutant KRAS degradation activity and a G12D mutant KRAS degradation activity were found (Test Examples 1-1 and 1-2). Furthermore, in some compounds of the formula (I), an inhibitory activity on phosphorylation of ERK located downstream of the KRAS signal was found (Test Example 2). Moreover, in some compounds of the formula (I), a cell growth inhibitory activity on human G12V mutant KRAS-positive pancreatic cancer line, human G12D mutant KRAS-positive pancreatic cancer line and human G12C mutant KRAS-positive pancreatic cancer line was found (Test Example 3). In addition, in some compounds of the formula (I), an anti-tumor activity in a mouse bearing human G12V mutant KRAS-positive pancreatic cancer line or human G12D mutant KRAS-positive pancreatic cancer line was found (Test Example 4). Accordingly, the compound of the formula (I) can be used for the treatment or the like of pancreatic cancer, in particular, mutant KRAS-positive pancreatic cancer, in particular, G12V mutant, G12D mutant and G12C mutant KRAS-positive pancreatic cancer.

A pharmaceutical composition that contains one or two or more compounds of the formula (I) or salts thereof as active ingredients can be prepared by a usually used method using an excipient usually used in the art, that is, a pharmaceutical excipient, a pharmaceutical carrier or the like.

The administration may be either oral administration with a tablet, pill, capsule, granule, powder, liquid or other agent or parenteral administration with an intraarticular, intravenous, intramuscular or other injection, a transmucosal agent, an inhalant or the like.

As a solid composition for oral administration, a tablet, powder, granular or other agent is used. In such a solid composition, one or two or more active ingredients are mixed with at least one inactive excipient. The composition may contain an inactive additive, for example, a lubricant, a disintegrator, a stabilizer or a dissolution aid, according to an ordinary method. A tablet or pill may be coated with a sugar coating or a film soluble in the stomach or intestine, when needed.

Liquid compositions for oral administration include a pharmaceutically acceptable emulsion, solution, suspension, syrup or elixir agent and the like and contain a generally used inactive diluent, for example, purified water or EtOH. The liquid composition may contain, in addition to the inactive diluent, an adjuvant, such as a solubilizer, a wetting agent and a suspending agent, a sweetening agent, a flavor, a fragrant or a preservative.

The injection agents for parenteral administration include a sterile aqueous or nonaqueous solution, suspension or emulsion agent. Examples of the aqueous solvent include distilled water for injection or physiological saline. An example of the nonaqueous solvent is an alcohol, such as EtOH. Such a composition may further contain an isotonizing agent, a preservative, a wetting agent, an emulsifier, a dispersant, a stabilizer or a dissolution aid. These are sterilized, for example, by filtration through a bacteria keeping filter, incorporation of a microbicide or irradiation. In addition, such a composition can be produced as a sterile solid composition, which is dissolved or suspended in sterile water or a sterile solvent for injection before use.

The transmucosal agent, such as an inhalant or a transnasal agent, is used in a solid, liquid or semi-solid form and can be produced according to a conventionally known method. For example, a known excipient and in addition, a pH modifier, a preservative, a surfactant, a lubricant, a stabilizer, a thickener or the like may be appropriately added. The administration can be performed by using an appropriate device for inhalation or insufflation. For example, the agent can be administered using a known device, such as a metering and administering inhalation device, or an atomizer, as a compound alone or a powder of a mixture formulated, or as a solution or a suspension in combination with a pharmaceutically acceptable carrier. A dry powder inhaler or the like may be for a single administration or multiple administrations, and dry powder or powder-containing capsule can be used. Alternatively, the agent may be used in a form of a pressurized aerosol spray or the like using an appropriate ejection agent, for example, a suitable gas, such as a chlorofluoroalkane or carbon dioxide.

In the case of a common oral administration, the daily dose is appropriately about 0.001 to 100 mg/kg body weight, preferably 0.1 to 30 mg/kg body weight, further preferably 0.1 to 10 mg/kg body weight, and the dose is given at once or is divided into two to four times. In the case of intravenous administration, the daily dose is appropriately about 0.0001 to 10 mg/kg body weight and is given at once or is divided into multiple times in a day. In addition, the daily dose of a transmucosal agent is about 0.001 to 100 mg/kg body weight and is given at once or is divided into multiple times in a day. The dose is appropriately decided depending on the individual case taking the symptom, age, sex and the like into account.

Depending on the route of administration, dosage form, site of administration and types of excipient and additive, the pharmaceutical composition of the present invention contains 0.01 to 100% by weight, in an aspect, 0.01 to 50% by weight, of one or more compounds of the formula (I) or salts thereof which are active ingredients.

The compound of the formula (I) can be used in combination with various therapeutic agents or preventive agents for a disease to which the compound of the formula (I) is considered to have an effectiveness. The combination use may be simultaneous administration or separate administration either sequential or with a desired interval. A simultaneous administration preparation may be a formulated agent or may be separately formulated.

### [Examples]

The production method of the compound of the formula (I) will be explained in further detail below based on the Examples. Note that, the present invention is not to be limited to the compounds described in the following Examples. The production methods of raw material compounds are also shown in the Production Examples. The production method of the compound of the formula (I) is not limited only to the production methods of specific Examples described below, and the compound of the formula (I) can also be produced by a combination of the production methods or a method that is obvious to a person skilled in the art.

Note that, in this specification, a compound is sometimes named by using a naming soft, such as ACD/Name (registered trademark, Advanced Chemistry Development, Inc.).

For the purpose of convenience, the concentration mol/L is shown as M. For example, 1M aqueous sodium hydroxide solution means an aqueous sodium hydroxide solution of 1 mol/L.

The "amorphous solid form" described in this specification includes both a form showing no peak in a powder X-ray diffraction (XRD) pattern and a form having low crystallinity.

### Production Example 1

In THF (310 mL), 7-bromo-2,4-dichloro-8-fluoro-6-iodoquinazoline (30.9 g) was suspended, and sodium hydroxide (1M aqueous solution, 147 mL) was added dropwise under ice cooling to keep the internal temperature at 12°C or lower. The mixture was stirred under ice cooling for two hours. The reaction solution was poured into a conical flask containing hydrogen chloride (1M aqueous solution, 147 mL) and water (700 mL) under ice cooling, and the mixture was stirred for two hours at room temperature. The insoluble matter was taken by filtration while washing it with water and dried at 40°C overnight under reduced pressure, thus obtaining 7-bromo-2-chloro-8-fluoro-6-iodoquinazolin-4-ol (24.6 g) as a solid.

### Production Example 2

Under nitrogen atmosphere, 2-tert-butyl-1,3-diisopropylisourea (73.4 g) was added dropwise over 15 minutes to a mixture of 7-bromo-2-chloro-8-fluoro-6-iodoquinazolin-4-ol (24.6 g) and THF (260 mL) heated to 60°C, and the mixture was stirred at the same temperature for 2.5 hours. The mixture was allowed to cool to room temperature, and the insoluble matter was removed by filtration while washing it with THF (500 mL). The filtrate was concentrated, and MeOH (210 mL) was added to the resulting solid. The mixture was stirred at room temperature for an hour, suspended and washed. The insoluble matter was taken by filtration using MeOH (100 mL), thus obtaining 7-bromo-4-tert-butoxy-2-chloro-8-fluoro-6-iodoquinazoline (23.2 g) as a solid.

### Production Example 3

At room temperature, trimethyl orthoacetate (32 mL) was added to a suspension of 2-amino-4-bromo-3-fluoro-5-iodobenzoic acid (30 g) in N-methyl-2-pyrrolidone (60 mL), and the mixture was stirred under argon atmosphere at 110°C overnight. The reaction mixture was brought to room temperature and then suspended in MeOH added thereto. The insoluble matter was taken by filtration and dried under reduced pressure at 50°C overnight, thus obtaining methyl 2-acetamide-4-bromo-3-fluoro-5-iodobenzoate (21.5 g) as a solid.

### Production Example 4

Under argon atmosphere under ice cooling, lithium bis(trimethylsilyl)amide (1M THF solution, 160 mL) was added using a dropping funnel over 20 minutes to a suspension of methyl 2-acetamide-4-bromo-3-fluoro-5-iodobenzoate (21.5 g) in THF (250 mL). Then, the mixture was stirred under argon atmosphere at 40°C for an hour. Water was added under ice cooling to stop the reaction, and the mixture was diluted with ethyl acetate and water. The organic layer and the aqueous layer were separated by a separation operation, and the organic layer was subjected to extraction twice with water. When hydrogen chloride (1M aqueous solution, 200 mL) was added slowly to the collected aqueous layer under ice cooling, a solid precipitated. The insoluble matter was taken by filtration, washed with water and MeOH and then dried under reduced pressure at 50°C overnight, thus obtaining 7-bromo-8-fluoro-6-iodoquinoline-2,4-diol (17.7 g) as a solid.

### Production Example 5

Under nitrogen atmosphere, DIPEA (30 mL) was added dropwise over five minutes to a suspension of 7-bromo-8-fluoro-6-iodoquinoline-2,4-diol (21.24 g) in phosphoryl chloride (95.2 mL) under ice cooling, and the mixture was stirred at 110°C for two hours. After the reaction mixture was allowed to cool to room temperature, MeCN (100 mL) was added, and the mixture was stirred under ice cooling for 30 minutes. The insoluble matter was taken by filtration using MeCN (100 mL). The resulting solid was suspended in MeCN (50 mL) and ice water (200 mL) and stirred for 30 minutes. The insoluble matter was taken by filtration using water/MeCN (5/1, 250 mL), thus obtaining 7-bromo-2,4-dichloro-8-fluoro-6-iodoquinoline (18.95 g) as a solid.

### Production Example 6

To a suspension of 7-bromo-4-tert-butoxy-2-chloro-8-fluoro-6-iodoquinazoline (29 g) in dichloromethane (300 mL), ethanethiol (5 mL) and DABCO (11 g) were added at room temperature, and the mixture was stirred under argon atmosphere at room temperature overnight. Under ice cooling, water was added to stop the reaction. Chloroform was added, and the organic layer and the aqueous layer were separated by a separation operation. The aqueous layer was subjected to extraction with chloroform three times. The collected organic layer was dried over anhydrous sodium sulfate, filtered and concentrated under reduced pressure, thus obtaining 7-bromo-4-tert-butoxy-2-(ethylsulfanyl)-8-fluoro-6-iodoquinazoline (32 g) as a solid.

### Production Example 7

To a suspension of 7-bromo-4-tert-butoxy-2-chloro-8-fluoro-6-iodoquinazoline (5 g) in DMF (25 mL) and THF (25 mL), 4-hydroxytetrahydropyran (1.45 mL), DABCO (120 mg) and cesium carbonate (7 g) were added at room temperature, and the mixture was stirred under nitrogen atmosphere at room temperature overnight. The reaction mixture was diluted by adding ethyl acetate, and the insoluble matter was removed by filtration through celite (registered trademark). Saturated aqueous ammonium chloride solution was added to the filtrate, followed by extraction with ethyl acetate. The organic layer was washed with water and saturated aqueous sodium chloride solution, then dried over anhydrous magnesium sulfate and filtered. The filtrate was concentrated under reduced pressure, and the resulting residue was purified by silica gel chromatography (chloroform/ethyl acetate), thus obtaining 7-bromo-4-tert-butoxy-8-fluoro-6-iodo-2-[(oxan-4-yl)oxy]quinazoline (3.9 g) as a solid.

### Production Example 8

Under argon atmosphere, DABCO (1.3 g) was added to a suspension of 7-bromo-2,4-dichloro-8-fluoro-6-iodoquinoline (4.4 g) in N-methyl-2-pyrrolidone (45 mL), and after the mixture was stirred at 40°C for two hours, ethanethiol (850 µL) was added. The mixture was stirred at 60°C for four hours. After cooling to room temperature, water (200 mL) was added, and the mixture was stirred at room temperature for 30 minutes. The insoluble matter was taken by filtration and dried under reduced pressure, thus obtaining 7-bromo-4-chloro-2-(ethylsulfanyl)-8-fluoro-6-iodoquinoline (4.33 g) as a solid.

### Production Example 9

To a THF (400 mL) solution of 7-bromo-4-tert-butoxy-2-(ethylsulfanyl)-8-fluoro-6-iodoquinazoline (32 g) and (1S)-1-phenylethan-1-ol (11 mL), tBuOK (10 g) was added under ice cooling, and the mixture was stirred under argon atmosphere, under ice cooling for an hour. Saturated aqueous ammonium chloride solution was added under ice cooling to stop the reaction. Water and ethyl acetate were added, and the organic layer and the aqueous layer were separated. The organic layer was washed with saturated aqueous sodium chloride solution and dried over anhydrous sodium sulfate. After filtration and concentration under reduced pressure, the residue was purified by silica gel column chromatography (hexane/ethyl acetate), thus obtaining 7-bromo-4-tert-butoxy-2-(ethylsulfanyl)-6-iodo-8-[(1S)-1-phenylethoxy]quinazoline (36.6 g) as an oily substance.

### Production Example 12

Under nitrogen atmosphere, 7-bromo-4-chloro-2-(ethylsulfanyl)-8-fluoro-6-iodoquinoline (4.3 g) was dissolved in THF (40 mL), and (1S)-1-phenylethan-1-ol (1.28 mL) was added at room temperature. In an ice salt bath, tBuOK (1.14 g) was added, and the mixture was stirred at the same temperature for three hours. After the reaction mixture was poured to saturated aqueous ammonium chloride solution to which ice and ethyl acetate were added, the mixture was stirred and subjected to extraction with ethyl acetate twice. The combined organic layer was washed with saturated aqueous sodium chloride solution and dried over anhydrous magnesium sulfate. After the drying agent was removed by filtration, the mixture was concentrated under reduced pressure. The residue was purified by silica gel column chromatography (hexane/ethyl acetate), thus obtaining 7-bromo-4-chloro-2-(ethylsulfanyl)-6-iodo-8-[(1S)-1-phenylethoxy]quinoline (4.85 g) as an oily substance.

### Production Example 13

In DMAc (10 mL), 1-(tert-butoxycarbonyl)-3-hydroxyazetidine (1 g) was dissolved, and tBuOK (600 mg) was added under argon atmosphere. The mixture was stirred at room temperature for 10 minutes, and thus an alkoxide solution was prepared. In DMAc (10 mL), 7-bromo-4-chloro-2-(ethylsulfanyl)-6-iodo-8-[(1S)-1-phenylethoxy]quinoline (2.12 g) was dissolved, and the prepared alkoxide solution was added dropwise under argon atmosphere in an ice salt bath. The mixture was stirred at the same temperature for 30 minutes. Water was added to the reaction mixture under ice cooling, and the mixture was stirred at the same temperature for 30 minutes. The insoluble matter was taken by filtration. The resulting solid was purified by silica gel column chromatography (from hexane/chloroform to chloroform/MeOH), thus obtaining tert-butyl 3-({7-bromo-2-(ethylsulfanyl)-6-iodo-8-[(1S)-1-phenylethoxy]quinolin-4-yl}oxy)azetidine-1 -carboxylate (2.48 g) as a foam-like solid.

### Production Example 14

At room temperature, 7-bromo-4-tert-butoxy-2-(ethylsulfanyl)-6-iodo-8-[(1S)-1-phenylethoxy]quinazoline (36.6 g), cyclopropylboronic acid (7.5 g), PdCl₂(dppf)·CH₂Cl₂ (7.6 g), tripotassium phosphate (53 g), MeCN (440 mL) and water (80 mL) were mixed and stirred under argon atmosphere at 90°C for four hours. The reaction solution was brought to room temperature and then diluted with ethyl acetate and water. The organic layer and the aqueous layer were separated, and the organic layer was washed with saturated aqueous sodium chloride solution and dried over anhydrous sodium sulfate. After filtration and concentration under reduced pressure, the residue was purified by silica gel column chromatography (hexane/ethyl acetate), thus obtaining 7-bromo-4-tert-butoxy-6-cyclopropyl-2-(ethylsulfanyl)-8-[(1S)-1-phenylethoxy]quinazoline (22.9 g) as an oily substance.

### Production Example 17

Under argon atmosphere, a mixture of tert-butyl 3-({7-bromo-2-(ethylsulfanyl)-6-iodo-8-[(1S)-1-phenylethoxy]quinolin-4-yl}oxy)azetidine-1 -carboxylate (2.48 g), cyclopropylboronic acid (500 mg), PdCl₂(dppf)·CH₂Cl₂ (300 mg), tripotassium phosphate (2.9 g), MeCN (40 mL) and water (8 mL) was stirred at 90 °C for six hours. After allowing to cool to room temperature, ethyl acetate and water were added, and the mixture was filtered through celite (registered trademark). The filtrate was subjected to extraction with ethyl acetate, and the combined organic layer was washed with saturated aqueous sodium chloride solution and dried over anhydrous magnesium sulfate. After the drying agent was removed by filtration, the mixture was concentrated under reduced pressure. The residue was purified by basic silica gel column chromatography (hexane/chloroform), thus obtaining tert-butyl 3-({7-bromo-6-cyclopropyl-2-(ethylsulfanyl)-8-[(1S)-1-phenylethoxy]quinolin-4-yl}oxy)azet idine-1-carboxylate (2.09 g) as a foam-like solid.

### Production Example 18

To a mixture of 4-bromo-6-fluoro-1H-indazole (235 g), TEA (183 mL) and dichloromethane (1880 mL), 1,1',1"-(chloromethanetriyl)tribenzene (335 g) was added, and the mixture was stirred at 25°C for 16 hours. The reaction mixture was poured to ice water (1.5 L), and the organic layer and the aqueous layer were separated. The aqueous layer was subjected to extraction with dichloromethane (400 mL) three times. After the combined organic layer was dried over anhydrous sodium sulfate, the insoluble matter was removed by filtration, and the filtrate was concentrated under reduced pressure. The resulting residue was pulverized (0°C, two hours) by adding petroleum ether (550 mL), then taken by filtration and dried under reduced pressure, thus obtaining 4-bromo-6-fluoro-2-(triphenylmethyl)-2H-indazole (508.98 g) as a solid.

### Production Example 19

To a mixture of 4-bromo-6-fluoro-2-(triphenylmethyl)-2H-indazole (100 g) in 2-methyltetrahydrofuran (1000 mL), lithium diisopropylamide (2M THF solution, 214.28 mL) was added at -78°C under nitrogen atmosphere, and the mixture was stirred at -78°C for 2.5 hours. Methyl iodide (26.68 mL) was added at -78°C, and the mixture was stirred at 25°C for 2.5 hours. Water (2000 mL) was added to stop the reaction, and extraction with ethyl acetate (800 mL) was performed twice. After the combined organic layer was dried over anhydrous sodium sulfate, the insoluble matter was removed by filtration, and the filtrate was concentrated under reduced pressure. The resulting residue was pulverized by adding ethyl acetate (50 mL)/petroleum ether (50 mL), then taken by filtration and dried under reduced pressure, thus obtaining 4-bromo-6-fluoro-5-methyl-2-(triphenylmethyl)-2H-indazole (81 g) as a solid.

### Production Example 20

To a mixture of 4-bromo-6-fluoro-5-methyl-2-(triphenylmethyl)-2H-indazole (100 g), 4,4,4',4',5,5,5',5'-octamethyl-2,2'-bi-1,3,2-dioxaborolane (61.42 g), triphenylphosphine (10.57 g), potassium acetate (59.34 g) and DOX (1000 mL), palladium(II) acetate (4.52 g) was added under nitrogen atmosphere at room temperature. After the reaction mixture was deaerated and filled with nitrogen gas each three times, the mixture was stirred under nitrogen atmosphere at 100°C for 12 hours. After cooling, water (1500 mL) was added, and extraction with ethyl acetate (900 mL) was performed three times. After the combined organic layer was dried over anhydrous sodium sulfate, the insoluble matter was removed by filtration. Activated carbon (50 g) was added to the resulting solution, and the mixture was stirred at 20°C for an hour and filtered while washing it with ethyl acetate (50 mL) three times. The filtrate was concentrated, and the resulting residue was pulverized by adding MeOH (200 mL), taken by filtration and dried under reduced pressure, thus obtaining 6-fluoro-5-methyl-4-(4,4,5,5-tetramethyl-1,3,2-dioxaborolan-2-yl)-2-(triphenylmethyl)-2H-in dazole (110 g) as a solid.

### Production Example 21

To 7-bromo-4-tert-butoxy-6-cyclopropyl-2-(ethylsulfanyl)-8-[(1S)-1-phenylethoxy]quinazoline (14.21 g), 6-fluoro-5-methyl-4-(4,4,5,5-tetramethyl-1,3,2-dioxaborolan-2-yl)-2-(triphenylmethyl)-2H-in dazole (19.3 g), palladium(II) acetate (0.67 g), dicyclohexyl(2',6'-diisopropoxy-[1,1'-biphenyl]-2-yl)phosphine (2.67 g), anhydrous barium hydroxide (14.6 g), DOX (500 mL) and water (100 mL) were added, and after deaeration and substitution operation with argon gas were performed several times, the mixture was stirred with heating under argon atmosphere at 50°C overnight. The reaction suspension which was allowed to cool was filtered through celite (registered trademark) while washing it with ethyl acetate, and the grey insoluble matter was removed by filtration. After the filtrate was concentrated under reduced pressure to around 1/4, water was added, and extraction with ethyl acetate was performed twice. The collected organic layer was washed with saturated aqueous sodium chloride solution and dried over anhydrous magnesium sulfate. After concentration under reduced pressure, the resulting residue was purified by silica gel column chromatography (hexane/ethyl acetate), thus obtaining 4-tert-butoxy-6-cyclopropyl-2-(ethylsulfanyl)-7-[6-fluoro-5-methyl-2-(triphenylmethyl)-2H-i ndazol-4-yl]-8-[(1S)-1-phenylethoxy]quinazoline (a diastereomer mixture of about 3.3:1 derived from axial chirality, 16.44 g) as a solid.

### Production Example 24

In DOX (100 mL), tert-butyl 3-({7-bromo-6-cyclopropyl-2-(ethylsulfanyl)-8-[(1S)-1-phenylethoxy]quinolin-4-yl}oxy)azet idine-1-carboxylate (4.98 g) was dissolved, and 6-fluoro-5-methyl-4-(4,4,5,5-tetramethyl-1,3,2-dioxaborolan-2-yl)-2-(triphenylmethyl)-2H-in dazole (5.7 g), palladium(II) acetate (238 mg), dicyclohexyl(2',6'-diisopropoxy-[1,1'-biphenyl]-2-yl)phosphine (1 g), barium hydroxide (4.3 g) and water (10 mL) were added at room temperature. The mixture was stirred under argon atmosphere at 50°C for an hour. To the reaction mixture, 6-fluoro-5-methyl-4-(4,4,5,5-tetramethyl-1,3,2-dioxaborolan-2-yl)-2-(triphenylmethyl)-2H-in dazole (1 g) was added at the same temperature, and the mixture was stirred under argon atmosphere at 50°C for an hour. The reaction mixture was allowed to cool to room temperature, and ethyl acetate was added. After filtration through celite (registered trademark) and washing with ethyl acetate, water was added to the filtrate, and extraction with ethyl acetate was performed twice. The combined organic layer was washed with saturated aqueous sodium chloride solution, dried over anhydrous sodium sulfate and filtered, and the filtrate was concentrated. The residue was purified by basic silica gel column chromatography (hexane/chloroform), thus obtaining tert-butyl 3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)azetidine-1-carboxylate (a diastereomer mixture of about 3.5:1 derived from axial chirality, 5.98 g) as a foam-like solid.

### Production Example 25

MeOH (120 mL) was added to tert-butyl 3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)azetidine-1-carboxylate (a diastereomer mixture of about 3.5:1 derived from axial chirality, 5.98 g), and the mixture was stirred at 50°C for an hour and then stirred at room temperature overnight. The insoluble matter, tert-butyl 3-({6-cyclopropyl-2-(ethylsulfanyl)-7-[6-fluoro-5-methyl-2-(triphenylmethyl)-2H-indazol-4-yl]-8-[(1S)-1-phenylethoxy]quinolin-4-yl}oxy)azetidine-1-carboxylate (a diastereomer mixture of about 1:1 derived from axial chirality, 1.82 g) was removed by filtration while washing it with MeOH, and the filtrate was concentrated under reduced pressure, thus obtaining the target tert-butyl 3-({(7M)-6-cyclopropyl-2-(ethylsulfanyl)-7-[6-fluoro-5-methyl-2-(triphenylmethyl)-2H-inda zol-4-yl]-8-[(1S)-1-phenylethoxy]quinolin-4-yl}oxy)azetidine-1-carboxylate (a single diastereomer, 3.43 g) as a foam-like solid.

### Production Example 27

In dichloromethane (300 mL), 4-tert-butoxy-6-cyclopropyl-2-(ethylsulfanyl)-7-[6-fluoro-5-methyl-2-(triphenylmethyl)-2H-i ndazol-4-yl]-8-[(1S)-1-phenylethoxy]quinazoline (a diastereomer mixture of about 3.7:1 derived from axial chirality, 22.6 g) was dissolved, and m-chloroperbenzoic acid (about 30% water content, 15 g) was added (internal temperature: 5-10°C) under ice cooling. The mixture was stirred under nitrogen atmosphere for two hours at room temperature. An aqueous solution (300 mL) of sodium thiosulfate pentahydrate (14 g) and saturated aqueous sodium hydrogen carbonate solution (300 mL) were poured to the reaction mixture under ice cooling, and after the mixture was stirred for 30 minutes at room temperature, extraction with ethyl acetate was performed twice. The combined organic layer was washed with saturated aqueous sodium hydrogen carbonate solution and saturated aqueous sodium chloride solution, dried over anhydrous magnesium sulfate, filtered and concentrated. To the resulting residue, iPrOH (600 mL) was added, and the mixture was stirred at room temperature overnight. The resulting insoluble matter was taken by filtration, washed with iPrOH and dried under reduced pressure, thus obtaining 4-tert-butoxy-6-cyclopropyl-2-(ethanesulfonyl)-7-[6-fluoro-5-methyl-2-(triphenylmethyl)-2H -indazol-4-yl]-8-[(1S)-1-phenylethoxy]quinazoline (a diastereomer mixture of about 1:1 derived from axial chirality, 9.45 g) as a solid. The filtrate was concentrated, thus obtaining 4-tert-butoxy-6-cyclopropyl-2-(ethanesulfonyl)-7-[6-fluoro-5-methyl-2-(triphenylmethyl)-2H -indazol-4-yl]-8-[(1S)-1-phenylethoxy]quinazoline (a single diastereomer with undetermined configuration of axial chirality, 15.5 g) as a foam-like solid.

### Production Example 29

In MeOH (30 mL), tert-butyl 3-({(7M)-6-cyclopropyl-2-(ethylsulfanyl)-7-[6-fluoro-5-methyl-2-(triphenylmethyl)-2H-inda zol-4-yl]-8-[(1S)-1-phenylethoxy]quinolin-4-yl}oxy)azetidine-1-carboxylate (2.93 g) was suspended, and methanesulfonic acid (1.1 mL) was added at room temperature. The mixture was stirred at room temperature for 30 minutes and stirred at 40°C overnight. The reaction mixture was allowed to cool to room temperature, and saturated aqueous sodium hydrogen carbonate solution was added. Extraction with chloroform/iPrOH (9/1) was performed three times, and the combined organic layer was dried over anhydrous sodium sulfate, filtered and concentrated. The residue was dissolved in THF (40 mL), and 4-methylbenzene-1-sulfonic acid monohydrate (740 mg) and 3,4-dihydro-2H-pyran (3 mL) were added at room temperature. The mixture was stirred at room temperature overnight. Basic silica gel was added to the reaction mixture, and after concentration, the residue was purified by basic silica gel column chromatography (hexane/chloroform), thus obtaining (7M)-4-[(azetidin-3-yl)oxy]-6-cyclopropyl-2-(ethylsulfanyl)-7-[6-fluoro-5-methyl-1-(oxan-2-yl)-1H-indazol-4-yl]-8-[(1S)-1-phenylethoxy]quinoline (1.65 g) as an oily substance.

### Production Example 30

In DMF (15 mL), (7M)-4-[(azetidin-3-yl)oxy]-6-cyclopropyl-2-(ethylsulfanyl)-7-[6-fluoro-5-methyl-1-(oxan-2-yl)-1H-indazol-4-yl]-8-[(1S)-1-phenylethoxy]quinoline (1.65 g) was dissolved, and TEA (825 µL) and N-[2-(trimethylsilyl)ethoxycarbonyloxy]succinimide (750 mg) were added under ice cooling. The mixture was stirred under nitrogen atmosphere under ice cooling for 30 minutes. Water was added to the reaction mixture under ice cooling, and after the mixture was stirred at the same temperature for 30 minutes, the resulting powder was taken by filtration and dried under reduced pressure, thus obtaining 2-(trimethylsilyl)ethyl 3-({(7M)-6-cyclopropyl-2-(ethylsulfanyl)-7-[6-fluoro-5-methyl-1-(oxan-2-yl)-1H-indazol-4-yl]-8-[(1S)-1-phenylethoxy]quinolin-4-yl}oxy)azetidine-1-carboxylate (2.11 g) as a solid.

### Production Example 31

In dichloromethane (50 mL), 2-(trimethylsilyl)ethyl 3-({(7M)-6-cyclopropyl-2-(ethylsulfanyl)-7-[6-fluoro-5-methyl-1-(oxan-2-yl)-1H-indazol-4-yl]-8-[(1S)-1-phenylethoxy]quinolin-4-yl}oxy)azetidine-1-carboxylate (2.1 g) was dissolved, and m-chloroperbenzoic acid (about 30% water content, 1.6 g) was added under ice cooling. The mixture was stirred at the same temperature for 30 minutes. Aqueous sodium thiosulfate solution was added under ice cooling, and after the mixture was stirred at room temperature for 30 minutes, extraction with chloroform was performed twice. The combined organic layer was washed with saturated aqueous sodium chloride solution, dried over anhydrous sodium sulfate and filtered, and the filtrate was concentrated. The residue was purified by silica gel column chromatography (hexane/ethyl acetate), thus obtaining 2-(trimethylsilyl)ethyl 3-({(7M)-6-cyclopropyl-2-(ethanesulfonyl)-7-[6-fluoro-5-methyl-1-(oxan-2-yl)-1H-indazol-4 -yl]-8-[(1S)-1-phenylethoxy]quinolin-4-yl}oxy)azetidine-1-carboxylate (1.57 g) as a foam-like solid.

### Production Example 33

Under argon atmosphere, sodium hydrogen carbonate (1.55 g) and 10% Pd/C (about 50% water content, 994 mg) were added to a MeOH (70 mL) and THF (70 mL) solution of 4-tert-butoxy-6-cyclopropyl-2-(ethanesulfonyl)-7-[6-fluoro-5-methyl-2-(triphenylmethyl)-2H -indazol-4-yl]-8-[(1S)-1-phenylethoxy]quinazoline (3 g) at room temperature. The mixture was stirred under hydrogen atmosphere at normal temperature under normal pressure overnight. After substitution with argon, the reaction mixture was filtered through celite (registered trademark) using chloroform/iPrOH (4/1, 100 mL) and EtOH/water (10/1, 100 mL) and concentrated. Ethyl acetate was added to the residue, and the mixture was washed with water and saturated aqueous sodium chloride solution. The organic layer was dried over anhydrous magnesium sulfate. Filtration and concentration were performed, thus obtaining 4-tert-butoxy-6-cyclopropyl-2-(ethanesulfonyl)-7-[6-fluoro-5-methyl-2-(triphenylmethyl)-2H -indazol-4-yl]quinazolin-8-ol (2.7 g) as a foam-like solid.

### Production Example 36

In MeOH (20 mL) and THF (20 mL), 2-(trimethylsilyl)ethyl 3-({(7M)-6-cyclopropyl-2-(ethanesulfonyl)-7-[6-fluoro-5-methyl-1-(oxan-2-yl)-1H-indazol-4 -yl]-8-[(1S)-1-phenylethoxy]quinolin-4-yl}oxy)azetidine-1-carboxylate (1.57 g) was dissolved, and 10% Pd/C (about 50% water content, 800 mg) was added at room temperature. The mixture was stirred under hydrogen atmosphere at normal temperature under normal pressure overnight. At room temperature, 10% Pd/C (about 50% water content, 1200 mg) was added, and the mixture was further stirred under hydrogen atmosphere at normal temperature under normal pressure overnight. The reaction mixture was substituted with argon, and celite (registered trademark) and chloroform were added. After filtration through celite (registered trademark) and washing with chloroform, the filtrate was concentrated, thus obtaining 2-(trimethylsilyl)ethyl 3-({(7M)-6-cyclopropyl-2-(ethanesulfonyl)-7-[6-fluoro-5-methyl-1-(oxan-2-yl)-1H-indazol-4 -yl]-8-hydroxyquinolin-4-yl}oxy)azetidine-1-carboxylate (1.42 g) as a foam-like solid.

### Production Example 37

In DMF (50 mL), 4-tert-butoxy-6-cyclopropyl-2-(ethanesulfonyl)-7-[6-fluoro-5-methyl-2-(triphenylmethyl)-2H -indazol-4-yl]quinazolin-8-ol (8.25 g) was dissolved, and cesium carbonate (14 g) and 1-(chloromethyl)-4-ethynylbenzene (1.95 g) were added at room temperature. The mixture was stirred under nitrogen atmosphere at room temperature overnight. Water and ethyl acetate were added to the reaction mixture, and extraction with ethyl acetate was performed twice. The combined organic layer was washed with saturated aqueous sodium chloride solution, dried over anhydrous magnesium sulfate, filtered and concentrated. The residue was purified by silica gel column chromatography (hexane/ethyl acetate), thus obtaining 4-tert-butoxy-6-cyclopropyl-2-(ethanesulfonyl)-8-[(4-ethynylphenyl)methoxy]-7-[6-fluoro-5-methyl-2-(triphenylmethyl)-2H-indazol-4-yl]quinazoline (6.07 g) as a solid.

### Production Example 40

In DMF (30 mL), 2-(trimethylsilyl)ethyl 3-({(7M)-6-cyclopropyl-2-(ethanesulfonyl)-7-[6-fluoro-5-methyl-1-(oxan-2-yl)-1H-indazol-4 -yl]-8-hydroxyquinolin-4-yl}oxy)azetidine-1-carboxylate (1.41 g) was dissolved, and cesium carbonate (3.2 g) and 1-(chloromethyl)-4-ethynylbenzene (0.32 g) were added at room temperature. The mixture was stirred under nitrogen atmosphere at room temperature for 20 hours. Water was added to the reaction mixture, and extraction with ethyl acetate was performed twice. The combined organic layer was washed with saturated aqueous sodium chloride solution, dried over anhydrous magnesium sulfate and filtered, and the filtrate was concentrated. The residue was purified by basic silica gel column chromatography (hexane/chloroform), thus obtaining 2-(trimethylsilyl)ethyl 3-({(7M)-6-cyclopropyl-2-(ethanesulfonyl)-8-[(4-ethynylphenyl)methoxy]-7-[6-fluoro-5-met hyl-1-(oxan-2-yl)-1H-indazol-4-yl]quinolin-4-yl}oxy)azetidine-1-carboxylate (1.19 g) as a foam-like solid.

### Production Example 41

To a THF (30 mL) solution of 4-tert-butoxy-6-cyclopropyl-2-(ethanesulfonyl)-8-[(4-ethynylphenyl)methoxy]-7-[6-fluoro-5-methyl-2-(triphenylmethyl)-2H-indazol-4-yl]quinazoline (2 g) and (2S)-2-methoxypropan-1-ol (0.35 mL), tBuOK (340 mg) was added under ice cooling, and the mixture was stirred under argon atmosphere under ice cooling for 30 minutes. Saturated aqueous ammonium chloride solution was added under ice cooling to stop the reaction. Water and ethyl acetate were added, and the organic layer and the aqueous layer were separated. The aqueous layer was subjected to extraction twice with ethyl acetate. The collected organic layer was dried over anhydrous sodium sulfate, filtered and concentrated under reduced pressure. The residue was purified by silica gel column chromatography (hexane/ethyl acetate), thus obtaining (7M)-4-tert-butoxy-6-cyclopropyl-8-[(4-ethynylphenyl)methoxy]-7-[6-fluoro-5-methyl-2-(tri phenylmethyl)-2H-indazol-4-yl]-2-[(2S)-2-methoxypropoxy]quinazoline (2.09 g) as a foam-like solid.

### Production Example 50

To a THF (25 mL) solution of (7M)-4-tert-butoxy-6-cyclopropyl-8-[(4-ethynylphenyl)methoxy]-7-[6-fluoro-5-methyl-2-(tri phenylmethyl)-2H-indazol-4-yl]-2-[(2S)-2-methoxypropoxy]quinazoline (2.09 g), 4-methylbenzene-1-sulfonic acid monohydrate (380 mg) and 3,4-dihydro-2H-pyran (0.5 mL) were added under ice cooling, and the mixture was stirred under argon atmosphere at room temperature overnight. Under ice cooling, TEA (2 mL) was added, and then the reaction mixture was concentrated under reduced pressure. The resulting residue was purified by silica gel column chromatography (hexane/ethyl acetate), thus obtaining (7M)-6-cyclopropyl-8-[(4-ethynylphenyl)methoxy]-7-[6-fluoro-5-methyl-1-(oxan-2-yl)-1H-i ndazol-4-yl]-2-[(2S)-2-methoxypropoxy]quinazolin-4-ol (1.35 g) as a foam-like solid.

### Production Example 58

In THF (2 mL), (2S)-2-methoxypropan-1-ol (70 mg) was dissolved, and tBuOK (85 mg) was added at room temperature. The mixture was stirred under nitrogen atmosphere at room temperature for 10 minutes, and thus an alkoxide solution was prepared. In THF (8 mL), 2-(trimethylsilyl)ethyl 3-({(7M)-6-cyclopropyl-2-(ethanesulfonyl)-8-[(4-ethynylphenyl)methoxy]-7-[6-fluoro-5-met hyl-1-(oxan-2-yl)-1H-indazol-4-yl]quinolin-4-yl}oxy)azetidine-1-carboxylate (400 mg) was dissolved, and the prepared alkoxide solution was added in a MeOH ice bath. The mixture was stirred under nitrogen atmosphere at the same temperature for 30 minutes. Water was poured to the reaction mixture, and extraction with ethyl acetate was performed twice. The organic layer was washed with saturated aqueous sodium chloride solution and then dried over anhydrous sodium sulfate. After filtration and concentration under reduced pressure, the residue was purified by silica gel column chromatography (hexane/ethyl acetate), thus obtaining 2-(trimethylsilyl)ethyl 3-({(7M)-6-cyclopropyl-8-[(4-ethynylphenyl)methoxy]-7-[6-fluoro-5-methyl-1-(oxan-2-yl)-1 H-indazol-4-yl]-2-[(2S)-2-methoxypropoxy]quinolin-4-yl}oxy)azetidine-1-carboxylate (361 mg) as a foam-like solid.

### Production Example 63

Under ice cooling, DIPEA (15 mL) and bis(4-nitrophenyl) carbonate (5.8 g) were added to a DMF (60 mL) solution of tert-butyl [(1s,3s)-3-hydroxycyclobutyl]carbamate (3.6 g), and the mixture was stirred under argon atmosphere at room temperature for an hour. Under ice cooling, benzyl azetidin-3-ylcarbamate (3 g) was added, and the mixture was stirred under argon atmosphere at room temperature for two hours. Under ice cooling, the reaction mixture was diluted with ethyl acetate and water. The organic layer and the aqueous layer were separated by a separation operation, and the organic layer was washed with saturated aqueous sodium chloride solution and dried over anhydrous sodium sulfate. After filtration and concentration under reduced pressure, the residue was purified by basic silica gel column chromatography (hexane/ethyl acetate), thus obtaining (1s,3s)-3-[(tert-butoxycarbonyl)amino]cyclobutyl 3-{[(benzyloxy)carbonyl]amino}azetidine-1-carboxylate (4.81 g) as a solid containing impurities.

### Production Example 67

Under ice cooling, CDI (940 mg) was added to a THF (30 mL) solution of 2-[(oxan-2-yl)oxy]ethan-1-amine (700 mg), and after the mixture was stirred at room temperature for two hours, the reaction mixture was concentrated under reduced pressure. To the residue, iPrOH (30 mL) and 3-{[tert-butyldi(methyl)silyl]oxy}azetidine (1.8 g) were added at room temperature, and the mixture was stirred under argon atmosphere at 80°C overnight. The reaction mixture was concentrated under reduced pressure, and the residue was purified by silica gel column chromatography (chloroform/MeOH), thus obtaining 3-{[tert-butyldi(methyl)silyl]oxy}-N-{2-[(oxan-2-yl)oxy]ethyl}azetidine-1-carboxyamide (1.96 g) as an oily substance.

### Production Example 74

Under ice cooling, DIPEA (4.1 mL) and triphosgene (630 mg) were added to a dichloromethane (30 mL) solution of N,N-dimethyl-3-azetidinemethanamine dihydrochloride (1100 mg), and the mixture was stirred under nitrogen atmosphere at room temperature for an hour. Under ice cooling, benzyl azetidin-3-ylcarbamate (1.5 g), pyridine (1 mL) and 4-(dimethylamino)pyridine (150 mg) were added, and the mixture was stirred under nitrogen atmosphere at room temperature for three hours. The reaction mixture was concentrated under reduced pressure, and the residue was purified by basic silica gel column chromatography (chloroform/MeOH), thus obtaining benzyl (1-{3-[(dimethylamino)methyl]azetidine-1-carbonyl}azetidin-3-yl)carbamate (2050 mg) as an oily substance containing impurities.

### Production Example 76

Under ice cooling, DIPEA (2.6 mL) and thiophosgene (0.29 mL) were added to a dichloromethane (20 mL) solution of N,N-dimethyl-3-azetidinemethanamine dihydrochloride (700 mg), and the mixture was stirred under argon atmosphere at room temperature for an hour. Under ice cooling, tert-butyl azetidin-3-ylcarbamate (780 mg), pyridine (0.6 mL) and 4-(dimethylamino)pyridine (90 mg) were added, and the mixture was stirred under argon atmosphere at room temperature for two hours. The reaction mixture was concentrated under reduced pressure, and the residue was purified by basic silica gel column chromatography (hexane/ethyl acetate), thus obtaining tert-butyl (1-{3-[(dimethylamino)methyl]azetidine-1-carbothioyl}azetidin-3-yl)carbamate (432 mg) as an oily substance.

### Production Example 77

To a suspension of benzyl azetidin-3-yl(methyl)carbamate (2.4 g) and 4-(dimethylamino)butanoic acid hydrochloride (2.19 g) in DMF (30 mL), DIPEA (8.6 mL) was added under ice cooling, and the mixture was stirred under nitrogen atmosphere at room temperature for five minutes. HATU (4.56 g) was added under ice cooling, and the mixture was stirred under nitrogen atmosphere at room temperature overnight. Saturated aqueous sodium hydrogen carbonate solution was added to the reaction mixture, and extraction with ethyl acetate was performed five times. The organic layer was washed with saturated aqueous sodium chloride solution, dried over anhydrous magnesium sulfate, then filtered and concentrated under reduced pressure. The residue was purified by silica gel column chromatography (chloroform/MeOH) and then purified by basic silica gel column chromatography (hexane/ethyl acetate), thus obtaining benzyl {1-[4-(dimethylamino)butanoyl]azetidin-3-yl}methylcarbamate (2504 mg) as an oily substance.

### Production Example 78

Sodium hydride (60%, dispersed in liquid paraffin, 85 mg) was added to a DMF (5 mL) solution of tert-butyl [(1r,3r)-3-hydroxycyclobutyl]carbamate (200 mg) and 2-chloro-N,N-dimethylacetamide (143 mg) under ice cooling, and the mixture was stirred under nitrogen atmosphere at room temperature overnight. At room temperature, water was added, and extraction with ethyl acetate was performed three times. The organic layer was washed with saturated aqueous sodium chloride solution and dried over anhydrous magnesium sulfate. After filtration and concentration, the residue was purified by silica gel column chromatography (from hexane/ethyl acetate to chloroform/MeOH), thus obtaining tert-butyl {(1r,3r)-3-[2-(dimethylamino)-2-oxoethoxy]cyclobutyl}carbamate (131 mg) as a solid.

### Production Example 79

At room temperature, 10% Pd/C (about 50% water content, 1.5 g) was added to a THF (50 mL) and MeOH (50 mL) solution of (1s,3s)-3-[(tert-butoxycarbonyl)amino]cyclobutyl 3-{[(benzyloxy)carbonyl]amino}azetidine-1-carboxylate (4.81 g), and the mixture was stirred under hydrogen atmosphere at normal temperature under normal pressure overnight. After substitution with argon, a small amount of celite (registered trademark) and water-containing EtOH were added, and then the mixture was stirred at room temperature for 10 minutes. After filtration through celite (registered trademark) using water-containing EtOH, toluene was added to the filtrate, and the mixture was concentrated under reduced pressure. The residue was purified by silica gel column chromatography (chloroform/MeOH/28% ammonia water), thus obtaining (1s,3s)-3-[(tert-butoxycarbonyl)amino]cyclobutyl 3-aminoazetidine-1-carboxylate (2.19 g) as a solid.

### Production Example 84

Under ice cooling, TBAF (1M THF solution, 11 mL) was added to a THF (25 mL) solution of 3-{[tert-butyldi(methyl)silyl]oxy}-N-{2-[(oxan-2-yl)oxy]ethyl}azetidine-1-carboxyamide (1.96 g), and the mixture was stirred at room temperature overnight. The reaction mixture was concentrated under reduced pressure, and the residue was purified by silica gel column chromatography (chloroform/MeOH), thus obtaining 3-hydroxy-N-{2-[(oxan-2-yl)oxy]ethyl}azetidine-1-carboxamide (1.46 g) as an oily substance.

### Production Example 85

TFA (3 mL) was added to a dichloromethane (15 mL) solution of tert-butyl (1-{[2-(dimethylamino)ethyl]carbamoyl}azetidin-3-yl)carbamate (1100 mg) at room temperature, and the mixture was stirred under nitrogen atmosphere at room temperature for six hours. The reaction mixture was concentrated under reduced pressure, thus obtaining 3-amino-N-[2-(dimethylamino)ethyl]azetidine-1-carboxamide n-(trifluoroacetic acid) salt (3000 mg) as an oily substance.

### Production Example 86

Hydrogen chloride (4M DOX solution, 14 mL) was added to a dichloromethane (16 mL) and MeOH (16 mL) solution of tert-butyl [(3S)-1-{[2-(dimethylamino)ethyl]carbamoyl}pyrrolidin-3-yl](methyl)carbamate (1600 mg) under ice cooling, and the mixture was stirred under nitrogen atmosphere at room temperature for four hours. The reaction mixture was concentrated under reduced pressure, thus obtaining (3S)-N-[2-(dimethylamino)ethyl] -3-(methylamino)pyrrolidine-1-carboxyamide n-hydrochloride (1800 mg) as a foam-like solid.

### Production Example 89

Under nitrogen atmosphere, borane dimethyl sulfide complex (2M THF solution, 361 µL) was added to a THF (10 mL) solution of tert-butyl {(1r,3r)-3-[2-(dimethylamino)-2-oxoethoxy]cyclobutyl}carbamate (131 mg) at room temperature, and the mixture was stirred at room temperature for eight hours. Borane dimethyl sulfide complex (2M THF solution, 361 µL) was added under nitrogen atmosphere at room temperature, and the mixture was stirred at room temperature overnight. Saturated aqueous sodium hydrogen carbonate solution and saturated aqueous sodium chloride solution were added at room temperature, and extraction with chloroform/iPrOH (4/1) was performed three times. The organic layer was dried over anhydrous magnesium sulfate, filtered and concentrated. The residue was purified by silica gel column chromatography (from hexane/ethyl acetate to chloroform/MeOH), thus obtaining tert-butyl {(1r,3r)-3-[2-(dimethylamino)ethoxy]cyclobutyl}carbamate (115 mg) as a solid.

### Production Example 91

To a MeOH (10 mL) and TEA (10 mL) solution of (2R)-2-amino-2-(6-chloropyridin-3-yl)ethan-1-ol dihydrochloride (3.58 g), di-tert-butyl dicarbonate (4.77 g) was added, and the mixture was stirred at 25°C for two hours. Water was added to the reaction mixture, and extraction with ethyl acetate was performed three times. The collected organic layer was dried over anhydrous sodium sulfate, filtered and concentrated under reduced pressure. The residue was purified by silica gel column chromatography (dichloromethane/MeOH), thus obtaining tert-butyl [(1R)-1-(6-chloropyridin-3-yl)-2-hydroxyethyl]carbamate (4.3 g) as a solid.

### Production Example 92

Under nitrogen atmosphere, boron trifluoride diethyl ether complex (520 µL) was added to a suspension of tert-butyl [(1R)-1-(4-bromophenyl)-2-hydroxyethyl]carbamate (20 g) and 2,2-dimethoxypropane (66 mL) in acetone (300 mL), and the mixture was stirred at room temperature for an hour. TEA (1.3 mL) was added, and after the mixture was stirred at room temperature for 10 minutes, the reaction mixture was concentrated under reduced pressure. The residue was purified by silica gel column chromatography (hexane/ethyl acetate), thus obtaining tert-butyl (4R)-4-(4-bromophenyl)-2,2-dimethyl-1,3-oxazolidine-3-carboxylate (21.4 g) as a solid.

### Production Example 94

In DMSO (10 mL), tert-butyl (4R)-4-(4-bromophenyl)-2,2-dimethyl-1,3-oxazolidine-3-carboxylate (3 g) was suspended, and 1,2,4-triazole (1.4 g), copper(I) iodide (315 mg), 8-quinolinol (495 mg) and potassium carbonate (2.4 g) were added at room temperature. The mixture was stirred under nitrogen atmosphere at 150°C overnight. Water was added to the reaction mixture, and extraction with ethyl acetate was performed three times. The combined organic layer was washed with saturated aqueous sodium chloride solution, dried over anhydrous magnesium sulfate, filtered and concentrated. The residue was purified by silica gel column chromatography (hexane/ethyl acetate), thus obtaining tert-butyl (4R)-2,2-dimethyl-4-[4-(1H-1,2,4-triazol-1-yl)phenyl]-1,3-oxazolidine-3-carboxylate (2.45 g) as a solid.

### Production Example 97

To a DMAc (80 mL) solution of tert-butyl [(1R)-1-(4-bromophenyl)-2-hydroxyethyl]carbamate (5.01 g), 4-methyl-1,3-thiazole (2.88 mL) and potassium acetate (3.11 g) were added at room temperature, and after performing deaeration and argon substitution each three times, palladium(II) acetate (356 mg) was added at room temperature. The mixture was stirred under argon atmosphere at 100°C for 16 hours. After cooling to room temperature, ethyl acetate and water were added to the reaction mixture, and the insoluble matter was removed by filtration through celite (registered trademark). Water was added to the filtrate, and the aqueous layer was subjected to extraction with ethyl acetate three times. The combined organic layer was washed with saturated aqueous sodium chloride solution and dried over anhydrous sodium sulfate. After filtration and concentration, the residue was purified by basic silica gel column chromatography (hexane/ethyl acetate), thus obtaining tert-butyl {(1R)-2-hydroxy-1-[4-(4-methyl-1,3-thiazol-5-yl)phenyl]ethyl}carbamate (4.66 g) as a solid.

### Production Example 100

Hydrogen chloride (4M DOX solution, 20 mL) was added portionwise to a dichloromethane (50 mL) and MeOH (40 mL) solution of tert-butyl {(1R)-2-hydroxy-1-[4-(4-methyl-1,3-thiazol-5-yl)phenyl]ethyl}carbamate (4.41 g) under ice cooling, and the mixture was stirred at room temperature for six hours. Diethyl ether was added to the reaction mixture, and the solid was taken by filtration, washed with diethyl ether and dried under reduced pressure, thus obtaining (2R)-2-amino-2-[4-(4-methyl-1,3-thiazol-5-yl)phenyl]ethan-1-ol n-hydrochloride (2.12 g) as a solid. The filtrate was concentrated under reduced pressure and dried by heating under reduced pressure, thus obtaining (2R)-2-amino-2-[4-(4-methyl-1,3-thiazol-5-yl)phenyl]ethan-1-ol n-hydrochloride (2.01 g) as a solid.

### Production Example 101

Under ice cooling, hydrogen chloride (4M DOX solution, 11 mL) was added to a dichloromethane (15 mL) solution of tert-butyl (4R)-2,2-dimethyl-4-[4-(1H-1,2,4-triazol-1-yl)phenyl]-1,3-oxazolidine-3-carboxylate (1.54 g), and the mixture was stirred at room temperature overnight. The reaction mixture was concentrated under reduced pressure, thus obtaining (2R)-2-amino-2-[4-(1H-1,2,4-triazol-1-yl)phenyl]ethan-1-ol n-hydrochloride (1.1 g) as a solid.

### Production Example 106

A suspension of tert-butyl [(1R)-1-(4-bromophenyl)-2-hydroxyethyl]carbamate (15 g) in dichloromethane (100 mL) was cooled with ice, and TEA (9 mL) and methanesulfonyl chloride (4.4 mL) were added. The mixture was stirred under argon atmosphere at the same temperature for an hour. Saturated aqueous sodium hydrogen carbonate solution was added, and the mixture was subjected to extraction with chloroform, dried over anhydrous magnesium sulfate, filtered and concentrated under reduced pressure, thus obtaining (2R)-2-(4-bromophenyl)-2-[(tert-butoxycarbonyl)amino]ethyl methanesulfonate (19 g) as a solid.

### Production Example 107

A mixture of (2R)-2-(4-bromophenyl)-2-[(tert-butoxycarbonyl)amino]ethyl methanesulfonate (13 g), dimethylamine (9.5M aqueous solution, 37 mL) and THF (30 mL) was stirred at room temperature for an hour and at 50°C overnight. After the reaction mixture was allowed to cool to room temperature, water was added, and the mixture was subjected to extraction with chloroform and dried over anhydrous magnesium sulfate. After filtration and concentration under reduced pressure, the residue was purified by silica gel column chromatography (chloroform/MeOH), thus obtaining tert-butyl [(1R)-1-(4-bromophenyl)-2-(dimethylamino)ethyl]carbamate (7.89 g) as a solid.

### Production Example 110

At room temperature, tert-butyl (4R)-4-(4-bromophenyl)-2,2-dimethyl-1,3-oxazolidine-3-carboxylate (3 g), 4-methyl-1,3-oxazole-5-carboxylic acid (2.16 g), tetra-n-butylammonium chloride (2.34 g), cesium carbonate (4.11 g), bis(tri-tert-butylphosphine)palladium(0) (225 mg) and N-methyl-2-pyrrolidone (30 mL) were added, and the mixture was stirred under argon atmosphere at 160°C for an hour. The reaction mixture was allowed to cool to room temperature, and ethyl acetate and water were added. After filtration through celite (registered trademark), the filtrate was subjected to extraction with ethyl acetate three times. The combined organic layer was washed with saturated aqueous sodium chloride solution, dried over anhydrous magnesium sulfate, filtered and concentrated. The residue was purified by silica gel column chromatography (hexane/ethyl acetate), thus obtaining tert-butyl (4R)-2,2-dimethyl-4-[4-(4-methyl-1,3-oxazol-5-yl)phenyl]-1,3-oxazolidine-3-carboxylate (1.51 g) as an oily substance.

### Production Example 112

Under ice cooling, HATU (13.8 g) was added to a DMF (150 mL) solution of N-(tert-butoxycarbonyl)-L-valyl-(4R)-4-hydroxy-L-proline (10 g), (2R)-2-amino-2-[4-(4-methyl-1,3-thiazol-5-yl)phenyl]ethan-1-ol n-hydrochloride (9.3 g) and DIPEA (21 mL), and the mixture was stirred under ice cooling for an hour. Under ice cooling, the reaction mixture was diluted with ethyl acetate and water, and the organic layer and the aqueous layer were separated by a separation operation. The organic layer was washed with saturated aqueous sodium chloride solution twice and dried over anhydrous sodium sulfate. After filtration and concentration under reduced pressure, the residue was purified by silica gel column chromatography (chloroform/MeOH), thus obtaining N-(tert-butoxycarbonyl)-L-valyl-(4R)-4-hydroxy-N-{(1R)-2-hydroxy-1-[4-(4-methyl-1,3-thia zol-5-yl)phenyl]ethyl}-L-prolinamide (16.47 g) as a foam-like solid.

### Production Example 113

Under ice cooling, DIPEA (4 mL) and HATU (2.2 g) were added to a suspension of (2R)-2-amino-2-[4-(1H-1,2,4-triazol-1-yl)phenyl]ethan-1-ol n-hydrochloride (1.1 g) and N-(tert-butoxycarbonyl)-L-valyl-(4R)-4-hydroxy-L-proline (3 g) in dichloromethane (20 mL), and the mixture was stirred under argon atmosphere at room temperature overnight. The reaction mixture was concentrated under reduced pressure, and the residue was purified by silica gel column chromatography (chloroform/MeOH), thus obtaining N-(tert-butoxycarbonyl)-L-valyl-(4R)-4-hydroxy-N-{(1R)-2-hydroxy-1-[4-(1H-1,2,4-triazol-1-yl)phenyl]ethyl}-L-prolinamide (1.77 g) as a solid.

### Production Example 118

Under ice cooling, 1-(fluorosulfonyl)-2,3-dimethyl-1H-imidazol-3-ium trifluoromethanesulfonate (14.8 g) was added to a methyl tert-butyl ether (70 mL), water (60 mL) and MeCN (10 mL) solution of sodium azide (4 g), and the mixture was stirred at room temperature for an hour. The mixture was moved to a separatory funnel using methyl tert-butyl ether and water, and the organic layer and the aqueous layer were separated. The organic layer was washed once with water, and thus a solution of an azidation reagent was prepared. Under ice cooling, TFA (23 mL) was added to a dichloromethane (200 mL) solution of N-(tert-butoxycarbonyl)-L-valyl-(4R)-4-hydroxy-N-{(1R)-2-hydroxy-1-[4-(4-methyl-1,3-thia zol-5-yl)phenyl]ethyl}-L-prolinamide (16.47 g), and after the mixture was stirred at room temperature for four hours, the reaction mixture was concentrated under reduced pressure. DMSO (150 mL), water (30 mL) and potassium hydrogen carbonate (12.5 g) were added to the residue under ice cooling, and the mixture was stirred under ice cooling for 10 minutes. The prepared solution of the azidation reagent was added dropwise from a separatory funnel to the reaction mixture under ice cooling, and the mixture was stirred at room temperature overnight. The reaction mixture was diluted with ethyl acetate and water, and the organic layer and the aqueous layer were separated by a separation operation. The organic layer was washed with saturated aqueous sodium chloride solution twice and dried over anhydrous sodium sulfate. After filtration and concentration under reduced pressure, the residue was purified by silica gel column chromatography (chloroform/MeOH), thus obtaining (4R)-1-[(2S)-2-azido-3-methylbutanoyl]-4-hydroxy-N-{(1R)-2-hydroxy-1-[4-(4-methyl-1,3-t hiazol-5-yl)phenyl]ethyl}-L-prolinamide (11.6 g) as a foam-like solid.

### Production Example 119

To a mixture of sodium azide (373 mg), methyl tert-butyl ether (6 mL) and water (6 mL), 1-(fluorosulfonyl)-2,3-dimethyl-1H-imidazol-3-ium trifluoromethanesulfonate (2304 mg) and MeCN (0.3 mL) were added under ice cooling, and the mixture was stirred at the same temperature for 30 minutes and allowed to stand at room temperature for an hour. The aqueous layer was suctioned with a pipette and removed, and thus a solution of an azidation reagent was prepared. TFA (3 mL) was added to a dichloromethane (15 mL) solution of N-(tert-butoxycarbonyl)-L-valyl-(4R)-4-hydroxy-N-{(1R)-2-hydroxy-1-[4-(1H-1,2,4-triazol-1-yl)phenyl]ethyl}-L-prolinamide (2417 mg), and the mixture was stirred at room temperature overnight. The reaction mixture was concentrated under reduced pressure. The residue was dissolved in DMSO (6 mL) and water (3 mL), and potassium hydrogen carbonate (7026 mg) and the prepared solution of the azidation reagent were added at room temperature using methyl tert-butyl ether (3 mL). The mixture was stirred at room temperature for three hours, and saturated aqueous sodium hydrogen carbonate solution and saturated aqueous sodium chloride solution were added at room temperature. Extraction with chloroform/iPrOH (4/1) was performed six times. The organic layer was dried over anhydrous magnesium sulfate, filtered and concentrated under reduced pressure, and the residue was purified by silica gel column chromatography (from hexane/ethyl acetate to chloroform/MeOH), thus obtaining (4R)-1-[(2S)-2-azido-3-methylbutanoyl]-4-hydroxy-N-{(1R)-2-hydroxy-1-[4-(1H-1,2,4-triaz ol-1-yl)phenyl]ethyl}-L-prolinamide (1503 mg) as a solid.

### Production Example 123

Hydrogen chloride (4M DOX solution, 5 mL) was added to a MeOH (5 mL) solution of N-(tert-butoxycarbonyl)-L-valyl-(4R)-N-{(1R)-2-(dimethylamino)-1-[4-(4-methyl-1,3-thiazo l-5-yl)phenyl]ethyl}-4-hydroxy-L-prolinamide (1.89 g), and the mixture was stirred at room temperature overnight. The reaction mixture was concentrated under reduced pressure, thus obtaining L-valyl-(4R)-N-{(1R)-2-(dimethylamino)-1-[4-(4-methyl-1,3-thiazol-5-yl)phenyl]ethyl}-4-h ydroxy-L-prolinamide n-hydrochloride (2.14 g) as a solid.

### Production Example 124

Under argon atmosphere, a mixture of (4R)-1-(tert-butoxycarbonyl)-4-hydroxy-L-proline (1.76 g), (2R)-2-amino-2-[4-(4-methyl-1,3-thiazol-5-yl)phenyl]ethan-1-ol n-hydrochloride (2.12 g) and DMF (22 mL) was cooled with ice, and DIPEA (4.7 mL) and HATU (3.02 g) were added portionwise in a manner that the internal temperature was kept at 5°C or lower. The reaction mixture was stirred under ice cooling for an hour and at room temperature for an hour. The reaction mixture was cooled with ice, and after water (120 mL) and saturated aqueous sodium chloride solution (50 mL) were added, extraction with ethyl acetate was performed three times. The organic layer was washed with saturated aqueous sodium chloride solution and dried over anhydrous magnesium sulfate. The aqueous layer was subjected to extraction with ethyl acetate/iPrOH (9/1) three times, and the organic layer was washed with saturated aqueous sodium chloride solution and dried over anhydrous magnesium sulfate. The organic layers were combined and concentrated under reduced pressure, and the residue was purified by silica gel column chromatography (chloroform/MeOH), thus obtaining tert-butyl (2S,4R)-4-hydroxy-2-({(1R)-2-hydroxy-1-[4-(4-methyl-1,3-thiazol-5-yl)phenyl]ethyl}carba moyl)pyrrolidine-1-carboxylate (3.09 g) as an oily substance.

### Production Example 125

In EtOH (100 mL), tert-butyl (2S,4R)-4-hydroxy-2-({(1R)-2-hydroxy-1-[4-(4-methyl-1,3-thiazol-5-yl)phenyl]ethyl}carba moyl)pyrrolidine-1-carboxylate (38.5 g) was dissolved, and methanesulfonic acid (26 mL) was added at room temperature. The mixture was stirred under nitrogen atmosphere at room temperature for three hours. The reaction mixture was concentrated, and the residue was purified by basic silica gel column chromatography (chloroform/MeOH), thus obtaining (4R)-4-hydroxy-N-{(1R)-2-hydroxy-1-[4-(4-methyl-1,3-thiazol-5-yl)phenyl]ethyl}-L-prolina mide (27.01 g) as a solid.

### Production Example 129

Under ice cooling, CDI (55 mg) was added to a THF (3 mL) solution of N-(tert-butoxycarbonyl)-N-methyl-1,2-ethylenediamine (60 mg), and the mixture was stirred under nitrogen atmosphere at room temperature for an hour. The reaction solution was concentrated under reduced pressure, and an iPrOH (3 mL) solution of (7M)-4-[(azetidin-3-yl)oxy]-6-cyclopropyl-7-[6-fluoro-5-methyl-1-(oxan-2-yl)-1H-indazol-4-yl]-2-[(2S)-2-methoxypropoxy]-8-[(1S)-1-phenylethoxy]quinoline (100 mg) was added to the residue at room temperature, and the mixture was stirred under nitrogen atmosphere at 90°C overnight. The reaction mixture was concentrated under reduced pressure, and the residue was purified by silica gel column chromatography (from hexane/ethyl acetate to hexane/chloroform and further chloroform/MeOH), thus obtaining tert-butyl (2-{[3-({(7M)-6-cyclopropyl-7-[6-fluoro-5-methyl-1-(oxan-2-yl)-1H-indazol-4-yl]-2-[(2S)-2-methoxypropoxy]-8-[(1S)-1-phenylethoxy]quinolin-4-yl}oxy)azetidine-1-carbonyl]amino}et hyl)methylcarbamate (117 mg) as a foam-like solid.

### Production Example 132

To an iPrOH (13 mL) and water (2 mL) solution of L-valyl-(4R)-N-{(1R)-2-(dimethylamino)-1-[4-(4-methyl-1,3-thiazol-5-yl)phenyl]ethyl}-4-h ydroxy-L-prolinamide n-hydrochloride (1.34 g), potassium carbonate (1.6 g), copper(II) sulfate pentahydrate (67 mg) and 1H-imidazole-1-sulfonyl=azide=mono(tetrafluoroboric acid) salt (715 mg) were added at room temperature, and the mixture was stirred at room temperature for 30 minutes. To the reaction mixture, iPrOH was added, and the filtrate obtained by filtration through celite (registered trademark) and washing with iPrOH was concentrated under reduced pressure. The residue was purified by basic silica gel column chromatography (chloroform/MeOH) and then purified by silica gel column chromatography (chloroform/MeOH), thus obtaining (4R)-1-[(2S)-2-azido-3-methylbutanoyl]-N-{(1R)-2-(dimethylamino)-1-[4-(4-methyl-1,3-thia zol-5-yl)phenyl]ethyl}-4-hydroxy-L-prolinamide (725 mg) as a foam-like solid.

### Production Example 133

In toluene (300 mL), 8-(benzyloxy)-4-tert-butoxy-6-cyclopropyl-7-[6-fluoro-5-methyl-1-(oxan-2-yl)-1H-indazol-4 -yl]-2-[(oxan-4-yl)oxy]quinazoline (30 g) was dissolved, and 4-methylbenzene-1-sulfonic acid monohydrate (759 mg) was added at 25°C. The mixture was stirred under nitrogen atmosphere at 60°C for two hours. The mixture was combined with a reaction mixture obtained in the same manner (using 13 g of the raw material compound), and saturated aqueous sodium hydrogen carbonate solution and water were added. Extraction with ethyl acetate was performed three times. The collected organic layer was dried over anhydrous sodium sulfate, filtered and concentrated under reduced pressure. The residue was purified by silica gel column chromatography (petroleum ether/ethyl acetate), thus obtaining a product (21.95 g).

TFA (4.74 mL) was added to a dichloromethane (20 mL) solution of a part (4 g) of the obtained product and triisopropylsilane (3.29 mL) at 0°C, and the mixture was stirred at 25°C for 18 hours. Saturated aqueous sodium hydrogen carbonate solution was added to the reaction mixture, and extraction with dichloromethane was performed three times. The collected organic layer was dried over anhydrous sodium sulfate, filtered and concentrated under reduced pressure. The reaction was performed again under the same conditions, and the crude products obtained from the two batches were combined and then purified by silica gel column chromatography (dichloromethane/MeOH) twice. The residue was suspended in dichloromethane (24 mL), and the suspension was stirred at 25°C for 12 hours. The insoluble matter was removed by filtration, and the filtrate was concentrated under reduced pressure. The residue was purified by supercritical fluid chromatography (Cellulose-2 column, 0.1% ammonia water/MeOH), and a fraction including a peak at the high polarity side was collected, thus obtaining a product (a single diastereomer, 2.8 g).

To a dichloromethane (5 mL) solution of a part (500 mg) of the obtained product and 3,4-dihydro-2H-pyran (127 µL), 4-methylbenzene-1-sulfonic acid monohydrate (48 mg) was added, and the mixture was stirred at 25°C for 52 hours. Water was added to the reaction mixture, and extraction with dichloromethane was performed three times. The organic layer was washed with saturated aqueous sodium chloride solution and dried over anhydrous sodium sulfate. After filtration and concentration under reduced pressure, the residue was purified by silica gel column chromatography (petroleum ether/ethyl acetate), thus obtaining (7M)-8-(benzyloxy)-6-cyclopropyl-7-[6-fluoro-5-methyl-1-(oxan-2-yl)-1H-indazol-4-yl]-2-[( oxan-4-yl)oxy]quinazolin-4-ol (480 mg) as a solid.

### Production Example 134

To a THF (15 mL) solution of (7M)-6-cyclopropyl-8-[(4-ethynylphenyl)methoxy]-7-[6-fluoro-5-methyl-1-(oxan-2-yl)-1H-i ndazol-4-yl]-2-[(2S)-2-methoxypropoxy]quinazolin-4-ol (380 mg), PyBOP (560 mg) and cesium carbonate (340 mg) were added under ice cooling, and the mixture was stirred under argon atmosphere at room temperature for an hour. Under ice cooling, (1s,3s)-3-[(tert-butoxycarbonyl)amino]cyclobutyl 3-aminoazetidine-1-carboxylate (600 mg) and DIPEA (1.3 mL) were added, and the mixture was stirred under argon atmosphere at 50°C for an hour. The reaction mixture was filtered through celite (registered trademark) using chloroform. The filtrate was concentrated under reduced pressure, and the residue was purified by basic silica gel column chromatography (hexane/ethyl acetate), thus obtaining (1s,3R)-3-[(tert-butoxycarbonyl)amino]cyclobutyl 3-({(7M)-6-cyclopropyl-8-[(4-ethynylphenyl)methoxy]-7-[6-fluoro-5-methyl-1-(oxan-2-yl)-1 H-indazol-4-yl]-2-[(2S)-2-methoxypropoxy]quinazolin-4-yl}amino)azetidine-1-carboxylate (573 mg) as a solid.

### Production Example 162

To a tBuOH (5 mL), water (5 mL) and THF (5 mL) solution of (1s,3R)-3-[(tert-butoxycarbonyl)amino]cyclobutyl 3-({(7M)-6-cyclopropyl-8-[(4-ethynylphenyl)methoxy]-7-[6-fluoro-5-methyl-1-(oxan-2-yl)-1 H-indazol-4-yl]-2-[(2S)-2-methoxypropoxy]quinazolin-4-yl}amino)azetidine-1-carboxylate (573 mg), (4R)-1-[(2S)-2-azido-3-methylbutanoyl]-4-hydroxy-N-{(1R)-2-hydroxy-1-[4-(4-methyl-1,3-t hiazol-5-yl)phenyl]ethyl}-L-prolinamide (320 mg) and sodium ascorbate (320 mg), copper(II) sulfate pentahydrate (170 mg) was added at room temperature, and the mixture was stirred at room temperature for an hour. Disodium ethylenediamine tetraacetate (1.2 g) was added at room temperature, and after the mixture was stirred at room temperature for an hour, saturated aqueous sodium hydrogen carbonate solution was added. The mixture was filtered through celite (registered trademark) using chloroform. The filtrate was concentrated under reduced pressure, and the residue was purified by silica gel column chromatography (chloroform/MeOH), thus obtaining (1s,3R)-3-[(tert-butoxycarbonyl)amino]cyclobutyl 3-({(7M)-6-cyclopropyl-7-[6-fluoro-5-methyl-1-(oxan-2-yl)-1H-indazol-4-yl]-8-{[4-(1-{(2S) -1-[(2S,4R)-4-hydroxy-2-({ (1R)-2-hydroxy-1-[4-(4-methyl- 1,3-thiazol-5-yl)phenyl]ethyl }car bamoyl)pyrrolidin-1-yl]-3-methyl-1-oxobutan-2-yl}-1H-1,2,3-triazol-4-yl)phenyl]methoxy}-2-[(2S)-2-methoxypropoxy]quinazolin-4-yl}amino)azetidine-1-carboxylate (773 mg) as a foam-like solid.

### Production Example 186

To a mixture of 2-(trimethylsilyl)ethyl 3-({(7M)-6-cyclopropyl-8-[(4-ethynylphenyl)methoxy]-7-[6-fluoro-5-methyl-1-(oxan-2-yl)-1 H-indazol-4-yl]-2-[(2S)-2-methoxypropoxy]quinolin-4-yl}oxy)azetidine-1-carboxylate (120 mg) and (4R)-1-[(2S)-2-azido-3-methylbutanoyl]-4-hydroxy-N-{(1R)-2-hydroxy-1-[4-(1H-1,2,4-triaz ol-1-yl)phenyl]ethyl}-L-prolinamide (90 mg) in tBuOH (1 mL), THF (1 mL) and water (1 mL), anhydrous copper(II) sulfate (25 mg) and sodium ascorbate (66 mg) were added at room temperature, and the mixture was stirred under nitrogen atmosphere at room temperature for 30 minutes. Basic silica gel was added to the reaction mixture, and after concentration, the residue was purified by basic silica gel column chromatography (from hexane/chloroform to chloroform/MeOH), thus obtaining 2-(trimethylsilyl)ethyl 3-({(7M)-6-cyclopropyl-7-[6-fluoro-5-methyl-1-(oxan-2-yl)-1H-indazol-4-yl]-8-{[4-(1-{(2S) -1-[(2S,4R)-4-hydroxy-2-({(1R)-2-hydroxy-1-[4-(1H-1,2,4-triazol-1-yl)phenyl]ethyl}carbam oyl)pyrrolidin-1-yl]-3-methyl-1-oxobutan-2-yl}-1H-1,2,3-triazol-4-yl)phenyl]methoxy}-2-[( 2S)-2-methoxypropoxy]quinolin-4-yl}oxy)azetidine-1-carboxylate (197 mg) as a solid.

### Production Example 188

To a tBuOH (5 mL), THF (10 mL) and water (5 mL) solution of tert-butyl 3-({(7M)-6-cyclopropyl-8-[(4-ethynylphenyl)methoxy]-7-[6-fluoro-5-methyl-2-(triphenylmet hyl)-2H-indazol-4-yl]-2-[(2S)-2-methoxypropoxy]quinolin-4-yl}oxy)azetidine-1-carboxylate (700 mg) and (4R)-1-[(2S)-2-azido-3-methylbutanoyl]-4-hydroxy-N-{(1R)-2-hydroxy-1-[4-(1H-1,2,4-triaz ol-1-yl)phenyl]ethyl}-L-prolinamide (350 mg), anhydrous copper(II) sulfate (126 mg) and sodium ascorbate (350 mg) were added at room temperature, and the mixture was stirred under nitrogen atmosphere at room temperature for 30 minutes. Basic silica gel was added to the reaction mixture, and after concentration, the residue was purified by basic silica gel column chromatography (chloroform/MeOH). The target fraction was concentrated under reduced pressure.

The residue was dissolved in MeOH (10 mL), and methanesulfonic acid (469 µL) was added at room temperature. The mixture was stirred under nitrogen atmosphere at room temperature for 30 minutes and at 40°C for four hours. DIPEA (1.4 mL) was added to the reaction mixture at room temperature, and the reaction mixture was concentrated. The residue was purified by basic silica gel column chromatography (chloroform/MeOH), thus obtaining (4R)-1-[(2S)-2-(4-{4-[({(7M)-4-[(azetidin-3-yl)oxy]-6-cyclopropyl-7-(6-fluoro-5-methyl-1H-indazol-4-yl)-2-[(2S)-2-methoxypropoxy]quinolin-8-yl}oxy)methyl]phenyl}-1H-1,2,3-triazol -1-yl)-3-methylbutanoyl]-4-hydroxy-N-{(1R)-2-hydroxy-1-[4-(1H-1,2,4-triazol-1-yl)phenyl] ethyl}-L-prolinamide (723 mg) as a solid.

### Production Example 190

To a tBuOH (1 mL), THF (1 mL) and water (1 mL) solution of 2-(trimethylsilyl)ethyl 3-({(7M)-6-cyclopropyl-8-[(4-ethynylphenyl)methoxy]-7-[6-fluoro-5-methyl-1-(oxan-2-yl)-1 H-indazol-4-yl]-2-[(2S)-2-methoxypropoxy]quinolin-4-yl}oxy)azetidine-1-carboxylate (47 mg) and (4R)-1-[(2S)-2-azido-3-methylbutanoyl]-4-hydroxy-N-{(1R)-2-hydroxy-1-[4-(4-methyl-1,3-o xazol-5-yl)phenyl]ethyl}-L-prolinamide (33 mg), anhydrous copper(II) sulfate (11 mg) and sodium ascorbate (30 mg) were added at room temperature, and the mixture was stirred under nitrogen atmosphere at room temperature for 20 minutes. Basic silica gel was added to the reaction mixture, and after concentration, the residue was purified by basic silica gel column chromatography (chloroform/MeOH). The target fraction was concentrated.

The residue was dissolved in THF (3 mL), and TBAF (1M THF solution, 300 µL) was added at room temperature. The mixture was stirred under nitrogen atmosphere at 40°C for an hour. The reaction mixture was concentrated and purified by basic silica gel column chromatography (chloroform/MeOH), thus obtaining (4R)-1-[(2S)-2-(4-{4-[({(7M)-4-[(azetidin-3-yl)oxy]-6-cyclopropyl-7-[6-fluoro-5-methyl-1-( oxan-2-yl)-1H-indazol-4-yl]-2-[(2S)-2-methoxypropoxy]quinolin-8-yl}oxy)methyl]phenyl}-1H-1,2,3-triazol-1-yl)-3-methylbutanoyl]-4-hydroxy-N-{ (1R)-2-hydroxy- 1-[4-(4-methyl-1,3-oxazol-5-yl)phenyl]ethyl}-L-prolinamide (54 mg) as a solid.

### Production Example 192

Under ice cooling, methanesulfonic acid (2 mL) was added to an iPrOH (15 mL) solution of (1s,3R)-3-[(tert-butoxycarbonyl)amino]cyclobutyl 3-({(7M)-6-cyclopropyl-7-[6-fluoro-5-methyl-1-(oxan-2-yl)-1H-indazol-4-yl]-8-{[4-(1-{(2S) -1-[(2S,4R)-4-hydroxy-2-({ (1R)-2-hydroxy-1-[4-(4-methyl- 1,3-thiazol-5-yl)phenyl]ethyl }car bamoyl)pyrrolidin-1-yl]-3-methyl-1-oxobutan-2-yl}-1H-1,2,3-triazol-4-yl)phenyl]methoxy}-2-[(2S)-2-methoxypropoxy]quinazolin-4-yl}amino)azetidine-1-carboxylate (773 mg), and the mixture was stirred at 40°C for four hours. Under ice cooling, TEA (15 mL) was added, and then the reaction mixture was concentrated under reduced pressure. The residue was purified by basic silica gel column chromatography (chloroform/MeOH), thus obtaining (1s,3R)-3-aminocyclobutyl 3-({(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrol idin-1-yl]-3-methyl-1-oxobutan-2-yl}-1H-1,2,3-triazol-4-yl)phenyl]methoxy}-2-[(2S)-2-met hoxypropoxy]quinazolin-4-yl}amino)azetidine-1-carboxylate (769 mg) as a solid.

### Production Example 193

Under ice cooling, TBAF (1M THF solution, 300 µL) was added to a THF (4 mL) solution of 2-(trimethylsilyl)ethyl 3-({(7M)-6-cyclopropyl-7-[6-fluoro-5-methyl-1-(oxan-2-yl)-1H-indazol-4-yl]-8-{[4-(1-{(2S) -1-[(2S,4R)-4-hydroxy-2-({(1R)-2-hydroxy-1-[4-(1H-1,2,4-triazol-1-yl)phenyl]ethyl}carbam oyl)pyrrolidin-1-yl]-3-methyl-1-oxobutan-2-yl}-1H-1,2,3-triazol-4-yl)phenyl]methoxy}-2-[( 2S)-2-methoxypropoxy]quinolin-4-yl}oxy)azetidine-1-carboxylate (195 mg), and the mixture was stirred at room temperature for an hour. TBAF (1M THF solution, 300 µL) was added at room temperature, and the mixture was stirred at room temperature overnight. The reaction mixture was concentrated under reduced pressure and purified by basic silica gel column chromatography (chloroform/MeOH), thus obtaining (4R)-1-[(2S)-2-(4-{4-[({(7M)-4-[(azetidin-3-yl)oxy]-6-cyclopropyl-7-[6-fluoro-5-methyl-1-( oxan-2-yl)-1H-indazol-4-yl]-2-[(2S)-2-methoxypropoxy]quinolin-8-yl}oxy)methyl]phenyl}-1H-1,2,3-triazol-1-yl)-3-methylbutanoyl]-4-hydroxy-N-{(1R)-2-hydroxy-1-[4-(1H-1,2,4-triaz ol-1-yl)phenyl]ethyl}-L-prolinamide (157 mg) as a foam-like solid.

### Production Example 195

To a THF (6 mL) solution of 2-(trimethylsilyl)ethyl 4-{[(7M)-6-cyclopropyl-4-[(1-{[2-(dimethylamino)ethyl]carbamoyl}azetidin-3-yl)oxy]-7-[6-fluoro-5-methyl-1-(oxan-2-yl)-1H-indazol-4-yl]-8-{[4-(1-{(2S)-1-[(2S,4R)-4-hydroxy-2-({(1 R)-2-hydroxy-1-[4-(4-methyl-1,3-thiazol-5-yl)phenyl]ethyl }carbamoyl)pyrrolidin-1-yl]-3-me thyl-1-oxobutan-2-yl}-1H-1,2,3-triazol-4-yl)phenyl]methoxy}quinazolin-2-yl]oxy}piperidine -1-carboxylate (317 mg), TBAF (1M THF solution, 800 µL) and acetic acid (30 µL) were added at room temperature, and the mixture was stirred under argon atmosphere at 60°C for 15 hours. After allowing to cool to room temperature, chloroform/MeOH (5/1) and saturated aqueous ammonium chloride solution were added, and the organic layer was separated. The organic layer was dried over anhydrous sodium sulfate, filtered and concentrated under reduced pressure. The residue was purified by basic silica gel column chromatography (chloroform/MeOH), thus obtaining 4R)-1-[(2S)-2-(4-{4-[({(7M)-6-cyclopropyl-4-[(1-{[2-(dimethylamino)ethyl]carbamoyl}azet idin-3-yl)oxy]-7-[6-fluoro-5-methyl-1-(oxan-2-yl)-1H-indazol-4-yl]-2-[(piperidin-4-yl)oxy]q uinazolin-8-yl}oxy)methyl]phenyl}-1H-1,2,3-triazol-1-yl)-3-methylbutanoyl]-4-hydroxy-N-{ (1R)-2-hydroxy-1-[4-(4-methyl-1,3-thiazol-5-yl)phenyl]ethyl}-L-prolinamide (348 mg) as a foam-like solid.

### Production Example 196

In 1,2-dichloroethane (15 mL), (4R)-1-[(2S)-2-(4-{4-[({6-cyclopropyl-4-[(1-{[2-(dimethylamino)ethyl]carbamoyl}azetidin-3 -yl)oxy]-7-[6-fluoro-5-methyl-1-(oxan-2-yl)-1H-indazol-4-yl]-2-[(oxan-4-yl)oxy]quinazolin-8-yl}oxy)methyl]phenyl}-1H-1,2,3-triazol-1-yl)-3-methylbutanoyl]-4-hydroxy-L-proline methyl ester (820 mg) was dissolved, and trimethyltin hydroxide (681 mg) was added. The mixture was stirred at 85°C for 24 hours. Water, saturated aqueous ammonium chloride solution and saturated aqueous sodium chloride solution were added at room temperature, and extraction with chloroform/iPrOH (4/1) was performed five times. The organic layer was dried over anhydrous magnesium sulfate, filtered and concentrated, thus obtaining (4R)-1-[(2S)-2-(4-{4-[({6-cyclopropyl-4-[(1-{[2-(dimethylamino)ethyl]carbamoyl}azetidin-3 -yl)oxy]-7-[6-fluoro-5-methyl-1-(oxan-2-yl)-1H-indazol-4-yl]-2-[(oxan-4-yl)oxy]quinazolin-8-yl}oxy)methyl]phenyl}-1H-1,2,3-triazol-1-yl)-3-methylbutanoyl]-4-hydroxy-L-proline (1119 mg) as a foam-like solid.

### Production Example 197

To a dichloromethane (5 mL) solution of (4R)-1-[(2S)-2-(4-{4-[({6-cyclopropyl-4-[(1-{[2-(dimethylamino)ethyl]carbamoyl}azetidin-3 -yl)oxy]-7-[6-fluoro-5-methyl-1-(oxan-2-yl)-1H-indazol-4-yl]-2-[(oxan-4-yl)oxy]quinazolin-8-yl}oxy)methyl]phenyl}-1H-1,2,3-triazol-1-yl)-3-methylbutanoyl]-4-hydroxy-L-proline (50 mg), HATU (27 mg), DIPEA (64 µL) and (2R)-2-amino-2-[4-(1H-pyrazol-1-yl)phenyl]ethan-1-ol n-hydrochloride (26 mg) were added at room temperature, and the mixture was stirred at room temperature overnight. The reaction mixture was concentrated under reduced pressure, and the residue was purified by basic silica gel column chromatography (from hexane/ethyl acetate to chloroform/MeOH), thus obtaining (4R)-1-[(2S)-2-(4-{4-[({6-cyclopropyl-4-[(1-{[2-(dimethylamino)ethyl]carbamoyl}azetidin-3 -yl)oxy]-7-[6-fluoro-5-methyl-1-(oxan-2-yl)-1H-indazol-4-yl]-2-[(oxan-4-yl)oxy]quinazolin-8-yl]oxy)methyl|phenyl]-1H-1,2,3-triazol-1-yl)-3-methylbutanoyl]-4-hydroxy-N-[(1R)-2-hy droxy-1-[4-(1H-pyrazol-1-yl)phenyl]ethyl}-L-prolinamide (34 mg) as a foam-like solid.

### Production Example 198

To a DMF (2 mL) solution of 4R)-1-[(2S)-2-(4-{4-[({(7M)-6-cyclopropyl-4-[(1-{[2-(dimethylamino)ethyl]carbamoyl}azet idin-3-yl)oxy]-7-[6-fluoro-5-methyl-1-(oxan-2-yl)-1H-indazol-4-yl]-2-[(piperidin-4-yl)oxy]q uinazolin-8-yl}oxy)methyl]phenyl}-1H-1,2,3-triazol-1-yl)-3-methylbutanoyl]-4-hydroxy-N-{ (1R)-2-hydroxy-1-[4-(4-methyl-1,3-thiazol-5-yl)phenyl]ethyl}-L-prolinamide (50 mg), DIPEA (40 µL) and 2,2-difluoroethyl triflate (15 µL) were added under ice cooling, and the mixture was stirred under argon atmosphere at the same temperature for an hour. Saturated aqueous sodium chloride solution and chloroform/MeOH (4/1) were added to the reaction mixture, and the organic layer was separated. The organic layer was dried over anhydrous sodium sulfate and filtered, and the filtrate was concentrated under reduced pressure, thus obtaining (4R)-1-[(2S)-2-(4-{4-[({(7M)-6-cyclopropyl-2-{[1-(2,2-difluoroethyl)piperidin-4-yl]oxy}-4-[ (1-{[2-(dimethylamino)ethyl]carbamoyl}azetidin-3-yl)oxy]-7-[6-fluoro-5-methyl-1-(oxan-2-yl)-1H-indazol-4-yl]quinazolin-8-yl}oxy)methyl]phenyl}-1H-1,2,3-triazol-1-yl)-3-methylbut anoyl]-4-hydroxy-N-{(1R)-2-hydroxy-1-[4-(4-methyl-1,3-thiazol-5-yl)phenyl]ethyl}-L-proli namide (58.5 mg) as an oily substance.

### Production Example 199

To a THF (1 mL) and MeCN (1 mL) solution of methyl 4-[({6-cyclopropyl-4-[(1-{[2-(dimethylamino)ethyl]carbamoyl}azetidin-3-yl)oxy]-7-[6-fluor o-5-methyl-1-(oxan-2-yl)-1H-indazol-4-yl]-2-[(2S)-2-methoxypropoxy]quinazolin-8-yl}oxy) methyl]benzoate (81 mg), sodium hydroxide (1M aqueous solution, 500 µL) was added at room temperature, and the mixture was stirred at 50°C for five hours. After hydrogen chloride (1M aqueous solution, 0.5 mL) and water were added, extraction with chloroform/iPrOH (4/1) was performed, and the organic layer was dried over anhydrous sodium sulfate, filtered and concentrated under reduced pressure, thus obtaining 4-[({6-cyclopropyl-4-[(1-{[2-(dimethylamino)ethyl]carbamoyl}azetidin-3-yl)oxy]-7-[6-fluor o-5-methyl-1-(oxan-2-yl)-1H-indazol-4-yl]-2-[(2S)-2-methoxypropoxy]quinazolin-8-yl}oxy) methyl]benzoic acid (72 mg) as a solid.

In the same manner as in the production methods of the Production Examples shown above, the compounds shown in the tables below were produced. In addition, the production methods, the structures and the physiochemical data of the compounds of the Production Examples are shown in the tables below.

Note that configurations for axial chirality of the compounds of Production Examples 27, 33, 37, 196, 197 and 199 were found as follows.

### Production Example 27:

(7M)-4-tert-butoxy-6-cyclopropyl-2-(ethanesulfonyl)-7-[6-fluoro-5-methyl-2-(triphenylmeth yl)-2H-indazol-4-yl]-8-[(1S)-1-phenylethoxy]quinazoline

### Production Example 33:

(7M)-4-tert-butoxy-6-cyclopropyl-2-(ethanesulfonyl)-7-[6-fluoro-5-methyl-2-(triphenylmeth yl)-2H-indazol-4-yl]quinazolin-8-ol

### Production Example 37:

(7M)-4-tert-butoxy-6-cyclopropyl-2-(ethanesulfonyl)-8-[(4-ethynylphenyl)methoxy]-7-[6-flu oro-5-methyl-2-(triphenylmethyl)-2H-indazol-4-yl]quinazoline

### Production Example 196:

4R)-1-[(2S)-2-(4-{4-[({(7M)-6-cyclopropyl-4-[(1-{[2-(dimethylamino)ethyl]carbamoyl}azet idin-3-yl)oxy]-7-[6-fluoro-5-methyl-1-(oxan-2-yl)-1H-indazol-4-yl]-2-[(oxan-4-yl)oxy]quina zolin-8-yl}oxy)methyl]phenyl}-1H-1,2,3-triazol-1-yl)-3-methylbutanoyl]-4-hydroxy-L-proli ne

### Production Example 197:

4R)-1-[(2S)-2-(4-{4-[({(7M)-6-cyclopropyl-4-[(1-{[2-(dimethylamino)ethyl]carbamoyl}azet idin-3-yl)oxy]-7-[6-fluoro-5-methyl-1-(oxan-2-yl)-1H-indazol-4-yl]-2-[(oxan-4-yl)oxy]quina zolin-8-yl}oxy)methyl]phenyl}-1H-1,2,3-triazol-1-yl)-3-methylbutanoyl]-4-hydroxy-N-{(1R )-2-hydroxy-1-[4-(1H-pyrazol-1-yl)phenyl]ethyl }-L-prolinamide

### Production Example 199:

4-[({(7M)-6-cyclopropyl-4-[(1-{[2-(dimethylamino)ethyl]carbamoyl}azetidin-3-yl)oxy]-7-[6 -fluoro-5-methyl-1-(oxan-2-yl)-1H-indazol-4-yl]-2-[(2S)-2-methoxypropoxy]quinazolin-8-yl }oxy)methyl]benzoic acid

### Example 1

Under ice cooling, methanesulfonic acid (180 µL) was added to an iPrOH (5 mL) solution of (1s,3R)-3-[(tert-butoxycarbonyl)amino]cyclobutyl 3-({(7M)-6-cyclopropyl-7-[6-fluoro-5-methyl-1-(oxan-2-yl)-1H-indazol-4-yl]-8-{[4-(1-{(2S) -1-[(2S,4R)-4-hydroxy-2-({ (1R)-2-hydroxy-1-[4-(4-methyl- 1,3-oxazol-5-yl)phenyl]ethyl } car bamoyl)pyrrolidin-1-yl]-3-methyl-1-oxobutan-2-yl}-1H-1,2,3-triazol-4-yl)phenyl]methoxy}-2-[(2S)-2-methoxypropoxy]quinazolin-4-yl}amino)azetidine-1-carboxylate (95 mg), and the mixture was stirred at 50°C for four hours. The reaction mixture was diluted with chloroform and then filtered through a short column filled with basic silica gel using chloroform/MeOH (9/1), and the filtrate was concentrated under reduced pressure.

THF (5 mL), formaldehyde (37% aqueous solution, 50 µL) and sodium triacetoxyborohydride (60 mg) were added to the residue under ice cooling, and the mixture was stirred at room temperature for 30 minutes. Saturated aqueous sodium hydrogen carbonate solution was added under ice cooling to stop the reaction. After dilution with chloroform/iPrOH (3/1), the organic layer and the aqueous layer were separated by a separation operation, and the aqueous layer was subjected to extraction with chloroform/iPrOH (3/1) three times. The collected organic layer was dried over anhydrous sodium sulfate, filtered and concentrated under reduced pressure. The residue was purified by ODS chromatography (0.1% formic acid MeCN/0.1% aqueous formic acid solution), and a fraction containing the target substance was collected. After dilution with chloroform/iPrOH (3/1) at room temperature, saturated aqueous sodium hydrogen carbonate solution was added. The organic layer and the aqueous layer were separated by a separation operation, and the aqueous layer was subjected to extraction with chloroform/iPrOH (3/1) three times. The collected organic layer was dried over anhydrous sodium sulfate, filtered and concentrated under reduced pressure. When the residue was dissolved in a small amount of dichloromethane/iPrOH (10/1) and hexane was added, a solid precipitated. The insoluble matter was taken by filtration, washed with water and then dried under reduced pressure at 50°C overnight, thus obtaining (1s,3R)-3-(dimethylamino)cyclobutyl 3-({(7M)-6-cyclopropyl-7-(6-fluoro-5-methyl-1H-indazol-4-yl)-8-{ [4-(1-{(2S)-1-[(2S,4R)-4-hydroxy-2-({(1R)-2-hydroxy-1-[4-(4-methyl-1,3-oxazol-5-yl)phenyl]ethyl}carbamoyl)pyrrol idin-1-yl]-3-methyl-1-oxobutan-2-yl}-1H-1,2,3-triazol-4-yl)phenyl]methoxy}-2-[(2S)-2-met hoxypropoxy]quinazolin-4-yl}amino)azetidine-1-carboxylate (33.9 mg) as a solid.

### Example 2

Under ice cooling, methanesulfonic acid (0.13 mL) was added to an iPrOH (5 mL) solution of (1s,3R)-3-[(tert-butoxycarbonyl)amino]cyclobutyl 3-({(7M)-6-cyclopropyl-7-[6-fluoro-5-methyl-1-(oxan-2-yl)-1H-indazol-4-yl]-8-{[4-(1-{(2S) -1-[(2S,4R)-4-hydroxy-2-({(1R)-2-hydroxy-1-[4-(1H-1,2,4-triazol-1-yl)phenyl]ethyl}carbam oyl)pyrrolidin-1-yl]-3-methyl-1-oxobutan-2-yl}-1H-1,2,3-triazol-4-yl)phenyl]methoxy}-2-[( 2S)-2-methoxypropoxy]quinazolin-4-yl}amino)azetidine-1-carboxylate (52 mg), and the mixture was stirred at 50°C for four hours. The reaction mixture was diluted with chloroform and then filtered through a short column filled with basic silica gel using chloroform/MeOH (9/1), and the filtrate was concentrated under reduced pressure.

THF (5 mL), formaldehyde (37% aqueous solution, 30 µL) and sodium triacetoxyborohydride (40 mg) were added to the residue under ice cooling, and the mixture was stirred at room temperature for 30 minutes. Saturated aqueous sodium hydrogen carbonate solution was added under ice cooling to stop the reaction. After dilution with chloroform/iPrOH (3/1), the organic layer and the aqueous layer were separated by a separation operation, and the aqueous layer was subjected to extraction with chloroform/iPrOH (3/1) three times. The collected organic layer was dried over anhydrous sodium sulfate, filtered and concentrated under reduced pressure. The residue was purified by ODS chromatography (0.1% formic acid MeCN/0.1% aqueous formic acid solution), and a fraction containing the target substance was collected. After dilution with chloroform/iPrOH (3/1) at room temperature, saturated aqueous sodium hydrogen carbonate solution was added. The organic layer and the aqueous layer were separated by a separation operation, and the aqueous layer was subjected to extraction with chloroform/iPrOH (3/1) three times. The collected organic layer was dried over anhydrous sodium sulfate, filtered and concentrated under reduced pressure. When the residue was dissolved in a small amount of dichloromethane/iPrOH (10/1) and hexane was added, a solid precipitated. The insoluble matter was taken by filtration, washed with water and then dried under reduced pressure at 50°C overnight, thus obtaining (1s,3R)-3-(dimethylamino)cyclobutyl 3-({(7M)-6-cyclopropyl-7-(6-fluoro-5-methyl-1H-indazol-4-yl)-8-{ [4-(1-{(2S)-1-[(2S,4R)-4-hydroxy-2-({(1R)-2-hydroxy-1-[4-(1H-1,2,4-triazol-1-yl)phenyl]ethyl}carbamoyl)pyrrolidin-1-yl]-3-methyl-1-oxobutan-2-yl}-1H-1,2,3-triazol-4-yl)phenyl]methoxy}-2-[(2S)-2-methoxy propoxy]quinazolin-4-yl}amino)azetidine-1-carboxylate (38.2 mg) as a solid.

### Example 5

DIPEA (220 µL) and triphosgene (31 mg) were added to a mixture of N,N-dimethyl-3-azetidinemethanamine dihydrochloride (60 mg) in dichloromethane (3 mL) under ice cooling, and the mixture was stirred under nitrogen atmosphere at room temperature for an hour. Under ice cooling, (4R)-1-[(2S)-2-(4-{4-[({(7M)-4-[(azetidin-3-yl)oxy]-6-cyclopropyl-7-[6-fluoro-5-methyl-1-( oxan-2-yl)-1H-indazol-4-yl]-2-[(2S)-2-methoxypropoxy]quinolin-8-yl}oxy)methyl]phenyl}-1H-1,2,3-triazol-1-yl)-3-methylbutanoyl]-4-hydroxy-N-{(1R)-2-hydroxy-1-[4-(1H-1,2,4-triaz ol-1-yl)phenyl]ethyl}-L-prolinamide (60 mg) was added, and the mixture was stirred under nitrogen atmosphere at room temperature for an hour. Saturated aqueous sodium hydrogen carbonate solution was added to the reaction mixture, and after extraction with chloroform three times, the organic layer was collected using a phase separator and concentrated. The residue was purified by basic silica gel column chromatography (chloroform/MeOH), and the target fraction was concentrated.

The residue was dissolved in MeOH (2 mL), and methanesulfonic acid (100 µL) was added at room temperature. The mixture was stirred under nitrogen atmosphere at room temperature overnight. The reaction mixture was concentrated and purified by ODS chromatography (MeCN/0.1% aqueous formic acid solution), and the target fraction was concentrated. The residue was dissolved in MeCN and water, and saturated aqueous sodium hydrogen carbonate solution was added. After extraction with chloroform/iPrOH (9/1) three times, the combined organic layer was dried over anhydrous sodium sulfate and filtered, and the filtrate was concentrated. The residue was suspended in ethyl acetate, and the insoluble matter was taken by filtration and dried under reduced pressure, thus obtaining (4R)-1-[(2S)-2-(4-{4-[({(7M)-6-cyclopropyl-4-[(1-{3-[(dimethylamino)methyl]azetidine-1-c arbonyl}azetidin-3-yl)oxy]-7-(6-fluoro-5-methyl-1H-indazol-4-yl)-2-[(2S)-2-methoxypropox y]quinolin-8-yl}oxy)methyl]phenyl}-1H-1,2,3-triazol-1-yl)-3-methylbutanoyl]-4-hydroxy-N-{(1R)-2-hydroxy-1-[4-(1H-1,2,4-triazol-1-yl)phenyl]ethyl}-L-prolinamide (33 mg) as a solid.

### Example 6

DIPEA (250 µL) and triphosgene (35 mg) were added to a mixture of N,N-dimethyl-3-azetidinemethanamine dihydrochloride (68 mg) in dichloromethane (3 mL) under ice cooling, and the mixture was stirred under nitrogen atmosphere at room temperature for an hour. Under ice cooling, (4R)-1-[(2S)-2-(4-{4-[({(7M)-4-[(azetidin-3-yl)oxy]-6-cyclopropyl-7-[6-fluoro-5-methyl-1-( oxan-2-yl)-1H-indazol-4-yl]-2-[(2S)-2-methoxypropoxy]quinolin-8-yl}oxy)methyl]phenyl}-1H-1,2,3-triazol-1-yl)-3-methylbutanoyl]-4-hydroxy-N-{ (1R)-2-hydroxy- 1-[4-(4-methyl-1,3-oxazol-5-yl)phenyl]ethyl}-L-prolinamide (52 mg) was added, and the mixture was stirred under nitrogen atmosphere at room temperature for an hour. Saturated aqueous sodium hydrogen carbonate solution was added to the reaction mixture, and after extraction with chloroform three times, the organic layer was collected using a phase separator and concentrated. The residue was purified by basic silica gel column chromatography (chloroform/MeOH), and the target fraction was concentrated.

The residue was dissolved in MeOH (3 mL), and methanesulfonic acid (150 µL) was added at room temperature. The mixture was stirred under nitrogen atmosphere at room temperature overnight. The reaction mixture was concentrated and purified by ODS chromatography (MeCN/0.1% aqueous formic acid solution), and the target fraction was concentrated. The residue was dissolved in MeCN and water, and saturated aqueous sodium hydrogen carbonate solution was added. After extraction with chloroform/iPrOH (9/1) three times, the combined organic layer was dried over anhydrous sodium sulfate and filtered, and the filtrate was concentrated. The residue was suspended in ethyl acetate, and the insoluble matter was taken by filtration and dried under reduced pressure, thus obtaining (4R)-1-[(2S)-2-(4-{4-[({(7M)-6-cyclopropyl-4-[(1-{3-[(dimethylamino)methyl]azetidine-1-c arbonyl}azetidin-3-yl)oxy]-7-(6-fluoro-5-methyl-1H-indazol-4-yl)-2-[(2S)-2-methoxypropox y]quinolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-oxazol-5-yl)phenyl]ethyl}-L-prolinamide (30 mg) as a solid.

### Example 11

Under ice cooling, formaldehyde (37% aqueous solution, 0.4 mL) and sodium triacetoxyborohydride (500 mg) were added to a THF (15 mL) solution of (1s,3R)-3-aminocyclobutyl 3-({(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrol idin-1-yl]-3-methyl-1-oxobutan-2-yl}-1H-1,2,3-triazol-4-yl)phenyl]methoxy}-2-[(2S)-2-met hoxypropoxy]quinazolin-4-yl}amino)azetidine-1-carboxylate (769 mg), and the mixture was stirred at room temperature for an hour. Saturated aqueous sodium hydrogen carbonate solution was added under ice cooling to stop the reaction. After dilution with chloroform/iPrOH (3/1) at room temperature, the organic layer and the aqueous layer were separated by a separation operation, and the aqueous layer was subjected to extraction with chloroform/iPrOH (3/1) three times. The collected organic layer was dried over anhydrous sodium sulfate, filtered and concentrated under reduced pressure. The residue was purified by ODS chromatography (0.1% formic acid MeCN/0.1% aqueous formic acid solution) twice, and a fraction containing the target substance was collected. Chloroform/iPrOH (3/1) and saturated aqueous sodium hydrogen carbonate solution were added at room temperature. The organic layer and the aqueous layer were separated by a separation operation, and the aqueous layer was subjected to extraction with chloroform/iPrOH (3/1) three times. The collected organic layer was dried over anhydrous sodium sulfate, filtered and concentrated under reduced pressure. When the residue was dissolved in a small amount of ethyl acetate/iPrOH (10/1) and hexane was added, a solid precipitated. The insoluble matter was taken by filtration, washed with water and then dried under reduced pressure at 50°C overnight, thus obtaining (1s,3R)-3-(dimethylamino)cyclobutyl 3-({(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrol idin-1-yl]-3-methyl-1-oxobutan-2-yl}-1H-1,2,3-triazol-4-yl)phenyl]methoxy}-2-[(2S)-2-met hoxypropoxy]quinazolin-4-yl}amino)azetidine-1-carboxylate (370 mg) as a solid.

### Example 12

In THF (15 mL), N,N-dimethylethylenediamine (230 µL) was dissolved, and CDI (335 mg) was added under ice cooling. The mixture was stirred at room temperature for an hour. Under ice cooling, an iPrOH (15 mL) solution of (4R)-1-[(2S)-2-(4-{4-[({(7M)-4-[(azetidin-3-yl)oxy]-6-cyclopropyl-7-(6-fluoro-5-methyl-1H-indazol-4-yl)-2-[(2S)-2-methoxypropoxy]quinolin-8-yl}oxy)methyl]phenyl}-1H-1,2,3-triazol -1-yl)-3-methylbutanoyl]-4-hydroxy-N-{(1R)-2-hydroxy-1-[4-(1H-1,2,4-triazol-1-yl)phenyl] ethyl}-L-prolinamide (720 mg) was added, and the mixture was stirred at room temperature for 30 minutes and at 50°C for an hour. The reaction mixture was allowed to cool, and saturated aqueous sodium hydrogen carbonate solution was added. The mixture was subjected to extraction with chloroform/iPrOH (9/1) three times, dried over anhydrous sodium sulfate, filtered and concentrated. The residue was purified by ODS chromatography (MeCN/0.1% aqueous formic acid solution), and the target fraction was concentrated to around 10 mL. Saturated aqueous sodium hydrogen carbonate solution was added to the residue, and the mixture was stirred at room temperature for an hour. The insoluble matter was taken by filtration, washed with water and dried under reduced pressure at 40°C, thus obtaining 4R)-1-[(2S)-2-(4-{4-[({(7M)-6-cyclopropyl-4-[(1-{[2-(dimethylamino)ethyl]carbamoyl}azet idin-3-yl)oxy]-7-(6-fluoro-5-methyl-1H-indazol-4-yl)-2-[(2S)-2-methoxypropoxy]quinolin-8-yl}oxy)methyl]phenyl}-1H-1,2,3-triazol-1-yl)-3-methylbutanoyl]-4-hydroxy-N-{(1R)-2-hydr oxy-1-[4-(1H-1,2,4-triazol-1-yl)phenyl]ethyl}-L-prolinamide (501 mg) as a solid.

### Example 13

Triphosgene (215 mg) was dissolved in dichloromethane (30 mL), and (1s,3s)-3-(dimethylamino)cyclobutan-1-ol (265 mg) and DIPEA (1.28 mL) were added under ice cooling. The mixture was stirred under nitrogen atmosphere under ice cooling for 20 minutes and at room temperature for 30 minutes, and thus a solution of an active substance was prepared. The prepared solution of the active substance was added dropwise to a mixture of (4R)-1-[(2S)-2-(4-{4-[({(7M)-4-[(azetidin-3-yl)oxy]-6-cyclopropyl-7-(6-fluoro-5-methyl-1H-indazol-4-yl)-2-[(2S)-2-methoxypropoxy]quinolin-8-yl}oxy)methyl]phenyl}-1H-1,2,3-triazol -1-yl)-3-methylbutanoyl]-4-hydroxy-N-{(1R)-2-hydroxy-1-[4-(1H-1,2,4-triazol-1-yl)phenyl] ethyl}-L-prolinamide (800 mg) in dichloromethane (20 mL) under ice cooling, and the mixture was stirred under nitrogen atmosphere under ice cooling for 30 minutes. Piperidine (160 µL) was added under ice cooling, and the mixture was stirred under ice cooling for 10 minutes and at room temperature for 30 minutes. Saturated aqueous sodium hydrogen carbonate solution was added to the reaction mixture, and after extraction with chloroform/iPrOH (9/1) three times, the organic layer was collected using a phase separator and concentrated. The residue was purified by ODS chromatography (MeCN/0.1% aqueous formic acid solution), and the target fraction was concentrated to around 5 mL. Saturated aqueous sodium hydrogen carbonate solution was added to the residue, and the mixture was stirred at room temperature for an hour. The insoluble matter was taken by filtration and dried under reduced pressure, thus obtaining (1s,3R)-3-(dimethylamino)cyclobutyl 3-({(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(1H-1,2,4-triazol-1-yl)phenyl]ethyl}carbamoyl)pyrrolidin-1-yl]-3-methyl-1-oxobutan-2-yl}-1H-1,2,3-triazol-4-yl)phenyl]methoxy}-2-[(2S)-2-methoxy propoxy]quinolin-4-yl}oxy)azetidine-1-carboxylate (635 mg) as a solid.

### Example 14

In dichloromethane (2 mL), N,N-dimethyl-3-azetidinemethanamine dihydrochloride (47 mg) was suspended, and DIPEA (200 µL) and triphosgene (24 mg) were added under ice cooling. The mixture was stirred under nitrogen atmosphere at room temperature for an hour. Under ice cooling, (4R)-1-[(2S)-2-(4-{4-[({(7M)-4-[(azetidin-3-yl)oxy]-6-cyclopropyl-7-(6-fluoro-5-methyl-1H-indazol-4-yl)-2-[(2S)-2-methoxypropoxy]quinolin-8-yl}oxy)methyl]phenyl}-1H-1,2,3-triazol -1-yl)-3-methylbutanoyl]-4-hydroxy-N-{(1R)-2-hydroxy-1-[4-(4-methyl-1,3-thiazol-5-yl)phe nyl]ethyl}-L-prolinamide (90 mg) was added, and the mixture was stirred under nitrogen atmosphere under ice cooling for an hour. In another flask, N,N-dimethyl-3-azetidinemethanamine dihydrochloride (47 mg) was suspended in dichloromethane (2 mL), and DIPEA (200 µL) and triphosgene (24 mg) were added under ice cooling. The mixture was stirred under nitrogen atmosphere at room temperature for an hour. This was added to the reaction mixture under ice cooling, and the mixture was stirred under nitrogen atmosphere under ice cooling for an hour and at room temperature for 30 minutes. Piperidine (60 µL) was added under ice cooling, and the mixture was stirred under ice cooling for 10 minutes and at room temperature for 30 minutes. Saturated aqueous sodium hydrogen carbonate solution was added to the reaction mixture, and after extraction with chloroform/iPrOH (9/1) three times, the organic layer was collected using a phase separator and concentrated. The residue was purified by ODS chromatography (MeCN/0.1% aqueous formic acid solution), and the target fraction was concentrated. The residue was dissolved in MeCN/water, and saturated aqueous sodium hydrogen carbonate solution was added. After extraction with chloroform/iPrOH (9/1) three times, the combined organic layer was dried over anhydrous sodium sulfate and filtered, and the filtrate was concentrated, thus obtaining (4R)-1-[(2S)-2-(4-{4-[({(7M)-6-cyclopropyl-4-[(1-{3-[(dimethylamino)methyl]azetidine-1-c arbonyl}azetidin-3-yl)oxy]-7-(6-fluoro-5-methyl-1H-indazol-4-yl)-2-[(2S)-2-methoxypropox y]quinolin-8-yl}oxy)methyl]phenyl}-1H-1,2,3-triazol-1-yl)-3-methylbutanoyl]-4-hydroxy-N-{(1R)-2-hydroxy-1-[4-(4-methyl-1,3-thiazol-5-yl)phenyl]ethyl}-L-prolinamide (28 mg) as a solid.

### Example 15

TFA (300 µL) was added to a dichloromethane (3 mL) solution of (4R)-1-{(2S)-2-[4-(4-{[(6-cyclopropyl-7-[6-fluoro-5-methyl-1-(oxan-2-yl)-1H-indazol-4-yl]-2-[(oxan-4-yl)oxy]-4-{[1-(pyrimidin-2-yl)azetidin-3-yl]amino}quinazolin-8-yl)oxy]methyl}p henyl)-1H-1,2,3-triazol-1-yl]-3-methylbutanoyl}-4-hydroxy-N-{(1R)-2-hydroxy-1-[4-(4-met hyl-1,3-thiazol-5-yl)phenyl]ethyl}-L-prolinamide (76 mg) at room temperature, and the mixture was stirred under nitrogen atmosphere at room temperature for four hours. The reaction mixture was concentrated under reduced pressure, and THF (3 mL), ice and saturated aqueous sodium hydrogen carbonate solution (3 mL) were added to the residue. The mixture was stirred at room temperature for 30 minutes. The reaction mixture was subjected to extraction with chloroform/MeOH (10/1) three times and filtered using a phase separator. The filtrate was concentrated under reduced pressure, and the residue was purified by basic silica gel column chromatography (chloroform/MeOH), thus obtaining (4R)-1-[(2S)-2-{4-[4-({ [6-cyclopropyl-7-(6-fluoro-5-methyl-1H-indazol-4-yl)-2-[(oxan-4-yl) oxy]-4-{[1-(pyrimidin-2-yl)azetidin-3-yl]amino}quinazolin-8-yl]oxy}methyl)phenyl]-1H-1,2 ,3-triazol-1-yl }-3-methylbutanoyl]-4-hydroxy-N-{ (1R)-2-hydroxy- 1-[4-(4-methyl- 1,3-thiazol -5-yl)phenyl]ethyl}-L-prolinamide (37 mg) as a foam-like solid.

### Example 28

Under ice cooling, TFA (0.3 mL) was added to a dichloromethane (10 mL) solution of (4R)-1-{(2S,3S)-2-[4-(4-{[(6-cyclopropyl-4-{[(3S)-1-{[2-(dimethylamino)ethyl]carbamoyl}p yrrolidin-3-yl]oxy}-7-[6-fluoro-5-methyl-1-(oxan-2-yl)-1H-indazol-4-yl]-2-[(2S)-2-methoxy propoxy]quinazolin-8-yl)oxy]methyl}phenyl)-1H-1,2,3-triazol-1-yl]-3-methylpentanoyl}-4-h ydroxy-N-{(1R)-2-hydroxy-1-[4-(4-methyl-1,3-thiazol-5-yl)phenyl]ethyl}-L-prolinamide (260 mg), and the mixture was stirred at room temperature overnight. The reaction mixture was concentrated under reduced pressure, and the residue was dissolved in THF (5 mL). Saturated aqueous sodium hydrogen carbonate solution was added under ice cooling, and the mixture was stirred under ice cooling for 30 minutes. The reaction mixture was diluted with chloroform and water, and the organic layer and the aqueous layer were separated by a separation operation. The aqueous layer was subjected to extraction with chloroform/iPrOH (3/1) three times. The collected organic layer was dried over anhydrous sodium sulfate, filtered and concentrated under reduced pressure. The residue was purified by ODS chromatography (0.1% formic acid MeCN/0.1% aqueous formic acid solution), and a fraction including a peak at the low polarity side was collected and concentrated under reduced pressure to a liquid volume of 2-5 mL. When saturated aqueous sodium hydrogen carbonate solution was added to the residue under ice cooling, a solid precipitated. The insoluble matter was taken by filtration, washed with water and then dried under reduced pressure at 50°C overnight, thus obtaining (4R)-1-{(2S,3S)-2-[4-(4-{ [(6-cyclopropyl-4-{ [(3S)-1-{ [2-(dimethylamino)ethyl]carbamoyl}p yrrolidin-3-yl]oxy}-7-(6-fluoro-5-methyl-1H-indazol-4-yl)-2-[(2S)-2-methoxypropoxy]quina zolin-8-yl)oxy]methyl}phenyl)-1H-1,2,3-triazol-1-yl]-3-methylpentanoyl}-4-hydroxy-N-{(1 R)-2-hydroxy-1-[4-(4-methyl-1,3-thiazol-5-yl)phenyl]ethyl}-L-prolinamide (a single diastereomer with undetermined configuration of axial chirality, 41.2 mg) as a solid.

### Example 34

To a mixture of 2-(dimethylamino)ethyl 3-({(7M)-6-cyclopropyl-8-[(4-ethynylphenyl)methoxy]-7-[6-fluoro-5-methyl-1-(oxan-2-yl)-1 H-indazol-4-yl]-2-[(2S)-2-methoxypropoxy]quinazolin-4-yl}amino)azetidine-1-carboxylate (35 mg) in THF (1 mL), tBuOH (1 mL) and water (1 mL), (4R)-1-[(2S)-2-azido-3-methylbutanoyl]-4-hydroxy-N-{(1R)-2-hydroxy-1-[4-(4-methyl-1,3-t hiazol-5-yl)phenyl]ethyl}-L-prolinamide (21 mg), sodium ascorbate (19 mg) and anhydrous copper(II) sulfate (7 mg) were added at room temperature. The mixture was stirred under argon atmosphere at room temperature for two hours, and disodium ethylenediamine tetraacetate (15 mg) and ethyl acetate (about 5 mL) were added to the reaction mixture. The mixture was stirred vigorously at room temperature for an hour. Saturated aqueous sodium hydrogen carbonate solution and saturated aqueous sodium chloride solution were added, and extraction with chloroform/iPrOH (4/1) was performed three times. The organic layer was dried over anhydrous sodium sulfate, filtered and concentrated. The residue was purified by silica gel column chromatography (chloroform/MeOH).

The resulting residue was dissolved in dichloromethane (1 mL), and TFA (1 mL) was added at room temperature. The mixture was stirred for three hours. The reaction mixture was concentrated, and THF (3 mL) and saturated aqueous sodium hydrogen carbonate solution were added to the residue. The mixture was stirred for an hour. Saturated aqueous sodium hydrogen carbonate solution and saturated aqueous sodium chloride solution were added, and the reaction mixture was subjected to extraction with chloroform/iPrOH (4/1) three times. The organic layer was dried over anhydrous sodium sulfate, filtered and concentrated under reduced pressure. The residue was purified by ODS chromatography (MeCN/0.1% aqueous formic acid solution), and the target fraction was collected. After saturated aqueous sodium hydrogen carbonate solution was added, extraction with chloroform/iPrOH (4/1) was performed three times. The organic layer was dried over anhydrous sodium sulfate, filtered and concentrated, thus obtaining 2-(dimethylamino)ethyl 3-({(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrol idin-1-yl]-3-methyl-1-oxobutan-2-yl}-1H-1,2,3-triazol-4-yl)phenyl]methoxy}-2-[(2S)-2-met hoxypropoxy]quinazolin-4-yl}amino)azetidine-1-carboxylate (12 mg) as a solid.

### Example 39

A mixture of (4R)-1-[(2S)-2-(4-{4-[({6-cyclopropyl-4-[(1-{[2-(dimethylamino)ethyl]carbamoyl}azetidin-3 -yl)oxy]-7-[6-fluoro-5-methyl-1-(oxan-2-yl)-1H-indazol-4-yl]-2-[(oxan-4-yl)oxy]quinazolin-8-yl}oxy)methyl]phenyl}-1H-1,2,3-triazol-1-yl)-3-methylbutanoyl]-4-hydroxy-L-proline (50 mg), DIPEA (50 µL), (3S)-3-amino-3-[4-(4-methyl-1,3-thiazol-5-yl)phenyl]propan-1-ol n-hydrochloride (30 mg), HATU (30 mg) and dichloromethane (3 mL) was stirred at room temperature overnight. The reaction mixture was concentrated under reduced pressure, and the residue was purified by basic silica gel column chromatography (chloroform/MeOH).

TFA (75 µL) was added to a dichloromethane (3 mL) solution of the resulting solid, and the mixture was stirred at room temperature overnight. The reaction mixture was concentrated under reduced pressure, and the residue was dissolved in THF (3 mL). Saturated aqueous sodium hydrogen carbonate solution was added under ice cooling, and the mixture was stirred at room temperature for an hour. Chloroform and water were added to the reaction mixture, and after separation with a phase separator, the aqueous layer was subjected to extraction with chloroform/MeOH (4/1) three times. The filtrate was concentrated under reduced pressure, and the residue was purified by ODS chromatography (MeCN/0.1% aqueous formic acid solution). The target fraction was concentrated. Saturated aqueous sodium hydrogen carbonate solution was added to the residue, and after extraction with chloroform/iPrOH (5/1) twice, the collected organic layer was concentrated under reduced pressure, thus obtaining (4R)-1-[(2S)-2-(4-{4-[({6-cyclopropyl-4-[(1-{[2-(dimethylamino)ethyl]carbamoyl}azetidin-3 -yl)oxy]-7-(6-fluoro-5-methyl-1H-indazol-4-yl)-2-[(oxan-4-yl)oxy]quinazolin-8-yl}oxy)meth yl]phenyl}-1H-1,2,3-triazol-1-yl)-3-methylbutanoyl]-4-hydroxy-N-{(1S)-3-hydroxy-1-[4-(4-methyl-1,3-thiazol-5-yl)phenyl]propyl}-L-prolinamide (14.8 mg) as a solid.

### Example 40

To a DMF (2 mL) solution of 4-[({6-cyclopropyl-4-[(1-{[2-(dimethylamino)ethyl]carbamoyl}azetidin-3-yl)oxy]-7-[6-fluor o-5-methyl-1-(oxan-2-yl)-1H-indazol-4-yl]-2-[(2S)-2-methoxypropoxy]quinazolin-8-yl}oxy) methyl]benzoic acid (29 mg), L-valyl-(4R)-4-hydroxy-N-{(1R)-2-hydroxy-1-[4-(4-methyl-1,3-thiazol-5-yl)phenyl]ethyl}-L -prolinamide n-hydrochloride (26 mg), DIPEA (60 µL) and PyBOP (25 mg) were added under ice cooling, and the mixture was stirred under argon atmosphere at room temperature for three hours. Saturated aqueous sodium hydrogen carbonate solution and chloroform/MeOH (7/1) were added to the reaction mixture, and extraction with chloroform/MeOH (7/1) was performed twice using a phase separator. The filtrate was concentrated under reduced pressure, and the residue was purified by basic silica gel column chromatography (chloroform/MeOH).

The resulting foam-like solid was dissolved in dichloromethane (3 mL), and TFA (220 µL) was added at room temperature. The mixture was stirred at room temperature overnight. The reaction mixture was concentrated under reduced pressure, and THF (3 mL), ice and saturated aqueous sodium hydrogen carbonate solution (3 mL) were added to the residue. The mixture was stirred at room temperature for an hour. The reaction mixture was subjected to extraction with chloroform/MeOH (7/1) three times, filtered using a phase separator and concentrated under reduced pressure. The residue was purified by ODS chromatography (MeOH/aqueous ammonium hydrogen carbonate solution), thus obtaining N-{4-[({6-cyclopropyl-4-[(1-{[2-(dimethylamino)ethyl]carbamoyl}azetidin-3-yl)oxy]-7-(6-fl uoro-5-methyl-1H-indazol-4-yl)-2-[(2S)-2-methoxypropoxy]quinazolin-8-yl}oxy)methyl]ben zoyl}-L-valyl-(4R)-4-hydroxy-N-{(1R)-2-hydroxy-1-[4-(4-methyl-1,3-thiazol-5-yl)phenyl]et hyl}-L-prolinamide (5 mg) as a solid.

### Example 41

DIPEA (90 µL), 1-hydroxybenzotriazole (15 mg) and 1-(3-dimethylaminopropyl)-3-ethylcarbodiimide hydrochloride (21 mg) were added to a DMF (2 mL) solution of (4R)-1-[(2S)-2-(4-{4-[({(7M)-4-[(azetidin-3-yl)oxy]-6-cyclopropyl-7-(6-fluoro-5-methyl-1H-indazol-4-yl)-2-[(2S)-2-methoxypropoxy]quinolin-8-yl}oxy)methyl]phenyl}-1H-1,2,3-triazol -1-yl)-3-methylbutanoyl]-4-hydroxy-N-{(1R)-2-hydroxy-1-[4-(1H-1,2,4-triazol-1-yl)phenyl] ethyl}-L-prolinamide (80 mg) and 4-(dimethylamino)butanoic acid hydrochloride (25 mg), and mixture was stirred under argon atmosphere at room temperature overnight. Chloroform/iPrOH (4/1) was added, and water and saturated aqueous sodium chloride solution were added. The organic layer and the aqueous layer were separated, and the aqueous layer was subjected to extraction with ethyl acetate. The combined organic layer was dried over anhydrous sodium sulfate. After filtration and concentration under reduced pressure, the residue was purified by ODS chromatography (MeCN/0.1% aqueous formic acid solution). Saturated aqueous sodium hydrogen carbonate solution was added to the target fraction, and after extraction with chloroform/iPrOH (4/1) twice, the organic layer was dried over anhydrous sodium sulfate, filtered and concentrated under reduced pressure. The residue was washed with hexane and dried under reduced pressure, thus obtaining (4R)-1-[(2S)-2-(4-{4-[({(7M)-6-cyclopropyl-4-({1-[4-(dimethylamino)butanoyl]azetidin-3-yl }oxy)-7-(6-fluoro-5-methyl-1H-indazol-4-yl)-2-[(2S)-2-methoxypropoxy]quinolin-8-yl}oxy) methyl]phenyl}-1H-1,2,3-triazol-1-yl)-3-methylbutanoyl]-4-hydroxy-N-{(1R)-2-hydroxy-1-[ 4-(1H-1,2,4-triazol-1-yl)phenyl]ethyl}-L-prolinamide (28 mg) as a solid.

### Example 43

Anhydrous copper(II) sulfate (13 mg) and sodium ascorbate (33 mg) were added to a tBuOH (1 mL), THF (1 mL) and water (1 mL) solution of tert-butyl (2-{[3-({(7M)-6-cyclopropyl-8-[(4-ethynylphenyl)methoxy]-7-[6-fluoro-5-methyl-1-(oxan-2-yl)-1H-indazol-4-yl]-2-[(2S)-2-methoxypropoxy]quinolin-4-yl}oxy)azetidine-1-carbonyl]ami no }ethyl)methylcarbamate (60 mg) and (4R)-1-[(2S)-2-azido-3-methylbutanoyl]-4-hydroxy-N-{(1R)-2-hydroxy-1-[4-(4-methyl-1,3-t hiazol-5-yl)phenyl]ethyl}-L-prolinamide (40 mg) at room temperature, and the mixture was stirred under nitrogen atmosphere at room temperature for 20 minutes. Basic silica gel was added to the reaction mixture, and after concentration, the residue was purified by basic silica gel column chromatography (chloroform/MeOH). The target fraction was concentrated.

The residue was dissolved in MeOH (2 mL), and methanesulfonic acid (50 µL) was added at room temperature. The mixture was stirred at room temperature for 30 minutes and at 50°C for six hours. The reaction mixture was concentrated and purified by ODS chromatography (MeCN/0.1% aqueous formic acid solution), and the target fraction was concentrated. The residue was dissolved in MeCN/water, and saturated aqueous sodium hydrogen carbonate solution was added. After extraction with chloroform/iPrOH (9/1) twice, the combined organic layer was dried over anhydrous sodium sulfate, filtered and concentrated.

The residue was suspended in THF (2 mL), and formaldehyde (37% aqueous solution, 50 µL) and sodium triacetoxyborohydride (45 mg) were added at room temperature. After the mixture was stirred at room temperature for 10 minutes, water (0.1 mL) was added, and the mixture was stirred at room temperature for 30 minutes. Saturated aqueous sodium hydrogen carbonate solution was added to the reaction mixture, and after extraction with chloroform/iPrOH (9/1) three times, the combined organic layer was separated with a phase separator and concentrated. The residue was purified by basic silica gel column chromatography (chloroform/MeOH), and the target fraction was concentrated. The residue was suspended in water, filtered and dried under reduced pressure, thus obtaining 4R)-1-[(2S)-2-(4-{4-[({(7M)-6-cyclopropyl-4-[(1-{[2-(dimethylamino)ethyl]carbamoyl}azet idin-3-yl)oxy]-7-(6-fluoro-5-methyl-1H-indazol-4-yl)-2-[(2S)-2-methoxypropoxy]quinolin-8-yl}oxy)methyl]phenyl}-1H-1,2,3-triazol-1-yl)-3-methylbutanoyl]-4-hydroxy-N-{(1R)-2-hydr oxy-1-[4-(4-methyl-1,3-thiazol-5-yl)phenyl]ethyl}-L-prolinamide (15 mg) as a solid.

In the same manner as in the production methods of the Examples shown above, the compounds of the Examples shown in the tables below were produced. In addition, the production methods and the physiochemical data of the compounds of the Examples are shown in the tables below.

Note that configurations for axial chirality of the compounds of Production Examples 15, 28, 39 and 40 were found as follows.

### Example compound 15:

(4R)-1-[(2S)-2-{4-[4-({[(7M)-6-cyclopropyl-7-(6-fluoro-5-methyl-1H-indazol-4-yl)-2-[(oxan -4-yl)oxy]-4-{[1-(pyrimidin-2-yl)azetidin-3-yl]amino}quinazolin-8-yl]oxy}methyl)phenyl]-1 H-1,2,3-triazol-1-yl}-3-methylbutanoyl]-4-hydroxy-N-{(1R)-2-hydroxy-1-[4-(4-methyl-1,3-t hiazol-5-yl)phenyl]ethyl}-L-prolinamide

### Example compound 28:

(4R)-1-[(2S,3S)-2-(4-{4-[({(7M)-6-cyclopropyl-4-{ [(3S)-1-{ [2-(dimethylamino)ethyl]carba moyl}pyrrolidin-3-yl]oxy}-7-(6-fluoro-5-methyl-1H-indazol-4-yl)-2-[(2S)-2-methoxypropox y]quinazolin-8-yl}oxy)methyl]phenyl}-1H-1,2,3-triazol-1-yl)-3-methylpentanoyl]-4-hydroxy -N-{(1R)-2-hydroxy-1-[4-(4-methyl-1,3-thiazol-5-yl)phenyl]ethyl}-L-prolinamide

### Example compound 39:

4R)-1-[(2S)-2-(4-{4-[({(7M)-6-cyclopropyl-4-[(1-{[2-(dimethylamino)ethyl]carbamoyl}azet idin-3-yl)oxy]-7-(6-fluoro-5-methyl-1H-indazol-4-yl)-2-[(oxan-4-yl)oxy]quinazolin-8-yl}oxy )methyl]phenyl }-1H-1,2,3-triazol-1-yl)-3-methylbutanoyl]-4-hydroxy-N-{(1S)-3-hydroxy- 1-[4-(4-methyl-1,3-thiazol-5-yl)phenyl]propyl}-L-prolinamide

### Example compound 40:

N-{4-[({(7M)-6-cyclopropyl-4-[(1-{[2-(dimethylamino)ethyl]carbamoyl}azetidin-3-yl)oxy]-7-(6-fluoro-5-methyl-1H-indazol-4-yl)-2-[(2S)-2-methoxypropoxy]quinazolin-8-yl}oxy)meth yl]benzoyl }-L-valyl -(4R)-4-hydroxy-N-{(1R)-2-hydroxy-1-[4-(4-methyl-1,3-thiazol-5-yl)phenyl]ethyl}-L-prolin amide

In the tables presented below, the following abbreviations are sometimes used.

PEx: Production Example No., Ex: Example No., PSyn: Production Example No. produced by the same method, Syn: Example No. produced by the same method (for example, Syn 1 represents that it was produced by the same method as for Example 1), Str: chemical structural formula (A compound with "#" in the chemical structural formula represents that the compound is a mixture of diastereomers with axial chirality of about 3.5:1. A compound with "##" in the chemical structural formula represents that the axial chirality of the compound is single but the configuration is undetermined. A compound with "###" in the chemical structural formula represents that the compound is a mixture of diastereomers with axial chirality of about 3.5:1 but the configuration is undetermined.). n HCl: n-hydrochloride (A compound with a Production Example No. represents a monohydrochloride to a trihydrochloride.), n TFA: n-trifluoroacetate (A compound with a Production Example No. represents a monotrifluoroacetate to tritrifluoroacetate.), DAT: physiochemical data, ESI+: m/z value in mass spectrometry (ionization method ESI, [M+H]⁺ unless otherwise specified), ESI-: m/z value in mass spectrometry (ionization method ESI, [M-H]⁻ unless otherwise specified), NMR: δ value (ppm) of peak in ¹H-NMR (500 MHz) in DMSO-d₆ at 27°C, NMR (100°C): δ value (ppm) of peak in ¹H-NMR (500 MHz) in DMSO-d₆ at 100°C, s: singlet (spectrum), d: doublet (spectrum), dd: double doublet (spectrum), ddd: double double doublet (spectrum), t: triplet (spectrum), dt: double triplet (spectrum), q: quartet (spectrum), m: multiplet (spectrum), br: broad (spectrum).

**[Table 5-1]**

| PEx | Str | PEx | Str |
|---|---|---|---|
| 1 | 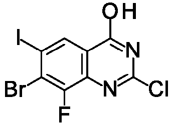 | 7 | 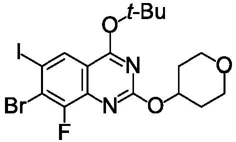 |
| 2 | 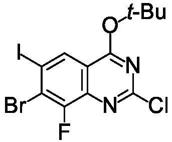 | 8 | 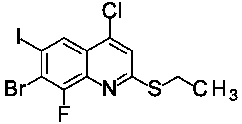 |
| 3 | 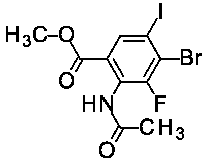 | 9 | 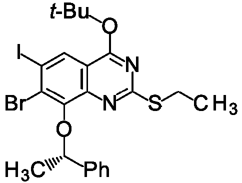 |
| 4 | 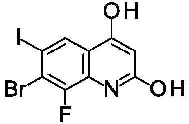 | 10 | 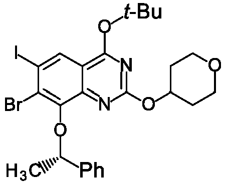 |
| 5 | 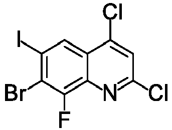 | 11 | 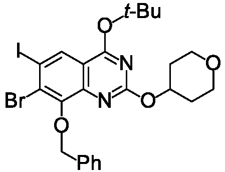 |
| 6 | 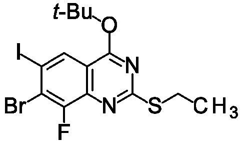 | 12 | 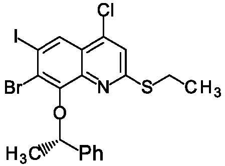 |

**[Table 5-2]**

| PEx | Str | PEx | Str |
|---|---|---|---|
| 13 | 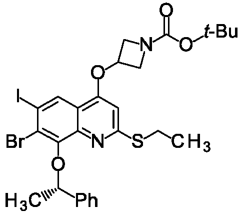 | 19 | 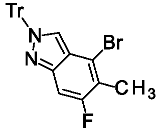 |
| 14 | 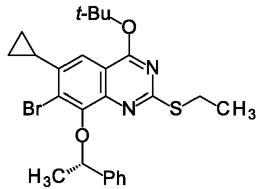 | 20 | 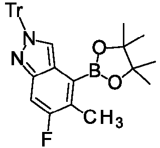 |
| 15 | 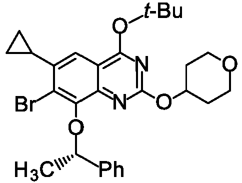 | 21 | 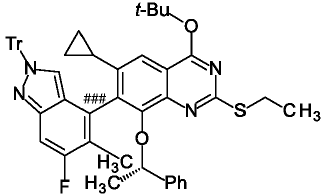 |
| 16 | 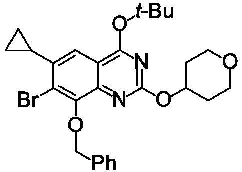 | 22 | 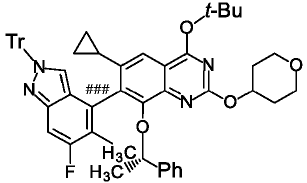 |
| 17 | 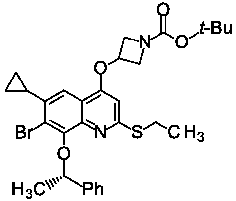 | 23 | 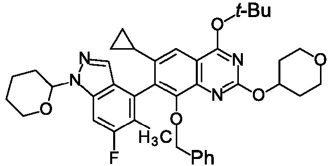 |
| 18 | 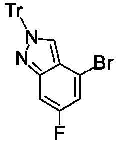 | 24 | 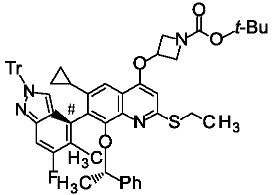 |

**[Table 5-3]**

| PEx | Str |
|---|---|
| 25 | 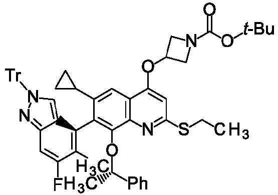 |
| 26 | 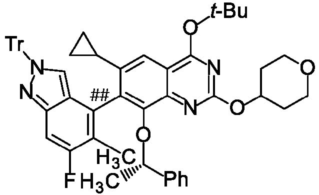 |
| 27 | 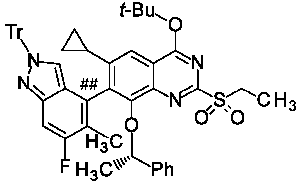 |
| 28 | 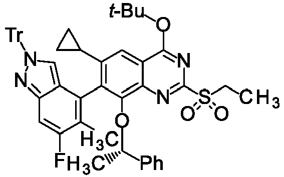 |
| 29 | 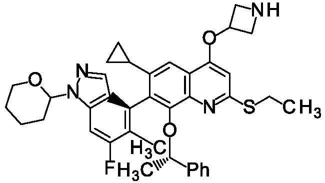 |
| 30 | 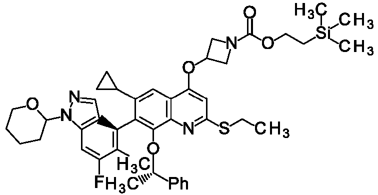 |

**[Table 5-4]**

| PEx | Str |
|---|---|
| 31 | 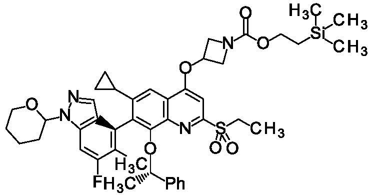 |
| 32 | 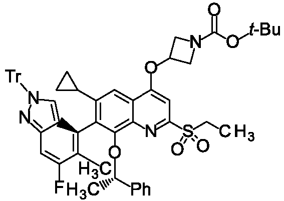 |
| 33 | 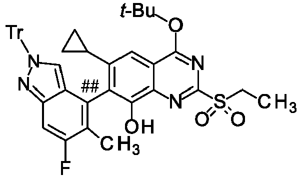 |
| 34 | 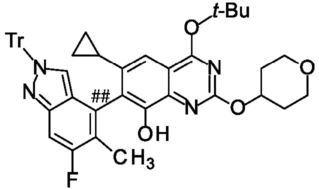 |
| 35 | 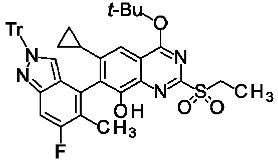 |
| 36 | 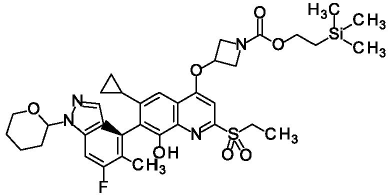 |

**[Table 5-5]**

| PEx | Str |
|---|---|
| 37 | 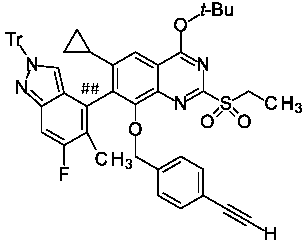 |
| 38 | 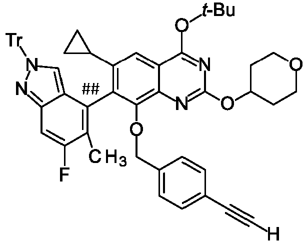 |
| 39 | 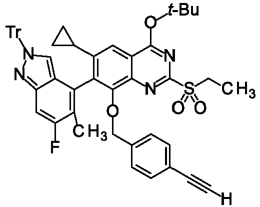 |
| 40 | 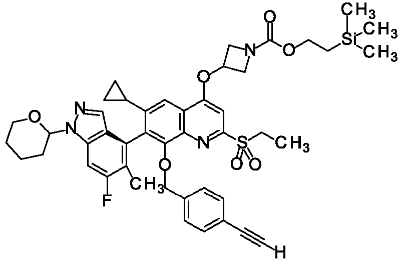 |
| 41 | 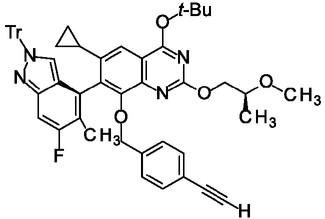 |

**[Table 5-6]**

| PEx | Str |
|---|---|
| 42 | 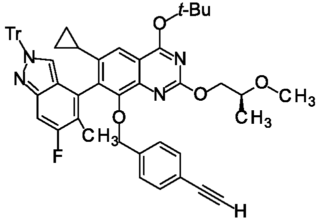 |
| 43 | 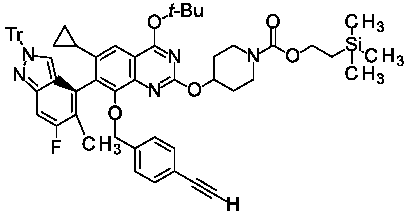 |
| 44 | 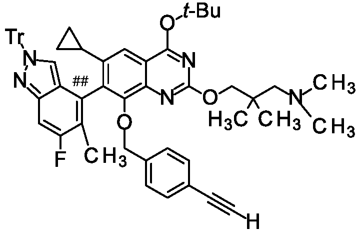 |
| 45 | 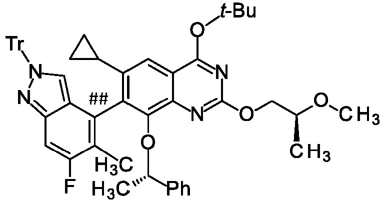 |
| 46 | 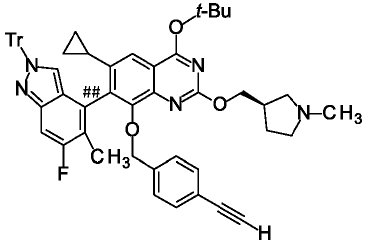 |

**[Table 5-7]**

| PEx | Str |
|---|---|
| 47 | 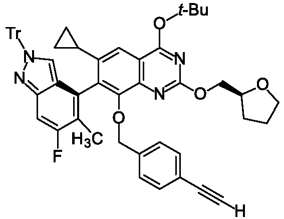 |
| 48 | 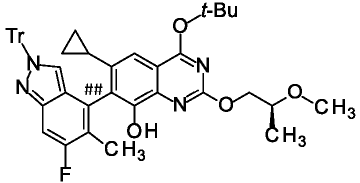 |
| 49 | 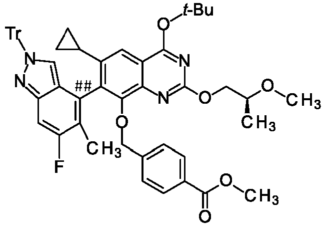 |
| 50 | 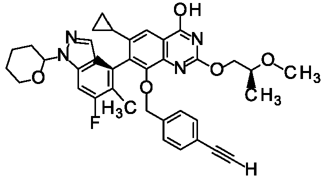 |
| 51 | 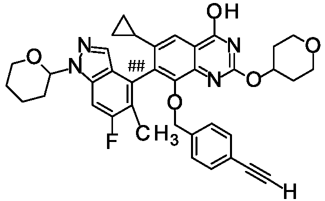 |
| 52 | 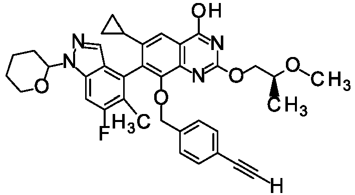 |

**[Table 5-8]**

| PEx | Str |
|---|---|
| 53 | 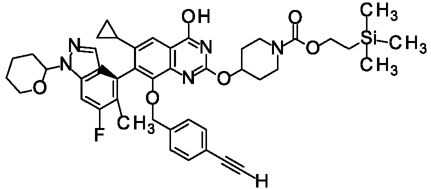 |
| 54 | 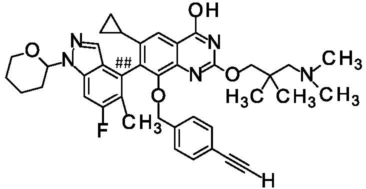 |
| 55 | 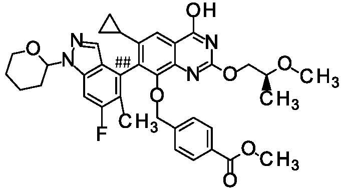 |
| 56 | 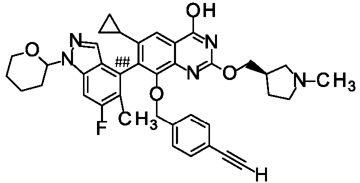 |
| 57 | 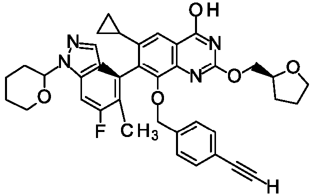 |
| 58 | 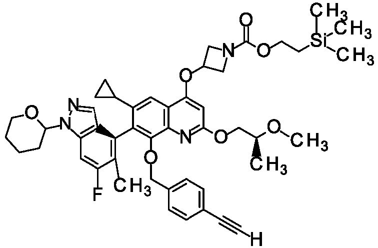 |

**[Table 5-9]**

| PEx | Str |
|---|---|
| 59 | 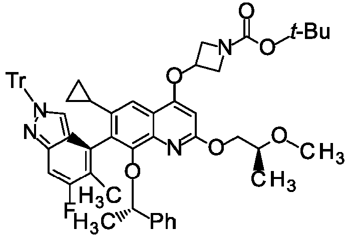 |
| 60 | 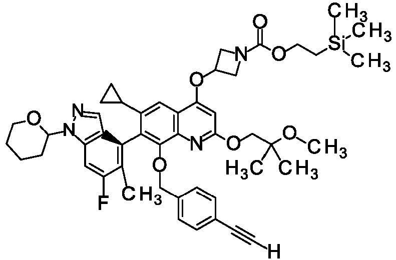 |
| 61 | 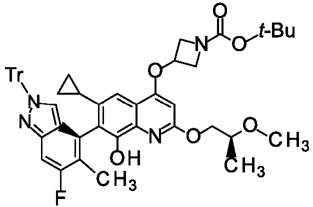 |
| 62 | 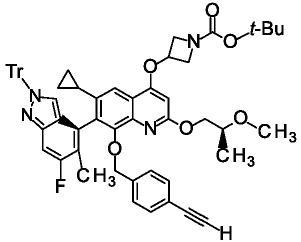 |
| 63 | 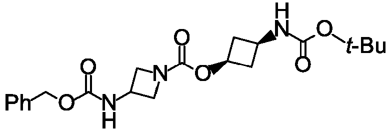 |
| 64 | 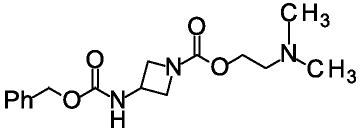 |

**[Table 5-10]**

| PEx | Str | PEx | Str |
|---|---|---|---|
| 65 | 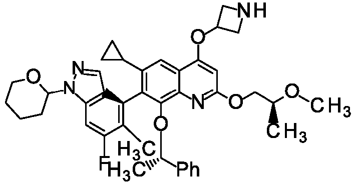 | 72 | 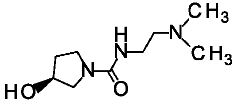 |
| 66 | 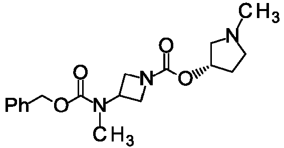 | 73 | 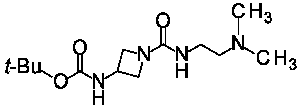 |
| 67 | 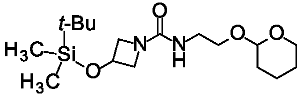 | 74 | 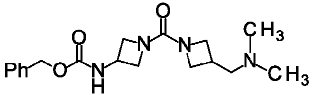 |
| 68 | 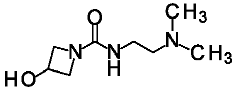 | 75 | 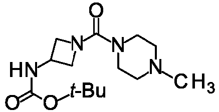 |
| 69 | 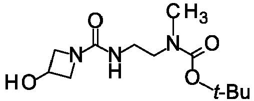 | 76 | 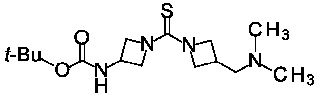 |
| 70 | 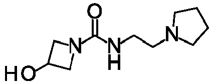 | 77 | 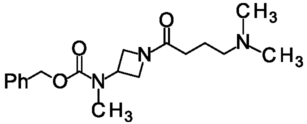 |
| 71 | 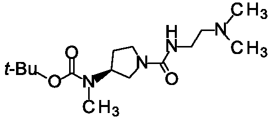 | 78 | 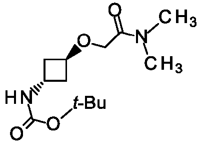 |

**[Table 5-11]**

| PEx | Str | PEx | Str |
|---|---|---|---|
| 79 | 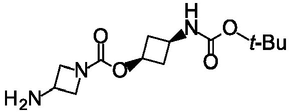 | 87 | 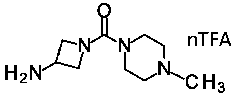 |
| 80 | 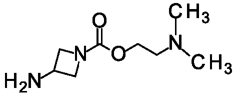 | 88 | 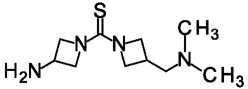 |
| 81 | 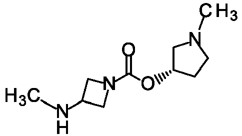 | 89 | 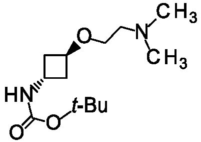 |
| 82 | 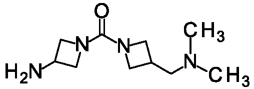 | 90 | 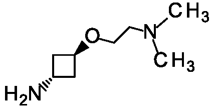 |
| 83 | 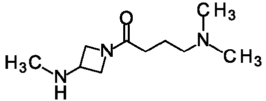 | 91 | 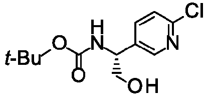 |
| 84 | 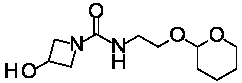 | 92 | 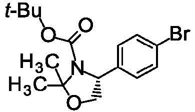 |
| 85 | 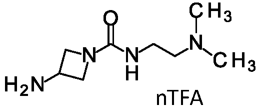 | 93 | 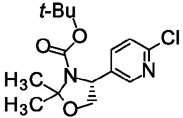 |
| 86 | 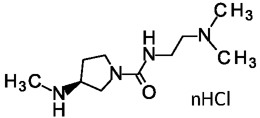 | 94 | 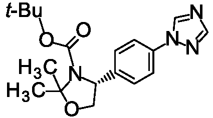 |

**[Table 5-12]**

| PEx | Str | PEx | Str |
|---|---|---|---|
| 95 | 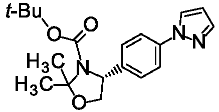 | 102 | 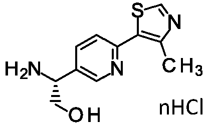 |
| 96 | 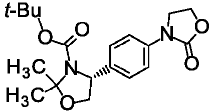 | 103 | 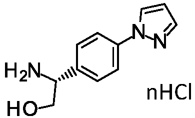 |
| 97 | 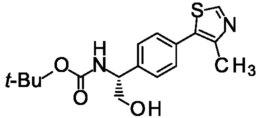 | 104 | 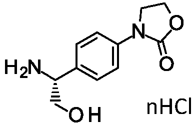 |
| 98 | 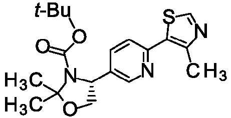 | 105 | 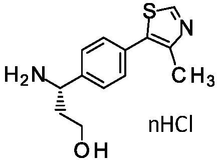 |
| 99 | 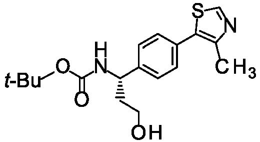 | 106 | 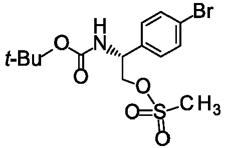 |
| 100 | 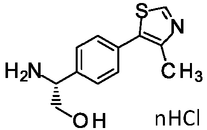 | 107 | 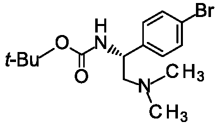 |
| 101 | 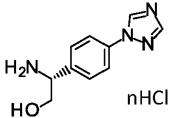 | 108 | 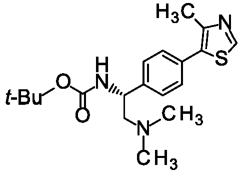 |

**[Table 5-13]**

| PEx | Str |
|---|---|
| 109 | 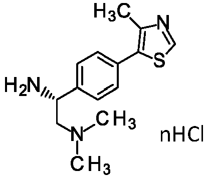 |
| 110 | 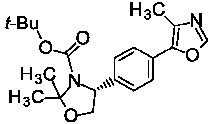 |
| 111 | 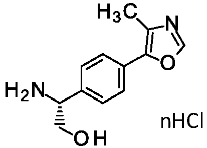 |
| 112 | 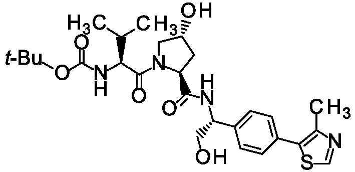 |
| 113 | 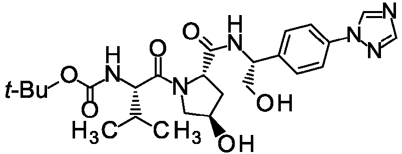 |
| 114 | 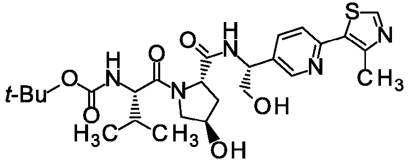 |
| 115 | 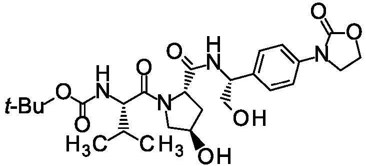 |

**[Table 5-14]**

| PEx | Str |
|---|---|
| 116 | 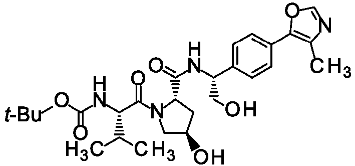 |
| 117 | 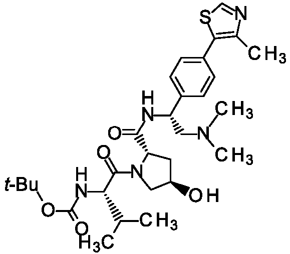 |
| 118 | 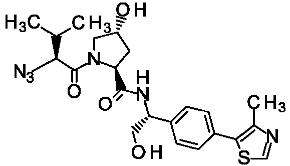 |
| 119 | 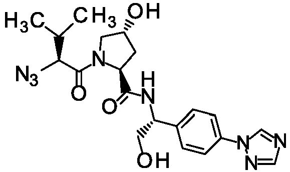 |
| 120 | 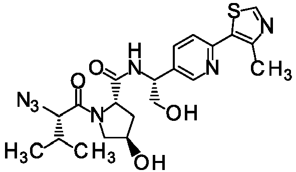 |
| 121 | 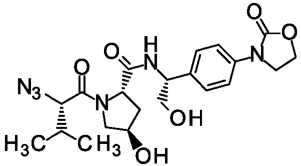 |

**[Table 5-15]**

| PEx | Str |
|---|---|
| 122 | 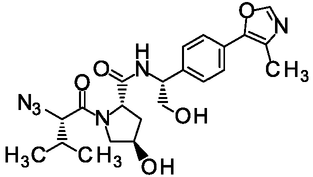 |
| 123 | 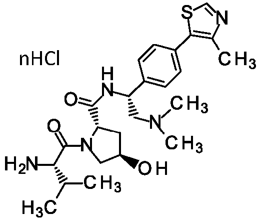 |
| 124 | 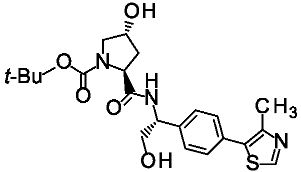 |
| 125 | 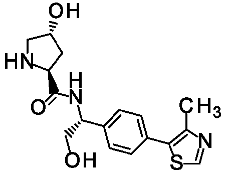 |
| 126 | 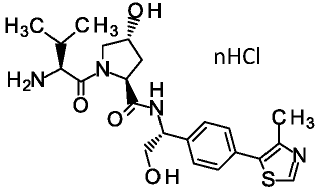 |
| 127 | 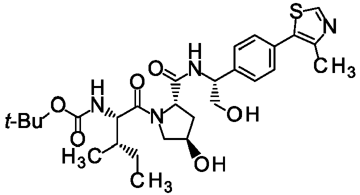 |

**[Table 5-16]**

| PEx | Str |
|---|---|
| 128 | 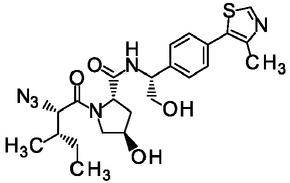 |
| 129 | 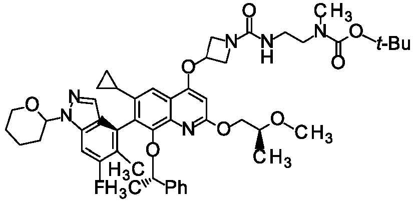 |
| 130 | 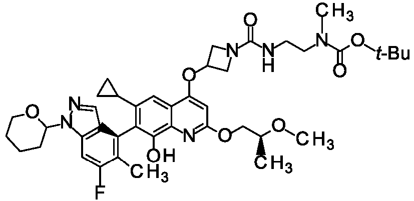 |
| 131 | 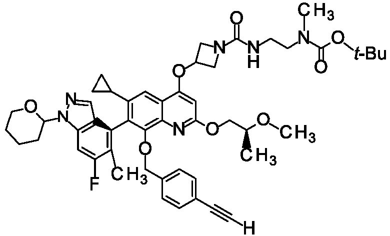 |
| 132 | 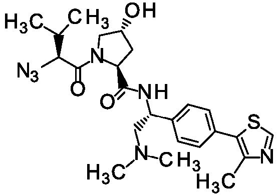 |
| 133 | 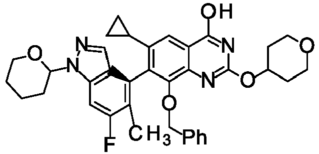 |

**[Table 5-17]**

| PEx | Str |
|---|---|
| 134 | 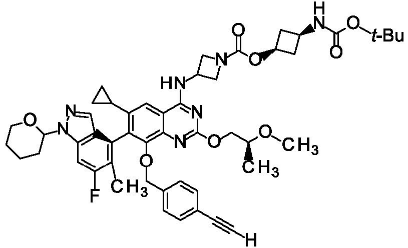 |
| 135 | 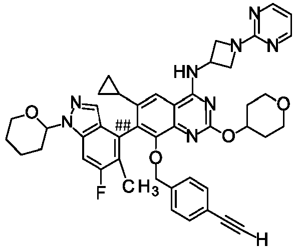 |
| 136 | 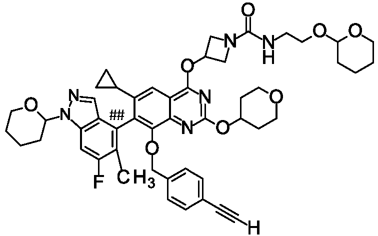 |
| 137 | 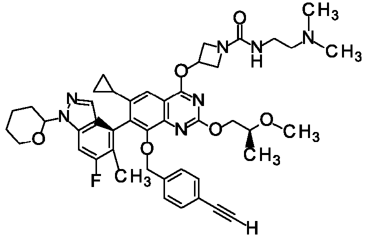 |
| 138 | 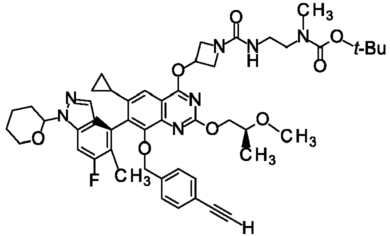 |

**[Table 5-18]**

| PEx | Str |
|---|---|
| 139 | 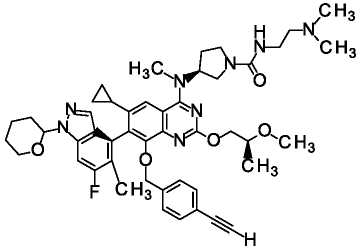 |
| 140 | 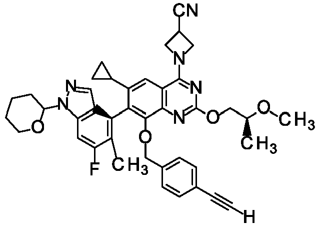 |
| 141 | 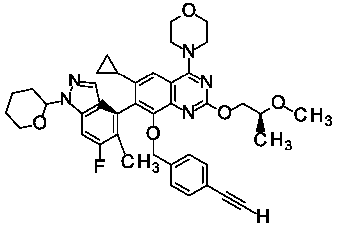 |
| 142 | 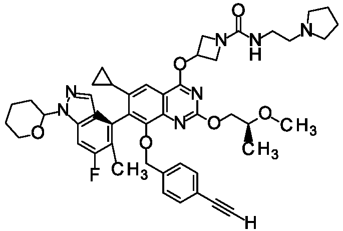 |
| 143 | 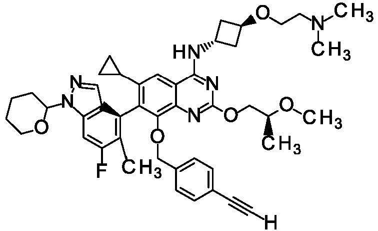 |

**[Table 5-19]**

| PEx | Str |
|---|---|
| 144 | 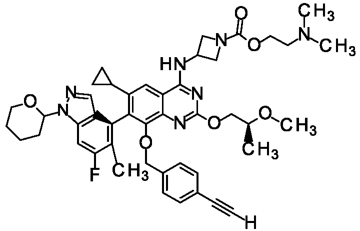 |
| 145 | 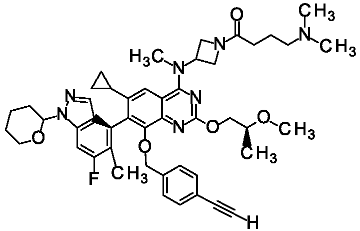 |
| 146 | 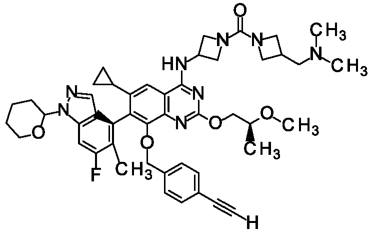 |
| 147 | 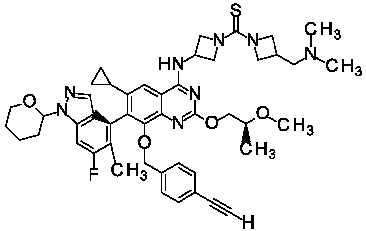 |
| 148 | 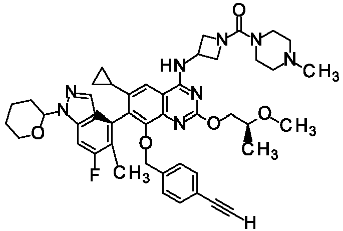 |

**[Table 5-20]**

| PEx | Str |
|---|---|
| 149 | 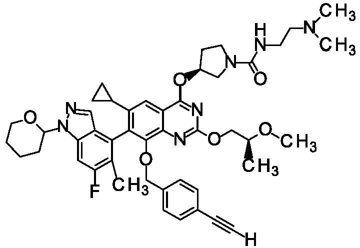 |
| 150 | 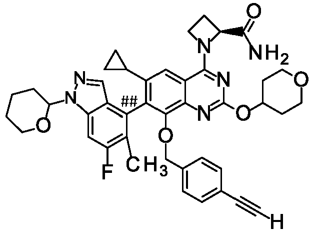 |
| 151 | 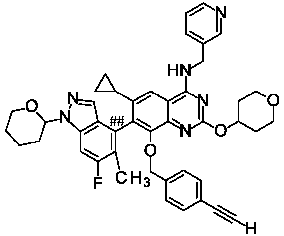 |
| 152 | 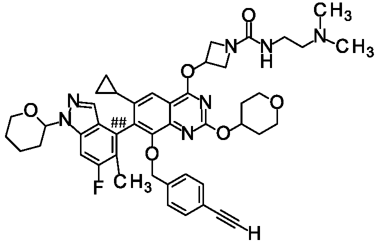 |
| 153 | 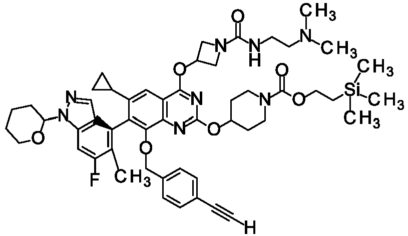 |

**[Table 5-21]**

| PEx | Str |
|---|---|
| 154 | 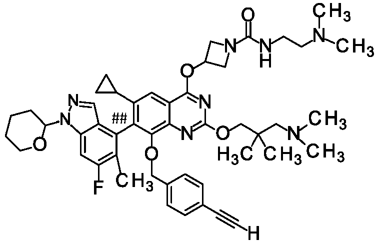 |
| 155 | 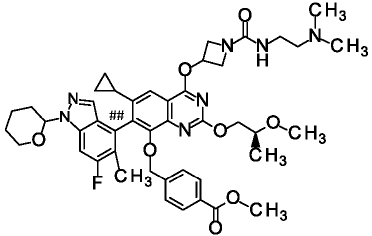 |
| 156 | 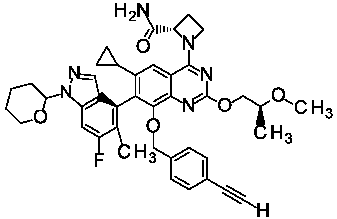 |
| 157 | 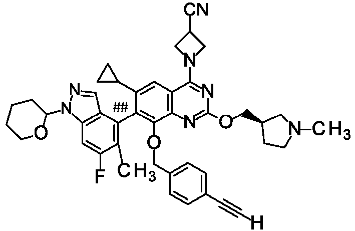 |
| 158 | 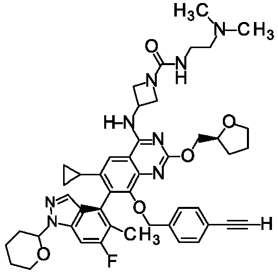 |

**[Table 5-22]**

| PEx | Str |
|---|---|
| 159 | 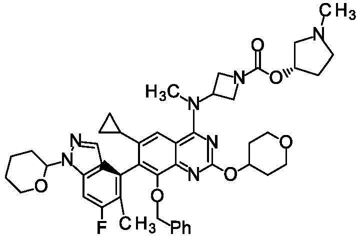 |
| 160 | 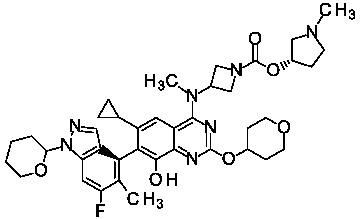 |
| 161 | 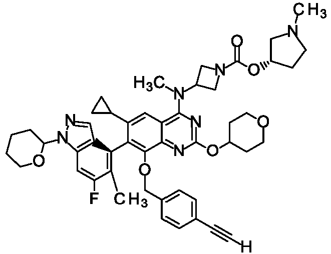 |
| 162 | 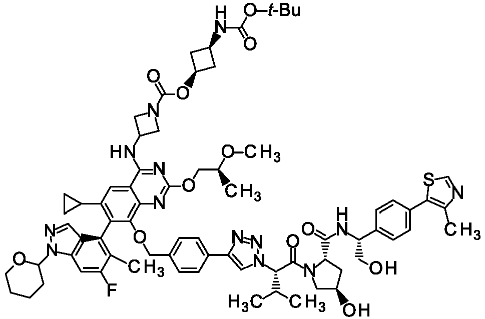 |

**[Table 5-23]**

| PEx | Str |
|---|---|
| 163 | 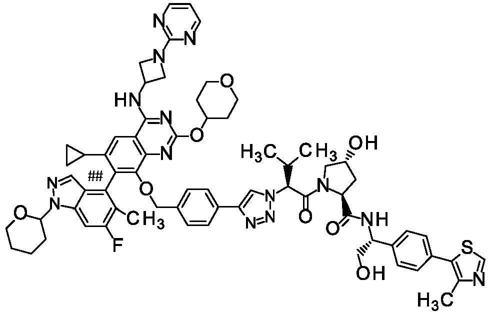 |
| 164 | 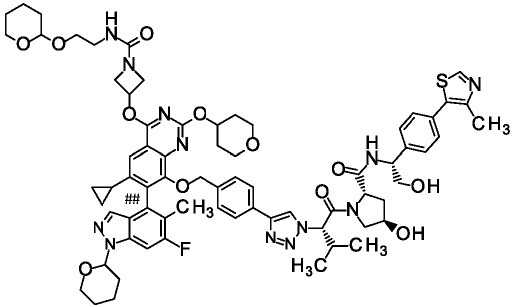 |
| 165 | 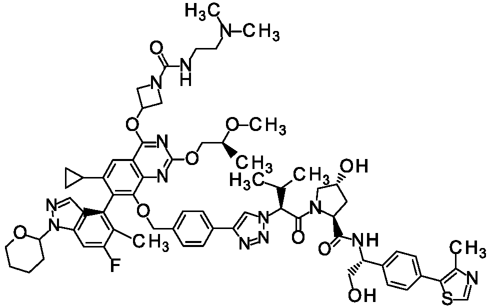 |
| 166 | 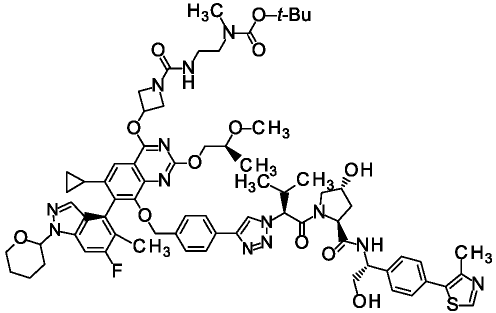 |

**[Table 5-24]**

| PEx | Str |
|---|---|
| 167 | 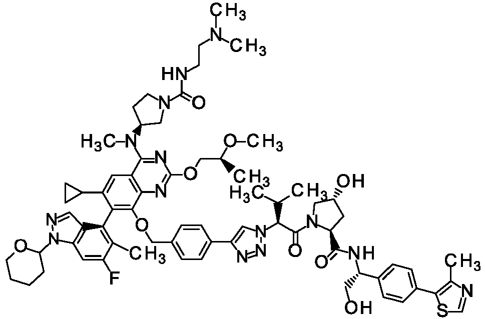 |
| 168 | 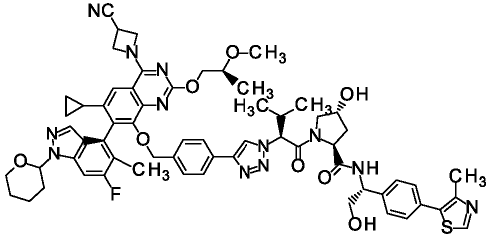 |
| 169 | 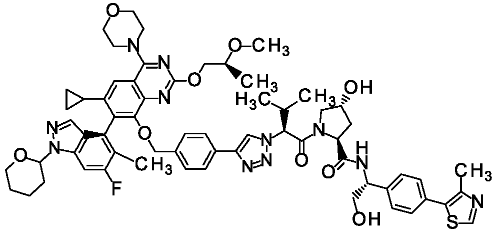 |
| 170 | 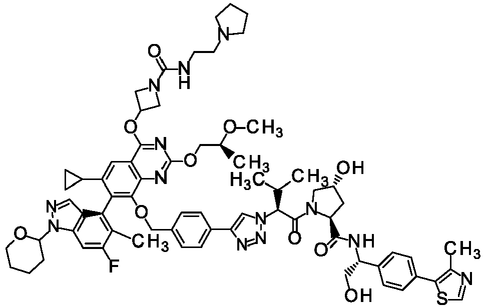 |

**[Table 5-25]**

| PEx | Str |
|---|---|
| 171 | 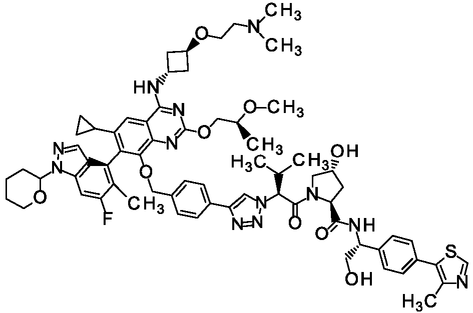 |
| 172 | 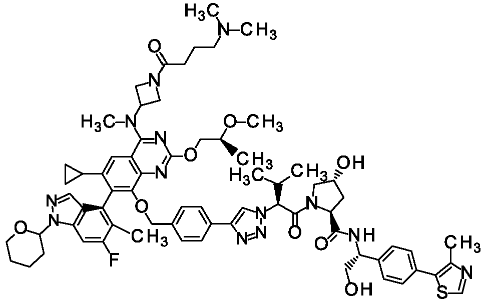 |
| 173 | 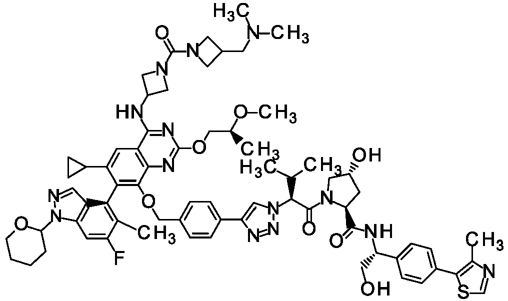 |
| 174 | 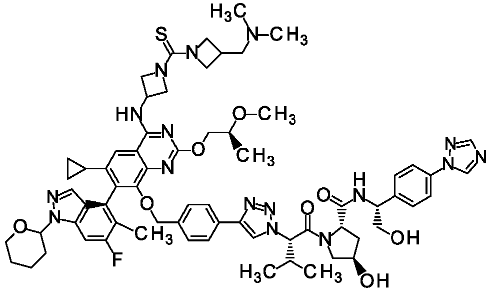 |

**[Table 5-26]**

| PEx | Str |
|---|---|
| 175 | 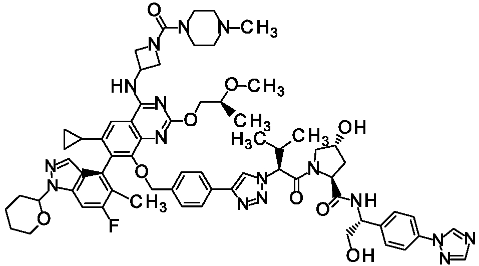 |
| 176 | 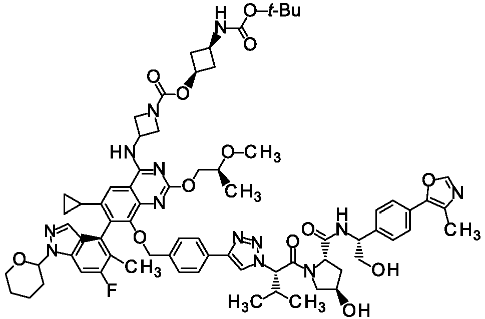 |
| 177 | 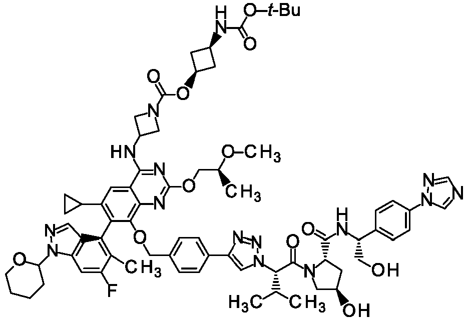 |
| 178 | 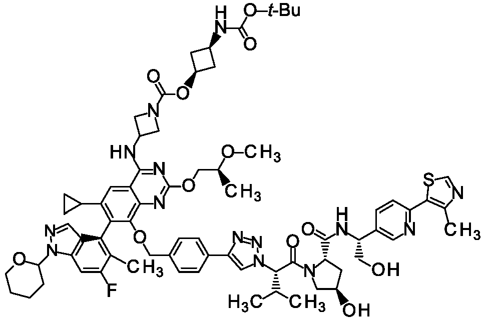 |

**[Table 5-27]**

| PEx | Str |
|---|---|
| 179 | 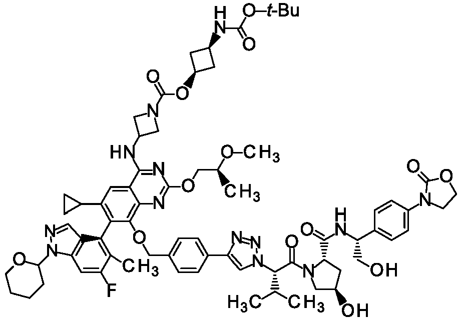 |
| 180 | 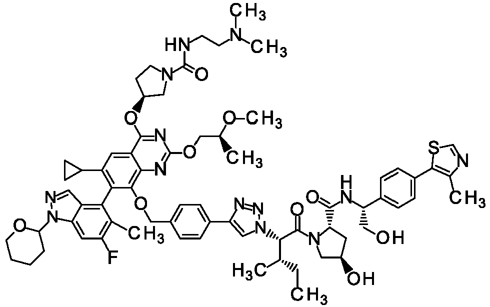 |
| 181 | 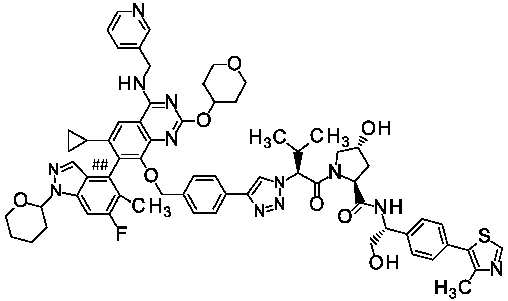 |
| 182 | 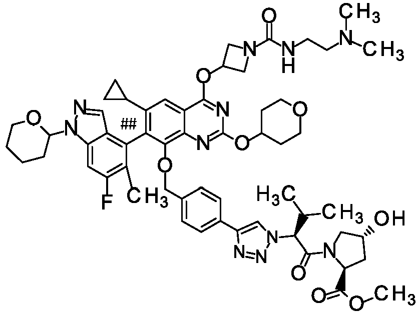 |

**[Table 5-28]**

| PEx | Str |
|---|---|
| 183 | 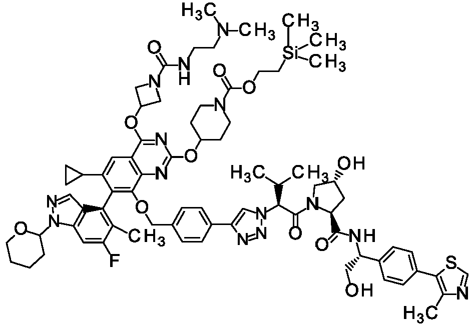 |
| 184 | 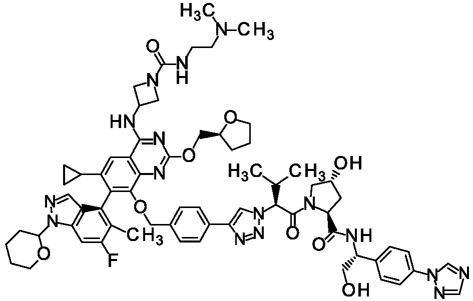 |
| 185 | 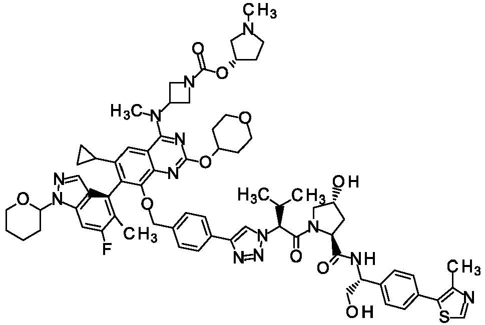 |
| 186 | 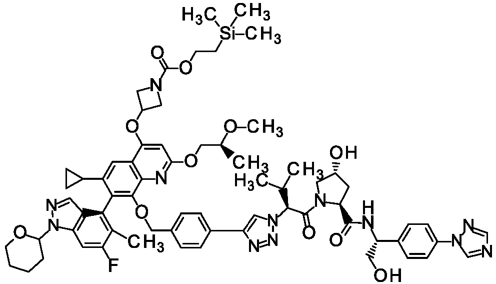 |

**[Table 5-29]**

| PEx | Str |
|---|---|
| 187 | 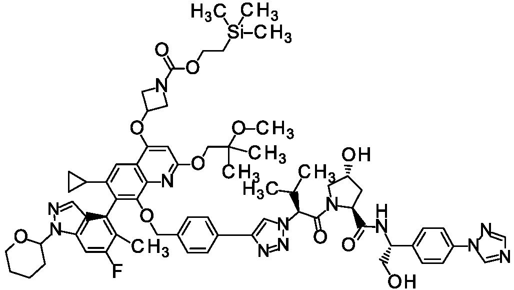 |
| 188 | 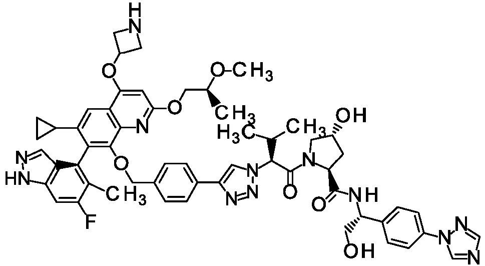 |
| 189 | 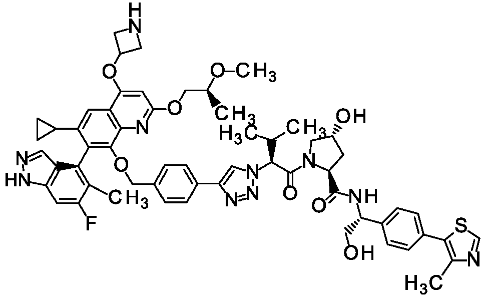 |
| 190 | 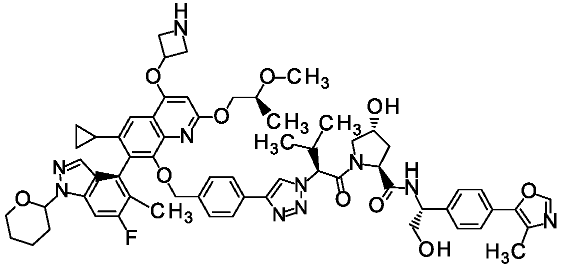 |

**[Table 5-30]**

| PEx | Str |
|---|---|
| 191 | 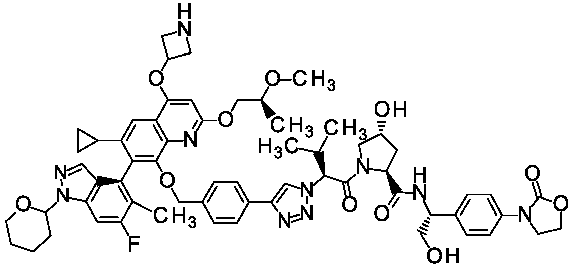 |
| 192 | 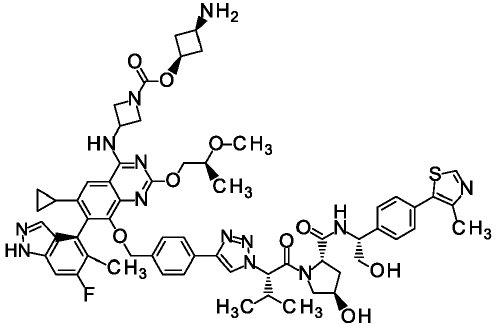 |
| 193 | 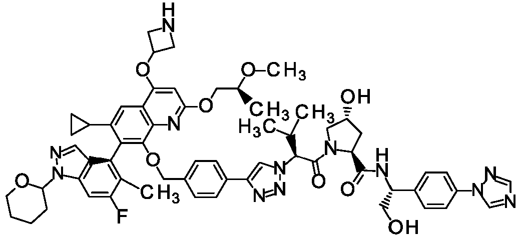 |
| 194 | 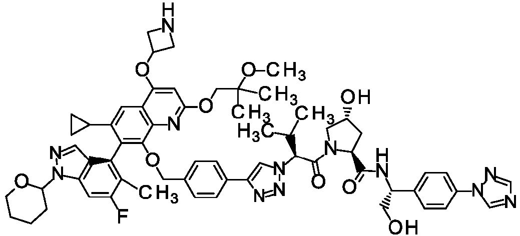 |

**[Table 5-31]**

| PEx | Str |
|---|---|
| 195 | 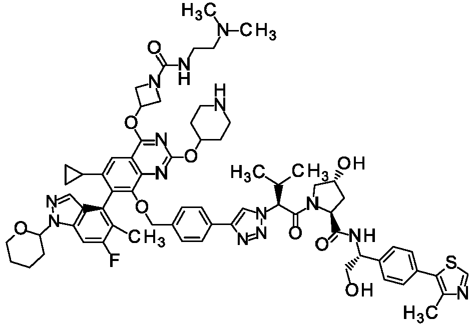 |
| 196 | 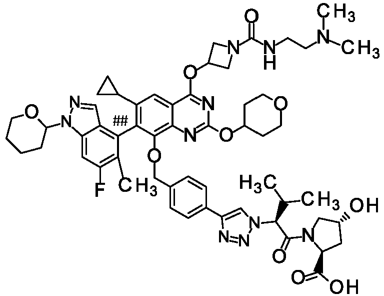 |
| 197 | 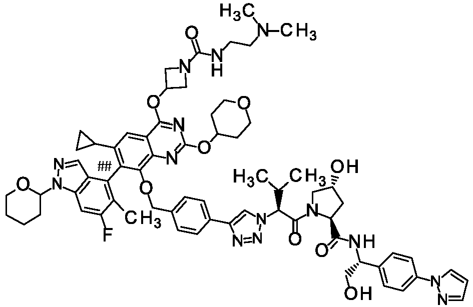 |
| 198 | 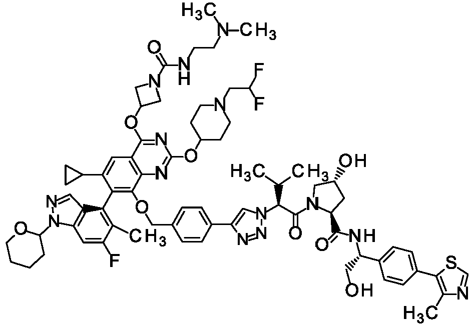 |

**[Table 5-32]**

| PEx | Str |
|---|---|
| 199 | 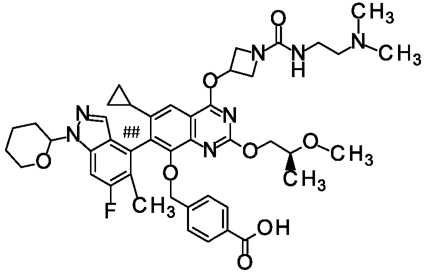 |

**[Table 6-1]**

| PEx | PSyn | DAT |
|---|---|---|
| 1 | 1 | ESI+: 402.9, 404.9 |
| 2 | 2 | ESI+: 480.9, 482.8 [M+Na]+ |
| 3 | 3 | ESI+: 416.1 |
| 4 | 4 | ESI+: 383.9 |
| 5 | 5 | ESI+: 419.9, 421.8 |
| 6 | 6 | ESI+: 509.0 [M+Na]+ |
| 7 | 7 | ESI+: 527.0 |
| 8 | 8 | ESI+: 446.0, 447.9 |
| 9 | 9 | ESI+: 587.2 |
| 10 | 9 | ESI+: 629.0 |
| 11 | 9 | ESI+: 615.1 |
| 12 | 12 | ESI+: 548.0, 550.1 |
| 13 | 13 | ESI+: 707.1, 709.2 [M+Na]+ |
| 14 | 14 | ESI+: 503.4 |
| 15 | 14 | ESI+: 543.2 |
| 16 | 14 | ESI+: 529.3 |
| 17 | 17 | ESI+: 601.5 |
| 18 | 18 | NMR: 7.08-7.15 (m, 6H), 7.36-7.44 (m, 10H), 7.49-7.54 (m, 1H), 7.89 (d, 1H) |
| 19 | 19 | NMR: 2.34 (d, 3H), 7.07-7.13 (m, 6H), 7.36-7.43 (m, 9H), 7.51 (d, 1H), 7.79 (d, 1H) |
| 20 | 20 | NMR: 1.21 (s, 12H), 2.44 (d, 3H), 7.04-7.11 (m, 6H), 7.34-7.44 (m, 9H), 7.49 (d, 1H), 8.09 (d, 1H) |
| 21 | 21 | ESI+: 813.4 |
| 22 | 21 | ESI+: 853.6 |
| 23 | 21 | ESI+: 681.5 |
| 24 | 24 | ESI+: 911.5 |
| 25 | 25 | ESI+: 911.5 |
| 26 | 25 | ESI+: 853.4 |
| 27 | 27 | ESI+: 867.5 [M+Na]+ |
| 28 | 27 | ESI+: 867.5 [M+Na]+ |
| 29 | 29 | ESI+: 653.5 |
| 30 | 30 | ESI+: 797.6 |
| 31 | 31 | ESI+: 829.7 |
| 32 | 31 | ESI+: 965.5 [M+Na]+ |
| 33 | 33 | ESI+: 763.5 [M+Na]+ |
| 34 | 33 | ESI+: 749.4 |
| 35 | 33 | ESI+: 763.5 [M+Na]+ |

**[Table 6-2]**

| PEx | PSyn | DAT |
|---|---|---|
| 36 | 36 | ESI+: 725.4 |
| 37 | 37 | ESI+: 877.5 [M+Na]+ |
| 38 | 37 | ESI+: 863.4 |
| 39 | 37 | ESI+: 877.6 [M+Na]+ |
| 40 | 40 | ESI+: 839.8 |
| 41 | 41 | ESI+: 851.5 |
| 42 | 41 | ESI+: 851.6 |
| 43 | 41 | ESI+: 1006.9 |
| 44 | 41 | ESI+: 892.5 |
| 45 | 41 | ESI+: 841.6 |
| 46 | 41 | ESI+: 876.6 |
| 47 | 41 | ESI+: 863.5 |
| 48 | 33 | ESI+: 737.7 |
| 49 | 37 | ESI+: 885.7 |
| 50 | 50 | ESI+: 637.5 |
| 51 | 50 | ESI+: 649.3 |
| 52 | 50 | ESI+: 637.4 |
| 53 | 50 | ESI+: 814.8 [M+Na]+ |
| 54 | 50 | ESI+: 678.5 |
| 55 | 50 | ESI+: 671.5 |
| 56 | 50 | ESI+: 662.7 |
| 57 | 50 | ESI+: 649.4 |
| 58 | 58 | ESI+: 835.6 |
| 59 | 58 | ESI+: 939.6 |
| 60 | 58 | ESI+: 849.5 |
| 61 | 33 | ESI+: 835.7 |
| 62 | 40 | ESI+: 949.5 |
| 63 | 63 | ESI+: 442.4 [M+Na]+ |
| 64 | 63 | ESI+: 322.4 |
| 65 | 29 | ESI+: 681.6 |
| 66 | 63 | ESI+: 348.3 |
| 67 | 67 | ESI+: 381.3 [M+Na]+ |
| 68 | 67 | ESI+: 188.1 |
| 69 | 67 | ESI+: 274.4 |
| 70 | 67 | ESI+: 214.2 |

**[Table 6-3]**

| PEx | PSyn | DAT |
|---|---|---|
| 71 | 67 | ESI+: 315.3 |
| 72 | 67 | ESI+: 202.2 |
| 73 | 67 | ESI+: 287.3 |
| 74 | 74 | ESI+: 347.4 |
| 75 | 74 | ESI+: 299.3 |
| 76 | 76 | ESI+: 329.2 |
| 77 | 77 | ESI+: 334.3 |
| 78 | 78 | ESI+: 295.2 [M+Na]+ |
| 79 | 79 | ESI+: 286.4 |
| 80 | 79 | ESI+: 188.1 |
| 81 | 79 | ESI+: 214.3 |
| 82 | 79 | ESI+: 213.3 |
| 83 | 79 | ESI+: 200.2 |
| 84 | 84 | ESI+: 267.1 [M+Na]+ |
| 85 | 85 | ESI+: 187.1 |
| 86 | 86 | ESI+: 215.2 |
| 87 | 85 | ESI+: 199.1 |
| 88 | 85 | ESI+: 229.2 |
| 89 | 89 | NMR: 1.37 (s, 9H), 2.02-2.22 (m, 4H), 2.53 (s, 6H), 2.88 (t, 2H), 3.59 (t, 2H), 3.90-4.08 (m, 2H), 7.17 (d, 1H) |
| 90 | 85 | ESI+: 159.2 |
| 91 | 91 | ESI+: 273.1 |
| 92 | 92 | ESI+: 380.2 [M+Na]+ |
| 93 | 92 | ESI+: 313.1 |
| 94 | 94 | ESI+: 345.4 |
| 95 | 94 | ESI+: 344.2 |
| 96 | 94 | ESI+: 385.2 [M+Na]+ |
| 97 | 97 | ESI+: 335.2 |
| 98 | 97 | ESI+: 376.2 |
| 99 | 97 | ESI+: 349.2 |
| 100 | 100 | ESI+: 235.2 |
| 101 | 101 | ESI+: 205.3 |
| 102 | 101 | ESI+: 236.1 |
| 103 | 101 | ESI+: 226.2 [M+Na]+ |
| 104 | 101 | ESI+: 245.1 [M+Na]+ |
| 105 | 100 | ESI+: 249.2 |

**[Table 6-4]**

| PEx | PSyn | DAT |
|---|---|---|
| 106 | 106 | ESI+: 416.1, 418.0 [M+Na]+ |
| 107 | 107 | ESI+: 345.1 |
| 108 | 97 | ESI+: 362.3 |
| 109 | 100 | ESI+: 262.3 |
| 110 | 110 | ESI+: 359.3 |
| 111 | 101 | ESI+: 219.2 |
| 112 | 112 | ESI+: 547.6 |
| 113 | 113 | ESI+: 517.6 |
| 114 | 112 | ESI+: 548.3 |
| 115 | 113 | ESI+: 535.5 |
| 116 | 113 | ESI+: 531.4 |
| 117 | 112 | ESI+: 574.6 |
| 118 | 118 | ESI+: 473.5 |
| 119 | 119 | ESI+: 443.5 |
| 120 | 119 | ESI+: 474.3 |
| 121 | 119 | ESI+: 461.4 |
| 122 | 119 | ESI+: 457.3 |
| 123 | 123 | ESI+: 474.4 |
| 124 | 124 | ESI+: 448.3 |
| 125 | 125 | ESI+: 348.2 |
| 126 | 123 | ESI+: 447.3 |
| 127 | 112 | ESI+: 583.5 [M+Na]+ |
| 128 | 118 | ESI+: 487.3 |
| 129 | 129 | ESI+: 881.7 |
| 130 | 36 | ESI+: 777.7 |
| 131 | 40 | ESI+: 891.8 |
| 132 | 132 | ESI+: 500.5 |
| 133 | 133 | ESI+: 625.2 |
| 134 | 134 | ESI+: 904.6 |
| 135 | 134 | ESI+: 781.4 |
| 136 | 134 | ESI+: 875.4 |
| 137 | 134 | ESI+: 806.7 |
| 138 | 134 | ESI+: 892.5 |
| 139 | 134 | ESI+: 833.8 |
| 140 | 134 | ESI+: 701.5 |

**[Table 6-5]**

| PEx | PSyn | DAT |
|---|---|---|
| 141 | 134 | ESI+: 706.5 |
| 142 | 134 | ESI+: 832.4 |
| 143 | 134 | ESI+: 777.7 |
| 144 | 134 | ESI+: 806.8 |
| 145 | 134 | ESI+: 818.6 |
| 146 | 134 | ESI+: 831.5 |
| 147 | 134 | ESI+: 847.5 |
| 148 | 134 | ESI+: 817.7 |
| 149 | 134 | ESI+: 820.8 |
| 150 | 134 | ESI+: 731.5 |
| 151 | 134 | ESI+: 739.7 |
| 152 | 134 | ESI+: 818.7 |
| 153 | 134 | ESI+: 961.8 |
| 154 | 134 | ESI+: 847.7 |
| 155 | 134 | ESI+: 840.7 |
| 156 | 134 | ESI+: 719.5 |
| 157 | 134 | ESI+: 726.7 |
| 158 | 134 | ESI+: 817.5 |
| 159 | 134 | ESI+: 820.6 |
| 160 | 33 | ESI+: 730.7 |
| 161 | 37 | ESI+: 844.7 |
| 162 | 162 | ESI+: 1398.8 [M+Na]+ |
| 163 | 162 | ESI+: 1253.3 |
| 164 | 162 | ESI+: 1370.6 [M+Na]+ |
| 165 | 162 | ESI+: 1278.9 |
| 166 | 162 | ESI+: 1365.1 |
| 167 | 162 | ESI+: 1305.6 |
| 168 | 162 | ESI+: 1173.9 |
| 169 | 162 | ESI+: 1178.7 |
| 170 | 162 | ESI+: 1304.6 |
| 171 | 162 | ESI+: 1250.1 |
| 172 | 162 | ESI+: 1290.7 |
| 173 | 162 | ESI+: 1326.1 [M+Na]+ |
| 174 | 162 | ESI+: 1311.5 [M+Na]+ |
| 175 | 162 | ESI+: 1281.7 [M+Na]+ |

**[Table 6-6]**

| PEx | PSyn | DAT |
|---|---|---|
| 176 | 162 | ESI-: 1358.7 |
| 177 | 162 | ESI+: 1368.7 [M+Na]+ |
| 178 | 162 | ESI-: 1375.8 |
| 179 | 162 | ESI+: 1364.6 |
| 180 | 162 | ESI+: 1328.7 [M+Na]+ |
| 181 | 162 | ESI+: 1211.1 |
| 182 | 162 | ESI+: 1088.9 |
| 183 | 162 | ESI+: 1433.6 |
| 184 | 162 | ESI+: 1259.9 |
| 185 | 162 | ESI-: 1314.8 |
| 186 | 186 | ESI+: 1299.5 [M+Na]+ |
| 187 | 186 | ESI+: 1292.4 |
| 188 | 188 | ESI+: 1050.7 |
| 189 | 188 | ESI+: 1080.5 |
| 190 | 190 | ESI+: 1147.4 |
| 191 | 190 | ESI+: 1151.6 |
| 192 | 192 | ESI+: 1214.4 [M+Na]+ |
| 193 | 193 | ESI+: 1155.7 [M+Na]+ |
| 194 | 193 | ESI+: 1147.6 |
| 195 | 195 | ESI+: 1290.8 |
| 196 | 196 | ESI-: 1072.6 |
| 197 | 197 | ESI+: 1260.1 |
| 198 | 198 | ESI+: 1354.3 |
| 199 | 199 | ESI+: 826.7 |

**[Table 7-1]**

| Ex | Str |
|---|---|
| 1 | 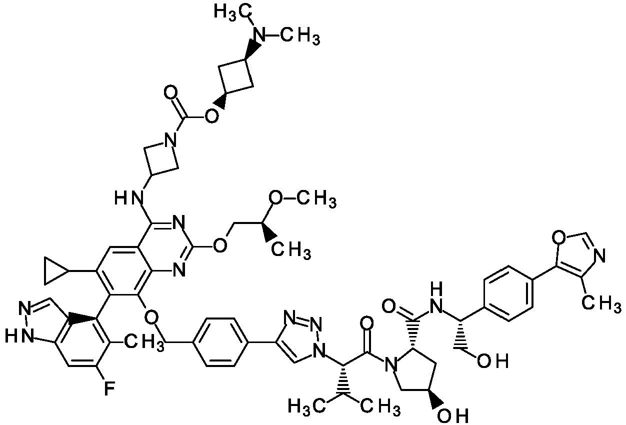 |
| 2 | 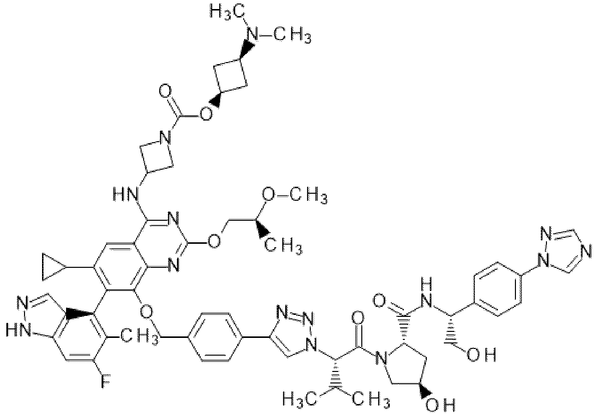 |
| 3 | 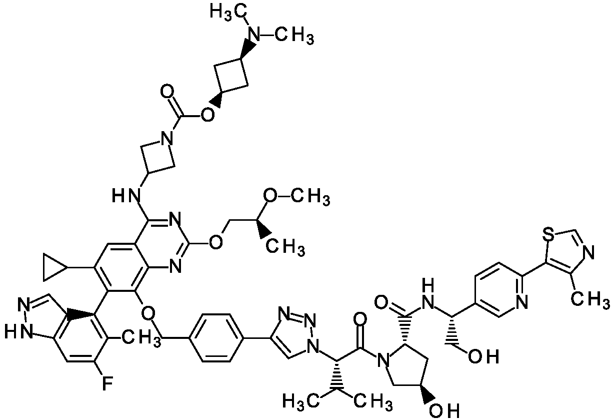 |

**[Table 7-2]**

| Ex | Str |
|---|---|
| 4 | 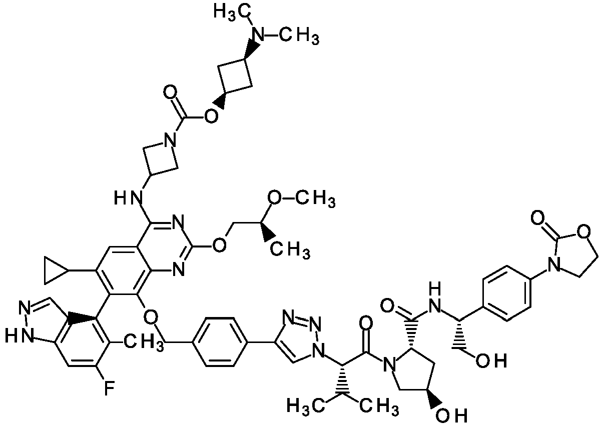 |
| 5 | 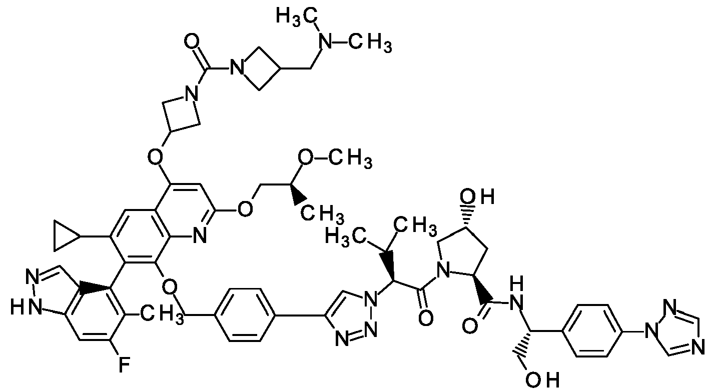 |
| 6 | 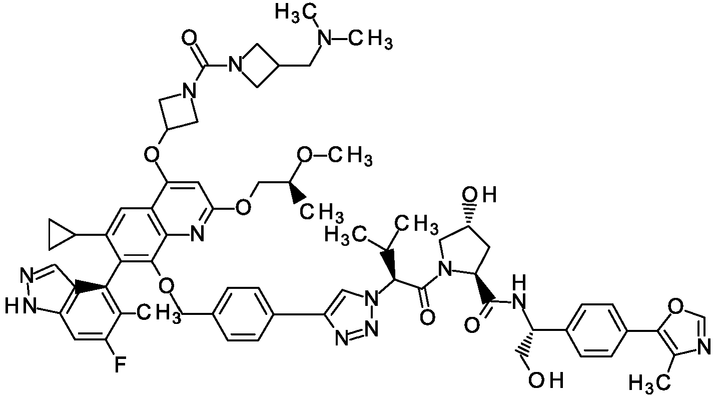 |

**[Table 7-3]**

| Ex | Str |
|---|---|
| 7 | 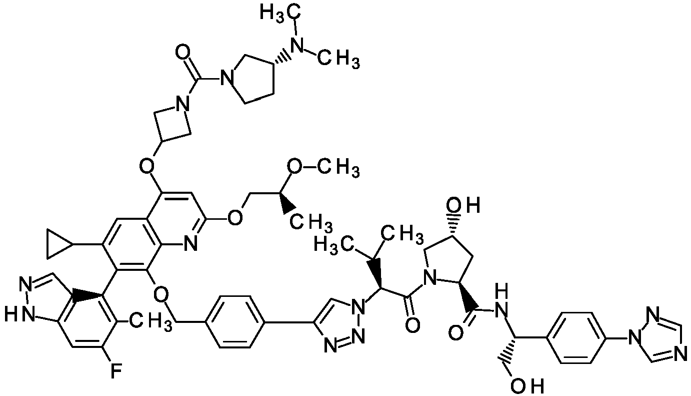 |
| 8 | 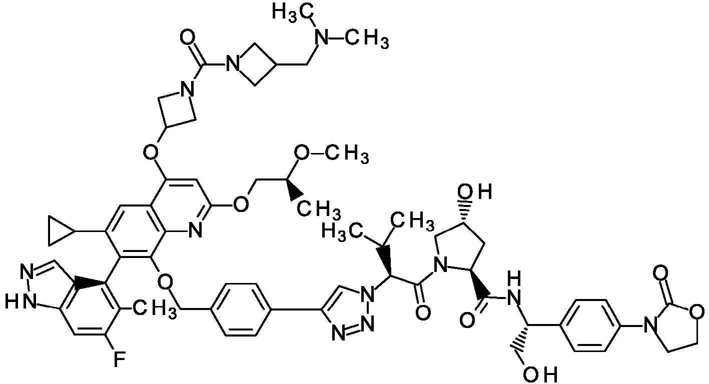 |
| 9 | 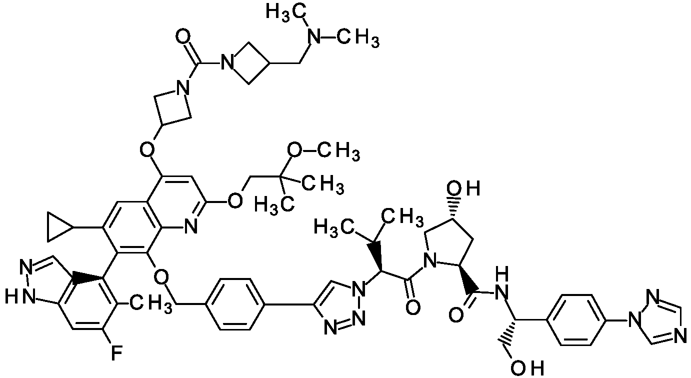 |

**[Table 7-4]**

| Ex | Str |
|---|---|
| 10 | 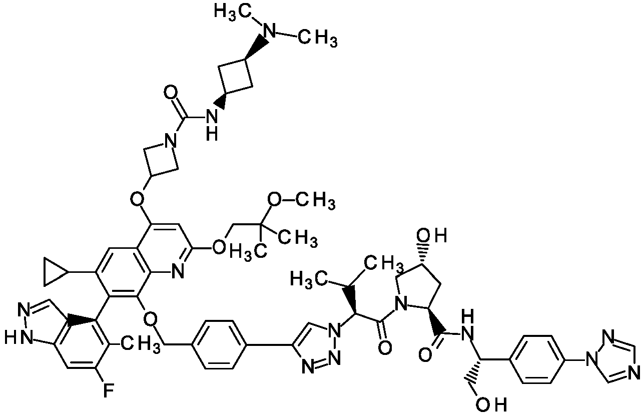 |
| 11 | 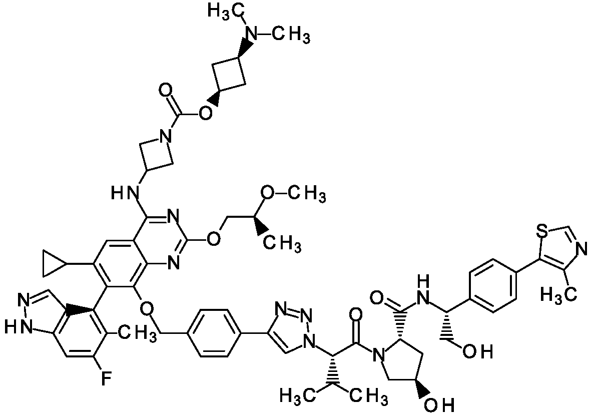 |
| 12 | 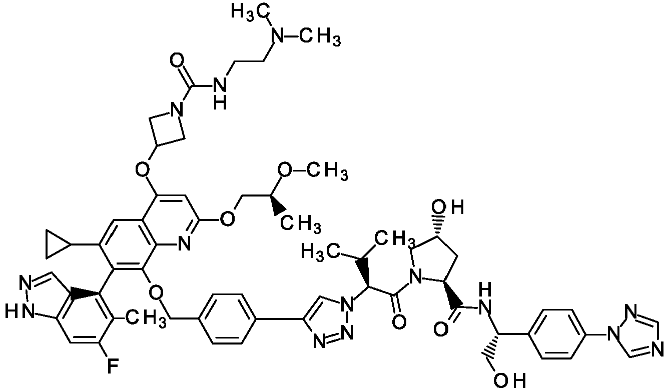 |

**[Table 7-5]**

| Ex | Str |
|---|---|
| 13 | 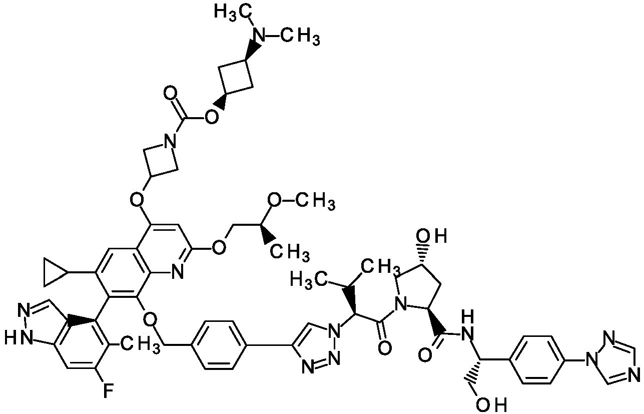 |
| 14 | 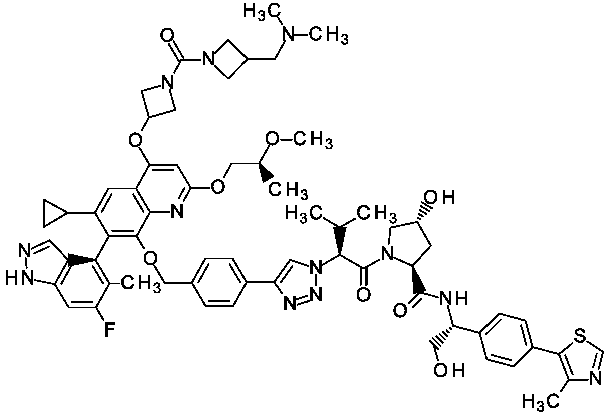 |
| 15 | 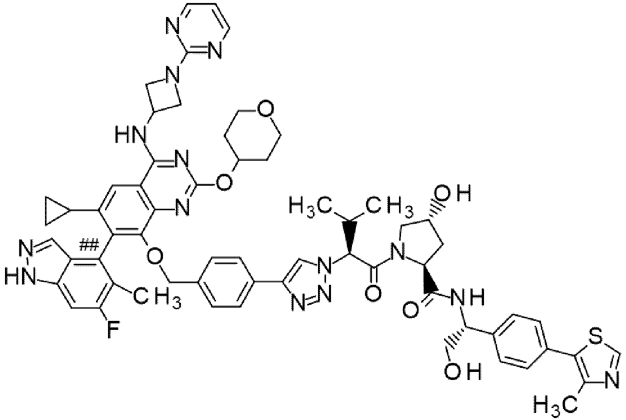 |

**[Table 7-6]**

| Ex | Str |
|---|---|
| 16 | 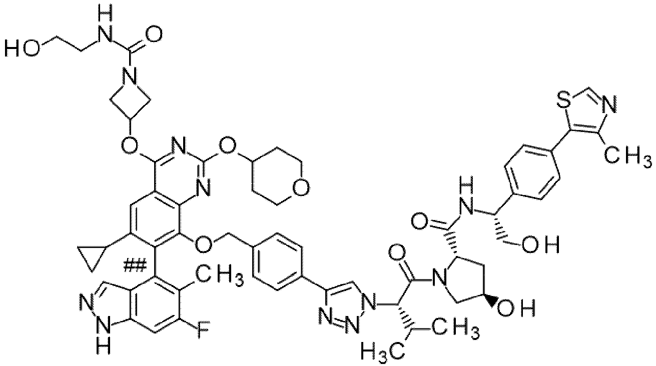 |
| 17 | 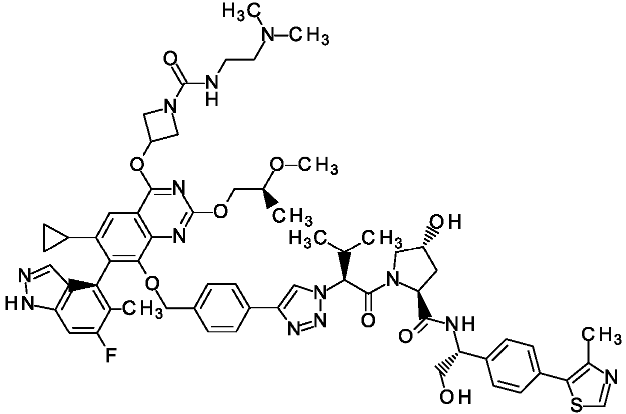 |
| 18 | 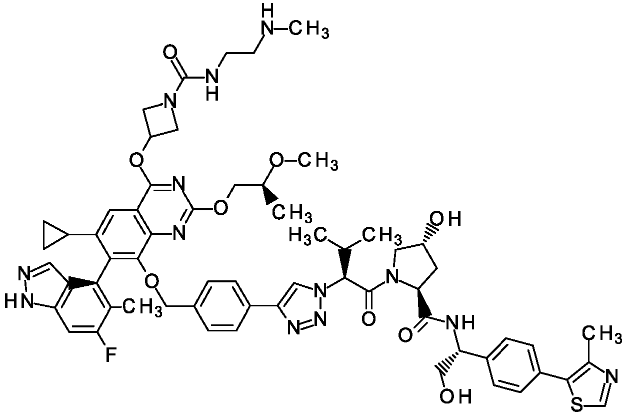 |

**[Table 7-7]**

| Ex | Str |
|---|---|
| 19 | 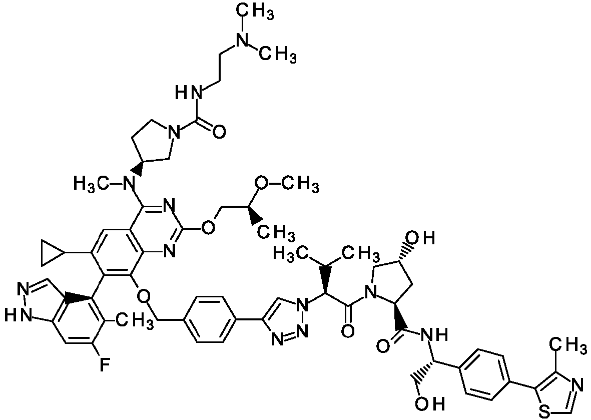 |
| 20 | 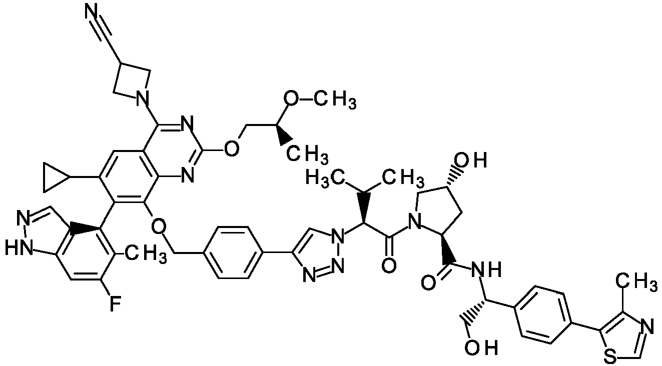 |
| 21 | 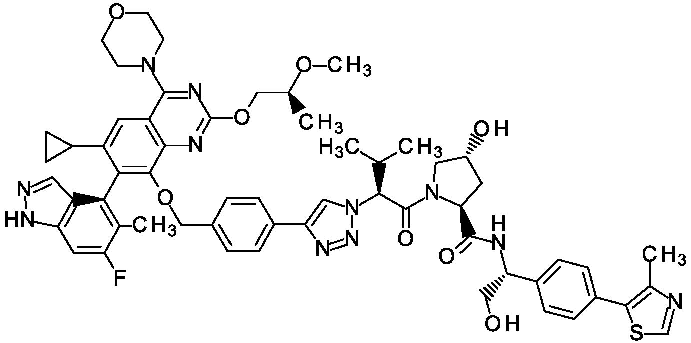 |

**[Table 7-8]**

| Ex | Str |
|---|---|
| 22 | 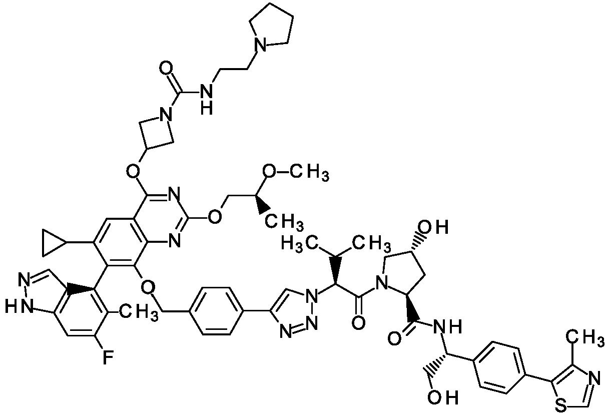 |
| 23 | 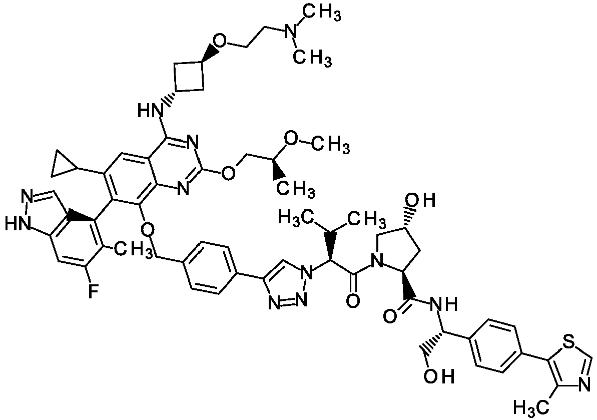 |
| 24 | 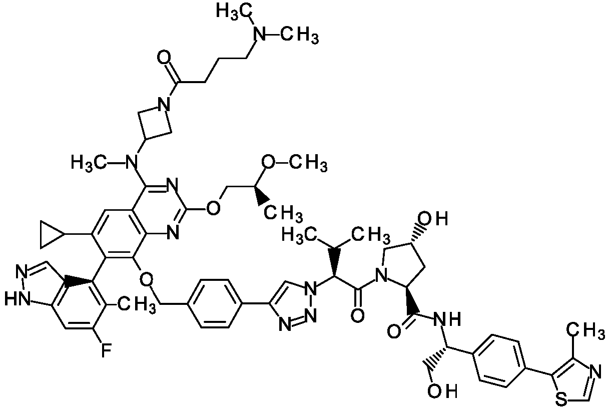 |

**[Table 7-9]**

| Ex | Str |
|---|---|
| 25 | 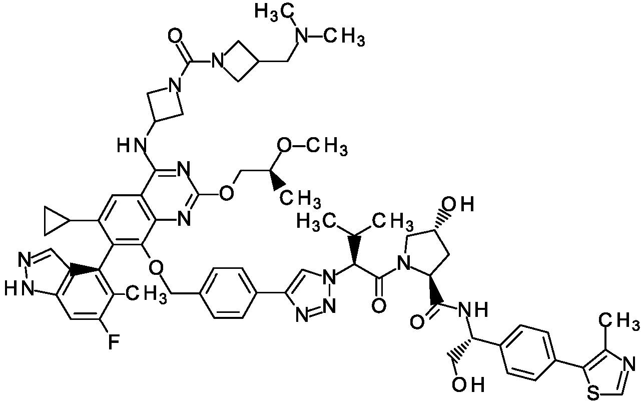 |
| 26 | 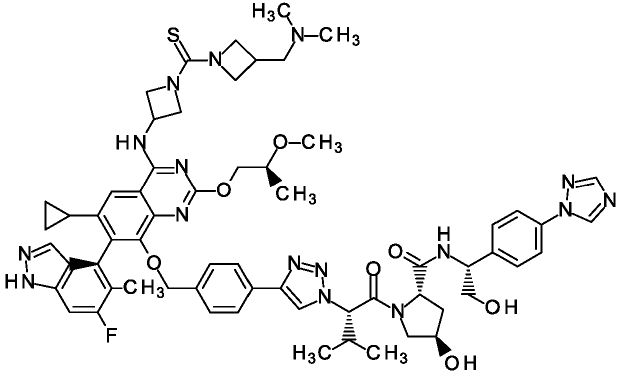 |
| 27 | 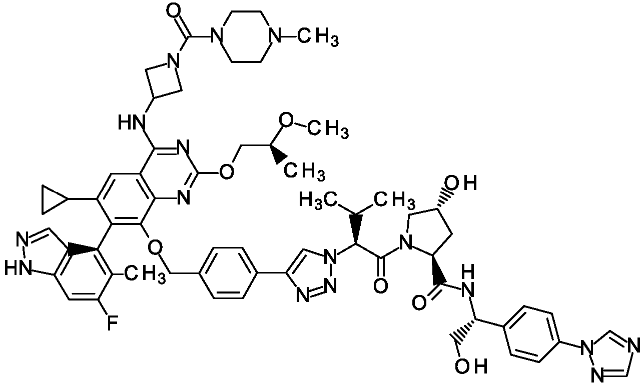 |

**[Table 7-10]**

| Ex | Str |
|---|---|
| 28 | 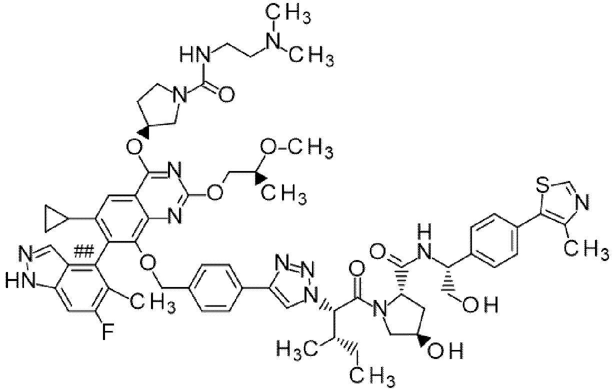 |
| 29 | 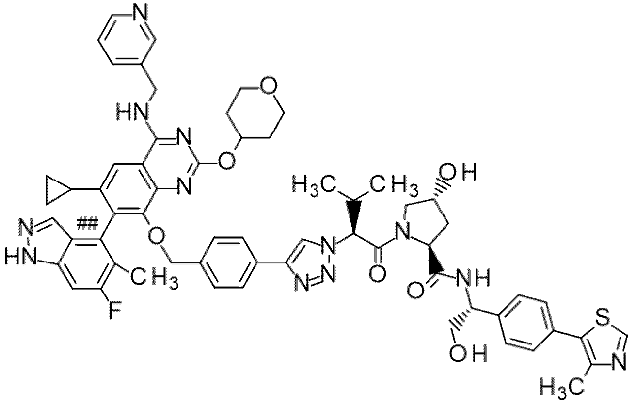 |
| 30 | 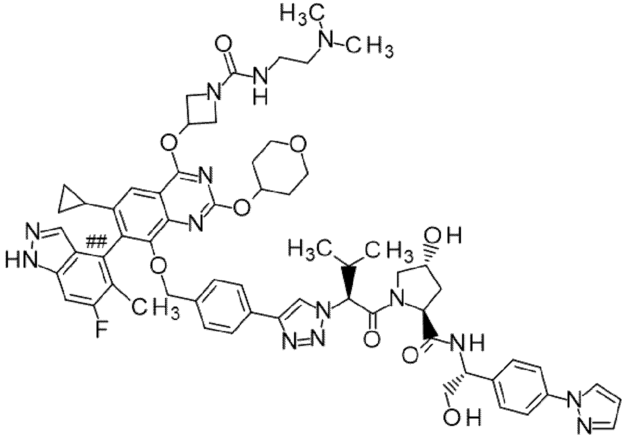 |

**[Table 7-11]**

| Ex | Str |
|---|---|
| 31 | 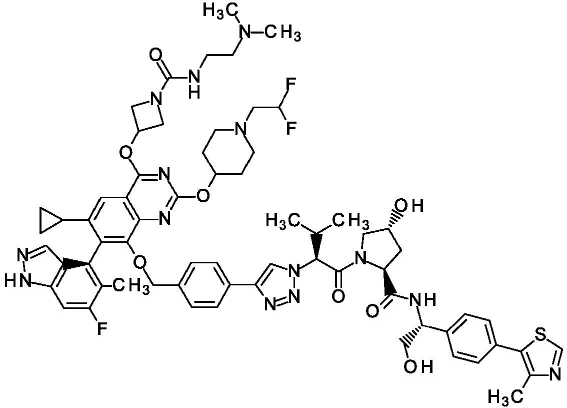 |
| 32 | 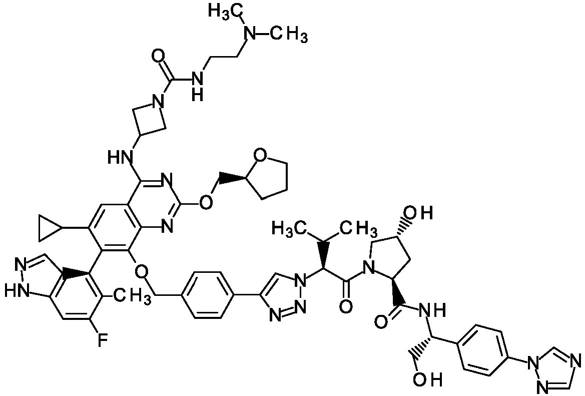 |
| 33 | 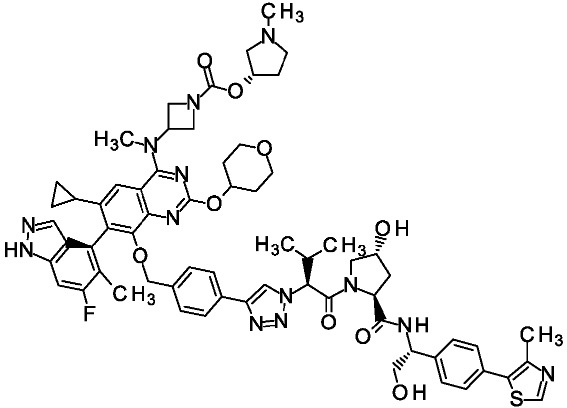 |

**[Table 7-12]**

| Ex | Str |
|---|---|
| 34 | 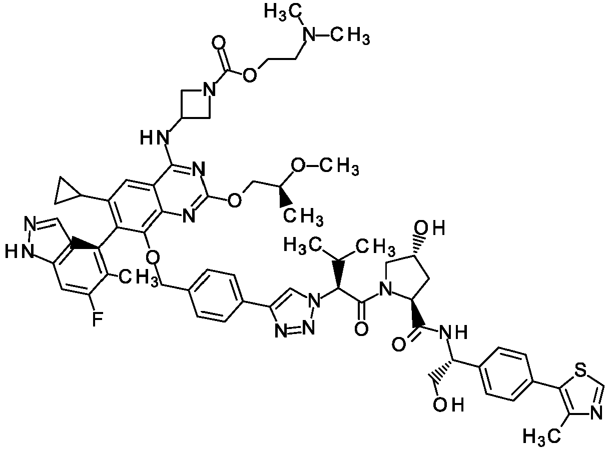 |
| 35 | 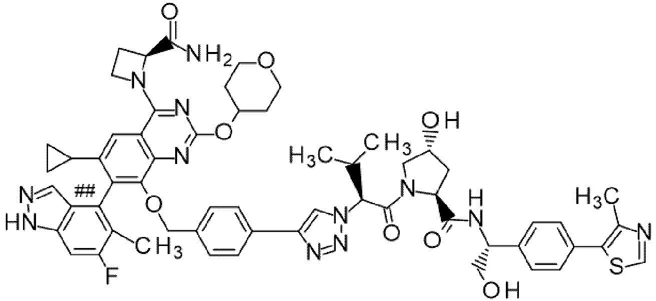 |
| 36 | 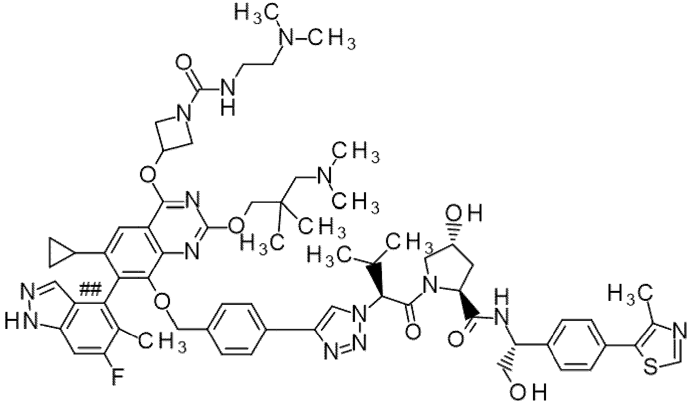 |

**[Table 7-13]**

| Ex | Str |
|---|---|
| 37 | 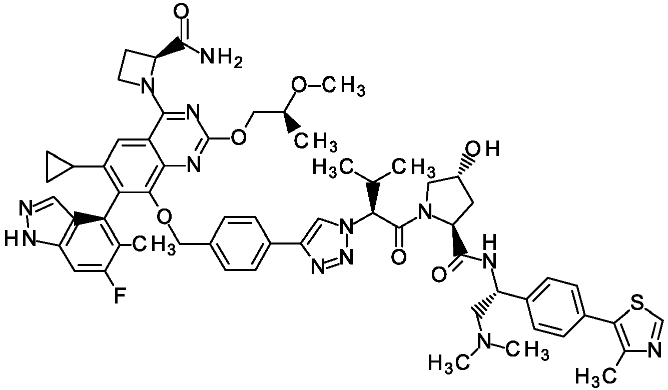 |
| 38 | 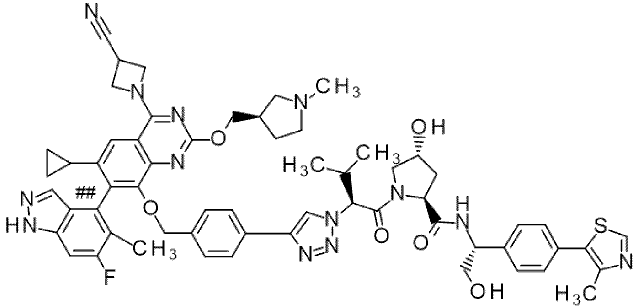 |
| 39 | 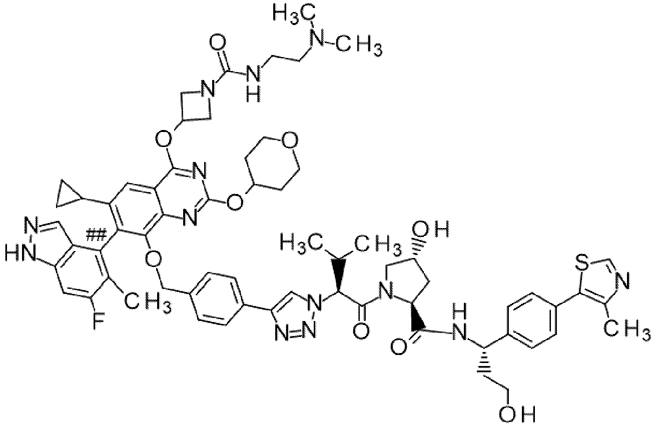 |

**[Table 7-14]**

| Ex | Str |
|---|---|
| 40 | 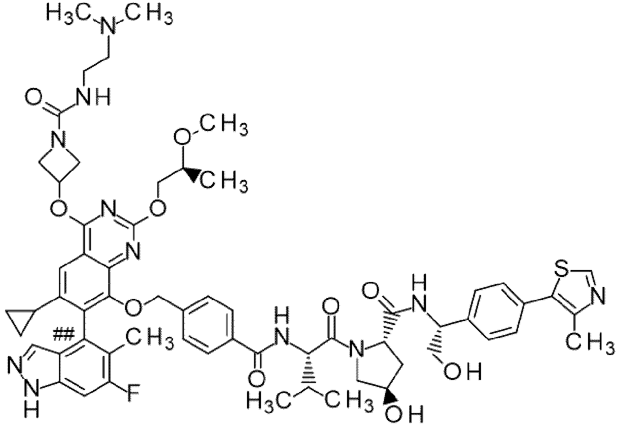 |
| 41 | 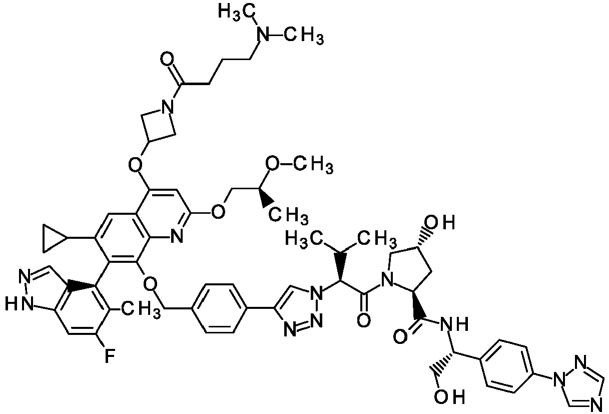 |
| 42 | 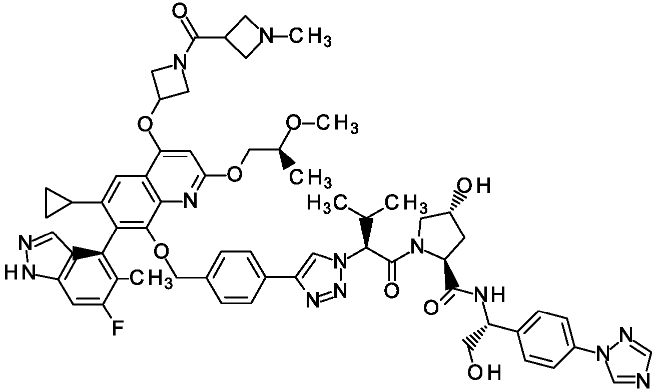 |

**[Table 7-15]**

| Ex | Str |
|---|---|
| 43 | 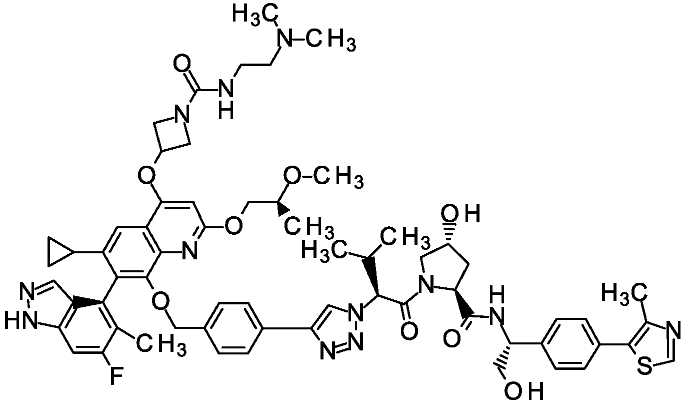 |

**[Table 8-1]**

| Ex | Syn | DAT |
|---|---|---|
| 1 | 1 | ESI+: 1204.7 |
| | | NMR: 0.53-0.62 (m, 1H), 0.68-0.82 (m, 6H), 1.08 (d, 3H), 1.13 (d, 3H), 1.29-1.38 (m, 1H), 1.73-1.83 (m, 3H), 1.98 (d, 3H), 2.01-2.11 (m, 7H), 2.22-2.32 (m, 1H), 2.36 (s, 3H), 2.41-2.56 (m, 3H), 3.29 (s, 3H), 3.55-3.66 (m, 2H), 3.66-3.74 (m, 2H), 3.74-3.83 (m, 1H), 4.04 (br, 2H), 4.24-4.40 (m, 5H), 4.42-4.49 (m, 1H), 4.57-4.66 (m, 1H), 4.80-4.88 (m, 3H), 4.95-5.03 (m, 1H), 5.15 (d, 1H), 5.25 (d, 1H), 5.32 (d, 1H), 6.81 (d, 2H), 7.36-7.43 (m, 3H), 7.48-7.50 (m, 1H), 7.55-7.59 (m, 2H), 7.61-7.69 (m, 3H), 8.30-8.32 (m, 1H), 8.47 (d, 1H), 8.63 (s, 1H), 8.68 (d, 1H), 13.07 (br, 1H) |
| 2 | 2 | ESI+: 1190.5 |
| | | NMR: 0.53-0.61 (m, 1H), 0.67-0.83 (m, 6H), 1.08 (d, 3H), 1.13 (d, 3H), 1.30-1.37 (m, 1H), 1.73-1.83 (m, 3H), 1.98 (d, 3H), 2.00-2.11 (m, 7H), 2.29 (br, 1H), 2.41-2.56 (m, 3H), 3.29 (s, 3H), 3.55-3.74 (m, 4H), 3.75-3.84 (m, 1H), 4.04 (br, 2H), 4.24-4.38 (m, 5H), 4.42-4.49 (m, 1H), 4.57-4.66 (m, 1H), 4.83-4.90 (m, 3H), 4.92-5.03 (m, 1H), 5.15 (d, 1H), 5.25 (d, 1H), 5.32 (d, 1H), 6.81 (d, 2H), 7.39 (d, 1H), 7.43-7.52 (m, 3H), 7.61-7.69 (m, 3H), 7.76-7.83 (m, 2H), 8.22 (s, 1H), 8.49 (d, 1H), 8.63 (s, 1H), 8.68 (d, 1H), 9.24 (s, 1H), 13.07 (br, 1H) |
| 3 | 2 | ESI+: 1221.6 |
| 4 | 2 | ESI+: 1208.8 |
| 5 | 5 | ESI+: 1211.7 [M+Na]+ |
| | | NMR: 0.51-0.62 (m, 1H), 0.64-0.79 (m, 6H), 1.09 (d, 3H), 1.14 (d, 3H), 1.33-1.46 (m, 1H), 1.72-1.83 (m, 1H), 2.00 (d, 3H), 2.04-2.17 (m, 7H), 2.42 (d, 2H), 2.46-2.57 (m, 1H), 2.65-2.80 (m, 1H), 3.29 (s, 3H), 3.47-3.56 (m, 2H), 3.56-3.88 (m, 5H), 3.91-3.99 (m, 2H), 3.99-4.10 (m, 2H), 4.26-4.40 (m, 3H), 4.40-4.51 (m, 3H), 4.81-4.94 (m, 3H), 5.16 (d, 1H), 5.21-5.37 (m, 3H), 6.30 (s, 1H), 6.83 (d, 2H), 7.41 (d, 1H), 7.43-7.55 (m, 4H), 7.68 (d, 2H), 7.79 (d, 2H), 8.22 (s, 1H), 8.50 (d, 1H), 8.64 (s, 1H), 9.25 (s, 1H), 13.08 (br, 1H) |

**[Table 8-2]**

| Ex | Syn | DAT |
|---|---|---|
| 6 | 6 | ESI-: 1201.6 |
| | | NMR: 0.53-0.59 (m, 1H), 0.66-0.76 (m, 6H), 1.08 (d, 3H), 1.14 (d, 3H), 1.34-1.42 (m, 1H), 1.75-1.83 (m, 1H), 1.99 (d, 3H), 2.04-2.14 (m, 7H), 2.36 (s, 3H), 2.42 (d, 2H), 2.46-2.54 (m, 1H), 2.66-2.76 (m, 1H), 3.28 (s, 3H), 3.49-3.55 (m, 2H), 3.56-3.67 (m, 2H), 3.67-3.83 (m, 3H), 3.91-3.97 (m, 2H), 4.00-4.06 (m, 2H), 4.25-4.39 (m, 3H), 4.39-4.50 (m, 3H), 4.81-4.90 (m, 3H), 5.15 (d, 1H), 5.24-5.36 (m, 3H), 6.29 (s, 1H), 6.82 (d, 2H), 7.37-7.45 (m, 3H), 7.48 (s, 1H), 7.51 (s, 1H), 7.57 (d, 2H), 7.67 (d, 2H), 8.31 (s, 1H), 8.47 (d, 1H), 8.64 (s, 1H), 13.07 (br, 1H) |
| 7 | 6 | ESI+: 1189.9 |
| 8 | 6 | ESI+: 1229.5 [M+Na]+ |
| 9 | 6 | ESI-: 1201.6 |
| 10 | 6 | ESI+: 1203.8 |
| 11 | 11 | ESI+: 1242.7 [M+Na]+ |
| | | NMR: 0.54-0.63 (m, 1H), 0.67-0.84 (m, 6H), 1.08 (d, 3H), 1.13 (d, 3H), 1.30-1.39 (m, 1H), 1.70-1.89 (m, 3H), 1.98 (d, 3H), 2.01-2.12 (m, 7H), 2.23-2.33 (m, 1H), 2.40-2.56 (m, 6H), 3.29 (s, 3H), 3.56-3.76 (m, 4H), 3.76-3.82 (m, 1H), 3.95-4.13 (m, 2H), 4.22-4.41 (m, 5H), 4.44-4.51 (m, 1H), 4.57-4.68 (m, 1H), 4.81-4.90 (m, 3H), 4.90-5.03 (m, 1H), 5.16 (d, 1H), 5.22-5.30 (m, 1H), 5.33 (d, 1H), 6.81 (d, 2H), 7.33-7.42 (m, 3H), 7.42-7.53 (m, 3H), 7.59-7.72 (m, 3H), 8.48 (d, 1H), 8.64 (s, 1H), 8.68 (d, 1H), 8.99 (s, 1H), 13.08 (br, 1H) |
| 12 | 12 | ESI+: 1163.9 |
| | | NMR: 0.51-0.61 (m, 1H), 0.64-0.78 (m, 6H), 1.08 (d, 3H), 1.14 (d, 3H), 1.33-1.45 (m, 1H), 1.74-1.84 (m, 1H), 2.00 (d, 3H), 2.04-2.11 (m, 1H), 2.15 (s, 6H), 2.28 (t, 2H), 2.44-2.57 (m, 1H), 3.11 (dt, 2H), 3.29 (s, 3H), 3.54-3.89 (m, 5H), 3.92-4.02 (m, 2H), 4.26-4.42 (m, 5H), 4.46 (dd, 1H), 4.83-4.92 (m, 3H), 5.17 (d, 1H), 5.24-5.37 (m, 3H), 6.33 (s, 1H), 6.37 (t, 1H), 6.83 (d, 2H), 7.41 (d, 1H), 7.47 (d, 2H), 7.50 (s, 1H), 7.51 (s, 1H), 7.68 (d, 2H), 7.77-7.82 (m, 2H), 8.22 (s, 1H), 8.50 (d, 1H), 8.65 (s, 1H), 9.25 (s, 1H), 13.09 (br, 1H) |

**[Table 8-3]**

| Ex | Syn | DAT |
|---|---|---|
| 13 | 13 | ESI+: 1190.6 |
| | | NMR: 0.51-0.61 (m, 1H), 0.64-0.78 (m, 6H), 1.09 (d, 3H), 1.14 (d, 3H), 1.34-1.45 (m, 1H), 1.73-1.84 (m, 3H), 2.00 (d, 3H), 2.02 (s, 6H), 2.05-2.15 (m, 1H), 2.21-2.32 (m, 1H), 2.41-2.58 (m, 3H), 3.29 (s, 3H), 3.56-3.86 (m, 5H), 4.13 (br, 2H), 4.26-4.41 (m, 3H), 4.42-4.57 (m, 3H), 4.57-4.68 (m, 1H), 4.81-5.01 (m, 3H), 5.07-5.39 (m, 4H), 6.32 (s, 1H), 6.83 (d, 2H), 7.41 (d, 1H), 7.44-7.56 (m, 4H), 7.68 (d, 2H), 7.77-7.82 (m, 2H), 8.22 (s, 1H), 8.53 (d, 1H), 8.65 (s, 1H), 9.25 (s, 1H), 13.10 (br, 1H) |
| 14 | 14 | ESI-: 1217.8 |
| | | NMR: 0.52-0.61 (m, 1H), 0.64-0.77 (m, 6H), 1.08 (d, 3H), 1.14 (d, 3H), 1.34-1.44 (m, 1H), 1.75-1.85 (m, 1H), 1.99 (d, 3H), 2.03-2.14 (m, 7H), 2.42 (d, 2H), 2.44-2.55 (m, 4H), 2.66-2.76 (m, 1H), 3.28 (s, 3H), 3.49-3.55 (m, 2H), 3.56-3.67 (m, 2H), 3.67-3.83 (m, 3H), 3.91-3.97 (m, 2H), 4.01-4.06 (m, 2H), 4,29-4.39 (m, 3H), 4.39-4.49 (m, 3H), 4.81-4.89 (m, 3H), 5.15 (d, 1H), 5.23-5.35 (m, 3H), 6.29 (s, 1H), 6.82 (d, 2H), 7.35-7.52 (m, 7H), 7.67(d, 2H), 8.47 (d, 1H), 8.64 (s, 1H), 8.99 (s, 1H), 13.07 (br, 1H) |
| 15 | 15 | ESI+: 1169.8 |
| 16 | 15 | ESI+: 1180.4 |
| 17 | 15 | ESI+: 1194.8 |
| 18 | 15 | ESI+: 1202.8 [M+Na]+ |
| 19 | 15 | ESI-: 1219.4 |
| 20 | 15 | ESI+: 1089.7 |
| 21 | 15 | ESI+: 1094.7 |
| 22 | 15 | ESI-: 1218.8 |
| 23 | 15 | ESI+: 1165.6 |
| 24 | 15 | ESI-: 1205.0 |
| 25 | 15 | ESI+: 1219.6 |
| 26 | 15 | ESI-: 1203.7 |
| 27 | 15 | ESI+: 1197.6 [M+Na]+ |
| 28 | 28 | ESI-: 1220.6 |
| 29 | 15 | ESI+: 1127.6 |
| 30 | 15 | ESI+: 1175.6 |

**[Table 8-4]**

| | | |
|---|---|---|
| 31 | 15 | ESI+: 1269.4 |
| 32 | 15 | ESI+: 1175.5 |
| 33 | 15 | ESI+: 1232.6 |
| 34 | 34 | ESI+: 1194.8 |
| 35 | 34 | ESI+: 1119.7 |
| 36 | 34 | ESI+: 1235.9 |
| 37 | 34 | ESI+: 1156.5 [M+Na]+ |
| 38 | 34 | ESI+: 1114.6 |
| 39 | 39 | ESI+: 1220.9 |
| 40 | 40 | ESI+: 1170.7 |
| 41 | 41 | ESI+: 1163.5 |
| 42 | 41 | ESI+: 1168.4 [M+Na]+ |
| 43 | 43 | ESI+: 1193.8 |

As examples of specific compounds of the formula (I) included in the present invention, compounds having any of the following structures are shown. These compounds can also be produced by the typical production methods shown above, the production methods of the Production Examples and the Examples, a combination of the production methods or a method that is obvious to a person skilled in the art.

Furthermore, the compounds are excellent in a degradation-inducing action on a mutant KRAS protein, in particular, in a degradation-inducing action on a G12V mutant, G12D mutant and G12C mutant KRAS protein, are useful as a mutant KRAS inhibitor, in particular, as a G12V mutant, G12D mutant and G12C mutant KRAS inhibitor and can be used as an active ingredient of a pharmaceutical composition, for example, a pharmaceutical composition for treating pancreatic cancer.

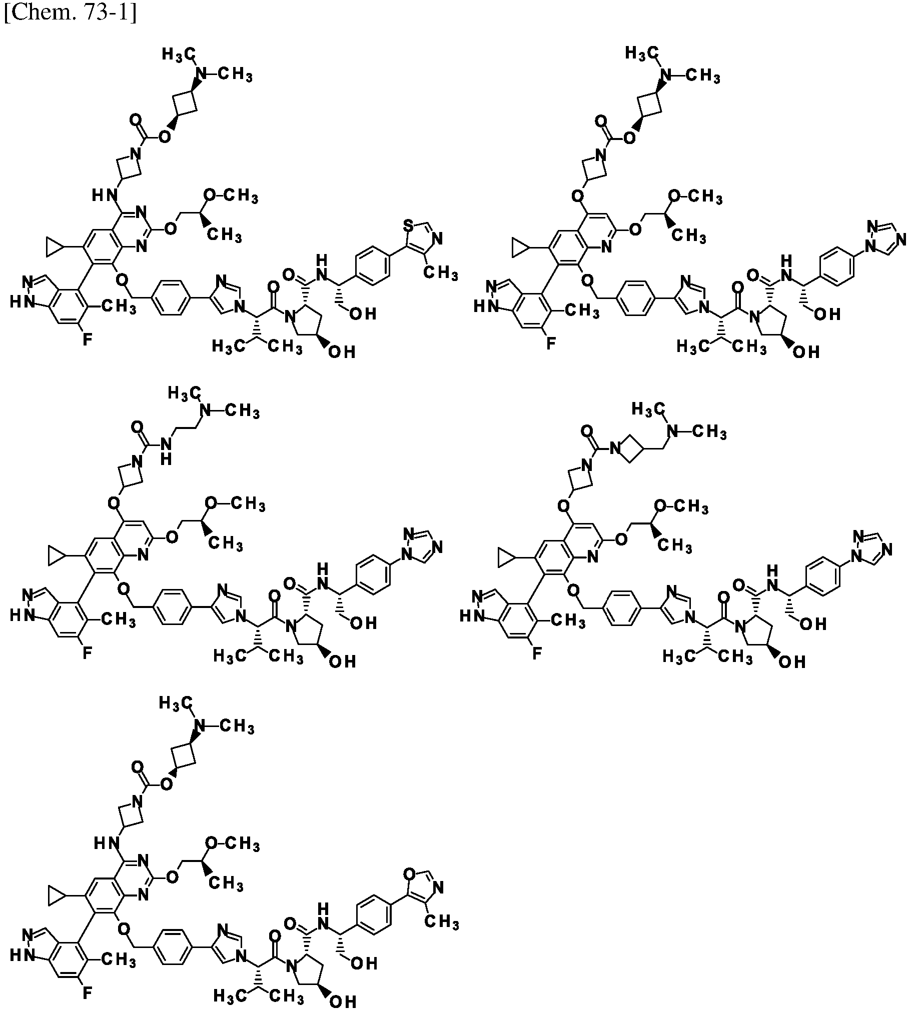

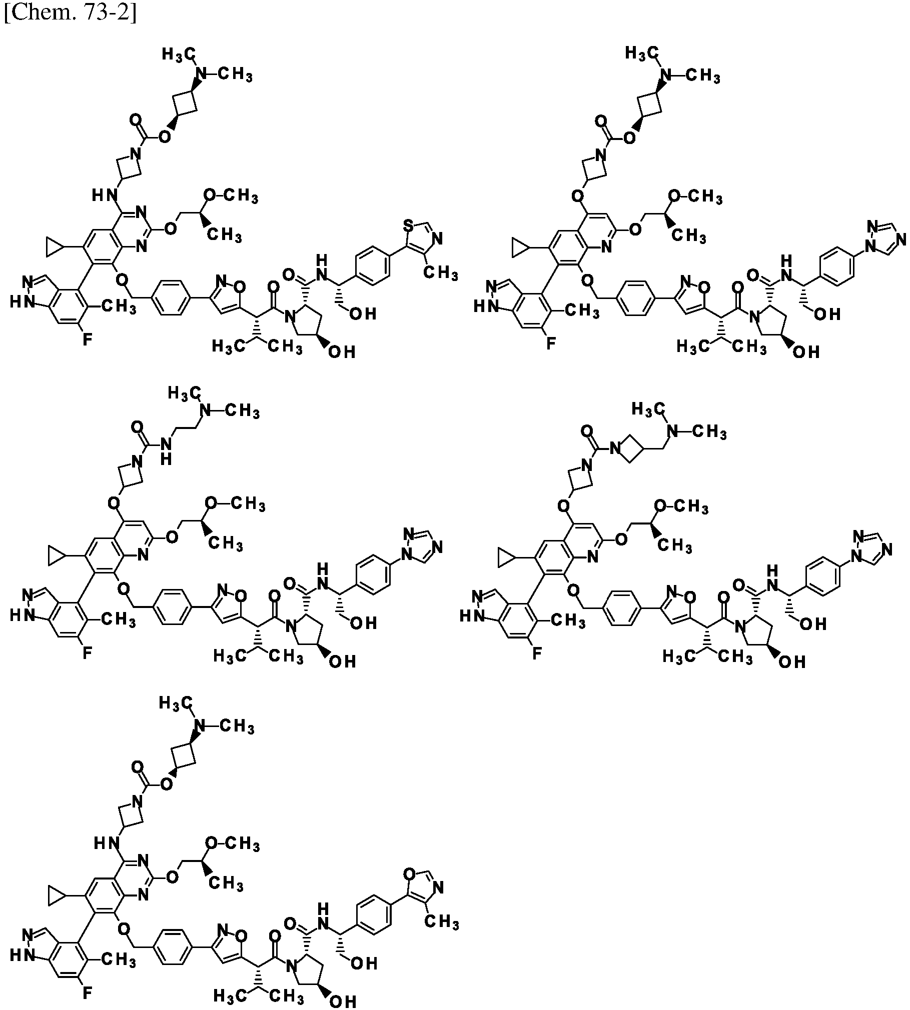

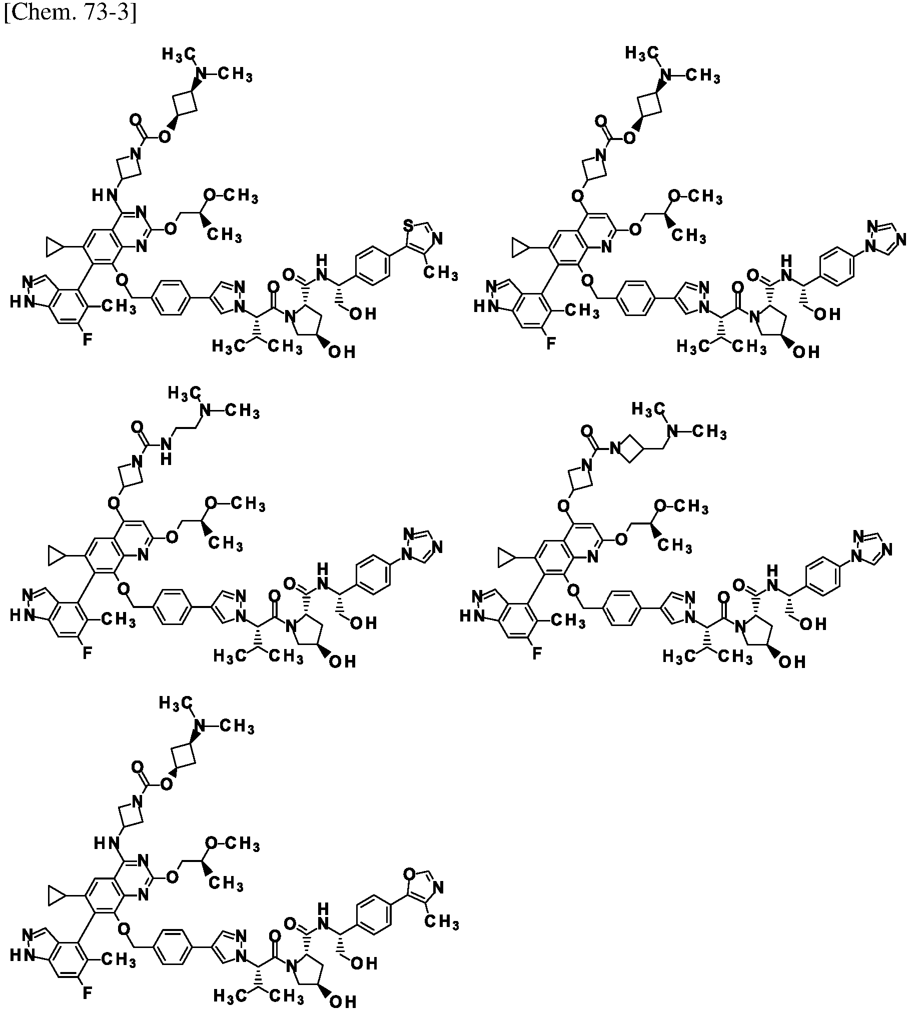

### [Industrial Applicability]

The compound of the present invention or a salt thereof is excellent in a degradation-inducing action on a mutant KRAS protein, in particular, in a degradation-inducing action on a G12V mutant, G12D mutant and G12C mutant KRAS protein, is useful as a mutant KRAS inhibitor, in particular, as a G12V mutant, G12D mutant and G12C mutant KRAS inhibitor and can be used as an active ingredient of a pharmaceutical composition, for example, a pharmaceutical composition for treating pancreatic cancer.

## Claims

1. A compound of the formula (I) or a salt thereof: 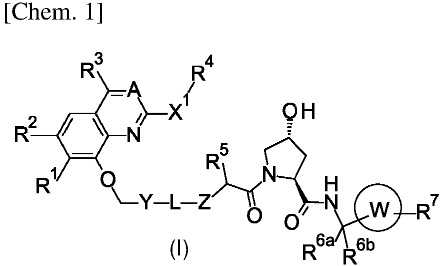 (in the formula,
A is CR^{A} or N,
wherein R^{A} is H or C₁₋₃ alkyl,
X¹ is -CH₂- or -O-,
R¹ is naphthyl optionally substituted with OH or the formula (II) below, 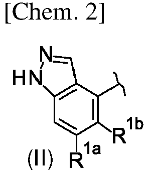
wherein R^{1a} is H, methyl, F or Cl, and
R^{1b} is F, Cl, methyl or ethyl,
R² is H, halogen, C₁₋₃ alkyl optionally substituted with a group selected from the group consisting of OH and OCH₃, cyclopropyl or vinyl,
R³ is a group selected from the group consisting of the formula (III), the formula (IV), the formula (V), the formula (VI), the formula (VII), the formula (VIII), the formula (IX), the formula (X), the formula (XI) and the formula (XXXV) below, 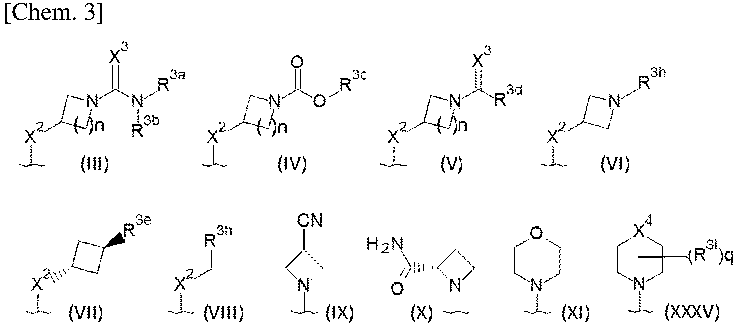
wherein R^{3a} is -(CH₂)ₚCHR^{3f}-NR^{N1}R^{N2}; -(CH₂)ₚCHR^{3f}-OR^{3g}; a 5-membered or 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3b} is H or C₁₋₃ alkyl,
R^{3c} and R^{3d} are -(CH₂)ₚCHR^{3f}-NR^{N1}R^{N2}; -(CH₂)ₚCHR^{3f}-OR^{3g}; a 4-membered to 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3e} is -O-C₂₋₃ alkylene-NR^{N1}R^{N2},
R^{3f} is H, F or C₁₋₃ alkyl,
R^{3g} is H or C₁₋₃ alkyl,
R^{3h} is optionally substituted 5-membered heteroaryl containing one to four hetero atoms selected from the group consisting of oxygen, sulfur and nitrogen or optionally substituted 6-membered heteroaryl containing one to three nitrogen atoms,
R³ⁱ, which are the same as or different from each other, are groups selected from the group consisting of H, OH, optionally substituted C₁₋₃ alkyl, -O-optionally substituted C₁₋₃ alkyl, -NH-optionally substituted C₁₋₃ alkyl, -N-(optionally substituted C₁₋₃ alkyl)₂, halogen, -CN and oxo, or
two R³ⁱ on a same carbon atom, together with the neighboring carbon atom, may form a ring selected from the group consisting of C₃₋₆ cycloalkane and a 4-membered to 6-membered saturated hetero ring containing one or two hetero atoms selected from the group consisting of oxygen, sulfur and nitrogen as ring-constituting atoms to form a spiro ring as the group in the formula (XXXV), wherein the spiro ring is optionally substituted with one or two groups selected from the group consisting of C₁₋₃ alkyl, -O-(C₁₋₃ alkyl), OH, halogen and oxo, or
R³ⁱ on two neighboring carbon atoms, together with the two carbon atoms, may form a ring selected from the group consisting of C₃₋₆ cycloalkane and a 4-membered to 6-membered saturated hetero ring containing one or two hetero atoms selected from the group consisting of oxygen, sulfur and nitrogen as ring-constituting atoms to form a condensed ring as the group in the formula (XXXV), wherein the condensed ring is optionally substituted with one or two groups selected from the group consisting of C₁₋₃ alkyl, -O-(C₁₋₃ alkyl), OH, halogen and oxo, or
R³ⁱ on two carbon atoms which are not neighboring, together with the two carbon atoms, may form a cross-linked structure composed of one or two carbon atoms, wherein the group in the formula (XXXV), which is a ring having the cross-linked structure, is optionally substituted with one or two groups selected from the group consisting of C₁₋₃ alkyl, -O-(C₁₋₃ alkyl), OH, halogen and oxo,
R^{N1} and R^{N2}, which are the same as or different from each other, are H or C₁₋₃ alkyl, or
R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms, or
R^{3f} and R^{N1}, together with the carbon atom and the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms,
X² is -O-, -NH- or -N(C₁₋₃ alkyl)-,
X³ is O or S,
X⁴ is -CH₂-, -CH₂-CH₂- or -O-CH₂-,
n is 1 or 2, p is 1 or 2, and
q is an integer of 1 to 8,
with the proviso that X² in the formula (IV) is -O-, -NH- or -N(C₂₋₃ alkyl)- when R^{3c} is -(CH₂)ₚCHR^{3f}-NR^{N1}R^{N2},
R⁴ is C₁₋₆ alkyl optionally substituted with a group selected from the group consisting of F, OH, OCH₃, R^{4a}, cyclopropyl, N(R^{4a})₂, pyrrolidinyl optionally substituted with R^{4a} and tetrahydrofuranyl optionally substituted with R^{4a}; piperidinyl optionally substituted with R^{4b}; or tetrahydropyranyl optionally substituted with R^{4a},
wherein R^{4a} is C₁₋₃ alkyl optionally substituted with F, and
R^{4b} is C₁₋₃ alkyl substituted with one to three F,
R⁵ is methyl, ethyl, isopropyl, isobutyl, sec-butyl, tert-butyl, C₃₋₆ cycloalkylmethyl or C₃₋₆ cycloalkyl,
R^{6a} and R^{6b}, which are the same as or different from each other, are H or C₁₋₆ alkyl optionally substituted with a group selected from the group consisting of F, OH, OCH₃ and N(CH₃)₂, or
R^{6a} and R^{6b}, together with the carbon to which they are attached, may form optionally substituted C₃₋₆ cycloalkane or an optionally substituted 4-membered to 6-membered saturated hetero ring containing one or two hetero atoms selected from the group consisting of oxygen, sulfur and nitrogen as ring-constituting atoms,
R⁷ is an optionally substituted 4-membered to 6-membered saturated heterocyclic group containing one or two hetero atoms selected from the group consisting of oxygen, sulfur and nitrogen as ring-constituting atoms, optionally substituted 5-membered heteroaryl containing one to four hetero atoms selected from the group consisting of oxygen, sulfur and nitrogen or 6-membered heteroaryl containing one to three nitrogen atoms,
W is optionally substituted phenylene or optionally substituted 6-membered heteroarenediyl containing one to three nitrogen atoms as ring-constituting atoms,
Y is phenylene optionally substituted with F or Cl or pyridinediyl,
L is -(L¹-L²-L³-L⁴)-,
wherein L¹, L², L³ and L⁴, which are the same as or different from each other, are groups selected from the group consisting of a bond, -O-, -NR^{L1}-, optionally substituted pyrrolidinediyl, optionally substituted piperidinediyl, optionally substituted piperazinediyl, optionally substituted C₁₋₃ alkylene and C=O,
wherein R^{L1} is H or C₁₋₃ alkyl, and
Z is NH or 5-membered heteroarenediyl containing one to four hetero atoms selected from the group consisting of oxygen, sulfur and nitrogen as ring-constituting atoms,
or Y-L-Z is the formula (XII) below.)

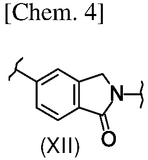

2. The compound according to claim 1 or a salt thereof,
wherein R³ is a group selected from the group consisting of the formula (III), the formula (IV), the formula (V), the formula (VI), the formula (VII), the formula (VIII), the formula (IX), the formula (X) and the formula (XI) below, 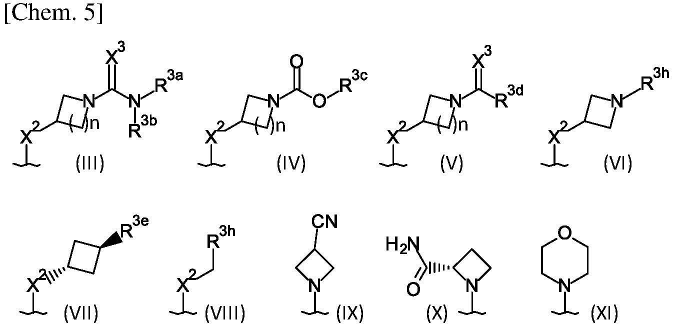
wherein R^{3a} is -(CH₂)ₚCHR^{3f}-NR^{N1}R^{N2}; -(CH₂)ₚCHR^{3f}-OR^{3g}; a 5-membered or 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3b} is H or C₁₋₃ alkyl,
R^{3c} and R^{3d} are -(CH₂)ₚCHR^{3f}-NR^{N1}R^{N2}; -(CH₂)ₚCHR^{3f}-OR^{3g}; a 4-membered to 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3e} is -O-C₂₋₃ alkylene-NR^{N1}R^{N2},
R^{3f} is H, F or C₁₋₃ alkyl,
R^{3g} is H or C₁₋₃ alkyl,
R^{3h} is optionally substituted 5-membered heteroaryl containing one to four hetero atoms selected from the group consisting of oxygen, sulfur and nitrogen or optionally substituted 6-membered heteroaryl containing one to three nitrogen atoms,
R^{N1} and R^{N2}, which are the same as or different from each other, are H or C₁₋₃ alkyl, or
R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms, or
R^{3f} and R^{N1}, together with the carbon atom and the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms,
X² is -O-, -NH- or -N(C₁₋₃ alkyl)-,
X³ is O or S,
n is 1 or 2, and p is 1 or 2,
with the proviso that X² in the formula (IV) is -O-, -NH- or -N(C₂₋₃ alkyl)- when R^{3c} is -(CH₂)ₚCHR^{3f}-NR^{N1}R^{N2},

3. The compound according to claim 2 or a salt thereof,
wherein Y is phenylene optionally substituted with F or Cl or pyridinediyl,
L is a bond, C₁₋₃ alkylene, C=O or a group selected from the group consisting of the formula (XIII), the formula (XIV), the formula (XV), the formula (XVI), the formula (XVII) and the formula (XVIII) below, 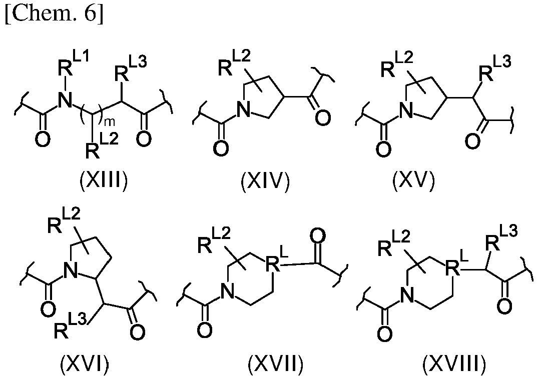
wherein R^{L1} is H or C₁₋₃ alkyl,
R^{L2} and R^{L3}, which are the same as or different from each other, are H, F, OH, OCH₃ or optionally substituted C₁₋₃ alkyl,
R^{L} is CH or N, and
m is 1 or 2, and
Z is NH or a group selected from the group consisting of the formula (XIX), the formula (XX), the formula (XXI) and the formula (XXII) below, 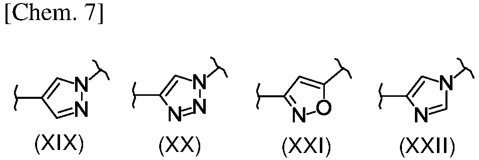
or Y-L-Z is the formula (XII) below.

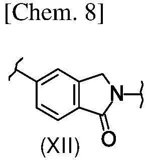

4. The compound according to claim 3 or a salt thereof,
wherein A is CR^{A} or N,
wherein R^{A} is H,
X¹ is -O-,
R^{6a} and R^{6b}, which are the same as or different from each other, are H or C₁₋₆ alkyl optionally substituted with a group selected from the group consisting of F, OH, OCH₃ and N(CH₃)₂, or
R^{6a} and R^{6b}, together with the carbon to which they are attached, may form optionally substituted C₃₋₆ cycloalkane,
R⁷ is a group selected from the group consisting of the formula (XXIII), the formula (XXIV), the formula (XXV), the formula (XXVI), the formula (XXVII), the formula (XXVIII), the formula (XXIX), the formula (XXX), the formula (XXXI), the formula (XXXII) and the formula (XXXIII) below, 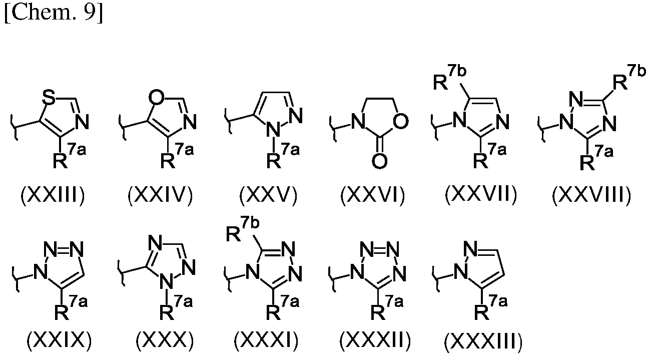
wherein R^{7a} and R^{7b}, which are the same as or different from each other, are H or C₁₋₃ alkyl optionally substituted with OH, and
W is the formula (XXXIV) below, 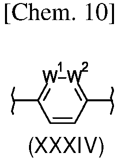
wherein W¹ is CH, CF, CCl or CCH₃, and
W² is CH, CF, CCl, CCH₃ or N.

5. The compound according to claim 4 or a salt thereof,
wherein R¹ is the formula (II) below, 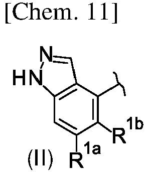
wherein R^{1a} is H, methyl, F or Cl, and
R^{1b} is F, Cl, methyl or ethyl,
R² is halogen, C₁₋₃ alkyl optionally substituted with a group selected from the group consisting of OH and OCH₃, cyclopropyl or vinyl,
R³ is a group selected from the group consisting of the formula (III), the formula (IV), the formula (V), the formula (VIII-a) and the formula (X) below, 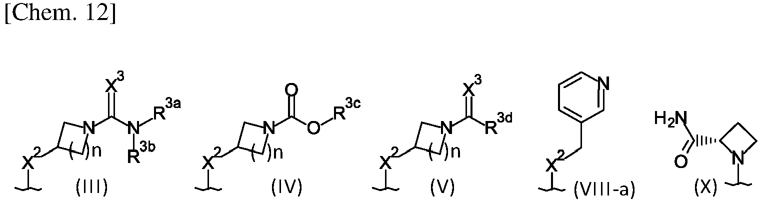
wherein R^{3a} is -(CH₂)ₚCHR^{3f}-NR^{N1}R^{N2}; -(CH₂)ₚCHR^{3f}-OR^{3g}; a 5-membered or 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3b} is H or methyl,
R^{3c} and R^{3d} are -(CH₂)ₚCHR^{3f}-NR^{N1}R^{N2}; -(CH₂)ₚCHR^{3f}-OR^{3g}; a 4-membered to 6-membered saturated heterocyclic group which is optionally substituted with a group selected from the group consisting of C₁₋₃ alkyl, C₁₋₃ alkylene-OR^{3g}, C₁₋₃ alkylene-NR^{N1}R^{N2} and -NR^{N1}R^{N2} and which contains one or two hetero atoms selected from the group consisting of oxygen, sulfur and nitrogen as ring-constituting atoms; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3f} is H, F or C₁₋₃ alkyl,
R^{3g} is H or C₁₋₃ alkyl,
R^{N1} and R^{N2}, which are the same as or different from each other, are H or C₁₋₃ alkyl, or
R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms, or
R^{3f} and R^{N1}, together with the carbon atom and the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms,
X² is -O-, -NH- or -N(C₁₋₃ alkyl)-,
X³ is O or S,
n is 1 or 2, and p is 1 or 2,
with the proviso that X² in the formula (IV) is -O-, -NH- or -N(C₂₋₃ alkyl)- when R^{3c} is -(CH₂)ₚCHR^{3f}-NR^{N1}R^{N2},
Y is phenylene optionally substituted with F or Cl or pyridinediyl,
L is a bond, C=O or a group selected from the group consisting of the formula (XIII) and the formula (XIV) below, 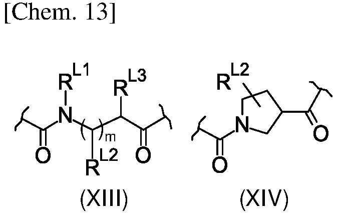
wherein R^{L1} is H or C₁₋₃ alkyl,
R^{L2} and R^{L3}, which are the same as or different from each other, are H, F, OH, OCH₃ or optionally substituted C₁₋₃ alkyl, and
m is 1 or 2, and
Z is NH or a group selected from the group consisting of the formula (XIX), the formula (XX), the formula (XXI) and the formula (XXII) below, 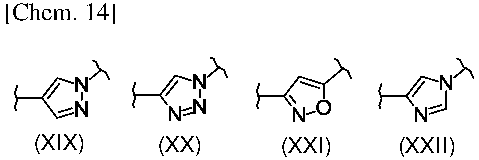
or Y-L-Z is the formula (XII) below.

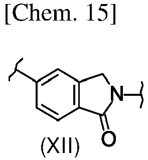

6. The compound according to claim 5 or a salt thereof,
wherein R³ is a group selected from the group consisting of the formula (III-a), the formula (IV-a), the formula (V-a), the formula (VIII-a) and the formula (X) below, 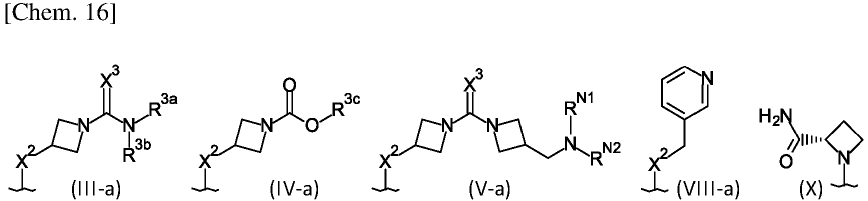
wherein R^{3a} is -(CH₂)ₚCHR^{3f}-NR^{N1}R^{N2}; -(CH₂)ₚCHR^{3f}-OR^{3g}; pyrrolidinyl optionally substituted with C₁₋₃ alkyl; piperidinyl optionally substituted with C₁₋₃ alkyl; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3b} is H or methyl,
R^{3c} is -(CH₂)ₚCHR^{3f}-NR^{N1}R^{N2}; -(CH₂)ₚCHR^{3f}-OR^{3g}; pyrrolidinyl optionally substituted with C₁₋₃ alkyl; piperidinyl optionally substituted with C₁₋₃ alkyl; or C₃₋₆ cycloalkyl optionally substituted with a group selected from the group consisting of C₁₋₃ alkyl, C₁₋₃ alkylene-OR^{3g}, C₁₋₃ alkylene-NR^{N1}R^{N2}, -OR^{3g} and -NR^{N1}R^{N2},
R^{3f} is H, For C₁₋₃ alkyl,
R^{3g} is H or C₁₋₃ alkyl,
R^{N1} and R^{N2}, which are the same as or different from each other, are H or C₁₋₃ alkyl, or
R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms, or
R^{3f} and R^{N1}, together with the carbon atom and the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms,
X² is -O- or -NH- or -N(CH₃)-,
X³ is O or S, and
p is 1 or 2,
with the proviso that X² in the formula (IV-a) is -O- or -NH- when R^{3c} is -(CH₂)ₚCHR^{3f}-NR^{N1}R^{N2},
R⁴ is C₁₋₆ alkyl optionally substituted with a group selected from the group consisting of F, OH, OCH₃, R^{4a}, cyclopropyl, N(R^{4a})₂, pyrrolidinyl optionally substituted with R^{4a} and tetrahydrofuranyl; piperidinyl optionally substituted with R^{4b}; or tetrahydropyranyl,
wherein R^{4a} is C₁₋₃ alkyl, and
R^{4b} is C₁₋₃ alkyl substituted with one to three F,
R⁷ is a group selected from the group consisting of the formula (XXIII), the formula (XXIV), the formula (XXVI), the formula (XXVIII) and the formula (XXXIII) below, 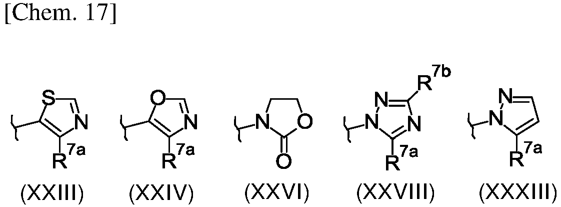
wherein R^{7a} and R^{7b}, which are the same as or different from each other, are H or C₁₋₃ alkyl optionally substituted with OH,
Y is phenylene optionally substituted with F or Cl, and
Z is NH or a group selected from the group consisting of the formula (XIX), the formula (XX), the formula (XXI) and the formula (XXII) below.

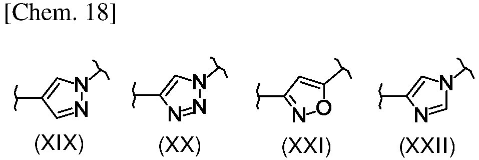

7. The compound according to claim 6 or a salt thereof,
wherein R¹ is the formula (II-a) below, 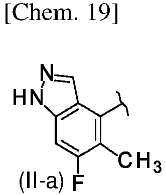
R² is cyclopropyl,
R³ is a group selected from the group consisting of the formula (III-a), the formula (IV-a), the formula (V-a), the formula (VIII-a) and the formula (X) below, 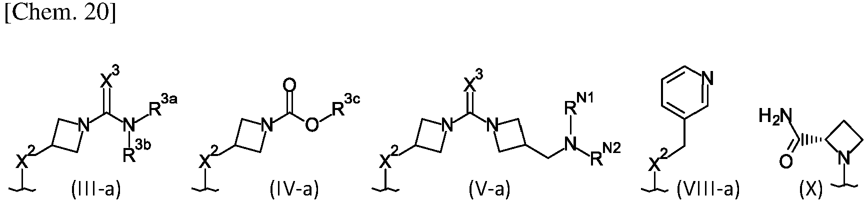
wherein R^{3a} is -(CH₂)ₚCHR^{3f}-NR^{N1}R^{N2} or -(CH₂)ₚCHR^{3f}-OR^{3g},
R^{3b} is H,
R^{3c} is -(CH₂)ₚCHR^{3f}-NR^{N1}R^{N2}; pyrrolidinyl optionally substituted with C₁₋₃ alkyl; or C₃₋₆ cycloalkyl optionally substituted with -NR^{N1}R^{N2},
R^{3f} is H,
R^{3g} is H,
R^{N1} and R^{N2}, which are the same as or different from each other, are H or C₁₋₃ alkyl, or
R^{N1} and R^{N2}, together with the nitrogen atom to which they are attached, may form an optionally substituted 4-membered to 6-membered saturated heterocyclic group containing one or two hetero atoms selected from the group consisting of oxygen, sulfur and nitrogen as ring-constituting atoms,
X² is -O- or -NH-,
X³ is O or S, and
p is 1 or 2,
R⁴ is C₁₋₆ alkyl optionally substituted with a group selected from the group consisting of OCH₃, R^{4a}, N(R^{4a})₂, pyrrolidinyl optionally substituted with R^{4a} and tetrahydrofuranyl; piperidinyl optionally substituted with R^{4b}; or tetrahydropyranyl,
wherein R^{4a} is C₁₋₃ alkyl, and
R^{4b} is C₁₋₃ alkyl substituted with one to three F,
R⁵ is isopropyl or sec-butyl,
R^{6a} and R^{6b}, which are the same as or different from each other, are H or C₁₋₆ alkyl optionally substituted with a group selected from the group consisting of OH and N(CH₃)₂,
R⁷ is a group selected from the group consisting of the formula (XXIII), the formula (XXIV), the formula (XXVI), the formula (XXVIII) and the formula (XXXIII) below, 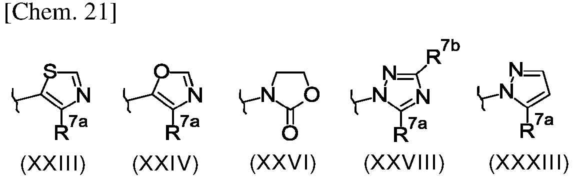
wherein R^{7a} and R^{7b}, which are the same as or different from each other, are H or C₁₋₃ alkyl,
W is the formula (XXXIV) below, 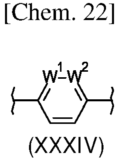
wherein W¹ is CH, and
W² is CH or N,
Y is phenylene, and
L is a bond or C=O.

8. The compound according to claim 7 or a salt thereof,
wherein R³ is a group selected from the group consisting of the formula (III-a), the formula (IV-a) and the formula (V-a) below, 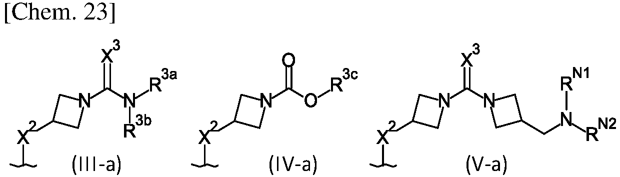
wherein R^{3a} is -(CH₂)ₚCHR^{3f}-NR^{N1}R^{N2},
R³⁶ is H,
R^{3c} is C₃₋₆cycloalkyl optionally substituted with -NR^{N1}R^{N2},
R^{3f} is H,
R^{N1} and R^{N2}, which are the same as or different from each other, are both C₁₋₃ alkyl,
X² is -O- or -NH-,
X³ is O, and
p is 1,
R⁴ is C₁₋₆ alkyl optionally substituted with OCH₃,
R⁵ is isopropyl,
R^{6a} is H,
R^{6b} is C₁₋₆ alkyl optionally substituted with OH,
R⁷ is a group selected from the group consisting of the formula (XXIII), the formula (XXIV) and the formula (XXVIII) below, 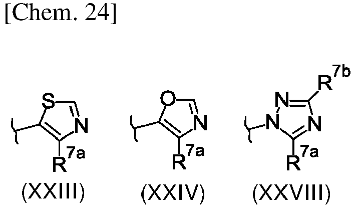
wherein R^{7a} and R^{7b}, which are the same as or different from each other, are H or C₁₋₃ alkyl,
W is the formula (XXXIV) below, 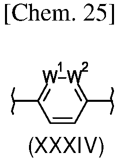
wherein W¹ and W² are both CH,
L is a bond, and
Z is a group selected from the group consisting of the formula (XIX), the formula (XX), the formula (XXI) and the formula (XXII) below.

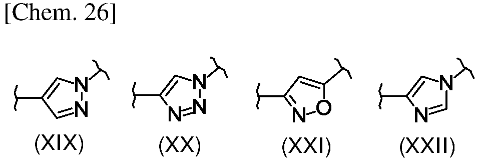

9. The compound according to claim 1 or a salt thereof,
wherein the compound of the formula (I) is selected from the group consisting of
(1s,3R)-3-(dimethylamino)cyclobutyl 3-({(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-oxazol-5-yl)phenyl]ethyl}carbamoyl)pyrrol idin-1-yl]-3-methyl-1-oxobutan-2-yl}-1H-1,2,3-triazol-4-yl)phenyl]methoxy}-2-[(2S)-2-met hoxypropoxy]quinazolin-4-yl}amino)azetidine-1-carboxylate,
(1s,3R)-3-(dimethylamino)cyclobutyl 3-({(7M)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(1H-1,2,4-triazol-1-yl)phenyl]ethyl}carbamoyl)pyrrolidin-1-yl]-3-methyl-1-oxobutan-2-yl}-1H-1,2,3-triazol-4-yl)phenyl}methoxy}-2-[(2S)-2-methoxy propoxy]quinazolin-4-yl}amino)azetidine-1-carboxylate,
(4R)-1-[(2S)-2-(4-{4-[({(7M)-6-cyclopropyl-4-[(1-{3-[(dimethylamino)methyl]azeti dine-1-carbonyl}azetidin-3-yl)oxy]-7-(6-fluoro-5-methyl-1H-indazol-4-yl)-2-[(2S)-2-methox ypropoxy]quinolin-8-yl}oxy)methyl]phenyl}-1H-1,2,3-triazol-1-yl)-3-methylbutanoyl]-4-hy droxy-N-{(1R)-2-hydroxy-1-[4-(1H-1,2,4-triazol-1-yl)phenyl]ethyl}-L-prolinamide,
(4R)-1-[(2S)-2-(4-{4-[({(7M)-6-cyclopropyl-4-[(1-{3-[(dimethylamino)methyl]azeti dine-1-carbonyl}azetidin-3-yl)oxy]-7-(6-fluoro-5-methyl-1H-indazol-4-yl)-2-[(2S)-2-methox ypropoxy]quinolin-8-yl}oxy)methyl]phenyl}-1H-1,2,3-triazol-1-yl)-3-methylbutanoyl]-4-hy droxy-N-{(1R)-2-hydroxy-1-[4-(4-methyl-1,3-oxazol-5-yl)phenyl]ethyl}-L-prolinamide,
(1s,3R)-3-(dimethylamino)cyclobutyl 3-({(7TM)-6-cyclopropyl-7-(6-fluoro-5-methyl-1H-indazol-4-yl)-8-{[4-(1-{(2S)-1-[(2S,4R)-4-hydroxy-2-({(1R)-2-hydroxy-1-[4-(4-methyl-1,3-thiazol-5-yl)phenyl]ethyl}carbamoyl)pyrrol idin-1-yl]-3-methyl-1-oxobutan-2-yl}-1H-1,2,3-triazol-4-yl)phenyl]methoxy}-2-[(2S)-2-met hoxypropoxy]quinazolin-4-yl}amino)azetidine-1-carboxylate,
(4R)-1-[(2S)-2-(4-{4-[({(7M)-6-cyclopropyl-4-[(1-{[2-(dimethylamino)ethyl]carba moyl}azetidin-3-yl)oxy]-7-(6-fluoro-5-methyl-1H-indazol-4-yl)-2-[(2S)-2-methoxypropoxy] quinolin-8-yl}oxy)methyl]phenyl}-1H-1,2,3-triazol-1-yl)-3-methylbutanoyl]-4-hydroxy-N-{( 1R)-2-hydroxy-1-[4-(1H-1,2,4-triazol-1-yl)phenyl]ethyl}-L-prolinamide,
(1s,3R)-3-(dimethylamino)cyclobutyl 3-({(7M)-6-cyclopropyl-7-(6-fluoro-5-methyl-lH-indazol-4-yl)-8-{[4-(1-{(2S)-1-[(2S,4R)-4-hydroxy-2-({(1R)-2-hydroxy-1-[4-(1H-1,2,4-triazol-1-yl)phenyl]ethyl}carbamoyl)pyrrolidin-1-yl]-3-methyl-1-oxobutan-2-yl}-1H-1,2,3-triazol-4-yl)phenyl]methoxy}-2-[(2S)-2-methoxy propoxy]quinolin-4-yl}oxy)azetidine-1-carboxylate and
(4R)-1-[(2S)-2-(4-{4-[({(7M)-6-cyclopropyl-4-[(1-{3-[(dimethylamino)methyl]azeti dine-1-carbonyl}azetidin-3-yl)oxy]-7-(6-fluoro-5-methyl-1H-indazol-4-yl)-2-[(2S)-2-methox ypropoxy]quinolin-8-yl}oxy)methyl]phenyl}-1H-1,2,3-triazol-1-yl)-3-methylbutanoyl]-4-hy droxy-N-{(1R)-2-hydroxy-1-[4-(4-methyl-1,3-thiazol-5-yl)phenyl]ethyl}-L-prolinamide.

10. A pharmaceutical composition containing the compound according to claim 1 or a salt thereof and one or more pharmaceutically acceptable excipients.

11. The pharmaceutical composition according to claim 10 which is a pharmaceutical composition for treating pancreatic cancer.

12. Use of the compound according to claim 1 or a salt thereof for producing a pharmaceutical composition for treating pancreatic cancer.

13. The compound according to claim 1 or a salt thereof for use in treatment of pancreatic cancer.

14. Use of the compound according to claim 1 or a salt thereof for treating pancreatic cancer.

15. A method for treating pancreatic cancer comprising administering an effective amount of the compound according to claim 1 or a salt thereof to a subject.
